# EUROPEAN PATENT SPECIFICATION

(11) **EP 3 057 991 B1**
(45) Date of publication and mention of the grant of the patent: **08.05.2019**
(21) Application number: 14853242.7
(22) Date of filing: 15.10.2014
(51) Int. Cl.: C07K 16/28, C07K 19/00, A61K 49/00, A61K 31/4188, A61K 39/385, A61K 39/395, A61K 31/352, C07K 16/32, C07K 16/40, C07K 16/44

(54) **CHIMERIC ANTIGEN RECEPTOR T CELL SWITCHES AND USES THEREOF**
CHIMÄRE ANTIGENREZEPTOR-T-ZELL-SCHALTER UND VERWENDUNGEN DAVON
COMMUTATEURS DE LYMPHOCYTES T DES RÉCEPTEURS D'ANTIGÈNE CHIMÉRIQUES ET LEUR UTILISATION

(30) Priority: 15.10.2013 US 201361891347 P; 25.10.2013 US 201361895704 P; 06.06.2014 US 201462009056 P
(43) Date of publication of application: 24.08.2016
(73) Proprietor: The California Institute for Biomedical Research, La Jolla, CA 92037 (US); The Scripps Research Institute, La Jolla, CA 92037 (US)
(72) Inventor: KIM, Chanhyuk, San Diego, CA 92129 (US); YOUNG, Travis, La Jolla, CA 92037-3231 (US); CAO, Yu, San Diego, CA 92111 (US); MA, Jennifer, La Jolla, CA 92037 (US); KIM, Minsoo, San Diego, CA 92122 (US); PINKERTON, Stephanie, San Diego, CA 92117 (US); SCHULTZ, Peter, G., La Jolla, CA 92037 (US)
(74) Representative: Schiweck Weinzierl Koch Patentanwälte Partnerschaft mbB
(86) International application number: PCT/US2014/060713
(87) International publication number: WO 2015/057852

(56) References cited:
- WO-A1-2012/079000
- WO-A1-2013/123061
- WO-A2-2012/082841
- K. TAMADA ET AL: "Redirecting Gene-Modified T Cells toward Various Cancer Types Using Tagged Antibodies", CLINICAL CANCER RESEARCH, vol. 18, no. 23, 2 October 2012 (2012-10-02), pages 6436-6445, XP055154500, ISSN: 1078-0432, DOI: 10.1158/1078-0432.CCR-12-1449
- CHAN HYUK KIM ET AL: "Synthesis of Bispecific Antibodies using Genetically Encoded Unnatural Amino Acids", JOURNAL OF THE AMERICAN CHEMICAL SOCIETY, vol. 134, no. 24, 20 June 2012 (2012-06-20), pages 9918-9921, XP055237288, US ISSN: 0002-7863, DOI: 10.1021/ja303904e
- CHAN HYUK KIM ET AL: "Protein conjugation with genetically encoded unnatural amino acids", CURRENT OPINION IN CHEMICAL BIOLOGY, vol. 17, no. 3, 1 June 2013 (2013-06-01), pages 412-419, XP055193458, ISSN: 1367-5931, DOI: 10.1016/j.cbpa.2013.04.017
- TAMADA ET AL.: 'Redirecting gene -modified T cells toward various cancer types using tagged antibodies' CLINICAL CANCER RESEARCH vol. 18, no. 23, 2012, pages 6436 - 6445, XP055154500
- GRADA ET AL.: 'TanCAR: a novel bispecific chimeric antigen receptor for cancer immunotherapy' MOLECULAR THERAPY-NUCLEIC ACIDS vol. 2, no. 7, 09 July 2013, pages 1 - 11, XP055152438

## Description

### BACKGROUND OF THE INVENTION

Immunotherapies, once considered "magic bullets" by Nobel laureate Paul Ehrlich, are rapidly becoming attractive alternatives to chemotherapies. Specifically, immunotherapies that use genetically modified T cells to "reteach" the immune system to recognize and eliminate malignant tumor cells are producing exciting results in early stage clinical trials. Such gene therapy circumvents many mechanisms of chemotherapy resistance and is active against relapsed/refractory disease, offering a realistic hope for a curative therapy. However, gene therapy techniques have encountered significant risks in the clinic including chronic immune dysregulation and even death. In the search for improved immunotherapies we have established a method of selectively activating and deactivating genetically modified T cells, which is both safer and more versatile than effector therapies currently being tested in the clinic.

Adoptive transfer of genetically engineered chimeric antigen receptor T cells (CAR-Ts) equips the immune system with the ability to recognize and eliminate tumor cells. This therapy has achieved sustained remissions in clinical trials for chronic lymphocytic leukemia (CLL) and acute lymphoblastic leukemia (ALL) patients, and is rapidly emerging as a powerful alternative to chemotherapy. Despite these successes, this therapy suffers from serious safety concerns due to persistent activity of the CAR-Ts leading to toxic lymphophenia, and chronic hypogammaglobulinemia for hematological targets, and fatal off-target cytolysis for solid tumor targets. Tanada et al.,2012 Clin Cancer Res, 18(23):6436-6445 discloses redirection of gene-modified T cells towards various cancer types using tagged antibodies. Kim et al., 2012; Journal of the American Chemical Society; 134(24):9918-9921 discloses synthesis of biospecific antibodies using genetically encoded unnatural amino acids. Kim et al., 2013, Current Opinoin in Chemical Biology, 17(3):412-419 discloses protein conjugation with geneically encoded unnatural amino acids.

### SUMMARY OF THE INVENTION

The present invention provides a switch for activating a chimeric antigen receptor-effector cell (CAR-EC), the switch comprising: (a) a chimeric antigen receptor-interacting domain (CAR-ID) that interacts with a chimeric antigen receptor on the CAR-EC; and (b) a target interacting domain (TID) comprising an unnatural amino acid, wherein the TID interacts with a surface molecule on a target cell, and wherein the CAR-ID is attached to the TID via the unnatural amino acid.

The present invention also provides a composition comprising a plurality of switches for activating a chimeric antigen receptor-effector cell (CAR-EC), wherein a switch of the plurality of switches comprises (a) a chimeric antigen receptor-interacting domain (CAR-ID) that interacts with a chimeric antigen receptor on the CAR-EC; and (b) a target interacting domain (TID) comprising an unnatural amino acid, wherein the TID interacts with a surface molecule on a target cell, wherein the CAR-ID is attached to the TID via the unnatural amino acid, and wherein at least about 60% of the switches are structurally homologous.

The present invention also provides a first chimeric antigen receptor-effector cell (CAR-EC) switch for use in a method of treating a disease, the switch comprising: (i) a first chimeric antigen receptor-interacting domain (CAR-ID) that interacts with a chimeric antigen receptor on the CAR-EC; and (ii) a first target interacting domain (TID) comprising an unnatural amino acid, wherein the TID interacts with a first surface molecule on a target cell and wherein the TID comprises a CAR-ID attached site-specifically to the TID via the unnatural amino acid, wherein the method comprises administering the first CAR-EC switch and further administering a first chimeric antigen receptor-effector cell comprising a chimeric antigen receptor that binds to the first CAR-ID of the first CAR-EC switch to a subject.

Disclosed herein are chimeric antigen receptor-effector cell (CAR-EC) switches comprising: (a) a chimeric antigen receptor-interacting domain (CAR-ID) comprising a small molecule; and (b) a target interacting domain (TID) comprising an unnatural amino acid. The CAR-ID may interact with a chimeric antigen receptor (CAR) on an effector cell. The effector cell may be a chimeric antigen receptor-effector cell (CAR-EC). The target interacting domain (TID) may interact with a surface molecule on a target cell. The CAR-ID may be attached to the TID. The CAR-ID may be site-specifically linked to the TID. The CAR-ID may be site-specifically linked to the unnatural amino acid of the TID. The TID may comprise an antibody or antibody fragment. The antibody fragment may be comprise a Fab. The antibody or antibody fragment may comprise at least a portion of an immunoglobulin. The CAR-EC switches may further comprise a linker. The linker may attach the CAR-ID to the TID. The linker may site-specifically link the CAR-ID to the TID. The linker may site-specifically link the CAR-ID to the unnatural amino acid of the TID. The linker may link the CAR-ID to the TID through one or more chemical groups. The chemical group may be selected from the group consisting of oxime, triazole, cyclooctyne, tetrazine, cyclopropene, norbornene, trans-cyclooctene, and selenocysteine. The chemical group may be formed between the linker and the TID. Alternatively, or additionally, the chemical group may be formed between the linker and the CAR-ID. The CAR-EC switches may further comprise one or more additional linkers. The one or more additional linkers may link an additional TID to the CAR-ID. Alternatively, or additionally, the one or more additional linkers may link an additional CAR-ID to the TID. The one or more additional linkers may be conjugated to the TID. Alternatively, the one or more additional linkers may be conjugated to the CAR-ID. The one or more additional linkers may connect the CAR-ID to a linker that is attached to the TID. The linker may possess a length of about 25 Å. The linker may provide a distance between the CAR-ID and TID of about 2.5 Å to about 100 Å. The CAR-ID may be a hapten. The CAR-ID may comprise FITC or derivatives thereof. The CAR-ID may be selected from DOTA, dinitrophenol, biotin and derivatives thereof. The TID may comprise a polypeptide comprising the unnatural amino acid. The polypeptide may be based on or derived from an antibody or fragment thereof. The unnatural amino acid may replace an amino acid residue of the antibody or fragment from which the polypeptide is based on or derived. The polypeptide may be based on or derived light chain of the antibody. The unnatural amino acid may replace an amino acid of the light chain of the antibody from which the polypeptide is based on or derived. For example, the unnatural amino acid may replace a serine residue of the antibody light chain. The unnatural amino acid may replace serine 202 of the antibody light chain or a homologue thereof. The unnatural amino acid may replace a glycine residue of the antibody light chain. The unnatural amino acid may replace glycine 68 of the antibody light chain or a homologue thereof. The unnatural amino acid may replace a threonine residue of the antibody light chain. The unnatural amino acid may replace threonine 109 of the antibody light chain or a homologue thereof. The polypeptide may be based on or derived from a heavy chain of the antibody. The unnatural amino acid may replace a lysine residue of the antibody heavy chain. The unnatural amino acid may replace lysine 136 of the antibody heavy chain or a homologue thereof. The unnatural amino acid may replace an alanine residue of the antibody heavy chain. The unnatural amino acid may replace alanine 123 of the antibody heavy chain or a homologue thereof. The unnatural amino acid may replace serine residue of the antibody heavy chain. The unnatural amino acid may replace serine 74 of the antibody heavy chain or a homologue thereof. The unnatural amino acid may replace an amino acid residue of the antibody light chain and an amino acid residue of the antibody heavy chain. The unnatural amino acid may replace a glycine residue of the antibody light chain and an amino acid of the antibody heavy chain. The unnatural amino acid may replace a glycine residue of the antibody light chain and a serine residue of the antibody heavy chain. The unnatural amino acid may replace a serine residue of the antibody heavy chain and an amino acid residue of the antibody light chain. The glycine residue of the antibody light chain may be glycine 68 or a homologue thereof. The serine residue of the antibody heavy chain may be serine 74 or a homologue thereof. The unnatural amino acid may replace a serine residue of the antibody light chain and an amino acid of the antibody heavy chain. The unnatural amino acid may replace a serine residue of the antibody light chain and a lysine residue of the antibody heavy chain. The unnatural amino acid may replace a lysine residue of the antibody heavy chain and an amino acid residue of the antibody light chain. The serine residue of the antibody light chain may be serine 202 or a homologue thereof. The serine residue of the antibody heavy chain may be lysine 136 or a homologue thereof. The TID may be based on or derived from an antibody selected from anti-EGFRvIII antibody, anti-CD33 antibody, anti-CLL-1 antibody, anti-CEA antibody, anti-CD19 antibody, anti-BCMA antibody, anti-CS1 antibody and fragments thereof. The TID may be selected from an anti-EGFR antibody, anti-Her2 antibody and fragments thereof. The CAR-ID may comprise FITC and the TID may comprise an anti-CD19 antibody or a fragment thereof. The CAR-ID may comprise FITC and the TID may comprise an anti-Her2 antibody or a fragment thereof. The CAR-ID may comprise FITC and the TID may be selected from an anti-CS1 antibody, anti-BCMA antibody, anti-CLL1 antibody, anti-CD33 antibody, or a fragment thereof. The TID may be encoded by a nucleotide of any one of SEQ ID NOs: 5-9. The TID may be encoded by a nucleotide sequence that is at least about 70% identical to any one of SEQ ID NOs: 5-9. The TID may comprise a polypeptide of any one of SEQ ID NOs: 10-17. The TID may comprise an amino acid sequence that is at least about 70% identical to any one of SEQ ID NOs: 10-17. The TID may comprise a polypeptide of any one of SEQ ID NOs: 18-56. The TID may comprise an amino acid sequence that is at least about 70% identical to any one of SEQ ID NOs: 18-56. The target cell may be a cancer cell. The purity of a plurality of CAR-EC switches may be at least about 90%. The homogeneity of a plurality of CAR-EC switches may be at least about 90%. The structural homogeneity of a plurality of CAR-EC switches may be at least about 90%.

Further disclosed herein are CAR-EC switches comprising (a) a chimeric antigen receptor-interacting domain (CAR-ID); and (b) a target interacting domain (TID) that binds a surface molecule on a target, wherein the CAR-ID and the TID do not comprise two or more amino acids connected by an amide bond. The CAR-ID may comprise one to five amino acids. The TID may comprise one to five amino acids. The CAR-EC switches may have a molar mass of less than about 1500 Da. The CAR-EC switches may have a molar mass of less than about 2500 Da. The CAR-EC switches may further comprise a linker. The linker may attach to CAR-ID to the linker. The CAR-ID and TID may be site-specifically linked through the linker. The CAR-EC switches may further comprise one or more additional linkers. The one or more additional linkers may link the CAR-ID to an additional TID. Alternatively, or additionally, the one or more linkers may link the TID to an additional CAR-ID. The one or more additional linkers may be conjugated to the TID. Alternatively, the one or more additional linkers may be conjugated to the CAR-ID. The one or more additional linkers may connect the CAR-ID to a linker that is attached to the TID. The CAR-ID may be selected from FITC, dinitrophenol, biotin and a derivative thereof. The CAR-ID may comprise FITC. The TID may bind a target that is at least 50% homologous to prostate specific membrane antigen (PSMA). The TID may be selected from 2-[3-(1,3-dicarboxypropyl)ureido] pentanedioic acid, a small molecule cholecystokinin B receptor antagonist, a 10-mer peptide luteinizing hormone releasing hormone, folate, a derivative thereof, or a modified version thereof. The TID may comprise 2-[3-(1,3-dicarboxypropyl)ureido] pentanedioic acid. The TID may comprise folate. The TID may bind a target that is at least 50% homologous to a receptor selected from a folate receptor, a luteinizing hormone releasing hormone receptor and a cholecystokinin B receptor. The CAR-ID may comprise FITC and the TID may bind to a surface molecule that is at least 50% homologous to prostate specific membrane antigen (PSMA). The CAR-ID may comprise FITC and the TID may comprise 2-[3-(1,3-dicarboxypropyl)ureido] pentanedioic acid. The CAR-ID may comprise FITC and the TID may bind a surface molecule that is at least 50% homologous to a folate receptor. The chimeric antigen receptor-binding component may comprise FITC and the TID may comprise folate or a derivative thereof. The target may be a cancer cell. The homogeneity of a plurality of CAR-EC switches may be at least about 90%.

Further disclosed herein are CAR-EC switches comprising: (a) a chimeric antigen receptor-interacting domain (CAR-ID) comprising FITC or a derivative thereof; (b) a target interacting domain (TID) comprising an antibody or antibody fragment; and (c) a linker that links the CAR-ID to the TID. The antibody or antibody fragment may be selected from an anti-Her2 antibody, anti-CD19 antibody or a fragment thereof. The geometry of a CAR-EC switch with the linker may be different from the geometry of a CAR-EC switch without the linker. For example, the angle of the CAR-ID to the TID may be altered by the linker as compared to the angle of the CAR-ID to the TID in a switch without a linker. The distance between the CAR-ID and the TID in a switch with a linker may be different from the distance between the CAR-ID and the TID in a switch without a linker. For example, the distance between the CAR-ID and the TID may be greater in a switch with a linker than the distance between the CAR-ID and the TID in a switch without the linker.

Disclosed herein is a switch for activating a chimeric antigen receptor-effector cell (CAR-EC), the switch comprising: (a) a chimeric antigen receptor-interacting domain (CAR-ID) that interacts with a chimeric antigen receptor on the CAR-EC; and (b) a target interacting domain (TID) comprising an unnatural amino acid, wherein the TID interacts with a surface molecule on a target cell. The CAR-ID may be attached to the TID. The CAR-ID may be attached to the TID via the unnatural amino acid. The CAR-ID may be site-specifically attached to the unnatural amino acid of the TID. The switch may further comprise a linker. The linker may attach the CAR-ID to the TID. The linker may attach the CAR-ID to the TID via the unnatural amino acid. The linker may site-specifically attach the CAR-ID to the unnatural amino acid of the TID. The linker may be a bifunctional linker. The linker may be a heterobifunctional linker. The linker may be a homobifunctional linker. The linker may comprise an aminooxy group, azide group cyclooctyne group, or a combination thereof at one or more termini. The linker may comprise one or more polyethylene glycol subunits. The linker may comprise triazole. The CAR-ID may comprise a small molecule. The small molecule may be a hapten. The small molecule may be fluorescein isothiocyanate (FITC). The TID may be conjugated to an isothiocyanate of FITC. The switch may further comprise a linker that may be conjugated to an isothiocyanate of FITC. The small molecule may be biotin. The TID may be based on or derived from at least a portion of an antibody. The TID may be based on or derived from at least a portion of a single chain variable fragment (scFv). The TID may be based on or derived from at least a portion of an anti-CD19 antibody. The TID may be based on or derived from at least a portion of a single chain variable domain (scFv) of an anti-CD19 antibody. The TID may be based on or derived from at least a portion of an antibody selected from the group consisting of anti-CD20, anti-CD22, anti-CD33, anti-BMSA, anti-CEA, anti-CLL1, anti-CS1, anti-EGFR, and anti-Her2. The TID may be based on or derived from at least a portion of a single chain variable domain (scFv) of an antibody selected from the group consisting of anti-CD20, anti-CD22, anti-CD33, anti-BMSA, anti-CEA, anti-CLL1, anti-CS1, anti-EGFR, and anti-Her2. The TID may be encoded by a nucleotide of any one of SEQ ID NOs: 5-9. The TID may be encoded by a nucleotide sequence that is at least about 70% identical to any one of SEQ ID NOs: 5-9. The TID may comprise a polypeptide of any one of SEQ ID NOs: 10-17. The TID may comprise an amino acid sequence that is at least about 70% identical to any one of SEQ ID NOs: 10-17. The TID may comprise a polypeptide of any one of SEQ ID NOs: 18-56. The TID may comprise an amino acid sequence that is at least about 70% identical to any one of SEQ ID NOs: 18-56. The unnatural amino acid may be inserted in the portion of the antibody from which the TID may be based or derived. The unnatural amino acid may replace an amino acid of the antibody from which the TID may be based or derived. The unnatural amino acid may be *p-*acetylphenylalanine (*p*AcF). The unnatural amino acid may be *p*-azidophenylalanine (*p*AzF). The unnatural amino acid may be inserted into the light chain of the antibody. The unnatural amino acid may be inserted into the heavy chain of the antibody.

Further disclosed herein are chimeric antigen receptor effector cells expressing one or more chimeric antigen receptors (CARs). The one or more CARs may comprise an anti-FITC antibody or fragment thereof. The anti-FITC antibody or fragment thereof may be selected from murine 4-4-20 scFv, chimeric 4D5Flu scFv, murine 4M5.3 scFv and human FITC-E2 scFv. The CAR may be encoded by one or more polynucleotides selected from SEQ ID NOs: 1-4. The CAR may be encoded by a polynucleotide that is at least about 70% identical to one or more polynucleotides selected from SEQ ID NOs: 1-4.

Disclosed herein is a composition comprising a plurality of switches for activating a chimeric antigen receptor-effector cell (CAR-EC), wherein a switch of the plurality of switches comprises (a) a chimeric antigen receptor-interacting domain (CAR-ID) that interacts with a chimeric antigen receptor on the CAR-EC; and (b) a target interacting domain (TID) that interacts with a surface molecule on a target cell, wherein at least about 60% of the switches are structurally homologous. At least about 80% of the switches may be structurally homologous. The CAR-ID may comprise a small molecule. The small molecule may be fluorescein isothiocyanate (FITC). The small molecule may be biotin. The small molecule may be dinitrophenol. The TID may comprise a small molecule. The small molecule may be 2-[3-(1,3-dicarboxypropyl)ureido] pentanedioic acid or a derivative thereof. The small molecule may be folate or a derivative thereof. The TID may be based on or derived from at least a portion of an antibody. The TID may be based on or derived from at least a portion of a single chain variable fragment (scFv). The TID may be based on or derived from at least a portion of an anti-CD19 antibody. The TID may be based on or derived from at least a portion of a single chain variable domain (scFv) of an anti-CD19 antibody. The TID may be based on or derived from at least a portion of an antibody selected from the group consisting of anti-CD20, anti-CD22, anti-CD33, anti-BMSA, anti-CEA, anti-CLL1, anti-CS1, anti-EGFR, and anti-Her2. The TID may be based on or derived from at least a portion of a single chain variable domain (scFv) of an antibody selected from the group consisting of anti-CD20, anti-CD22, anti-CD33, anti-BMSA, anti-CEA, anti-CLL1, anti-CS1, anti-EGFR, and anti-Her2. The TID may be encoded by a nucleotide of any one of SEQ ID NOs: 5-9. The TID may be encoded by a nucleotide sequence that is at least about 70% identical to any one of SEQ ID NOs: 5-9. The TID may comprise a polypeptide of any one of SEQ ID NOs: 10-17. The TID may comprise an amino acid sequence that is at least about 70% identical to any one of SEQ ID NOs: 10-17. The TID may comprise a polypeptide of any one of SEQ ID NOs: 18-56. The TID may comprise an amino acid sequence that is at least about 70% identical to any one of SEQ ID NOs: 18-56. The TID may comprise an unnatural amino acid. The unnatural amino acid may be *p*-acetylphenylalanine (*p*AcF). The unnatural amino acid may be *p*-azidophenylalanine (*p*AzF). The switch of the plurality of switches further may comprise a linker. The linker may attach the CAR-ID to the TID. The linker may attach the CAR-ID to the TID via an unnatural amino acid. The linker may site-specifically attach the CAR-ID to an unnatural amino acid of the TID. The linker may be a bifunctional linker. The linker may be a heterobifunctional linker. The linker may be a homobifunctional linker. The linker may comprise an aminooxy group, azide group cyclooctyne group, or a combination thereof at one or more termini. The linker may comprise one or more polyethylene glycol subunits. The linker may comprise triazole.

Disclosed herein is a composition comprising a plurality of switches for activating a chimeric antigen receptor-effector cell (CAR-EC), wherein a switch of the plurality of switches comprises (a) a chimeric antigen receptor-interacting domain (CAR-ID) that interacts with a chimeric antigen receptor on the CAR-EC; and (b) a target interacting domain (TID) comprising a polypeptide, wherein the CAR-ID is attached to the same predetermined site in the TID in at least 60% of the switches. The CAR-ID may be attached to the same predetermined site in the TID in at least about 80% of the switches. The CAR-ID may comprise a small molecule. The small molecule may be fluorescein isothiocyanate (FITC). The small molecule may be biotin. The small molecule may be dinitrophenol. The TID may comprise a small molecule. The small molecule may be 2-[3-(1,3-dicarboxypropyl)ureido] pentanedioic acid or a derivative thereof. The small molecule may be folate or a derivative thereof. The TID may comprise a small molecule. The TID may be based on or derived from at least a portion of an antibody. The TID may be based on or derived from at least a portion of a single chain variable fragment (scFv). The TID may be based on or derived from at least a portion of an anti-CD19 antibody. The TID may be based on or derived from at least a portion of a single chain variable domain (scFv) of an anti-CD19 antibody. The TID may be based on or derived from at least a portion of an antibody selected from the group consisting of anti-CD20, anti-CD22, anti-CD33, anti-BMSA, anti-CEA, anti-CLL1, anti-CS1, anti-EGFR, and anti-Her2. The TID may be based on or derived from at least a portion of a single chain variable domain (scFv) of an antibody selected from the group consisting of anti-CD20, anti-CD22, anti-CD33, anti-BMSA, anti-CEA, anti-CLL1, anti-CS1, anti-EGFR, and anti-Her2. The TID may be encoded by a nucleotide of any one of SEQ ID NOs: 5-9. The TID may be encoded by a nucleotide sequence that is at least about 70% identical to any one of SEQ ID NOs: 5-9. The TID may comprise a polypeptide of any one of SEQ ID NOs: 10-17. The TID may comprise an amino acid sequence that is at least about 70% identical to any one of SEQ ID NOs: 10-17. The TID may comprise a polypeptide of any one of SEQ ID NOs: 18-56. The TID may comprise an amino acid sequence that is at least about 70% identical to any one of SEQ ID NOs: 18-56. The TID may comprise an unnatural amino acid. The unnatural amino acid may be *p*-acetylphenylalanine (*p*AcF). The unnatural amino acid may be *p*-azidophenylalanine (*p*AzF). The switch of the plurality of switches further may comprise a linker. The linker may attach the CAR-ID to the TID. The linker may attach the CAR-ID to the TID via an unnatural amino acid. The linker may site-specifically attach the CAR-ID to an unnatural amino acid of the TID. The linker may be a bifunctional linker. The linker may be a heterobifunctional linker. The linker may be a homobifunctional linker. The linker may comprise an aminooxy group, azide group cyclooctyne group, or a combination thereof at one or more termini. The linker may comprise one or more polyethylene glycol subunits. The linker may comprise triazole. The predetermined site in the TID may be an amino acid residue. The amino acid residue may be an unnatural amino acid. The unnatural amino acid may be *p-*acetylphenylalanine (*p*AcF). The unnatural amino acid may be *p*-azidophenylalanine (*p*AzF).

Further disclosed herein are methods of producing CAR-EC switches, comprising site-specifically linking a chimeric antigen receptor-interacting domain (CAR-ID) to a target interacting domain (TID), thereby producing a CAR-EC switch. Alternatively, or additionally, the method of producing a CAR-EC switch may comprise (a) producing a target interacting domain (TID) by incorporating an unnatural amino acid into a polypeptide; and (b) contacting the TID with a chimeric antigen receptor-interacting domain (CAR-ID), thereby producing a CAR-EC switch. The method may further comprise coupling a first linker to the TID to produce a first switch intermediate comprising the TID and the first linker. The first linker may be coupled to the TID by oxime ligation. The method may further comprise coupling a second linker to the CAR-ID to produce a second switch intermediate comprising the CAR-ID and the second linker. The second linker may be coupled to the CAR-ID by oxime ligation. Contacting the TID with the CAR-ID may comprise contacting the first switch intermediate with the second switch intermediate. Contacting the first switch intermediate with the second switch intermediate may comprise conducting a click chemistry reaction. Contacting the first switch intermediate with the second switch intermediate may comprise a conducting a cycloaddition reaction. The cycloaddition reaction may be a [3+2] cycloaddition reaction.

Disclosed herein is a method of producing a switch comprising (a) a chimeric antigen receptor-interacting domain (CAR-ID); and (b) a target interacting domain (TID), the method comprising: (a) coupling a first linker to the TID to produce a first switch intermediate comprising the first linker conjugated to the TID; (b) coupling the first switch intermediate to the CAR-ID, thereby producing the switch. The method may further comprise coupling a second linker to the CAR-ID to produce a second switch intermediate comprising the second linker conjugated to the CAR-ID. Coupling the first switch intermediate to the CAR-ID may comprise coupling the first switch intermediate to the second switch intermediate. Coupling the first linker to the TID may comprise an oxime ligation. The second linker may comprise a cyclooctyne. Coupling the first switch intermediate to the second switch intermediate may comprise a cycloaddition reaction. The first linker may comprise an azide. The cycloaddition reaction may comprise reacting the cyclooctyne of the second linker with the azide of the first linker. The cycloaddition reaction may comprise a [3+2] cycloaddition reaction. The TID may comprise a small molecule. The small molecule may be 2-[3-(1,3-dicarboxypropyl)ureido]pentanedioic acid or a derivative thereof. The small molecule may be folate or a derivative thereof. The TID may be based on or derived from at least a portion of an antibody. The TID may be based on or derived from at least a portion of a single chain variable fragment (scFv). The TID may be based on or derived from at least a portion of an anti-CD 19 antibody. The TID may be based on or derived from at least a portion of a single chain variable domain (scFv) of an anti-CD19 antibody. The TID may be based on or derived from at least a portion of an antibody selected from the group consisting of anti-CD20, anti-CD22, anti-CD33, anti-BMSA, anti-CEA, anti-CLL1, anti-CS1, anti-EGFR, and anti-Her2. The TID may be based on or derived from at least a portion of a single chain variable domain (scFv) of an antibody selected from the group consisting of anti-CD20, anti-CD22, anti-CD33, anti-BMSA, anti-CEA, anti-CLL1, anti-CS1, anti-EGFR, and anti-Her2. The TID may be encoded by a nucleotide of any one of SEQ ID NOs: 5-9. The TID may be encoded by a nucleotide sequence that is at least about 70% identical to any one of SEQ ID NOs: 5-9. The TID may comprise a polypeptide of any one of SEQ ID NOs: 10-17. The TID may comprise an amino acid sequence that is at least about 70% identical to any one of SEQ ID NOs: 10-17. The TID may comprise a polypeptide of any one of SEQ ID NOs: 18-56. The TID may comprise an amino acid sequence that is at least about 70% identical to any one of SEQ ID NOs: 18-56. The TID may comprise an unnatural amino acid. The unnatural amino acid may be *p*-acetylphenylalanine (*p*AcF). The unnatural amino acid may be *p*-azidophenylalanine (*p*AzF). The CAR-ID may comprise fluorescein isothiocyanate (FITC). The small molecule may be biotin. The small molecule may be dinitrophenol. The first linker may be a bifunctional linker. The first linker may be a heterobifunctional linker. The first linker may be a homobifunctional linker. The first linker may comprise an aminooxy group, azide group cyclooctyne group, or a combination thereof at one or more termini. The first linker may comprise one or more polyethylene glycol subunits. The first linker may comprise cyclooctyne. The first linker may be a PEG-cyclooctyne linker. The first linker may comprise triazole. The triazole may be a 1,2,3-triazole or a 1,2,4-triazole. The second linker may be a bifunctional linker. The second linker may be a heterobifunctional linker. The second linker may be a homobifunctional linker. The second linker may comprise an aminooxy group, azide group cyclooctyne group, or a combination thereof at one or more termini. The second linker may comprise one or more polyethylene glycol subunits. The second linker may comprise cyclooctyne. The second linker may be a PEG-cyclooctyne linker. The second linker may comprise triazole. The triazole may be a 1,2,3-triazole or a 1,2,4-triazole.

Disclosed herein are kits comprising any of the chimeric antigen receptor-effector cell (CAR-EC) switches disclosed . The kit may further comprise a CAR-EC. The CAR-EC may comprise a chimeric antigen receptor (CAR) that interacts with a chimeric antigen receptor-interacting domain (CAR-ID) of the CAR-EC switch. The CAR-EC may comprise a T cell that expresses the CAR. The kit may further comprise one or more additional CAR-EC switches disclosed herein. The CAR-EC switches may be different. The CAR-EC switches may comprise different target interacting domains (TIDs). The CAR-EC switches may comprise different CAR-IDs. The CAR-EC switches may be similar. The CAR-EC switches may comprise the same TID. The CAR-EC switches may comprise the same CAR-ID.

Disclosed herein are kits comprising a first switch intermediate. The first switch intermediate may comprise a chimeric antigen receptor-interacting domain (CAR-ID). The first switch intermediate may further comprise a first linker. The first linker may be conjugated to the CAR-ID. The kit may further comprise a second switch intermediate. The second switch intermediate may comprise a first target interacting domain (TID). The second switch intermediate may further comprise a second linker. The second linker may be conjugated to the first TID. The kit may further comprise a CAR-EC. The CAR-EC may comprise a chimeric antigen receptor (CAR) that interacts with a chimeric antigen receptor-interacting domain (CAR-ID) of the CAR-EC switch. The CAR-EC may comprise a T cell that expresses the CAR. The kit may further comprise one or more additional switch intermediates. The one or more switch intermediates may comprise one or more additional CAR-IDs. The CAR-IDs of the one or more additional switch intermediates may be different from the CAR-ID of the first switch intermediate. The CAR-IDs of the one or more additional switch intermediates may be the same as the CAR-ID of the first switch intermediate. The one or more additional switch intermediates comprising the CAR-ID may further comprise a linker. The linker of the one or more additional switch intermediates comprising the CAR-ID may be different from the first linker of the first switch intermediate. The linker of the one or more additional switch intermediates comprising the CAR-ID may be the same as the first linker of the first switch intermediate. Alternatively, or additionally, the one or more switch intermediates may comprise one or more additional TIDs. The TIDs of the one or more additional switch intermediates may be different from the TID of the second switch intermediate. The TIDs of the one or more additional switch intermediates may be the same as the TID of the second switch intermediate. The one or more additional switch intermediates comprising the TID may further comprise a linker. The linker of the one or more additional switch intermediates comprising the TID may be different from the second linker of the second switch intermediate. The linker of the one or more additional switch intermediates comprising the TID may be the same as the second linker of the second switch intermediate.

Further disclosed herein are compounds of Formula XIII:

A-L-B (Formula XIII)

wherein:
A is a 2-[3-(1,3-dicarboxypropyl)ureido] pentanedioic acid derivative or a 2-(4-((2-amino-4-oxo 3,4-dihydropteridin-6-yl)methylamino)benzamido)pentanedioic acid (folate) derivative;
L is a bond or a linker; and
B is a fluorescein derivative or a biotin derivative.

In some embodiments, A of Formula XIII (A-L-B) is a 2-[3-(1,3-dicarboxypropyl)ureido] pentanedioic acid derivative. The 2-[3-(1,3-dicarboxypropyl)ureido] pentanedioic acid derivative may be of Formula XIV: 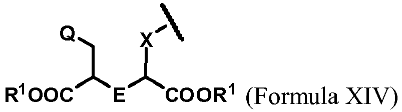 wherein:
Q is selected from the group consisting of: 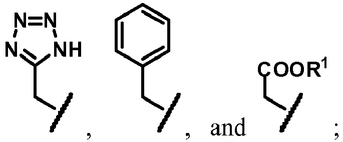 E is selected from the group consisting of: 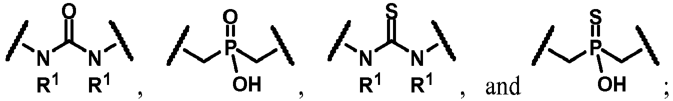
X is C₁-C₅alkylene or C₁-C₄alkylene(C=O)-; and
each R¹ is independently selected from hydrogen, C₁-C₄ alkyl, or C₁-C₄ haloalkyl.

The 2-[3-(1,3-dicarboxypropyl)ureido] pentanedioic acid derivative may be of Formula XIVa:

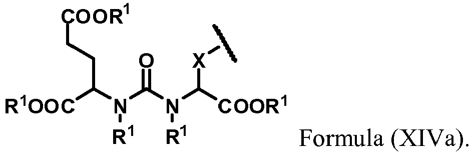

The 2-[3-(1,3-dicarboxypropyl)ureido] pentanedioic acid derivative may be 2-[3-(1,3-dicarboxypropyl)ureido] pentanedioic acid derivative is of Formula IIb:

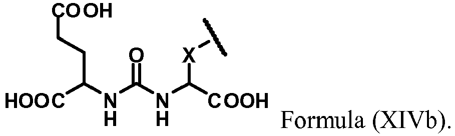

The 2-[3-(1,3-dicarboxypropyl)ureido] pentanedioic acid derivative may be of Formula XIVc:

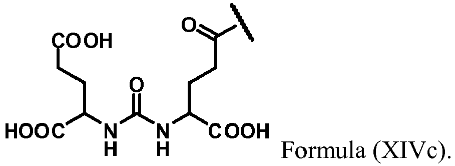

The 2-[3-(1,3-dicarboxypropyl)ureido] pentanedioic acid derivative may be of Formula XIVd:

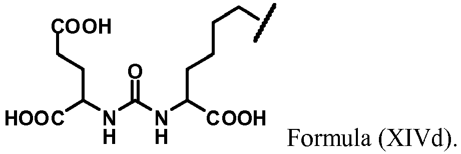

In some embodiments, A of Formula XIII (A-L-B) is a folate derivative. The folate derivative may be selected from the group consisting of: 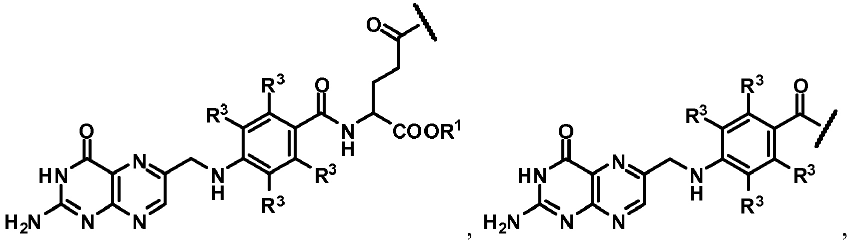 and 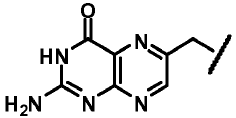 wherein:
R¹ is hydrogen, C₁-C₄ alkyl, or C₁-C₄ haloalkyl; and
each R³ is independently selected from hydrogen, halogen, C₁-C₄ alkyl and C₁-C₄ alkoxy.

The folate derivative may be:

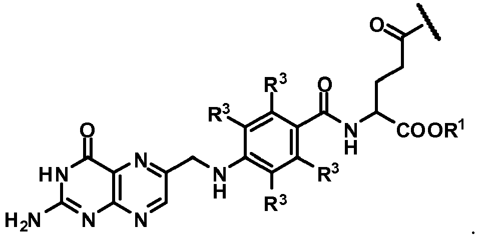

The folate derivative may be:

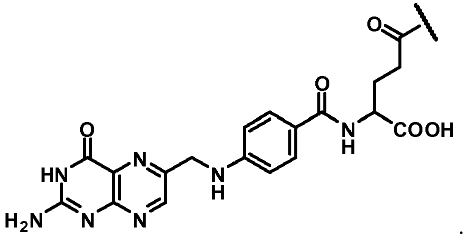

In some embodiments, B of Formula XIII (A-L-B) is a fluorescein derivative. The fluorescein derivate may be selected from Formula XV and Formula XVI: 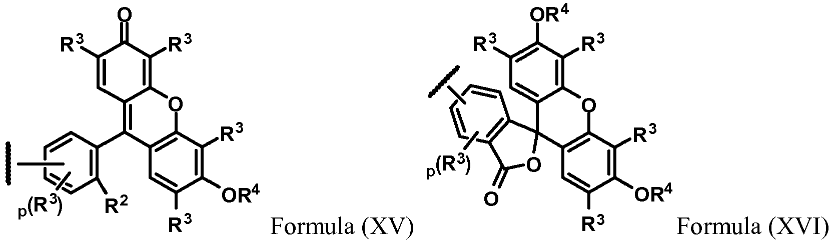 wherein:
R² is be -C(=O)O(R¹) or -C(=O)N(R^{a})₂;
each R^{a} is be independently selected from hydrogen and C₁-C₄ alkyl; or
two R^{a} taken together form an optionally substituted heterocyclyl ring;
each R³ is independently selected from hydrogen, halogen, C₁-C₄ alkyl and C₁-C₄ alkoxy; p is 0-3;
R⁴ is hydrogen, C₁-C₄ alkyl, -C(=O)C₁-C₄ alkyl or -CH₂-C(=O)OR¹; and
R¹ is hydrogen, C₁-C₄ alkyl, or C₁-C₄ haloalkyl.
The fluorescein derivative of Formula XV may be of Formula XVa: 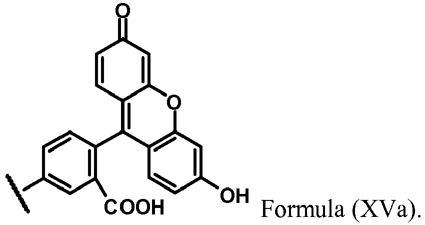 The fluorescein derivative of Formula XVI may be of Formula XVIa:

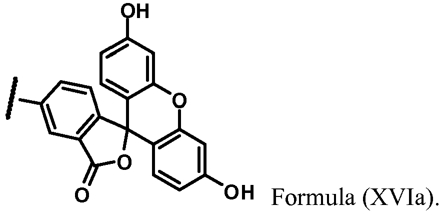

B of Formula XIII (A-L-B) may be a biotin derivative. The biotin derivative may be selected from the group consisting of:

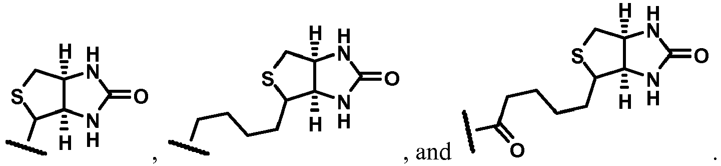

L of Formula XIII (A-L-B) may be a linker. The linker may comprise a triazole. The triazole may be a 1,2,3-triazole. The triazole may be a 1,2,4-triazole. The linker may comprise an aryl or a heteroaryl. The linker may comprise an aryl. The aryl may be phenyl. The phenyl may be disubstituted. The disubstituted phenyl may be 1,4-disubstituted phenyl. The disubstituted phenyl may be 1,3-disubstituted phenyl. The phenyl may be trisubstituted. The phenyl may be tetrasubstituted. Two of the substituents of the substituted phenyl may be NO₂. In some instances, the linker does not comprise a benzyl substituent. The linker may comprise one or more polyethylene glycol (PEG) units. The linker may comprise multiple polyethylene glycol (PEG) units. The linker may comprise 2 or more polyethylene glycol (PEG) units. The linker may comprise 3 or more polyethylene glycol (PEG) units. The linker may comprise 4 or more polyethylene glycol (PEG) units. The linker may comprise 5 or more polyethylene glycol (PEG) units. The linker may comprise 6 or more polyethylene glycol (PEG) units. The linker may comprise 7 or more polyethylene glycol (PEG) units. The linker may comprise 8 or more polyethylene glycol (PEG) units. The linker may comprise 9 or more polyethylene glycol (PEG) units. The linker may comprise 10 or more polyethylene glycol (PEG) units. The linker may comprise 11 or more polyethylene glycol (PEG) units. The linker may comprise 12 or more polyethylene glycol (PEG) units. The linker may comprise 13 or more polyethylene glycol (PEG) units. The linker may comprise 14 or more polyethylene glycol (PEG) units. The linker may comprise an amide on one end. The linker may comprise an amide on one end and an amine on the other end. The linker may comprise an amide on one end and a triazole on the other end.

The linker may be selected from the group consisting of: 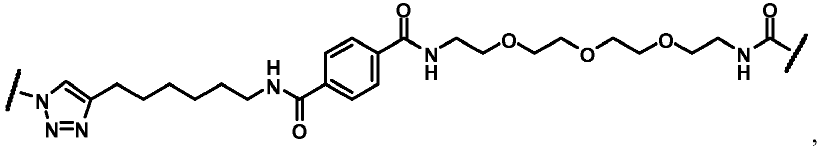 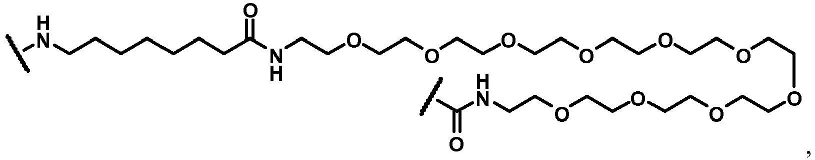 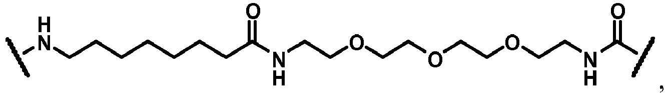 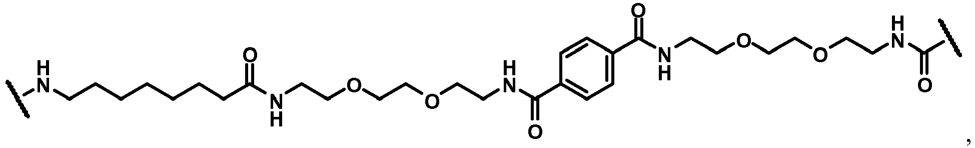 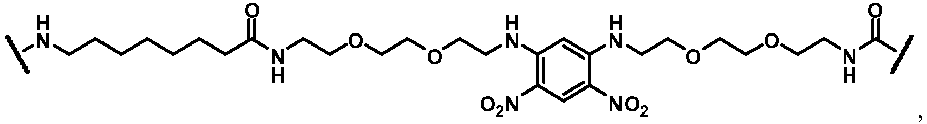 and

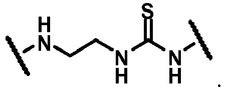

Formula XIII (A-L-B) may be of Formula XIIIa: 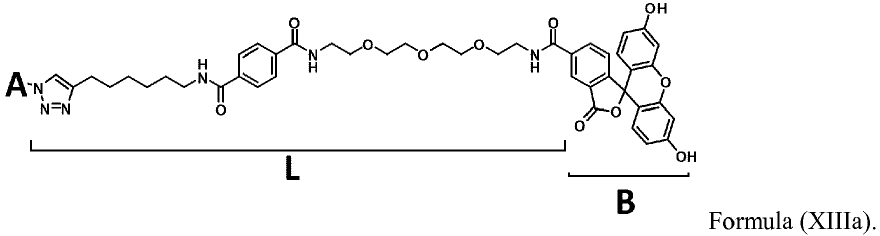 A of Formula XIIIa may be

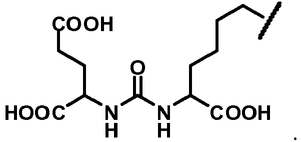

Formula XIII may be of Formula XIIIb: 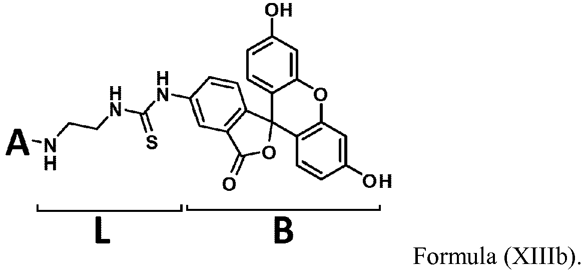 A may be

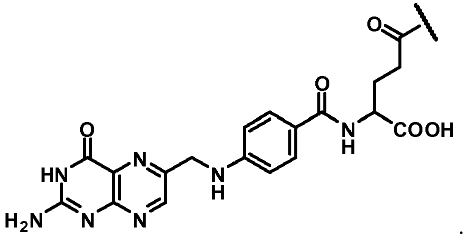

Disclosed herein is a switch intermediate for activating a chimeric antigen receptor-effector cell (CAR-EC), the switch intermediate comprising: (a) a chimeric antigen receptor-interacting domain (CAR-ID) comprising a small molecule, wherein the CAR-ID interacts with a chimeric antigen receptor on the CAR-EC; and (b) a linker connected to the CAR-ID, wherein the linker does not comprise a region that interacts with the CAR-EC and the linker does not comprise a region that interacts with a surface molecule on a target cell. The linker may be a bifunctional linker. The linker may be a heterobifunctional linker. The linker may be a homobifunctional linker. The linker may comprise an aminooxy group, azide group cyclooctyne group, or a combination thereof at one or more termini. The linker may comprise one or more polyethylene glycol subunits. The linker may comprise cyclooctyne. The linker may be a PEG-cyclooctyne linker. The linker may comprise triazole. The triazole may be a 1,2,3-triazole or a 1,2,4-triazole. The CAR-ID may comprise fluorescein isothiocyanate (FITC). The CAR-ID may comprise biotin. The CAR-ID may comprise dinitrophenol.

Disclosed herein is a switch intermediate for activating a chimeric antigen receptor-effector cell (CAR-EC), the switch intermediate comprising: (a) a target interacting domain (TID) comprising an unnatural amino acid, wherein the TID interacts with a surface molecule on a target cell; and (b) a linker connected to the TID, wherein the linker does not comprise a region that directly interacts with the CAR-EC and the linker does not comprise a region that directly interacts with the target cell. The linker may be a bifunctional linker. The linker may be a heterobifunctional linker. The linker may comprise a homobifunctional linker. The linker may comprise one or more polyethylene glycol (PEG) subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The linker may be attached to the TID via an oxime ligation. The TID may comprise a small molecule. The small molecule may be 2-[3-(1,3-dicarboxypropyl)ureido] pentanedioic acid or a derivative thereof. The small molecule may be folate or a derivative thereof. The TID may be based on or derived from at least a portion of an antibody. The TID may be based on or derived from at least a portion of a single chain variable fragment (scFv). The TID may be based on or derived from at least a portion of an anti-CD19 antibody. The TID may be based on or derived from at least a portion of a single chain variable domain (scFv) of an anti-CD19 antibody. The TID may be based on or derived from at least a portion of an antibody selected from the group consisting of anti-CD20, anti-CD22, anti-CD33, anti-BMSA, anti-CEA, anti-CLL1, anti-CS1, anti-EGFR, and anti-Her2. The TID may be based on or derived from at least a portion of a single chain variable domain (scFv) of an antibody selected from the group consisting of anti-CD20, anti-CD22, anti-CD33, anti-BMSA, anti-CEA, anti-CLL1, anti-CS1, anti-EGFR, and anti-Her2. The TID may be encoded by a nucleotide of any one of SEQ ID NOs: 5-9. The TID may be encoded by a nucleotide sequence that is at least about 70% identical to any one of SEQ ID NOs: 5-9. The TID may comprise a polypeptide of any one of SEQ ID NOs: 10-17. The TID may comprise an amino acid sequence that is at least about 70% identical to any one of SEQ ID NOs: 10-17. The TID may comprise a polypeptide of any one of SEQ ID NOs: 18-56. The TID may comprise an amino acid sequence that is at least about 70% identical to any one of SEQ ID NOs: 18-56. The TID may comprise an unnatural amino acid. The unnatural amino acid may be *p*-acetylphenylalanine (*p*AcF). The unnatural amino acid may be *p*-azidophenylalanine (*p*AzF).

Further disclosed herein are methods of treating a condition comprising administering one or more chimeric antigen receptor-effector cell (CAR-EC) switches disclosed herein or a pharmaceutical composition thereof to a subject in need thereof. The method may further comprise administering a chimeric antigen receptor-effector cell (CAR-EC) comprising a chimeric antigen receptor (CAR) to the subject in need thereof. The CAR-EC switch and the CAR-EC may be administered simultaneously. The CAR-EC switch and the CAR-EC may be administered sequentially. The CAR may interact with a chimeric antigen receptor-interacting domain (CAR-ID) of the CAR-EC switch. The CAR-ID may comprise a fluorescein derivative or the biotin derivative. The CAR-EC switch may comprise a target-interacting domain (TID). The TID may comprise a small molecule. The small molecule may be 2-[3-(1,3-dicarboxypropyl)ureido] pentanedioic acid or a derivative thereof. The small molecule may be folate or a derivative thereof. The TID may be based on or derived from at least a portion of an antibody. The TID may be based on or derived from at least a portion of a single chain variable fragment (scFv). The TID may be based on or derived from at least a portion of an anti-CD19 antibody. The TID may be based on or derived from at least a portion of a single chain variable domain (scFv) of an anti-CD19 antibody. The TID may be based on or derived from at least a portion of an antibody selected from the group consisting of anti-CD20, anti-CD22, anti-CD33, anti-BMSA, anti-CEA, anti-CLL1, anti-CS1, anti-EGFR, and anti-Her2. The TID may be based on or derived from at least a portion of a single chain variable domain (scFv) of an antibody selected from the group consisting of anti-CD20, anti-CD22, anti-CD33, anti-BMSA, anti-CEA, anti-CLL1, anti-CS1, anti-EGFR, and anti-Her2. The TID may be encoded by a nucleotide of any one of SEQ ID NOs: 5-9. The TID may be encoded by a nucleotide sequence that is at least about 70% identical to any one of SEQ ID NOs: 5-9. The TID may comprise a polypeptide of any one of SEQ ID NOs: 10-17. The TID may comprise an amino acid sequence that is at least about 70% identical to any one of SEQ ID NOs: 10-17. The TID may comprise a polypeptide of any one of SEQ ID NOs: 18-56. The TID may comprise an amino acid sequence that is at least about 70% identical to any one of SEQ ID NOs: 18-56. The TID may comprise an unnatural amino acid. The unnatural amino acid may be *p*-acetylphenylalanine (*p*AcF). The unnatural amino acid may be *p*-azidophenylalanine (*p*AzF). The switch may further comprise a linker. The linker may be a bifunctional linker. The linker may be a heterobifunctional linker. The linker may comprise a homobifunctional linker. The linker may comprise one or more polyethylene glycol (PEG) subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The CAR may comprise an anti-FITC antibody or fragment thereof. The condition may be a cancer.

Disclosed herein are methods of treating a disease or condition comprising administering a first chimeric antigen effector cell (CAR-EC) switch comprising a chimeric antigen receptor-interacting domain (CAR-ID) and a target interacting domain (TID) to a subject in need thereof. The method may further comprise administering a chimeric antigen receptor effector cell (CAR-EC) comprising a chimeric antigen receptor (CAR) that binds to the CAR-ID of the CAR-EC switch to the subject. The CAR-EC switch and the CAR-EC may be administered simultaneously. The CAR-EC switch and the CAR-EC may be administered sequentially. The method may further comprise administering a second CAR-EC switch. The first CAR-EC switch may bind a first surface molecule on a first target and the second CAR-EC switch may bind a second surface molecule on a second target. The first switch and the second switch may be administered simultaneously. The first switch and the second switch may be administered sequentially. The first surface molecule and the second surface molecule may be different. The first surface molecule and the second surface molecule may be the same. The first target and the second target may be different. The first target and the second target may be the same. The first surface molecule and the second surface molecule may be on different targets. The first surface molecule and the second surface molecule may be the same target. The first CAR-EC switch and the second CAR-EC switch may bind the same chimeric antigen receptor (CAR) on a CAR-EC. The method may further comprise administering one or more additional CAR-ECs. The CAR-ECs may be the same. The CAR-ECs may be different. The CAR-EC may be selected from a T cell, an effector B cell, a natural killer cell, a macrophage and a progenitor thereof. The CAR-EC may be a T cell. The T cell may be selected from a naive T cell, a memory stem cell T cell, a central memory T cell, an effector memory T cell, a helper T cell, a CD4+ T cell, a CD8+ T cell, a CD8/CD4+ T cell, an αβ T cell, a γδ T cell, a cytotoxic T cell, a natural killer T cell, a natural killer cell, a macrophage. The CAR of the CAR-EC may comprise an antibody or fragment thereof. The antibody may comprise an anti-FITC antibody or fragment thereof. The fragment thereof may be an scFv. The CAR of the CAR-EC may be encoded by one or more polynucleotides selected from SEQ ID NOs: 1-4. The CAR may be encoded by a polynucleotide that is at least about 70% identical to one or more polynucleotides selected from SEQ ID NOs: 1-4. The condition may be a cancer. The cancer may be selected from a relapsed cancer, a refractory cancer, a glioblastoma, an ovarian cancer, a prostate cancer, a hormone refractory prostate cancer and a breast cancer. The cancer may comprise a tumor selected from a liquid tumor or a solid tumor. The cancer may comprise a heterogeneous tumor. The cancer may be a hematological malignancy. The hematological malignancy may be selected from acute lymphoblastic leukemia, B cell lymphoma, a mantle cell lymphoma, a B cell prolymphocytic leukemia, a diffuse large cell lymphoma, a follicular lymphoma and multiple myeloma. The hematological malignancy may be a B cell malignancy. The hematological malignancy may be a CD19-positive hematological malignancy. The condition may be an infection.

### BRIEF DESCRIPTION OF THE DRAWINGS

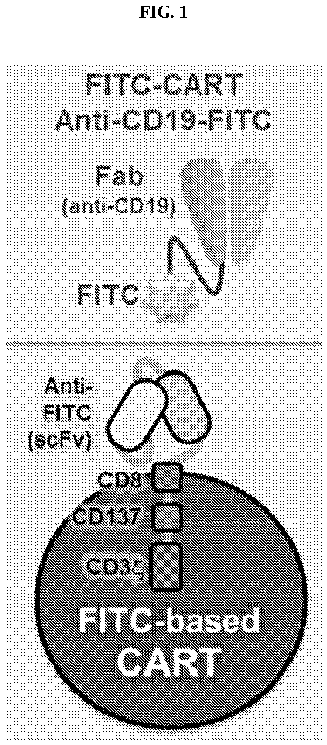

**FIG. 1** shows a non-limiting example of a FITC-based switchable CAR-EC platform.
**FIG. 2A** shows a schematic of target interacting domain (TID) comprising an anti-CD 19 Fab with non-limiting examples of natural amino acid sites that may be replaced with the unnatural amino acid p-acetylphenylalanine (pAcF). HC denotes heavy chain and LC denotes light chain.
**FIG. 2B** shows a two-step conjugation reaction consisting of an oxime reaction followed by "click" reaction in which a ketone of a p-acetylphenylalanine (pAcF) residue is used as a chemical handle to modify the protein with a heterobifunctional N3-TEG-ONH₂ linker. In this schematic, FITC is modified with a linker ending in a cyclooctyne, which can be clicked to the modified protein.

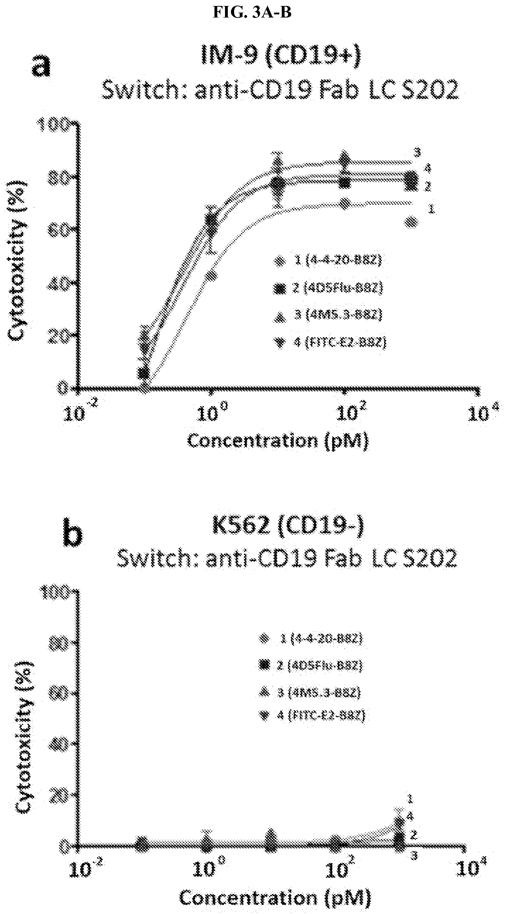

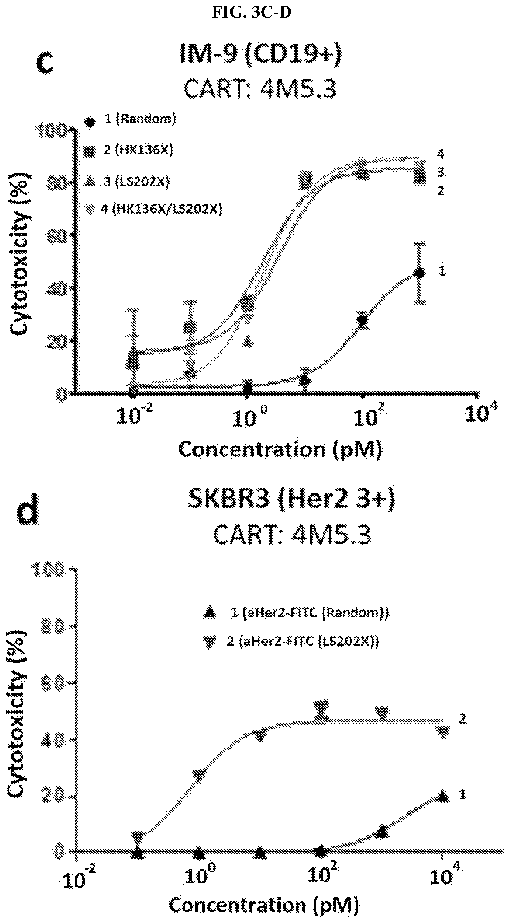

**FIG. 3** shows results from FITC-CAR-EC cytotoxicity assays at E:T ratios of 10:1 and 24 hour incubation. FIG. 3A shows the cytotoxicity of 4 FITC-CAR-EC constructs against IM-9 cells with an anti-CD19 FITC switch conjugated at LC S202. FIG. 3B shows the cytotoxicity of 4 FITC-CAR-EC constructs against K562 cells with an anti-CD19 FITC switch conjugated at LC S202. FIG. 3C shows the cytotoxicity of FITC-CAR-EC 4M5.3 against IM-9 cells with FITC switches conjugated at HC K136, LC S202, both, or nonspecific (e.g., random) conjugation. FIG. 3D shows cytotoxicity of FITC-CAR-EC 4M5.3 against SKBR3 cells with a Her2 switch conjugated at LC S202 or nonspecifically (e.g., random). LC denotes light chain.
**FIG. 4A** shows a schematic of conjugation via p-azidophenylalanine (pAzF). The pAzF unnatural amino acid is incorporated into anti-CD19 to produce a proteinogenic substrate for a single step "click" conjugation to a FITC molecule modified with a cyclooctyne linker.
**FIG. 4B** shows a flowchart for testing the efficacy of the CAR-ECs in three mouse models.
**FIG. 5A** depicts the structure of 2-[3-(1,3-dicarboxypropyl)ureido]pentanedioic acid and FITC linked by a TriA linker.
**FIG. 5B** depicts the synthetic scheme of the 2-[3-(1,3-dicarboxypropyl)ureido]pentanedioic acid-TriA-FITC CAR-EC switch.
**FIG. 5C** shows results for a 24hr cytotoxicity assay of anti-FITC-CAR T cells targeting C4-2 (PSMA+) prostate cancer cells with serial dilutions of P-TriA-FITC. Each concentration was carried out in triplicates. Results are shown as average percentage of cytotoxicity.
**FIG. 6A** depicts the structure of Folate-FITC switch.
**FIG. 6B-C** show the results for an in vitro cytotoxicity assay of anti-FITC-CAR-ECs co-incubated with KB (FR+) (**FIG.6B**) or A549 (FR-) (**FIG. 6C**) target cells in the presence of varying concentrations a Folate-FITC switch. Each concentration was carried out in triplicates. Results are shown as average percentage of cytotoxicity.
**FIG. 7** depicts CCK2R antagonist Z360.
**FIG. 8** depicts [D-Lys6]-LHRH, a proteolytically stable version of natural LHRH (luteinizing hormone releasing hormone).
**FIG. 9A** depicts sites of mutation in an anti-CD19 antibody for the incorporation of p-aziophenylalanine (pAzF) to produce a target interacting domain (TID) comprising an unnatural amino acid.
**FIG. 9B** depicts one-step, site-specific conjugation of a FITC-linker intermediate to various target interacting domains comprising mutated Fabs via a click reaction.
**FIG. 10A-N** show ESI-MS scans of FITC-anti-CD19 antibody switches.
**FIG. 11** shows SDS-PAGE size-exclusion chromatography of FITC-anti-CD19 antibody switches.
**FIG. 12** shows cytotoxicity of FITC-anti-CD19 antibody switches as measured by flow cytometry.
**FIG. 13** shows PSMA-positive tumor regression in mice treated with P-TriA-FITC-based switches and anti-FITC-CART cells.
**FIG. 14A-I** show a schematic of exemplary switches.
**FIG. 15A-H** show a schematic of exemplary switch intermediates.
**FIG. 16** shows an exemplary schematic of producing a switch.
**FIG. 17A-C** show schematics of CAR-EC regulators and CAR-ECs.
**FIG. 18** depicts exemplary linkers.
**FIG. 19** depicts heterobifunctional linkers.
**FIG. 20** shows a general scheme for synthesizing bifunctional linkers.
**FIG. 21** shows FITC-anti-CD19 antibody conjugates purified by size-exclusion chromatography and characterized by SDS-PAGE.
**FIG. 22** shows ESI-MS for FITC-anti-CD19 antibody conjugates.

### DETAILED DESCRIPTION OF THE INVENTION

Current CAR-T therapies suffer from cumbersome methods and unacceptable safety risks. These major limitations are often the result of insufficient methods to control CAR-T activity. For example, current anti-CD19 CAR-T therapies result in persistent B cell aplasia. Introduction of a switch which controls the activity of the CAR-Ts would allow the response to be turned off after neoplastic cells are eliminated and allow B cells to reproliferate. Recent preclinical studies have demonstrated that CAR-T systems can be controlled through an antibody-based switch, wherein the antibody binds the target cell (e.g. cancer cell), which enables activity to be turned off. While these systems conceptually allow for switchable targeting tumors using CAR-Ts, they suffer from a series of limitations, such as non-specific labeling of antibodies. Non-specific labeling of antibodies using cysteines or lysines often produces heterogeneous products which include variants that may be non-functional, have altered pharmacokinetics, potentially immunogenic, and/or are difficult to optimize. In addition, antibody-based switches comprising multiple CAR-T-binding regions on a single antibody produces a multivalent substrate for the T cell which may cause non-specific activation.

The immunogenicity of the chimeric antigen receptor (CAR) is also a concern. For instance, the expression of some foreign proteins (e.g. avidin) on the CAR-T cell surface may activate an undesired immune response. Furthermore, signal transduction by the CAR may be optimized to produce the greatest desired effect on the target (e.g. cancer cell).

Developing homogeneous CAR-T cell switches with precise geometry, controlled activity and specificity may be critical to develop a CAR-T therapy that has the potency and persistence necessary to eradicate malignancies while maintaining a safe, controllable treatment. The compositions, methods, platforms and kits disclosed herein address these needs.

Disclosed herein are switches for use in regulating the activity of a chimeric antigen receptor-effector cell (CAR-EC). Generally, the switch comprises (a) a chimeric antigen receptor-interacting domain (CAR-ID); and (b) a target interacting domain (TID). The switch may further comprise one or more linkers. The TID may be based on or derived from a polypeptide. The polypeptide may be modified to comprise one or more unnatural amino acids. The TID may comprise a small molecule. In some instances, the TID does not comprise two or more amino acids connected by an amide bond. The CAR-ID may comprise a small molecule. In some instances, the CAR-ID does not comprise two or more amino acids connected by an amide bond.

Disclosed herein are switches for regulating the activity of a chimeric antigen receptor-effector cell (CAR-EC), the switch comprising (a) a chimeric antigen receptor-interacting domain (CAR-ID) that interacts with a chimeric antigen receptor on the CAR-EC; and (b) a target interacting domain (TID) comprising an unnatural amino acid, wherein the TID interacts with a surface molecule on a target cell.

Disclosed herein are switches for regulating the activity of a chimeric antigen receptor-effector cell (CAR-EC), the switch comprising: (a) a chimeric antigen receptor-interacting domain (CAR-ID) that interacts with a chimeric antigen receptor on the CAR-EC; and (b) a target interacting domain (TID) that interacts with a surface molecule on a target cell, wherein the CAR-ID and the TID do not comprise two or more amino acids connected by an amide bond.

Disclosed herein are chimeric antigen receptor expressing effector cells (CAR-ECs) and chimeric antigen receptor expressing effector cell switches (CAR-EC switches) useful for the treatment of a variety of diseases and conditions. The CAR-EC switch may comprise (a) a chimeric antigen receptor-interacting domain (CAR-ID) comprising a small molecule; and (b) a target interacting domain (TID) comprising an antibody or antibody fragment. The antibody or antibody fragment may comprise a fragment antibody-binding (Fab fragment) or a full length antibody.

Further disclosed herein are CAR-EC switches comprising (a) a chimeric antigen receptor-interacting domain (CAR-ID) comprising a first small molecule; and (b) a target interacting domain (TID) comprising a second small molecule.

Disclosed herein are compositions comprising a plurality of switches for regulating the activity of a chimeric antigen receptor-effector cell (CAR-EC), wherein a switch of the plurality of switches comprises (a) a chimeric antigen receptor-interacting domain (CAR-ID) that interacts with a chimeric antigen receptor on the CAR-EC; and (b) a target interacting domain (TID) that interacts with a surface molecule on a target cell, wherein at least about 60% of the switches are structurally homologous.

Disclosed herein are compositions comprising a plurality of switches for regulating the activity of a chimeric antigen receptor-effector cell (CAR-EC), wherein a switch of the plurality of switches comprises (a) a chimeric antigen receptor-interacting domain (CAR-ID) that interacts with a chimeric antigen receptor on the CAR-EC; and (b) a target interacting domain (TID) comprising a polypeptide, wherein the CAR-ID is attached to the same amino acid residue of the TID in at least 60% of the switches.

Methods of producing the switches and switch intermediates disclosed herein may advantageously provide for control of CAR-EC cell activity, titration of off-target reactivity, abrogation of tumor lysis syndrome (TLS), attenuation of cytokine release syndrome (CRS), and/or optimization of CAR-EC switch binding by affinity, valency, geometry, linker length and/or linker chemistry through site-specific conjugation of CAR-EC switch components/regions.

Disclosed herein are methods of producing a switch for regulating the activity of a chimeric antigen receptor-effector cell (CAR-EC). The method may comprise (a) obtaining a target interacting domain (TID) comprising an unnatural amino acid; and (b) attaching a chimeric antigen receptor-interacting domain (CAR-ID) to the TID, thereby producing the switch.

Further disclosed herein are methods of producing a switch for regulating the activity of a chimeric antigen receptor-effector cell (CAR-EC) comprising (a) contacting a chimeric antigen receptor-interacting domain (CAR-ID) with a target interacting domain (TID); and (b) producing the switch by attaching the CAR-ID to a predetermined site on the TID.

Further disclosed herein are methods of producing a switch for regulating the activity of a chimeric antigen receptor-effector cell (CAR-EC) comprising (a) contacting a plurality of chimeric antigen receptor-interacting domains (CAR-IDs) with a plurality of target interacting domains (TIDs); and (b) attaching one or more CAR-IDs of the plurality of CAR-IDs to one or more TIDs of the plurality of TIDs, thereby producing a plurality of switches, wherein at least about 60% of the switches are structurally homologous.

Further disclosed herein are methods of producing a switch for regulating the activity of a chimeric antigen receptor-effector cell (CAR-EC) comprising (a) contacting a plurality of chimeric antigen receptor-interacting domains (CAR-IDs) with a plurality of target interacting domains (TIDs); and (b) attaching a CAR-ID of the plurality of CAR-IDs to a TID of the plurality of TIDs, thereby producing a plurality of switches, wherein the CAR-ID is attached to the same amino acid residue of the TID in at least 60% of the switches.

Further disclosed herein are methods of producing a switch of Formula IV: X- L1-L2-Y or Formula IVA: Y-L2-L1-X comprising (a) coupling L1 to X to produce a first intermediate of Formula IIA: L1-X, wherein: i. X comprises a chimeric antigen receptor-interacting domain (CAR-ID) that interacts with a chimeric antigen receptor on an effector cell; and ii. L1 comprises a first linker before being coupled to X; (b) coupling L2 to Y to produce a second intermediate of Formula VA: Y-L2, wherein: i. Y comprises a target interacting domain (TID) that interacts with a surface molecule on a target cell; and ii. L2 comprises a second linker before being coupled to X; and (c) linking the first intermediate to the second intermediate, thereby producing the switch of Formula IV (X- L1-L2-Y) or Formula IVA (Y-L2-L1-X).

Disclosed herein are switch intermediates. The switch intermediate may comprise (a) a chimeric antigen receptor-interacting domain (CAR-ID) comprising a small molecule, wherein the CAR-ID interacts with a chimeric antigen receptor on the CAR-EC; and (b) a linker connected to the CAR-ID, wherein the linker does not comprise a region that interacts with the CAR-EC and the linker does not comprise a region that interacts with a surface molecule on a target cell.

Further disclosed herein is a switch intermediate comprising (a) a chimeric antigen receptor-interacting domain (CAR-ID) comprising a small molecule, wherein the CAR-ID interacts with a chimeric antigen receptor on the CAR-EC; and (b) a linker connected to the CAR-ID, wherein the linker comprises an aminooxy group, azide group and/or cyclooctyne group at one or more termini.

Further disclosed herein is a switch intermediate comprising (a) a target interacting domain (TID) comprising an unnatural amino acid, wherein the TID interacts with a surface molecule on a target cell; and (b) a linker connected to the TID, wherein the linker does not comprise a region that directly interacts with the CAR-EC and the linker does not comprise a region that directly interacts with the target cell.

Further disclosed herein is a switch intermediate comprising (a) a target interacting domain (TID) comprising a polypeptide or a small molecule, wherein the TID interacts with a surface molecule on a target cell; and (b) a linker connected to the TID, wherein the linker comprises an aminooxy group, azide group and/or cyclooctyne group at one or more termini.

Further disclosed herein are methods of producing a switch intermediate for regulating the activity of a chimeric antigen receptor-effector cell (CAR-EC) comprising (a) contacting a target interacting domain (TID) with a linker, the linker comprising an aminooxy group, azide group and/or cyclooctyne group at one or more termini; and (b) attaching the linker to the TID, thereby producing the switch intermediate.

Further disclosed herein are methods of producing a switch intermediate for regulating the activity of a chimeric antigen receptor-effector cell (CAR-EC) comprising (a) contacting a chimeric antigen receptor-interacting domain (CAR-ID) with a linker, the linker comprising an aminooxy group, azide group and/or cyclooctyne group at one or more termini; and (b) attaching the linker to the CAR-ID, thereby producing the switch intermediate.

Further disclosed herein are universal chimeric antigen receptor-effector cell (CAR-EC) platforms. The CAR-EC platforms may comprise one or more CAR-EC switches, CAR-ECs, CAR-EC intermediates, and linkers. The CAR-EC may comprise a chimeric antigen receptor (CAR) comprising an ultra-high affinity antibody or antibody fragment (e.g. scFv) to the switch.

Further disclosed herein are methods of treating a disease or condition comprising administering a CAR-EC switch to a subject in need thereof. The method may further comprise administering a CAR-EC to the subject in need thereof. The CAR-EC switch may provide for a titratable response, improved safety and/or cessation of CAR-EC cell activity by controlling administration of switch. In contrast to previous approaches at controlling CAR-T cell activity, which "turn off' CAR-T activity, the CAR-EC switches disclosed herein generally function as CAR-EC activators or "on" switches.

Disclosed herein are methods of treating a disease or condition in a subject in need thereof, the method comprising administering to the subject a switch comprising: (a) a chimeric antigen receptor-interacting domain (CAR-ID) that interacts with a chimeric antigen receptor on the CAR-EC; and (b) a target interacting domain (TID) comprising an unnatural amino acid, wherein the TID interacts with a surface molecule on a target cell.

Further disclosed herein are uses of a switch in manufacturing a pharmaceutical composition for use in the treatment of a subject with cancer, the switch comprising: (a) a chimeric antigen receptor-interacting domain (CAR-ID) that interacts with a chimeric antigen receptor on the CAR-EC; and (b) a target interacting domain (TID) comprising an unnatural amino acid, wherein the TID interacts with a surface molecule on a target cell.

Methods, kits and compositions are provided for producing CAR-EC platforms, CAR-EC switches, and CAR-ECs. The methods, kits and compositions disclosed herein may be used to activate an effector cell. For example, the chimeric antigen receptor-interacting domain (CAR-ID) of a CAR-EC switch may interact with a chimeric antigen receptor (CAR) on an effector cell and the target interacting domain (TID) of the CAR-switch may interact with a surface molecule on a target, thereby activating the effector cell and directing the activity of the effector cell to the target. These methods, kits and compositions may find therapeutic use in a number of diseases. The CAR-EC platform may be used to produce a variety of CAR-EC switches with different lengths, valences, orientations, linkers, CAR-IDs and TIDs. The variability of the CAR-EC switches may provide for optimization of the CAR-EC switches in the treatment of a variety of diseases and conditions. For example, heterogeneous tumors and blood cell malignancies (e.g. acute lymphoblastic leukemia and chronic lymphocytic leukemia) may be more effectively treated with a CAR-EC and CAR-EC switch that has been optimized for particular targets. Heterogeneous tumors may also be more effectively treated with multiple CAR-EC switches that target more than one tumor antigen. Unlike previously seen CAR-EC therapy, the present invention provides for many, if not all, such optimizations. These and other objects, advantages, and features of the invention will become apparent to those persons skilled in the art upon reading the details of the compositions, methods, and kits as more fully described below.

Unless otherwise specified, the terms "switch" and "CAR-EC switch", as used herein, are used interchangeably and may refer to a small molecule-antibody switch, a hapten-antibody switch and/or a small molecule switch. The antibody portion of the hapten-antibody switch or hapten-small molecule switch may comprise at least a portion of an antibody or an entire antibody. For example, the antibody portion of the hapten-antibody switch may comprise at least a portion of a heavy chain, a portion of a light chain, a portion of a variable region, a portion of a constant region, a portion of a complementarity determining region (CDR), or a combination thereof. The antibody portion of the small molecule antibody switch and/or hapten antibody switch may comprise at least a portion of the Fc (fragment, crystallizable) region. The antibody portion of the small molecule antibody switch and/or hapten antibody switch may comprise at least a portion of the complementarity determining region (e.g., CDR1, CDR2, CDR3). The antibody portion of the small molecule antibody switch and/or hapten antibody switch may comprise at least a portion of the Fab (fragment, antigen-binding) region. The hapten-antibody switch may be a hapten-Fab switch.

Before the present methods, kits and compositions are described in greater detail, it is to be understood that this invention is not limited to particular method, kit or composition described, as such may, of course, vary. It is also to be understood that the terminology used herein is for the purpose of describing particular embodiments only, and is not intended to be limiting, since the scope of the present invention will be limited only by the appended claims. Examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and preferred methods and materials are now described.

It must be noted that as used herein and in the appended claims, the singular forms "a", "an", and "the" include plural referents unless the context clearly dictates otherwise. Thus, for example, reference to "a cell" includes a plurality of such cells and reference to "the peptide" includes reference to one or more peptides and equivalents thereof, e.g. polypeptides, known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### CAR-EC switches

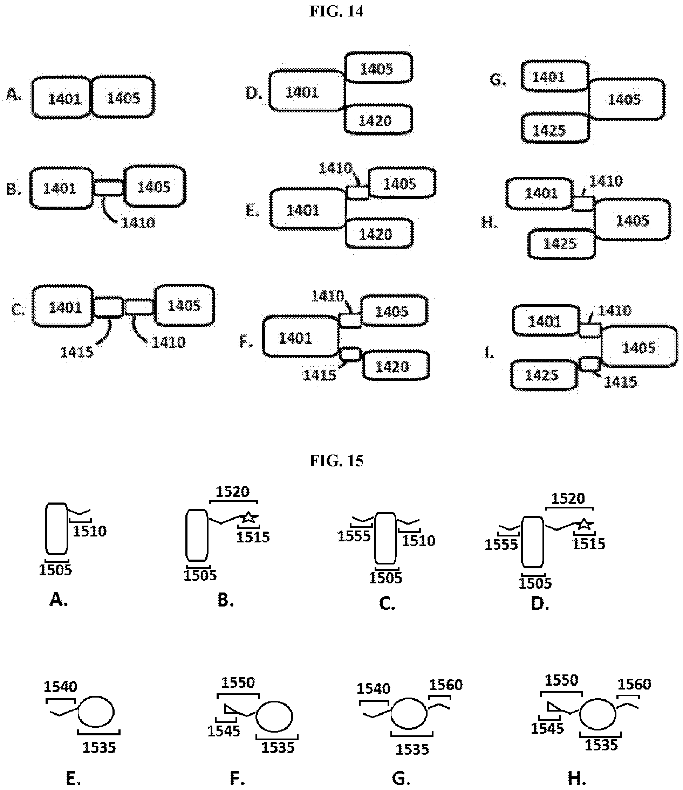

Disclosed herein are switches (e.g., chimeric antigen receptor-effector cell switches, CAR-EC switches), methods of producing such switches, and uses thereof. Generally, a switch may comprise (a) a chimeric antigen receptor-interacting domain (CAR-ID); and (b) a target interacting domain (TID). The switch may further comprise one or more additional CAR-IDs. The switch may further comprise one or more additional TIDs. The switch may further comprise one or more linkers. **FIG. 14A-I** depict exemplary switches. As shown **in** **FIG. 14A**, a switch may comprise a CAR-ID (**1401**) attached to a TID (**1405**)**.** As shown in **FIG. 14B**, a switch may comprise a CAR-ID (**1401**), a linker (**1410**) and a TID (**1405**). The linker (**1410**) may attach the CAR-ID (**1401**) to the TID (**1405**). As shown in **FIG. 14C**, a switch may comprise a CAR-ID (**1401**), a first linker (**1415**), a second linker (**1410**) and a TID (**1405**). The first linker (**1415**) and second linker (**1410**) may be connected to each other. In addition, the first linker (**1415**) may be attached to the CAR-ID (**1401**) and the second linker (**1410**) may be attached to the TID (**1405**), thereby resulting in attachment of the CAR-ID (**1401**) to the TID (**1405**). The first linker (**1410**) and the second linker (**1415**) may be the same. The first linker (**1410**) and the second linker (**1415**) maybe different.

The switch may comprise a CAR-ID and two or more TIDs. As shown in **FIG. 14D**, a switch may comprise a CAR-ID (**1401**), a first TID (**1405**), and a second TID (**1420**). The first TID (**1405**) and the second TID (**1420**) may be attached to the CAR-ID (**1401**). As shown in **FIG. 14E**, a switch may comprise a CAR-ID (**1401**), a linker (**1410**), a first TID (**1405**), and a second TID (**1420**). The linker (**1410**) may attach the first TID (**1405**) to the CAR-ID (**1401**). The second TID (**1420**) may be attached to the CAR-ID (**1401**). As shown in **FIG. 14F**, a switch may comprise a CAR-ID (**1401**), a first linker (**1410**), a second linker (**1415**), a first TID (**1405**), and a second TID (**1420**). The first linker (**1410**) may attach the first TID (**1405**) to the CAR-ID (**1401**). The second linker (**1415**) may attach the second TID (**1420**) to the CAR-ID (**1401**). The first TID (**1405**) and a second TID (**1420**) may be the same. The first TID (**1405**) and a second TID (**1420**) may be different. The first linker (**1410**) and the second linker (**1415**) may be the same. The first linker (**1410**) and the second linker (**1415**) may be different. The switch may further comprise one or more additional CAR-IDs. The switch may further comprise one or more additional TIDs. The switch may further comprise one or more linkers.

The switch may comprise a TID and two or more CAR-IDs. As shown in **FIG. 14G**, a switch may comprise a TID (**1405**), a first CAR-ID (**1401**), and a second CAR-ID (**1425**). The first CAR-ID (**1401**) and the second CAR-ID (**1425**) may be attached to the TID (**1405**). As shown in **FIG. 14H**, a switch may comprise a TID (**1405**), a linker (**1410**), a first CAR-ID (**1401**), and a second CAR-ID (**1425**). The linker (**1410**) may attach the first CAR-ID (**1401**) to the TID (**1405**). The second CAR-ID (**1425**) may be attached to the TID (**1405**). As shown in **FIG. 14I**, a switch may comprise a TID (**1405**), a first linker (**1410**), a second linker (**1415**), a first CAR-ID (**1401**), and a second CAR-ID (**1425**). The first linker (**1410**) may attach the first CAR-ID (**1401**) to the TID (**1405**). The second linker (**1415**) may attach the second CAR-ID (**1425**) to the TID (**1405**). The first CAR-ID (**1401**) and the second CAR-ID (**1425**) may be the same. The first CAR-ID (**1401**) and the second CAR-ID (**1425**) may be different. The first linker (**1410**) and the second linker (**1415**) may be the same. The first linker (**1410**) and the second linker (**1415**) may be different. The switch may further comprise one or more additional CAR-IDs. The switch may further comprise one or more additional TIDs. The switch may further comprise one or more linkers.

The CAR-ID may be attached to the TID. Attachment of the CAR-ID to the TID may occur by any method known in the art. For example, the CAR-ID may be attached to the TID by fusion, insertion, grafting, or conjugation. The CAR-ID may be fused to the TID. The CAR-ID may be inserted into the TID. The CAR-ID may be conjugated to the TID.

A switch may comprise (a) a chimeric antigen receptor-interacting domain (CAR-ID); and (b) a target interacting domain (TID). The CAR-ID may comprise fluorescein isothiocyanate (FITC). The TID may comprise 2-[3-(1,3-dicarboxypropyl)ureido] pentanedioic acid, folate or a derivative thereof. Switches that comprise a CAR-ID comprising a hapten and a TID comprising a small molecule may be referred to as hapten-small molecule switches.

A switch may comprise a chimeric antigen receptor-interacting domain (CAR-ID); and (b) a target interacting domain (TID). The CAR-ID may interact with a chimeric antigen receptor (CAR) on an effector cell. The TID may interact with a surface molecule on a target. The TID may comprise an unnatural amino acid. A TID may comprise a polypeptide that is based on or derived from an antibody or antibody fragment. The antibody or antibody fragment may be modified to contain one or more unnatural amino acids. The CAR-ID may be attached to the TID. The CAR-ID may be site-specifically attached to the TID. The CAR-ID may be site-specifically attached to the unnatural amino acid in the TID. Switches that comprise a CAR-ID comprising a small molecule and a TID comprising an antibody or antibody fragment may be referred to as small molecule-antibody switches.

A switch may comprise (a) a chimeric antigen receptor-interacting domain (CAR-ID) comprising FITC or a derivative thereof; and (b) a target interacting domain (TID). The TID may be based on or derived from a heavy chain of an antibody. The TID may be based on or derived from a light chain of an antibody. The TID may be based on or derived from a Fab of an antibody. The TID may comprise an anti-CD 19 antibody or a fragment thereof. The TID may be based on or derived from an anti-Her2 antibody or a fragment thereof. The TID may be based on or derived from an anti-CS1 antibody or a fragment thereof. The TID may be based on or derived from an anti-BCMA antibody or a fragment thereof. The TID may be based on or derived from an anti-EGFRvIII antibody or a fragment thereof. The TID may be based on or derived from an antibody or antibody fragment selected from an anti-CD20 antibody, an anti-EGFR antibody, an anti-CEA antibody, an anti-CLL-1 antibody, an anti-CD33 antibody or a fragment thereof, or a combination thereof. The TID may an anti-CD19 Fab. The TID may an anti-CD22 Fab. The TID may an anti-Her2 Fab. The TID may an anti-CSl Fab. The TID may an anti-BCMA Fab. The TID may an anti- EGFRvIII Fab. The TID may be based on or derived from a Fab selected from an anti-CD20 Fab, an anti-EGFR Fab, an anti-CEA Fab, an anti-CLL-1 Fab, an anti-CD33 Fab, or a combination thereof. The CAR-ID may be attached to the TID. The CAR-ID may be site-specifically attached to the TID. The CAR-ID may be fused to the TID. The CAR-ID may be inserted into the TID. The TID may be inserted into the CAR-ID. The CAR-EC switch may further comprise one or more linkers. The one or more linkers may attach the CAR-ID to the TID. The CAR-EC switch may further comprise one or more unnatural amino acids. The CAR-ID may comprise one or more unnatural amino acids. The TID may comprise one or more unnatural amino acids. The CAR-ID and the TID may comprise one or more unnatural amino acids. The CAR-ID may be attached to the TID via the one or more unnatural amino acids in the CAR-ID. The CAR-ID may be attached to the TID via the one or more unnatural amino acids in the TID. The CAR-ID may be attached to the TID via the one or more unnatural amino acids in the CAR-ID and one or more unnatural amino acids in the TID.

A switch may comprise (a) a chimeric antigen receptor-interacting domain (CAR-ID); and a target interacting domain (TID), wherein the CAR-ID and the TID do not comprise two or more amino acids linked by an amide bond. The CAR-ID may comprise a first small molecule. The TID may comprise a second small molecule. The first small molecule and the second small molecule may be different. The switch may further comprise one or more linkers. The switch may further comprise one or more additional CAR-IDs. The switch may further comprise one or more additional TIDs. The CAR-ID may comprise one amino acid. The CAR-ID may comprise about 1, about 2, about 3, about 4 or about five amino acids. The CAR-ID may comprise two or more amino acids. The two or more amino acids may be adjacent to each other. The two or more amino acids may be connected to each other. The two or more amino acids may be non-adjacent to each other. The two or more amino acids may not be linked by an amide bond.

A switch may comprise (a) a chimeric antigen receptor-interacting domain (CAR-ID) that interacts with a chimeric antigen receptor on the CAR-EC; and (b) a target interacting domain (TID) that interacts with a surface molecule on a target cell, wherein the CAR-ID and the TID do not comprise two or more amino acids connected by an amide bond. The switch may further comprise one or more additional CAR-IDs. The CAR-ID may comprise a small molecule. The CAR-ID may be selected from the group consisting of DOTA, dinitrophenol, quinone, biotin, aniline, atrazine, an aniline-derivative, o-aminobenzoic acid, p-aminobenzoic acid, m-aminobenzoic acid, hydralazine, halothane, digoxigenin, benzene arsonate, lactose, trinitrophenol, biotin or a derivative thereof. The CAR-ID may comprise fluorescein isothiocyanate (FITC). The CAR-ID may comprise biotin. The CAR-ID may comprise dinitrophenol. The switch may further comprise one or more additional TIDs. The TID may comprise a small molecule. The TID may comprise 2-[3-(1,3-dicarboxypropyl)ureido] pentanedioic acid or a derivative thereof. The TID may comprise folate or a derivative thereof. The switch may further comprise a linker. The switch may further comprise two or more linkers. The linker may be a bifunctional linker. The linker may be a heterobifunctional linker. The linker may be a homobifunctional linker. The linker may comprise an aminooxy group, azide group cyclooctyne group, or a combination thereof at one or more termini. The linker may comprise one or more polyethylene glycol subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The linker may comprise cyclooctyne. The linker may be a PEG-cyclooctyne linker. The linker may comprise triazole. The triazole may be a 1,2,3-triazole or a 1,2,4-triazole. The linker may be a NHS-ester linker. The linker may be a TriA linker.

A switch may comprise (a) a chimeric antigen receptor-interacting domain (CAR-ID) comprising FITC; and (b) a target interacting domain (TID) that binds to a surface molecule that is at least 50% homologous to prostate specific membrane antigen (PSMA). The TID may comprise a small molecule. The TID may comprise 2-[3-(1,3-dicarboxypropyl)ureido] pentanedioic acid or a derivative or a modified version thereof. The TID may bind to a surface molecule that is at least 50%, 55%, 57%, 60%, 62%, 65%, 67%, 70%, 72%, 75%, 77%, 80%, 82%, 85%, 87%, 90%, 92%, 95%, 97% or about 100% homologous to prostate specific membrane antigen (PSMA). The TID may bind to a surface molecule that is at least 70% homologous to prostate specific membrane antigen (PSMA). The TID may bind to a surface molecule that is at least 80% homologous to prostate specific membrane antigen (PSMA). The TID may bind to a surface molecule that is at least 90% homologous to prostate specific membrane antigen (PSMA). The TID may bind to a surface molecule that is at least 95% homologous to prostate specific membrane antigen (PSMA).

A switch may comprise (a) a chimeric antigen receptor-interacting domain (CAR-ID) comprising FITC and (b) a target interacting domain (TID) that binds to a target that is at least 50% homologous to a folate receptor. The TID may comprise a small molecule. The TID may comprise folate or a derivative or a modified version thereof. The TID may bind to a surface molecule that is about 50%, 55%, 57%, 60%, 62%, 65%, 67%, 70%, 72%, 75%, 77%, 80%, 82%, 85%, 87%, 90%, 92%, 95%, 97% or about 100% homologous to a folate receptor. The TID may bind to a surface molecule that is about 70% homologous to a folate receptor. The TID may bind to a surface molecule that is about 80% homologous to a folate receptor. The TID may bind to a surface molecule that is about 90% homologous to a folate receptor. The TID may bind to a surface molecule that is about 95% homologous to a folate receptor.

The switch may be less than about 1000 kDa, 1100 kDa, 1200 kDa, 1300 kDa, 1400 kDa, 1500 kDa, 1600 kDa, 1700 kDa, 1800 kDa, 1900, kDa, 2000 kDa, 2100 kDa, 2200 kDa, 2300 kDa, 2400 kDa, 2500 kDa, 2600 kDa, 2700 kDa, 2800 kDa, 2900, kDa or less than about 3000 kDa. The switch may be less than about 1200 kDa. The switch may be less than about 1500 kDa. The CAR-EC switch may be less than about 2000 kDa.

The percent cytotoxicity of a switch comprising a CAR-ID attached in a site-specific manner to a TID may be at least equal to the percent cytotoxicity of a switch comprisng a CAR-ID attached in a random manner to the TID. The percent cytotoxicity of a switch comprising a CAR-ID attached in a site-specific manner to a TID may be greater than the percent cytotoxicity of a switch comprisng a CAR-ID attached in a random manner to the TID. The percent cytotoxicity of a switch comprising a CAR-ID attached in a site-specific manner to a TID may be at least about 5%, 10%, 15%, 20%, 25%, 30%, 35%, 40%, 45%, 50%, 55%, 60%, 65%, 70%, 75%, 80%, 85%, 90%, 95%, 97% or 99% greater than the percent cytotoxicity of a switch comprisng a CAR-ID attached in a random manner to the TID. The percent cytotoxicity of a switch comprising a CAR-ID attached in a site-specific manner to a TID may be at least about 10% greater than the percent cytotoxicity of a switch comprisng a CAR-ID attached in a random manner to the TID. The percent cytotoxicity of a switch comprising a CAR-ID attached in a site-specific manner to a TID may be at least about 25% greater than the percent cytotoxicity of a switch comprisng a CAR-ID attached in a random manner to the TID. The percent cytotoxicity of a switch comprising a CAR-ID attached in a site-specific manner to a TID may be at least about 50% greater than the percent cytotoxicity of a switch comprisng a CAR-ID attached in a random manner to the TID. The percent cytotoxicity of a switch comprising a CAR-ID attached in a site-specific manner to a TID may be at least about 75% greater than the percent cytotoxicity of a switch comprisng a CAR-ID attached in a random manner to the TID. The percent cytotoxicity of a switch comprising a CAR-ID attached in a site-specific manner to a TID may be at least about 85% greater than the percent cytotoxicity of a switch comprisng a CAR-ID attached in a random manner to the TID. The percent cytotoxicity of a switch comprising a CAR-ID attached in a site-specific manner to a TID may be at least about 90% greater than the percent cytotoxicity of a switch comprisng a CAR-ID attached in a random manner to the TID.

The percent cytotoxicity of a switch comprising a CAR-ID attached in a site-specific manner to a TID may be at least about 1.5, 2, 2.5, 3, 3.5, 4, 4.5, 5, 5.5, 6, 6.5, 7, 7.5, 8, 8.5, 9, 9.5, or 10-fold greater than the percent cytotoxicity of a switch comprisng a CAR-ID attached in a random manner to the TID. The percent cytotoxicity of a switch comprising a CAR-ID attached in a site-specific manner to a TID may be at least about 15, 20, 25, 30, 35, 40, 45, 50, 55, 60, 65, 70, 75, 80, 85, 90, 95, or 100-fold greater than the percent cytotoxicity of a switch comprisng a CAR-ID attached in a random manner to the TID. The percent cytotoxicity of a switch comprising a CAR-ID attached in a site-specific manner to a TID may be at least about 200, 300, 400, 500, 600, 700, 800, 900, or 1000-fold greater than the percent cytotoxicity of a switch comprisng a CAR-ID attached in a random manner to the TID. The percent cytotoxicity of a switch comprising a CAR-ID attached in a site-specific manner to a TID may be at least about 2-fold greater than the percent cytotoxicity of a switch comprisng a CAR-ID attached in a random manner to the TID. The percent cytotoxicity of a switch comprising a CAR-ID attached in a site-specific manner to a TID may be at least about 3-fold greater than the percent cytotoxicity of a switch comprisng a CAR-ID attached in a random manner to the TID. The percent cytotoxicity of a switch comprising a CAR-ID attached in a site-specific manner to a TID may be at least about 4-fold greater than the percent cytotoxicity of a switch comprisng a CAR-ID attached in a random manner to the TID. The percent cytotoxicity of a switch comprising a CAR-ID attached in a site-specific manner to a TID may be at least about 5-fold greater than the percent cytotoxicity of a switch comprisng a CAR-ID attached in a random manner to the TID.

### Switch Intermediates

Disclosed herein are switch intermediates (e.g., chimeric antigen receptor-effector cell switch intermediates, CAR-EC switch intermediates), methods of producing such switches, and uses thereof. Generally, a switch intermediate comprises (a) a chimeric antigen receptor-interacting domain (CAR-ID); and (b) a linker. The switch intermediate may further comprise one or more additional CAR-IDs. The switch intermediate may further comprise one or more additional linkers. The CAR-ID may comprise one or more functional groups. The linker may comprise one or more functional groups. Switch intermediates that comprise a CAR-ID may be referred to as CAR-ID switch intermediates. The switch intermediate may further comprise one or more additional CAR-IDs. The CAR-ID may comprise a small molecule. The CAR-ID may be selected from the group consisting of DOTA, dinitrophenol, quinone, biotin, aniline, atrazine, an aniline-derivative, o-aminobenzoic acid, p-aminobenzoic acid, m-aminobenzoic acid, hydralazine, halothane, digoxigenin, benzene arsonate, lactose, trinitrophenol, biotin or a derivative thereof. The CAR-ID may comprise fluorescein isothiocyanate (FITC). The CAR-ID may comprise biotin. The CAR-ID may comprise dinitrophenol. The switch intermediate may further comprise one or more additional linkers. The linker may be a bifunctional linker. The linker may be a heterobifunctional linker. The linker may be a homobifunctional linker. The linker may further comprise one or more polyethylene glycol subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The linker may comprise cyclooctyne at one end. The linker may be a PEG-cyclooctyne linker. The linker may comprise triazole. The triazole may be a 1,2,3-triazole or a 1,2,4-triazole. The linker may be a NHS-ester linker. The linker may be a TriA linker. The linker may be attached to the CAR-ID by oxime ligation.

A switch intermediate may comprise (a) a chimeric antigen receptor-interacting domain (CAR-ID) comprising a small molecule, wherein the CAR-ID interacts with a chimeric antigen receptor on the CAR-EC; and (b) a linker connected to the CAR-ID, wherein the linker comprises an aminooxy group, azide group and/or cyclooctyne group at one or more termini. The switch intermediate may further comprise one or more additional CAR-IDs. The CAR-ID may comprise a small molecule. The CAR-ID may be selected from the group consisting of DOTA, dinitrophenol, quinone, biotin, aniline, atrazine, an aniline-derivative, o-aminobenzoic acid, p-aminobenzoic acid, m-aminobenzoic acid, hydralazine, halothane, digoxigenin, benzene arsonate, lactose, trinitrophenol, biotin or a derivative thereof. The CAR-ID may comprise fluorescein isothiocyanate (FITC). The CAR-ID may comprise biotin. The CAR-ID may comprise dinitrophenol. The switch intermediate may further comprise one or more additional linkers. The linker may be a bifunctional linker. The linker may be a heterobifunctional linker. The linker may be a homobifunctional linker. The linker may further comprise one or more polyethylene glycol subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The linker may comprise cyclooctyne at one end. The linker may be a PEG-cyclooctyne linker. The linker may be attached to the CAR-ID by oxime ligation.

Alternatively, or additionally, a switch intermediate may comprise (a) a target interacting domain (TID); and (b) a linker. The switch intermediate may further comprise one or more additional TIDs. The switch intermediate may further comprise one or more additional linkers. The TID may comprise one or more functional groups. The linker may comprise one or more functional groups. Switch intermediates that comprise a TID may be referred to as TID switch intermediates. The switch intermediate may further comprise one or more additional TIDs. The TID may comprise a small molecule. The TID may comprise 2-[3-(1,3-dicarboxypropyl)ureido] pentanedioic acid or a derivative thereof. The TID may comprise folate or a derivative thereof. The TID may comprise a polypeptide based on or derived from an antibody or antibody fragment. The antibody may be selected from the group consisting of an anti-CD19 antibody, an anti-CD22 antibody, an anti-CD20 antibody, an anti-EGFR antibody, an anti-EGFRvIII antibody, an anti-Her2 antibody, an anti-CS1 antibody, an anti-BCMA antibody, an anti-CEA antibody, an anti-CLL-1 antibody and an anti-CD33 antibody. The antibody may be an anti-CD19 antibody. The antibody may be an anti-EGFR antibody. The antibody may be an anti-CD20 antibody. The antibody may be an anti-HER2 antibody. The TID may comprise an antibody fragment. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56. The TID may comprise one or more unnatural amino acids. The switch intermediate may further comprise one or more additional linkers. The linker may be a bifunctional linker. The linker may be a heterobifunctional linker. The linker may be a homobifunctional linker. The linker may comprise an aminooxy group, azide group cyclooctyne group, or a combination thereof at one or more termini. The linker may comprise one or more polyethylene glycol subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The linker may comprise cyclooctyne. The linker may be a PEG-cyclooctyne linker. The linker may comprise triazole. The triazole may be a 1,2,3-triazole or a 1,2,4-triazole. The linker may be a NHS-ester linker. The linker may be a TriA linker. The linker may be attached to the TID by oxime ligation.

A switch intermediate may comprise (a) a target interacting domain (TID) comprising a polypeptide or a small molecule, wherein the TID interacts with a surface molecule on a target cell; and (b) a linker connected to the TID, wherein the linker comprises an alkoxy-amine (or aminooxy) group, azide group and/or cyclooctyne group at one or more termini. The switch intermediate may further comprise one or more additional TIDs. The TID may comprise a small molecule. The TID may comprise 2-[3-(1,3-dicarboxypropyl)ureido] pentanedioic acid or a derivative thereof. The TID may comprise folate or a derivative thereof. The TID may comprise a polypeptide based on or derived from an antibody or antibody fragment. The antibody may be selected from the group consisting of an anti-CD19 antibody, an anti-CD22 antibody, an anti-CD20 antibody, an anti-EGFR antibody, an anti-EGFRvIII antibody, an anti-Her2 antibody, an anti-CSl antibody, an anti-BCMA antibody, an anti-CEA antibody, an anti-CLL-1 antibody and an anti-CD33 antibody. The antibody may be an anti-CD19 antibody. The antibody may be an anti-EGFR antibody. The antibody may be an anti-CD20 antibody. The antibody may be an anti-HER2 antibody. The TID may comprise an antibody fragment. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56. The TID may comprise one or more unnatural amino acids. The switch intermediate may further comprise one or more additional linkers. The linker may be a bifunctional linker. The linker may be a heterobifunctional linker. The linker may be a homobifunctional linker. The linker may comprise an aminooxy group, azide group cyclooctyne group, or a combination thereof at one or more termini. The linker may comprise one or more polyethylene glycol subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The linker may comprise cyclooctyne. The linker may be a PEG-cyclooctyne linker.

**FIG. 15A-H** depict exemplary switch intermediates. **FIG. 15A** shows a switch intermediate comprising a CAR-ID (**1505**) and a linker (**1510**). **FIG. 15B** shows a switch intermediate comprising a CAR-ID (**1505**) and a linker (**1520**), wherein the linker comprises a functional group (**1515**). **FIG. 15C** shows a switch intermediate comprising a CAR-ID (**1505**), a first linker (**1510**), and a second linker (**1555**). **FIG. 15D** shows a switch intermediate comprising a CAR-ID (**1505**), a first linker (**1520**), and a second linker (**1555**), wherein the first linker (**1520**) comprises a functional group (**1515**). **FIG. 15E** shows a switch intermediate comprising a TID (**1535**) and a linker (**1540**). **FIG. 15F** shows a switch intermediate comprising a TID (**1535**) and a linker (**1550**), wherein the linker comprises a functional group (**1545**). **FIG. 15G** shows a switch intermediate comprising a TID (**1535**), a first linker (**1540**), and a second linker (**1560**). **FIG. 15H** shows a switch intermediate comprising a TID (**1535**), a first linker (**1550**), and a second linker (**1560**), wherein the first linker (**1550**) comprises a functional group (**1545**).

A switch intermediate may comprise a compound of Formula IIIA: L1-Y or Formula III: Y-L1, wherein Y comprises a TID that interacts with a surface molecule that is at least 50% homologous to PSMA and L1 comprises a linker. The switch intermediate may comprise a compound of Formula IIIA: L1-Y or Formula III: Y-L1, wherein Y comprises 2-[3-(1,3-dicarboxypropyl)ureido] pentanedioic acid and L1 comprises a linker. The TID may interact with a surface molecule that is at least 50%, 55%, 57%, 60%, 62%, 65%, 67%, 70%, 72%, 75%, 77%, 80%, 82%, 85%, 87%, 90%, 92%, 95%, 97% or about 100% homologous to prostate specific membrane antigen (PSMA). The TID may interact with a surface molecule that is at least 70% homologous to prostate specific membrane antigen (PSMA). The TID may interact with a surface molecule that is at least 80% homologous to prostate specific membrane antigen (PSMA). The TID may interact with a surface molecule that is at least 90% homologous to prostate specific membrane antigen (PSMA). The TID may interact with a surface molecule that is at least 95% homologous to prostate specific membrane antigen (PSMA).

The switch intermediate, by non-limiting example, may comprise a compound selected from the compounds of Formula VI, Formula VII, Formula VIII and Formula IX: 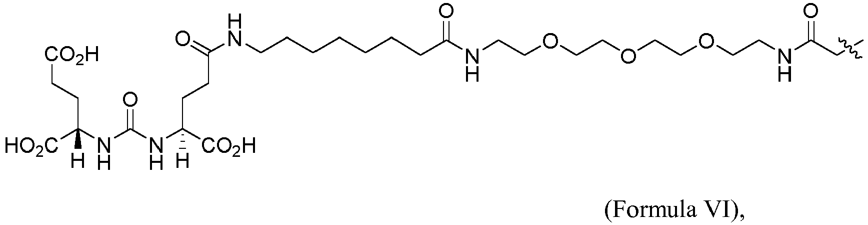 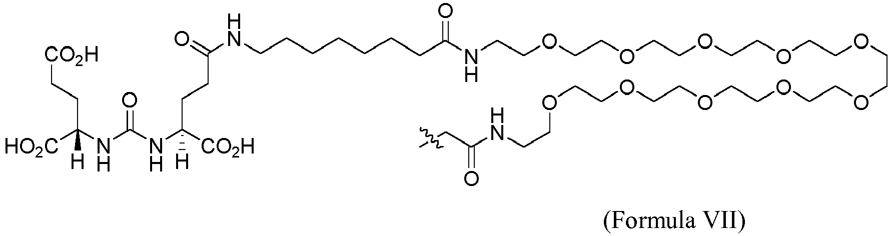 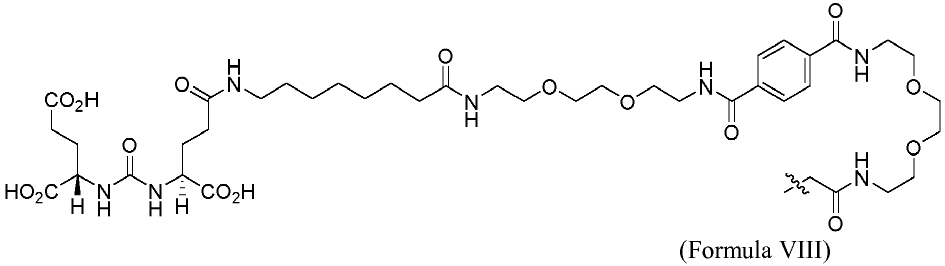 and 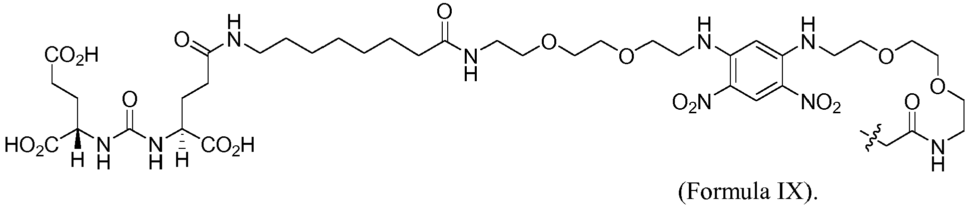 The switch intermediate, by non-limiting example, may comprise a compound selected from a derivative, analog, isomer, rearrangement or modification of the compounds of Formula VI, Formula VII, Formula VIII and Formula IX.

The switch intermediate may comprise a compound of Formula X:

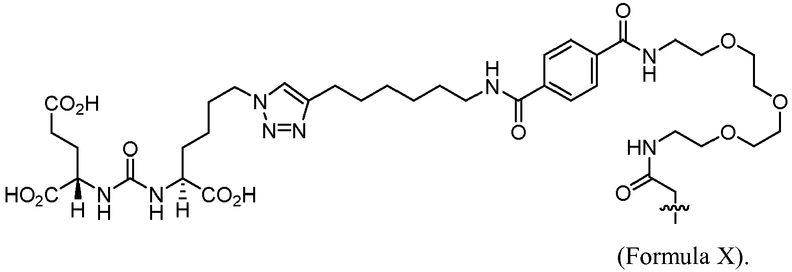

The switch intermediate may comprise a compound of Formula XI:

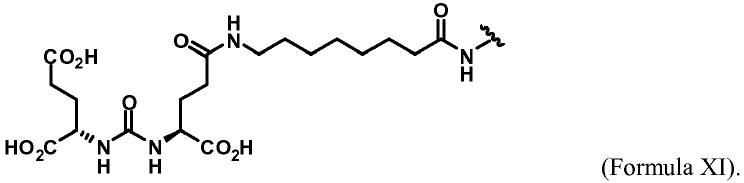

The switch intermediate may comprise a compound of Formula XII:

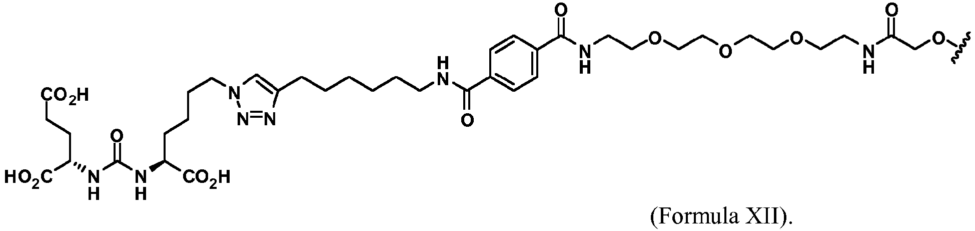

The switch intermediate, by non-limiting example, may comprise a compound selected from a derivative, analog, isomer, rearrangement or modification of the compounds of Formula X, Formula XI, and Formula XII:

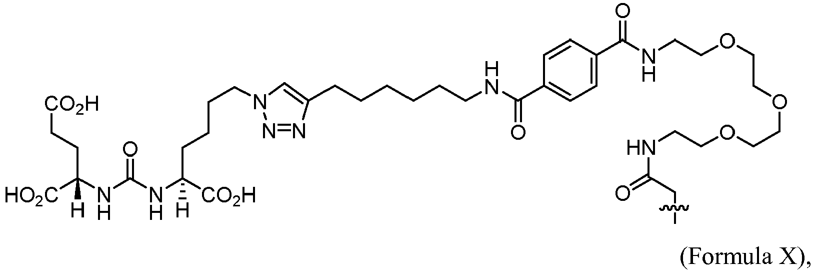

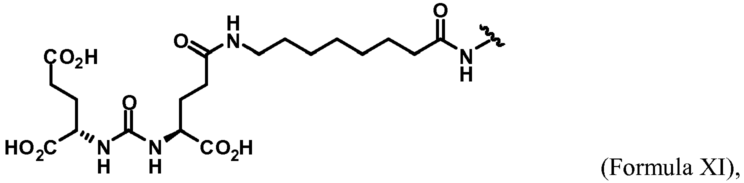

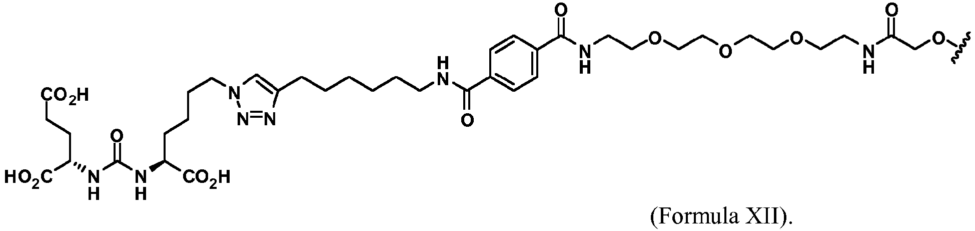

Further disclosed herein are CAR-EC switches of Formula XIII:

A-L-B (Formula XIII)

wherein:
A is a 2-[3-(1,3-dicarboxypropyl)ureido] pentanedioic acid derivative or a 2-(4-((2-amino-4-oxo 3,4-dihydropteridin-6-yl)methylamino)benzamido)pentanedioic acid (folate) derivative;
L is a bond or a linker; and
B is a fluorescein derivative or a biotin derivative.

In some embodiments, A of Formula XIII (A-L-B) is a 2-[3-(1,3-dicarboxypropyl)ureido] pentanedioic acid derivative. The 2-[3-(1,3-dicarboxypropyl)ureido] pentanedioic acid derivative may be of Formula XIV: 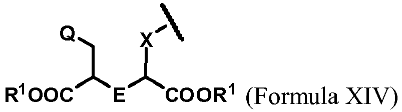 wherein:
Q is selected from the group consisting of: 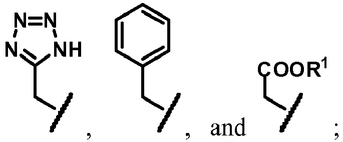 E is selected from the group consisting of: 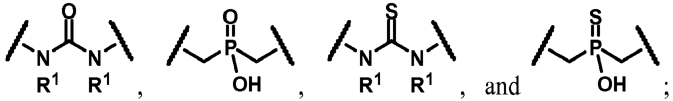
X is C₁-C₅alkylene or C₁-C₄alkylene(C=O)-; and
each R¹ is independently selected from hydrogen, C₁-C₄ alkyl, or C₁-C₄ haloalkyl.

The 2-[3-(1,3-dicarboxypropyl)ureido] pentanedioic acid derivative may be of Formula XIVa:

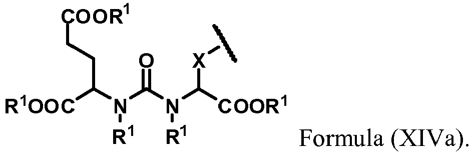

The 2-[3-(1,3-dicarboxypropyl)ureido] pentanedioic acid derivative may be 2-[3-(1,3-dicarboxypropyl)ureido] pentanedioic acid derivative is of Formula IIb:

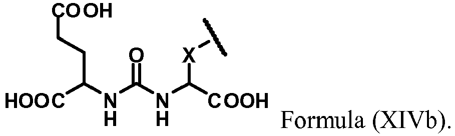

The 2-[3-(1,3-dicarboxypropyl)ureido] pentanedioic acid derivative may be of Formula XIVc:

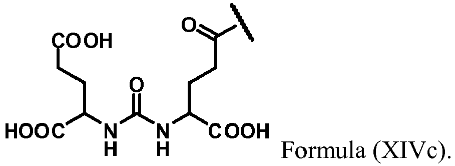

The 2-[3-(1,3-dicarboxypropyl)ureido] pentanedioic acid derivative may be of Formula XIVd:

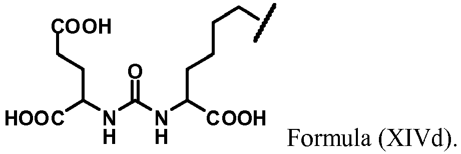

In some embodiments, A of Formula XIII (A-L-B) is a folate derivative. The folate derivative may be selected from the group consisting of: 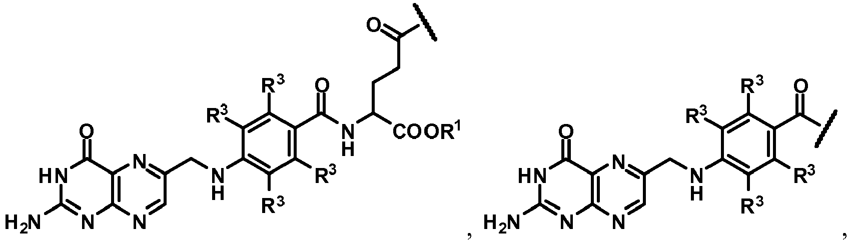 and 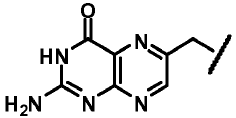 wherein:
R¹ is hydrogen, C₁-C₄ alkyl, or C₁-C₄ haloalkyl; and
each R³ is independently selected from hydrogen, halogen, C₁-C₄ alkyl and C₁-C₄ alkoxy.

The folate derivative may be:

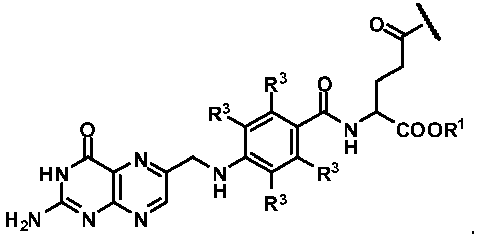

The folate derivative may be:

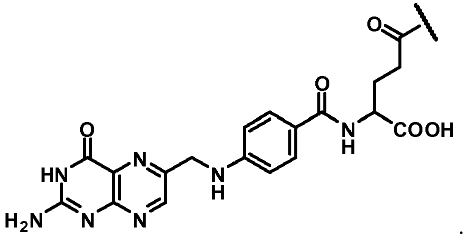

In some embodiments, B of Formula XIII (A-L-B) is a fluorescein derivative. The fluorescein derivate may be selected from Formula XV and Formula XVI: 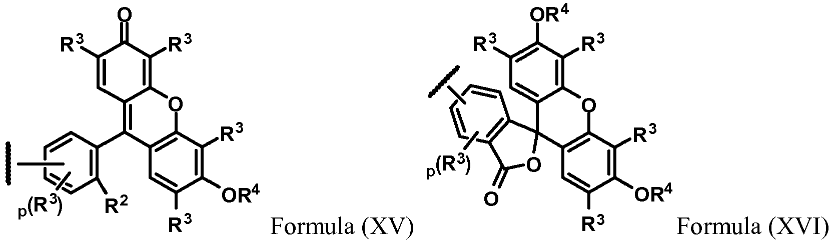 wherein:
R² is be -C(=O)O(R¹) or -C(=O)N(R^{a})₂;
each R^{a} is be independently selected from hydrogen and C₁-C₄ alkyl; or
two R^{a} taken together form an optionally substituted heterocyclyl ring;
each R³ is independently selected from hydrogen, halogen, C₁-C₄ alkyl and C₁-C₄ alkoxy;
p is 0-3;
R⁴ is hydrogen, C₁-C₄ alkyl, -C(=O)C₁-C₄ alkyl or -CH₂-C(=O)OR¹; and
R¹ is hydrogen, C₁-C₄ alkyl, or C₁-C₄ haloalkyl.

The fluorescein derivative of Formula XV may be of Formula XVa:

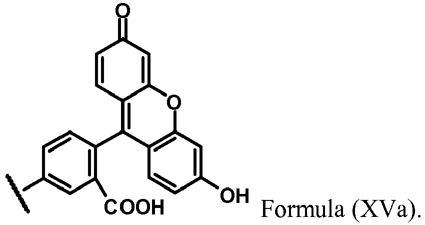

The fluorescein derivative of Formula XVI may be of Formula XVIa:

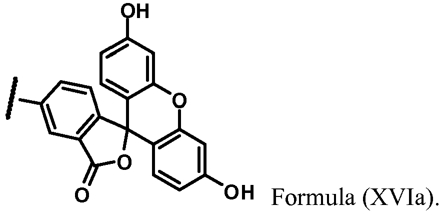

B of Formula XIII (A-L-B) may be a biotin derivative. The biotin derivative may be selected from the group consisting of:

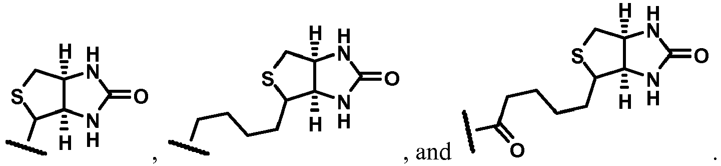

L of Formula XIII (A-L-B) may be a linker. The linker may comprise a triazole. The triazole may be a 1,2,3-triazole. The triazole may be a 1,2,4-triazole. The linker may comprise an aryl or a heteroaryl. The linker may comprise an aryl. The aryl may be phenyl. The phenyl may be disubstituted. The disubstituted phenyl may be 1,4-disubstituted phenyl. The disubstituted phenyl may be 1,3-disubstituted phenyl. The phenyl may be trisubstituted. The phenyl may be tetrasubstituted. Two of the substituents of the substituted phenyl may be NO₂. In some instances, the linker does not comprise a benzyl substituent. The linker may comprise one or more polyethylene glycol (PEG) units. The linker may comprise multiple polyethylene glycol (PEG) units. The linker may comprise 2 or more polyethylene glycol (PEG) units. The linker may comprise 3 or more polyethylene glycol (PEG) units. The linker may comprise 4 or more polyethylene glycol (PEG) units. The linker may comprise 5 or more polyethylene glycol (PEG) units. The linker may comprise 6 or more polyethylene glycol (PEG) units. The linker may comprise 7 or more polyethylene glycol (PEG) units. The linker may comprise 8 or more polyethylene glycol (PEG) units. The linker may comprise 9 or more polyethylene glycol (PEG) units. The linker may comprise 10 or more polyethylene glycol (PEG) units. The linker may comprise 11 or more polyethylene glycol (PEG) units. The linker may comprise 12 or more polyethylene glycol (PEG) units. The linker may comprise 13 or more polyethylene glycol (PEG) units. The linker may comprise 14 or more polyethylene glycol (PEG) units. The linker may comprise an amide on one end. The linker may comprise an amide on one end and an amine on the other end. The linker may comprise an amide on one end and a triazole on the other end.

The linker may be selected from the group consisting of: 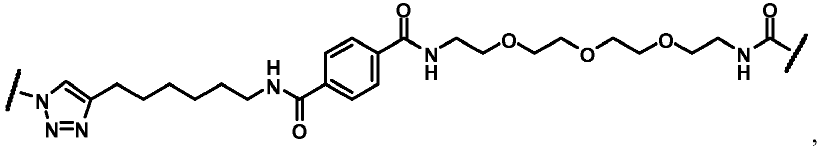 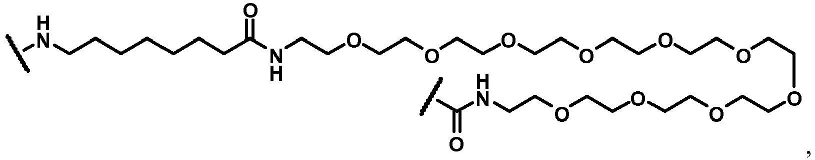 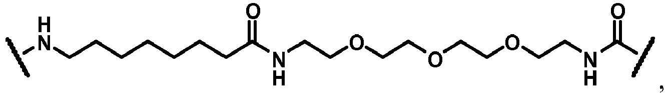 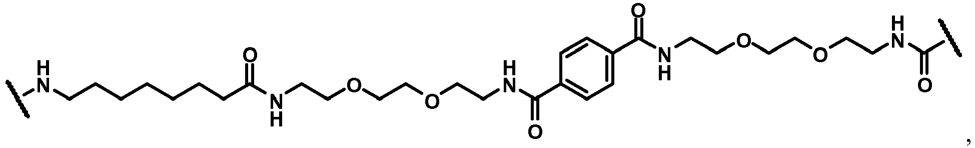 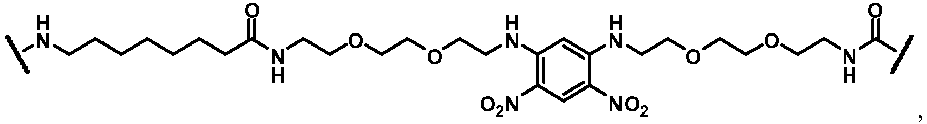 and

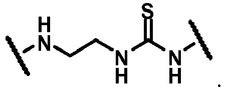

Formula XIII (A-L-B) may be of Formula XIIIa: 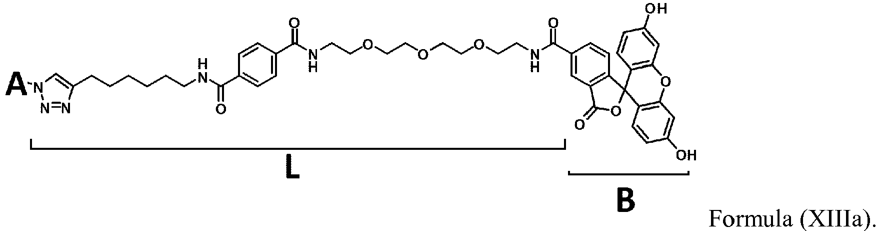 A of Formula XIIIa may be

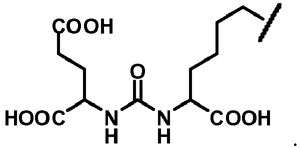

Formula XIII may be of Formula XIIIb: 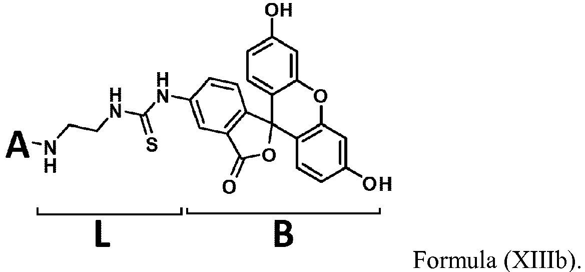 A may be

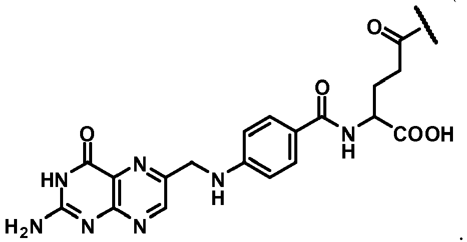

In some embodiments of CAR-EC switches of Formula XIII, the CAR-EC switch is:

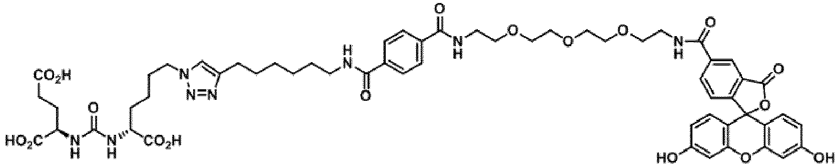

In other embodiments of CAR-EC switches of Formula XIII, the CAR-EC switch is:

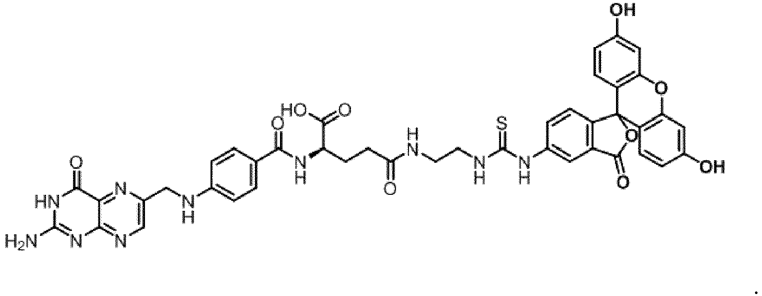

### Chimeric antigen receptor-interacting domains (CAR-IDs)

The switches disclosed herein may comprise one or more chimeric antigen receptor-interacting domains (CAR-IDs). The switches disclosed herein may comprise two or more chimeric antigen receptor-interacting domains (CAR-IDs). The switches disclosed herein may comprise three or more chimeric antigen receptor-interacting domains (CAR-IDs). The switches disclosed herein may comprise four or more chimeric antigen receptor-interacting domains (CAR-IDs). The switches disclosed herein may comprise 5, 6, 7, 8, 9, 10 or more chimeric antigen receptor-interacting domains (CAR-IDs). The two or more CAR-IDs may be the same. At least two of the three or more CAR-IDs may be the same. The two or more CAR-IDs may be different. At least two of the three or more CAR-IDs may be different. The number of CAR-IDs may be optimized for safety and efficacy. For example, one or two CAR-IDs per TID may yield efficient CAR-T activation while three or four CAR-IDs per TID may result in nonspecific activation of the CAR-T may result in nonspecific activation of the CAR-T.

The switch intermediates disclosed herein may comprise one or more chimeric antigen receptor-interacting domains (CAR-IDs). The switch intermediates disclosed herein may comprise two or more chimeric antigen receptor-interacting domains (CAR-IDs). The switch intermediates disclosed herein may comprise three or more chimeric antigen receptor-interacting domains (CAR-IDs). The switch intermediates disclosed herein may comprise four or more chimeric antigen receptor-interacting domains (CAR-IDs). The switch intermediates disclosed herein may comprise 5, 6, 7, 8, 9, 10 or more chimeric antigen receptor-interacting domains (CAR-IDs). The two or more CAR-IDs may be the same. At least two of the three or more CAR-IDs may be the same. The two or more CAR-IDs may be different. At least two of the three or more CAR-IDs may be different.

The CAR-ID may be a naturally-occurring molecule. For example, the CAR-ID may be based or derived from an endogenous protein. Alternatively, the CAR-ID may comprise a wild-type version of a protein. The CAR-ID may be an artificial or synthetic molecule. For example, the CAR-ID may comprise a protein comprising one or more unnatural amino acids. Alternatively, the CAR-ID may comprise a modified version of a wild-type protein that is not naturally occurring. The CAR-ID may be able to cross a cell membrane when not attached to a target interacting domain (TID) of the CAR-EC switch. In some instances, at least a portion of a CAR-ID is not be genetically encoded. At least a portion of a CAR-ID may be synthetic. The CAR-ID may comprise a polypeptide that is not naturally occurring. The CAR-ID may be an organic molecule. The CAR-BC may be inorganic molecule.

The CAR-ID may be a small molecule. The small molecule may be an organic compound. The small molecule may have a size on the order of about 10⁻⁸ m, about 10⁻⁹ m, about 10⁻¹⁰ m. The small molecule may have a size of less than about 10⁻⁷ m. The small molecule may have a size of less than about 10⁻⁸ m. The small molecule may have a size of less than about 10⁻⁹ m. The small molecule may have a size of less than about 10⁻¹⁰ m. The small molecule may have a size of less than about 10⁻¹¹ m.

The small molecule may have a mass of less than about 5000 kDa, less than about 4500 kDa, less than about 4000 kDa, less than about 3500 kDa, less than about 3000 kDa, less than about 2500 kDa, less than about 2000 kDa, less than about 1500 kDa, less than about 1000 kDa, less than about 900 kD, less than about 500 kDa or less than about 100 kDa.

In some instances, the small molecule does not comprise a polypeptide. In some instances, the small molecule does comprise two or more amino acids that are linked by an amide bond. The small molecule may be a chemical compound.

The CAR-ID may comprise a hapten. The CAR-ID may induce an immune response when attached to a larger carrier molecule, such as a protein, antibody or antibody fragment. The CAR-ID may be FITC or a derivative thereof. The CAR-ID may be selected from DOTA, dinitrophenol, quinone, biotin, aniline, atrazine, an aniline-derivative, o-aminobenzoic acid, p-aminobenzoic acid, m-aminobenzoic acid, hydralazine, halothane, digoxigenin, benzene arsonate, lactose, trinitrophenol, biotin or a derivative thereof. The CAR-ID may be a penicillin drug or a derivative thereof. The CAR-ID may be a quinone or a derivative thereof. The CAR-ID may be DOTA or a derivative thereof. The CAR-ID may be dinitrophenol or a derivative thereof. The CAR-ID may be biotin or a derivative thereof.

Alternatively, the CAR-ID does not comprise a hapten. The CAR-ID may be selected from a steroid, a vitamin, a vitamer, a metabolite, an antibiotic, a monosaccharide, a disaccharide, a lipid, a fatty acid, a nucleic acid, an alkaloid, a glycoside, a phenzine, a polyketide, a terpene and a tetrapyrrole and a portion thereof.

### Target interacting domains (TIDs)

The switches disclosed herein may comprise one or more target interacting domains (TIDs). The switches disclosed herein may comprise two or more target interacting domains (TIDs). The switches disclosed herein may comprise three or more target interacting domains (TIDs). The switches disclosed herein may comprise four or more target interacting domains (TIDs). The switches disclosed herein may comprise 5, 6, 7, 8, 9, 10 or more target interacting domains (TIDs). The two or more TIDs may be the same. At least two of the three or more TIDs may be the same. The two or more TIDs may be different. At least two of the three or more TIDs may be different.

The switch intermediates disclosed herein may comprise one or more target interacting domains (TIDs). The switch intermediates disclosed herein may comprise two or more target interacting domains (TIDs). The switch intermediates disclosed herein may comprise three or more target interacting domains (TIDs). The switch intermediates disclosed herein may comprise four or more target interacting domains (TIDs). The switch intermediates disclosed herein may comprise 5, 6, 7, 8, 9, 10 or more target interacting domains (TIDs). The two or more TIDs may be the same. At least two of the three or more TIDs may be the same. The two or more TIDs may be different. At least two of the three or more TIDs may be different.

Generally, the TID interacts with a molecule on or from a target. The TID, by non-limiting example, may be selected from a small molecule, a ligand, a receptor agonist, a receptor antagonist, an enzyme inhibitor, a DNA aptamer, a PNA aptamer, a vitamin, a substrate and a substrate analog. In some instances, the TID does not comprise an antibody or an antibody fragment.

The TID may comprise a vitamin or a derivative thereof. The vitamin, by non-limiting example may be selected from Vitamin A, Vitamin B, Vitamin C, Vitamin D, Vitamin E and Vitamin K. The vitamin may be Vitamin C. The vitamin may be Vitamin D. The vitamin may comprise folate or a derivative thereof. The vitamin may be folate or a derivative thereof. The TID may be folate. The TID may comprise a vitamer. The TID may comprise a vitamin metabolite or vitamin precursor. The vitamin, by non-limiting example, may be selected from retinol, retinal, beta carotene, a carotenoid, thiamine, riboflavin, niacin, niacinamide, pantothenic acid, pyridoxine, pyridoxamine, pyridozl, biotin, folic acid, folinic acid, cyanocobalamin, hydroxycobalamin, methylcobalamin, ascorbic acid, cholecalciferol, ergocalciferol, a tocopherol, a tocotrienol, a phylloquinone, and a menaquinone or a derivative thereof. The TID may comprise an antioxidant or a derivative thereof.

The TID may be an enzyme inhibitor. The TID may be selected, by non-limiting example, from a tyrosine kinase inhibitor, a protease inhibitor, a growth factor receptor inhibitor, a hormone receptor inhibitor, a janus kinase inhibitor, an anaplastic lymphoma kinase (ALK) inhibitor, a Bcl-2 inhibitor, a poly ADP ribose polymerase (PARP) inhibitor, a PI3K inhibitor, a Braf inhibitor, a MAP kinase inhibitor, a cyclin dependent kinase inhibitor and a heat shock protein inhibitor. The enzyme inhibitor may be selected from apatinib, bortezomib, imatinib, ibrutinib, seliciclib, bosutinib, cabozantinib, crizotinib, dabrafenib, dasatinib, doxorubicin, erlotinib, everolimus, gefitinib, imatinib, iniparib, lapatinib, LEE011, LGX818, milotinib, obatoclax, olaparib, pazopanib, PD-0332991, perifosine, ponatinib, regorafenib, ruxolitinib, salinomycin, sorafebnib, sunitinib, tamoxifen, temsirolimus, tofacitinib, trametinib, vandetanib and vemurafenib or a derivative thereof.

The TID may comprise 2-[3-(1,3-dicarboxypropy)ureidol] pentanedioic acid or a derivative thereof. The TID may be sufficiently small to penetrate a tumor.

The TID may comprise a polypeptide. The TID may be based on or derived from a polypeptide. The TID may comprise a synthetic polypeptide. A synthetic polypeptide may refer to a polypeptide that is not genetically encoded. The TID may comprise a naturally occurring polypeptide. A naturally occurring polypeptide may refer to a polypeptide that is genetically encoded. The polypeptide may comprise about 5, about 10, about 15, about 20, about 25, about 30, about 35, about 40, about 45, about 50, about 55, about 60, about 65, about 70, about 75, about 80, about 85 or about 90 or more amino acids.

The TID may be selected from a 10-mer peptide hormone leutenizing hormone releasing hormone, bombesin, gonadotropin-releasing hormone, pentagastrin, cRGD, octreotide, octreotate and analogs thereof. The TID may bind a target at least 50% homologous to gonadotropin releasing hormone receptor, a prostate specific membrane antigen, a c-type lectin-like molecule, a folate receptor, a cholecystokinin B receptor, a somatostatin receptor, an αvβ3 integrin, a gastrin-releasing peptide receptor, a neurokinin receptor, a melanocortin receptor, a neurotenin receptor, a leutenizing hormone releasing hormone receptor and a neuropeptide receptor.

The TID may comprise an amino acid sequence that is based on or derived from an amino acid sequence selected from any one of SEQ ID NOs: 18-56. The TID may comprise an amino acid sequence that is at least 60% identical to an amino acid sequence selected from any one of SEQ ID NOs: 18-56. The TID may comprise an amino acid sequence that is at least 70% identical to an amino acid sequence selected from any one of SEQ ID NOs: 18-56. The TID may comprise an amino acid sequence that is at least 75% identical to an amino acid sequence selected from any one of SEQ ID NOs: 18-56. The TID may comprise an amino acid sequence that is at least 80% identical to an amino acid sequence selected from any one of SEQ ID NOs: 18-56. The TID may comprise an amino acid sequence that is at least 85% identical to an amino acid sequence selected from any one of SEQ ID NOs: 18-56. The TID may comprise an amino acid sequence that is at least 90% identical to an amino acid sequence selected from any one of SEQ ID NOs: 18-56. The TID may comprise an amino acid sequence that is at least 95% identical to an amino acid sequence selected from any one of SEQ ID NOs: 18-56.

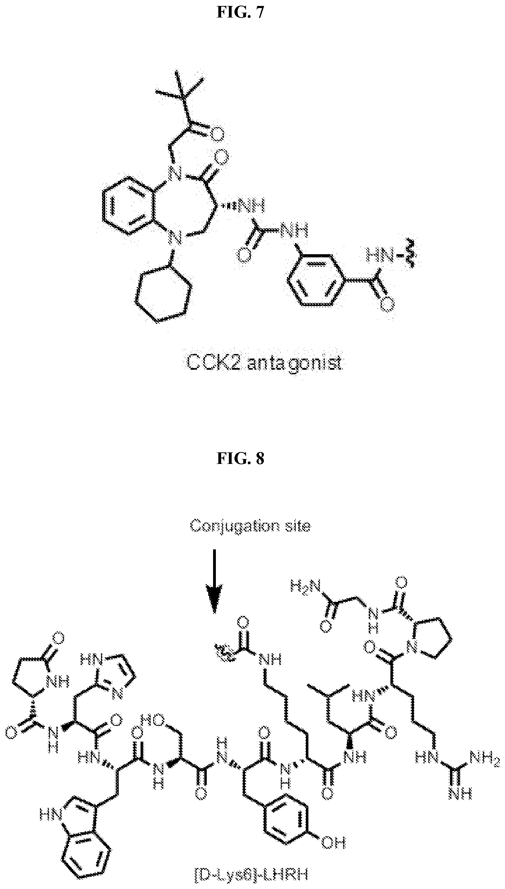

The TID may comprise a cholecystokinin B receptor agonist. The TID may comprise a cholecystokinin B receptor antagonist (e.g. CCK2 antagonist). By non-limiting example, the TID may comprise be a cholecystokinin B receptor antagonist as depicted in **FIG. 7**.

The TID may comprise a polypeptide that is based on or derived from an antibody or antibody fragment. As used herein, the term "antibody fragment" refers to any form of an antibody other than the full-length form. Antibody fragments herein include antibodies that are smaller components that exist within full-length antibodies, and antibodies that have been engineered. Antibody fragments include, but are not limited to, Fv, Fc, Fab, and (Fab')2, single chain Fv (scFv), diabodies, triabodies, tetrabodies, bifunctional hybrid antibodies, CDR1, CDR2, CDR3, combinations of CDRs, variable regions, framework regions, constant regions, heavy chains, light chains, alternative scaffold non-antibody molecules, and bispecific antibodies. Unless specifically noted otherwise, statements and claims that use the term "antibody" or "antibodies" may specifically include "antibody fragment" and "antibody fragments."

The TID may comprise an antibody or antibody fragment may be selected from Fv, Fc, Fab, and (Fab')2, single chain Fv (scFv), diabodies, triabodies, tetrabodies, bifunctional hybrid antibodies, monoclonal solution TCRs, complementary determining region 1 (CDR1), CDR2, CDR3, combinations of CDRs, variable regions, framework regions, constant regions, heavy chains, light chains, alternative scaffold non-antibody molecules, and bispecific antibodies. The polypeptide may be an immunoglobulin. The polypeptide may be a Fab. The TID may be a monoclonal TCR.

The TID may be human, fully human, humanized, human engineered, non-human, and/or chimeric antibody. The non-human antibody may be humanized to reduce immunogenicity to humans, while retaining the specificity and affinity of the parental non-human antibody. Generally, a humanized antibody comprises one or more variable domains in which CDRs (or portions thereof) are derived from a non-human antibody, and FRs (or portions thereof) are derived from human antibody sequences. A humanized antibody optionally also comprises at least a portion of a human constant region. In some embodiments, some FR residues in a humanized antibody are substituted with corresponding residues from a non-human antibody (*e*.*g*., the antibody from which the CDR residues are derived), *e*.*g*., to restore or improve antibody specificity or affinity.

The TID may be based on or derived from a chimeric antibody. Chimeric antibodies may refer to antibodies created through the joining of two or more antibody genes which originally encoded for separate antibodies. A chimeric antibody may comprise at least one amino acid from a first antibody and at least one amino acid from a second antibody, wherein the first and second antibodies are different. At least a portion of the antibody or antibody fragment may be from a bovine species, a human species, or a murine species. At least a portion of the antibody or antibody fragment may be from a rat, a goat, a guinea pig or a rabbit. At least a portion of the antibody or antibody fragment may be from a human. At least a portion of the antibody or antibody fragment antibody may be from cynomolgus monkey.

The TID may be based on or derived from an antibody or antibody fragment from a mammal, bird, fish, amphibian, and reptile. Mammals include, but are not limited to, carnivores, rodents, elephants, marsupials, rabbits, bats, primates, seals, anteaters, cetaceans, odd-toed ungulates and even-toed ungulates. The mammal may be a human, non-human primate, mouse, sheep, cat, dog, cow, horse, goat, or pig.

The TID may be based on or derived from an antibody or antibody fragment selected from the group consisting of anti-CD19 antibody, anti-Her2 antibody, anti-EGFR antibody, anti-CD22 antibody, anti-CS1 antibody, anti-CLL-1 antibody, anti-CD33 antibody and anti-CEA antibody. The TID may be based on or derived from an anti-CD19 antibody or a fragment thereof. The TID may be based on or derived from an anti-Her2 antibody or fragment thereof. The TID may be based on or derived from an anti-EGFR antibody or fragment thereof. The TID may be based on or derived from an anti-EGFRvIII antibody or fragment thereof. The TID may be based on or derived from an anti-BCMA antibody or fragment thereof. The TID may be based on or derived from an anti-CD22 antibody or fragment thereof. The TID may be based on or derived from an anti-CS1 antibody or fragment thereof. The TID may be based on or derived from an anti-CLL-1 antibody or fragment thereof. The TID may be based on or derived from an anti-CD33 antibody or fragment thereof. The TID may be based on or derived from an anti-CEA antibody or fragment thereof.

The TID may be based on or derived from an immunoglobulin (Ig). The immunoglobulin may be selected from an IgG, an IgA, an IgD, an IgE, an IgM, a fragment thereof or a modified version thereof. The immunoglobulin may be IgG. The IgG may be IgG1. The IgG may be IgG2. The IgG may have one or more Fc mutations for reduced Fc receptor (FcR) binding. The Fc mutations may be in the heavy chain of the IgG. The Fc mutations in the IgG1 may be L234A and L235A. The Fc mutations in the IgG1 may be L234A and L235E. The Fc mutation in the IgG1 may be N297A. The Fc mutation in the IgG2 may be V234A and V237A. The TID may comprise an Fc null immunoglobulin or a fragment thereof.

The TID may comprise an antibody selected from the group consisting of adotrastuzumab emtansine, alemtuzumab, bevacizumab, brentuximab vedotin, gemtuzumab ozogamicin, ipilimumab ibritumomab tiuxetan or panitumumab or a fragment thereof. In some instances, the TID does not comprise cetuximab. The TID may be erbitux or a fragment thereof. The TID may not be erbitux. The TID may be rituximab or a fragment thereof. The TID may not be rituximab. The TID may be trastuzumab or a fragment thereof. The TID may not be trastuzumab.

The TID may be an anti-CD19 antibody or a fragment thereof. The TID may be an anti-CD22 antibody or a fragment thereof. The TID may not be an anti-CD19 antibody or a fragment thereof. The TID may target an antigen selected from, by non-limiting example, Her2, CLL-1, CD33, EGFRvIII, CD20, BCMA, CS1 or a fragment thereof. The antigen may comprise a wildtype antigen. The antigen may comprise one or more mutations.

The TID may comprise an anti-CD19 antibody or fragment thereof. The light chain of the anti-CD19 antibody or fragment thereof may comprise SEQ ID NO: 16 or a homologous amino acid sequence. The amino acid sequence may be about 99%, about 98%, about 97%, about 96%, about 95%, about 92%, about 90%, about 85%, about 80%, about 75%, about 70%, about 65%, about 60%, about 55%, about 50%, about 45%, about 40%, about 35%, about 30%, about 25%, about 20%, about 15%, about 10%, about 5% or about 2% homologous to SEQ ID NO: 16. The heavy chain of the anti-CD19 antibody or fragment thereof may comprise SEQ ID NO: 17 or a homologous amino acid sequence. The amino acid sequence may be about 99%, about 98%, about 97%, about 96%, about 95%, about 92%, about 90%, about 85%, about 80%, about 75%, about 70%, about 65%, about 60%, about 55%, about 50%, about 45%, about 40%, about 35%, about 30%, about 25%, about 20%, about 15%, about 10%, about 5% or about 2% homologous to SEQ ID NO: 17.

The TID may be an anti-BCMA antibody or a fragment thereof. The TID may be an anti-CS1 antibody or a fragment thereof. The TID may be an anti-EGFRvIII antibody or a fragment thereof. The TID may be an anti-Her2 antibody or a fragment thereof. The TID may comprise an anti-CD20 antibody or antibody fragment. The TID may comprise an anti-CEA antibody or antibody fragment. The TID may comprise an anti-EGFR antibody or antibody fragment. The TID may comprise an anti-CEA antibody or antibody fragment. The TID may comprise an anti-CLL-1 antibody or antibody fragment. The TID may comprise an anti-CD33 antibody or antibody fragment. The TID may comprise trastuzumab or fragment thereof. The TID may not comprise cetuximab or fragment thereof. The TID may not comprise trastuzumab or fragment thereof. The TID may not comprise rituximab or fragment thereof. The TID may not comprise an antibody to EpCAM or fragment thereof. The TID may be an antibody or antibody fragment encoded by a nucleotide or amino acids sequence selected from SEQ ID NOs: 5-9, wherein the unnatural amino acid may replace any amino acid of the antibody or antibody fragment.

The TID may be a small molecule. The small molecule may be 2-[3-(1,3-dicarboxypropyl)ureido] pentanedioic acid. The small molecule may be folate. The CAR-ID may be FITC or a derivative thereof. The CAR-EC switch may further comprise a second TID. The CAR-EC switch may comprise more than 1, 2, 3, 4 or 5 TIDs.

The TID may be based on or derived from a polypeptide selected from the group consisting of protein A, a lipocalin, a fibronectin domain, an ankyrin consensus repeat domain, and a thioredoxin.

The CAR-EC switches disclosed herein may comprise one or more unnatural amino acids. The one or more CAR-IDs may comprise one or more unnatural amino acids. The one or more TIDs may comprise one or more unnatural amino acids. The one or more linkers may comprise one or more unnatural amino acids. Attachment of the CAR-ID to the TID may occur via the one or more unnatural amino acids. The one or more linkers may link the one or more CAR-IDs to the one or more TIDs site-specifically through the one or more unnatural amino acids. Alternatively, or additionally, the one or more linkers may link the one or more TIDs to the one or more TIDs site-specifically, wherein an unnatural amino acid is not required to link the one or more TIDs to the one or more TIDs. The TID may be linked to 1, 2, 3, 4, 5 or more unnatural amino acids on the TID. The TID may be linked to 1, 2, 3, 4, 5 or more unnatural amino acids on the TID site-specifically. Alternatively, the TID may be linked to 1, 2, 3, 4, 5 or more unnatural amino acids on the TID. The TID may be linked to 1, 2, 3, 4, 5 or more unnatural amino acids on the TID site-specifically.

The chimeric antigen receptor-interacting domain (CAR-ID) may comprise one or more unnatural amino acids. The CAR-IDs disclosed herein may comprise 1, 2, 3, 4, 5, 6, 7, 8, 9, 10 or more unnatural amino acids. The TID may comprise one or more unnatural amino acids. The targeting antibodies or antibody fragments disclosed herein may comprise 2, 3, 4, 5, 6, 7, 8, 9, 10 or more unnatural amino acids. The unnatural amino acid may react with the linker to create a chemical bond.

The one or more unnatural amino acids may be inserted between two naturally occurring amino acids in the TID. The one or more unnatural amino acids may replace one or more naturally occurring amino acids in the TID. The one or more unnatural amino acids may be incorporated at the N terminus of the TID. The one or more unnatural amino acids may be incorporated at the C terminus of the TID. The unnatural amino acid may be incorporated distal to the region of the TID that interacts with a molecule on or from a target. The unnatural amino acid may be incorporated near the region of the TID that interacts with a molecule on or from a target. The unnatural amino acid may be incorporated in the region of the TID that interacts with a molecule on or from a target.

The one or more unnatural amino acids may replace one or more amino acids in the TID. The one or more unnatural amino acids may replace any natural amino acid in the TID.

The one or more unnatural amino acids may be incorporated in a light chain of the immunoglobulin from which the TID is based or derived. The one or more unnatural amino acids may be incorporated in a heavy chain of the immunoglobulin from which the TID is based or derived. The one or more unnatural amino acids may be incorporated in a heavy chain and a light chain of the immunoglobulin from which the TID is based or derived. The one or more unnatural amino acids may replace an amino acid in the light chain of the immunoglobulin from which the TID is based or derived. The one or more unnatural amino acids may replace an amino acid in a heavy chain of the immunoglobulin from which the TID is based or derived. The one or more unnatural amino acids may replace an amino acid in a heavy chain and a light chain of the immunoglobulin from which the TID is based or derived.

The one or more unnatural amino acids may replace an alanine of a light chain of the immunoglobulin from which the TID is based or derived. The one or more unnatural amino acids may replace a cysteine of a light chain of the immunoglobulin from which the TID is based or derived. The one or more unnatural amino acids may replace a serine of a light chain of the immunoglobulin from which the TID is based or derived. The one or more unnatural amino acids may replace a lysine of a light chain of the immunoglobulin from which the TID is based or derived. The one or more unnatural amino acids may replace an asparagine of a light chain of the immunoglobulin from which the TID is based or derived. The one or more unnatural amino acids may replace a threonine of a light chain of the immunoglobulin from which the TID is based or derived. The one or more unnatural amino acids may replace an alanine of a heavy chain of the immunoglobulin from which the TID is based or derived. The one or more unnatural amino acids may replace a cysteine of a heavy chain of the immunoglobulin from which the TID is based or derived. The one or more unnatural amino acids may replace a serine of a heavy chain of the immunoglobulin from which the TID is based or derived. The one or more unnatural amino acids may replace a lysine of a heavy chain of the immunoglobulin from which the TID is based or derived. The one or more unnatural amino acids may replace an asparagine of a heavy chain of the immunoglobulin from which the TID is based or derived. The one or more unnatural amino acids may replace a threonine of a heavy chain of the immunoglobulin from which the TID is based or derived.

The one or more unnatural amino acids may replace Ser 202 of the light chain of the immunoglobulin from which the TID is based or derived. The one or more unnatural amino acids may replace Lys 136 of the heavy chain of the immunoglobulin from which the TID is based or derived. The one or more unnatural amino acids may replace Ala 123 of the heavy chain of the immunoglobulin from which the TID is based or derived. The one or more unnatural amino acids may replace Ser 202 of the light chain and the Lys 136 of the heavy chain of the immunoglobulin from which the TID is based or derived. The one or more unnatural amino acids may replace Thr 109 of the light chain of the immunoglobulin from which the TID is based or derived. The unnatural amino acid may replace an amino acid of the light chain of the antibody from which the polypeptide is based on or derived. For example, the unnatural amino acid may replace a serine residue of the antibody light chain. The unnatural amino acid may replace serine 202 of the antibody light chain or a homologue thereof. The unnatural amino acid may replace a glycine residue of the antibody light chain. The unnatural amino acid may replace glycine 68 of the antibody light chain or a homologue thereof. The unnatural amino acid may replace a threonine residue of the antibody light chain. The unnatural amino acid may replace threonine 109 of the antibody light chain or a homologue thereof. The polypeptide may be based on or derived from a heavy chain of the antibody. The unnatural amino acid may replace a lysine residue of the antibody heavy chain. The unnatural amino acid may replace lysine 136 of the antibody heavy chain or a homologue thereof. The unnatural amino acid may replace an alanine residue of the antibody heavy chain. The unnatural amino acid may replace alanine 123 of the antibody heavy chain or a homologue thereof. The unnatural amino acid may replace serine residue of the antibody heavy chain. The unnatural amino acid may replace serine 74 of the antibody heavy chain or a homologue thereof. The unnatural amino acid may replace an amino acid residue of the antibody light chain and an amino acid residue of the antibody heavy chain. The unnatural amino acid may replace a glycine residue of the antibody light chain and an amino acid of the antibody heavy chain. The unnatural amino acid may replace a glycine residue of the antibody light chain and a serine residue of the antibody heavy chain. The unnatural amino acid may replace a serine residue of the antibody heavy chain and an amino acid residue of the antibody light chain. The glycine residue of the antibody light chain may be glycine 68 or a homologue thereof. The serine residue of the antibody heavy chain may be serine 74 or a homologue thereof. The unnatural amino acid may replace a serine residue of the antibody light chain and an amino acid of the antibody heavy chain. The unnatural amino acid may replace a serine residue of the antibody light chain and a lysine residue of the antibody heavy chain. The unnatural amino acid may replace a lysine residue of the antibody heavy chain and an amino acid residue of the antibody light chain. The serine residue of the antibody light chain may be serine 202 or a homologue thereof. The serine residue of the antibody heavy chain may be lysine 136 or a homologue thereof.

The one or more unnatural amino acids may replace an amino acid of the TID, wherein the TID is an anti-CD 19 antibody or fragment thereof. The one or more unnatural amino acids may replace any serine of a light chain of the anti-CD 19 antibody or fragment thereof. The one or more unnatural amino acids may replace any lysine of a light chain of the anti-CD 19 antibody or fragment thereof. The one or more unnatural amino acids may replace any asparagine of a light chain of the anti-CD 19 antibody or fragment thereof. The one or more unnatural amino acids may replace any threonine of a light chain of the anti-CD 19 antibody or fragment thereof. The one or more unnatural amino acids may replace any serine of a heavy chain of the anti-CD19 antibody or fragment thereof. The one or more unnatural amino acids may replace any lysine of a heavy chain of the anti-CD 19 antibody or fragment thereof. The one or more unnatural amino acids may replace any asparagine of a heavy chain of the anti-CD 19 antibody or fragment thereof. The one or more unnatural amino acids may replace any threonine of a heavy chain of the anti-CD 19 antibody or fragment thereof. The antibody or antibody fragment may be an anti-CD 19 antibody or fragment thereof, wherein the one or more unnatural amino acids may replace one or more amino acids of a light chain of the anti-CD 19 antibody or fragment thereof. The light chain of the anti-CD 19 antibody or fragment thereof may comprise SEQ ID NO: 16. The one or more unnatural amino acids may replace one or more amino acids of SEQ ID NO: 16. The one or more amino acids of SEQ ID NO: 16 may be selected from the group comprising G68, K107, T109, E152, S156, K169 and S202. The one or more unnatural amino acids may replace one or more amino acids of a heavy chain of the anti-CD 19 antibody or fragment thereof. The heavy chain of the anti-CD 19 antibody or fragment thereof may comprise SEQ ID NO: 17. The one or more unnatural amino acids may replace one or more amino acids of SEQ ID NO: 17. The one or more amino acids of SEQ ID NO: 17 may be selected from the group consisting of S74, A121, and K136.

The one or more unnatural amino acids may be encoded by a codon that does not code for one of the twenty natural amino acids. The one or more unnatural amino acids may be encoded by a nonsense codon (stop codon). The stop codon may be an amber codon. The amber codon may comprise a UAG sequence. The stop codon may be an ochre codon. The ochre codon may comprise a UAA sequence. The stop codon may be an opal or umber codon. The opal or umber codon may comprise a UGA sequence. The one or more unnatural amino acids may be encoded by a four-base codon.

The one or more unnatural amino acids may be *p*-acetylphenylalanine (pAcF or pAcPhe). The one or more unnatural amino acids may be selenocysteine. The one or more unnatural amino acids may be *p*-fluorophenylalanine (pFPhe). The one or more unnatural amino acids may be selected from the group comprising *p*-azidophenylalanine (pAzF),p-azidomethylphenylalanine(pAzCH₂F), *p*-benzoylphenylalanine (pBpF), *p-*propargyloxyphenylalanine (pPrF), *p*-iodophenylalanine (pIF), *p*-cyanophenylalanine (pCNF), *p*-carboxylmethylphenylalanine (pCmF), 3-(2-naphthyl)alanine (NapA), *p*-boronophenylalanine (pBoF), o-nitrophenylalanine (oNiF), (8-hydroxyquinolin-3-yl)alanine (HQA), selenocysteine, and (2,2'-bipyridin-5-yl)alanine (BipyA).). The one or more unnatural amino acids may be 4-(6-methyl-*s*-tetrazin-3-yl)aminopheynlalanine.

The one or more unnatural amino acids may be β-amino acids (β3 and β2), homo-amino acids, proline and pyruvic acid derivatives, 3-substituted alanine derivatives, glycine derivatives, ring-substituted phenylalanine and tyrosine derivatives, linear core amino acids, diamino acids, D-amino acids, N-methyl amino acids, or a combination thereof.

Additional examples of unnatural amino acids include, but are not limited to, 1) various substituted tyrosine and phenylalanine analogues such as *O*-methyl-L-tyrosine, *p*-amino-L-phenylalanine, 3-nitro-L-tyrosine, *p*-nitro-L-phenylalanine, *m*-methoxy-L-phenylalanine and *p-*isopropyl-L-phenylalanine; 2) amino acids with aryl azide and benzophenone groups that may be photo-cross-linked; 3) amino acids that have unique chemical reactivity including acetyl-L-phenylalanine and *m*-acetyl-L-phenylalanine, *O*-allyl-L-tyrosine, *O*-(2-propynyl)- L-tyrosine, *p-*ethylthiocarbonyl-L-phenylalanine and *p*-(3-oxobutanoyl)-L-phenylalanine; 4) heavy-atom-containing amino acids for phasing in X-ray crystallography including *p*-iodo and *p*-bromo-L-phenylalanine; 5) the redox-active amino acid dihydroxy-L-phenylalanine; 6) glycosylated amino acids including b-N-acetylglucosamine-*O*-serine and a-N-acetylgalactosamine-*O-*threonine; 7) fluorescent amino acids with naphthyl, dansyl, and 7-aminocoumarin side chains; 8) photocleavable and photoisomerizable amino acids with azobenzene and nitrobenzyl Cys, Ser, and Tyr side chains; 9) the phosphotyrosine mimetic *p*-carboxymethyl-L-phenylalanine; 10) the glutamine homologue homoglutamine; and 11) 2-aminooctanoic acid. The unnatural amino acid may be modified to incorporate a chemical group. The unnatural amino acid may be modified to incorporate a ketone group.

The one or more unnatural amino acids may comprise at least one oxime, carbonyl, dicarbonyl, hydroxylamine group or a combination thereof. The one or more unnatural amino acids may comprise at least one carbonyl, dicarbonyl, alkoxy-amine, hydrazine, acyclic alkene, acyclic alkyne, cyclooctyne, aryl/alkyl azide, norbornene, cyclopropene, trans-cyclooctene, or tetrazine functional group or a combination thereof.

The one or more unnatural amino acids may be incorporated into the TID and/or the CAR-ID by methods known in the art. Cell-based or cell-free systems may be used to alter the genetic sequence of the TID and/or the CAR-ID, thereby producing the TID and/or the CAR-ID with one or more unnatural amino acids. Auxotrophic strains may be used in place of engineered tRNA and synthetase. The one or more unnatural amino acids may be produced through selective reaction of one or more natural amino acids. The selective reaction may be mediated by one or more enzymes. In one non-limiting example, the selective reaction of one or more cysteines with formylglycine generating enzyme (FGE) may produce one or more formylglycines (see Rabuka et al., Nature Protocols 7:1052-1067 (2012)).

The one or more unnatural amino acids may take part in a chemical reaction to form a linker. The chemical reaction to form the linker may be a bioorthogonal reaction. The chemical reaction to form the linker may be click chemistry.

Additional unnatural amino acids are disclosed in Liu et al. (Annu Rev Biochem, 79:413-44, 2010), Wang et al. (Angew Chem Int Ed, 44:34-66, 2005) and PCT application numbers PCT/US2012/039472, PCT/US2012/039468, PCT/US2007/088009, PCT/US2009/058668, PCT/US2007/089142, PCT/US2007/088011, PCT/US2007/001485, PCT/US2006/049397, PCT/US2006/047822 and PCT/US2006/044682.

One skilled in the art will envision that CAR-EC platform can be expanded to a variety of molecules on a cell to be targeted in a disease or condition. By way of non-limiting example, CAR-EC switches disclosed herein may comprise TIDs based on or derived from antibodies targeting EGFR for colon and lung cancer, EGFRvIII for glioma and glioblastoma, BCMA, and CS1 for multiple myeloma, and CLL-1 and CD33 for acute myeloid leukemia. CAR-EC switches disclosed herein may comprise TIDs comprising small molecules used to target cancer cells. CAR-EC switches comprising a TID comprising folate may be used to treat ovarian cancer. CAR-EC switches comprising a TID comprising 2-[3-(1,3-dicarboxypropyl)ureido] pentanedioic acid may be used to treat PSMA positive prostate cancer cells.

### Linkers

The switches disclosed herein may comprise one or more linkers. The switches disclosed herein may comprise two or more linkers. The switches disclosed herein may comprise three or more linkers. The switches disclosed herein may comprise four or more linkers. The switches disclosed herein may comprise 5, 6, 7, 8, 9, 10 or more linkers. The two or more linkers may be the same. At least two of the three or more linkers may be the same. The two or more linkers may be different. At least two of the three or more linkers may be different.

The switch intermediates disclosed herein may comprise one or more linkers. The switch intermediates disclosed herein may comprise two or more linkers. The switch intermediates disclosed herein may comprise three or more linkers. The switch intermediates disclosed herein may comprise four or more linkers. The switch intermediates disclosed herein may comprise 5, 6, 7, 8, 9, 10 or more linkers. The two or more linkers may be the same. At least two of the three or more linkers may be the same. The two or more linkers may be different. At least two of the three or more linkers may be different.

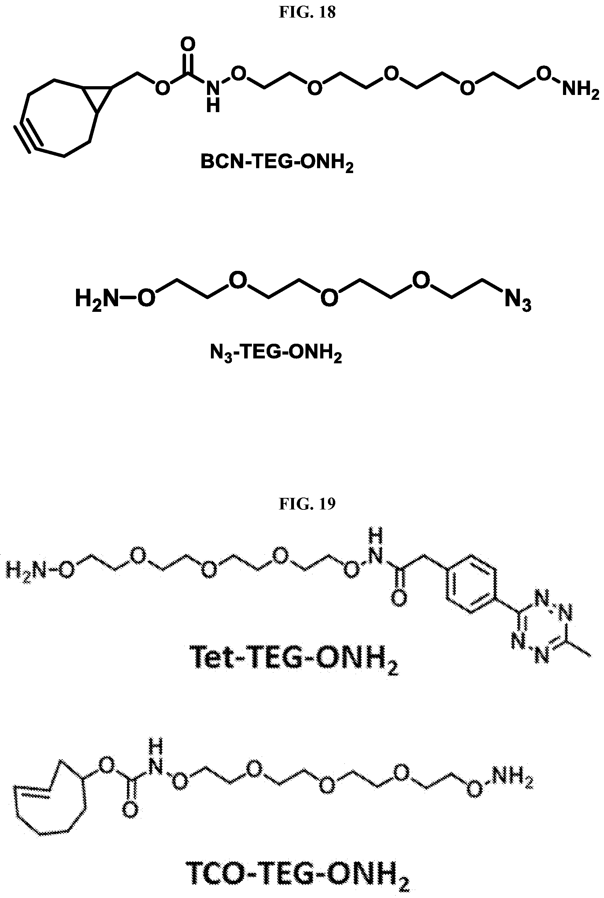

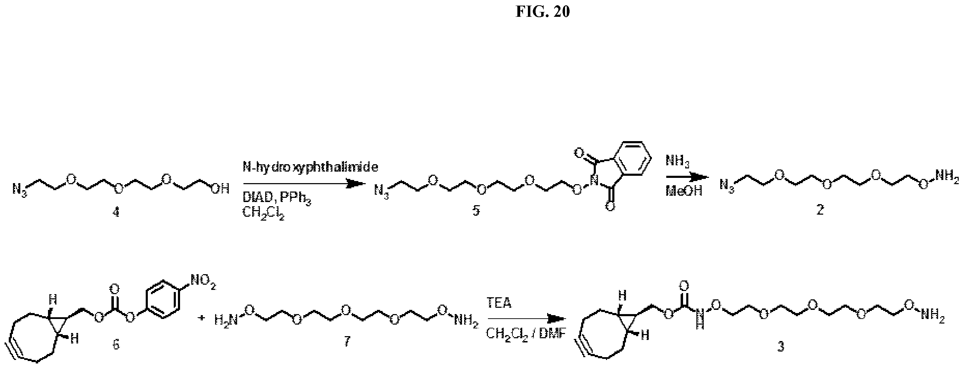

**FIG. 18** depicts exemplary linkers. **FIG. 19** depicts exemplary heterobifunctional linkers. **FIG. 20** shows a general scheme for synthesizing bifunctional linkers. Additional exemplary linkers and methods of constructing linkers can be found in WO2014/153002.

The linker may be attached to a chimeric antigen receptor-interacting domain (CAR-ID). The linker may be attached to a target interacting domain (TID). The linker may attach a CAR-ID to a TID. The one or more linkers may attach the one or more CAR-IDs to the one or more TIDs. The one or more linkers may attach the one or more CAR-IDs to the one or more TIDs in a site-specific manner. Attachment in a site-specific manner may comprise attaching the one or more CAR-IDs to a predetermined site on the one or more TIDs. Alternatively, or additionally, attachment in a site-specific manner may comprise attaching the one or more CAR-IDs to an unnatural amino acid in the one or more TIDs. The one or more linkers may attach the one or more CAR-IDs to the one or more TIDs in a site-independent manner. Attachment in a site-independent manner may comprise attaching the one or more CAR-IDs to a random site on the one or more TIDs. The CAR-ID may be attached to 1, 2, 3, 4, 5 or more TIDs in a site-specific manner. The CAR-ID may be attached to 1, 2, 3, 4, 5 or more TIDs in a site-independent manner. Alternatively, the TID may be attached to 1, 2, 3, 4, 5 or more CAR-IDs in a site-specific manner. Attachment in a site-specific manner may comprise attaching the one or more TIDs to a predetermined site on the one or more CAR-IDs. The TID may be attached to 1, 2, 3, 4, 5 or more CAR-IDs in a site-independent manner. Attachment in a site-independent manner may comprise attaching the one or more TIDs to a random site on the one or more CAR-IDs.

The one or more linkers may be coupled to the CAR-ID, the TID, or a combination thereof. The one or more linkers may be coupled to the CAR-ID to form one or more switch intermediates of the Formula IIA: L1-X or Formula II: X-L1, wherein X is the CAR-ID and L1 is the linker. The one or more linkers may be coupled to the CAR-ID by an oxime. The one or more linkers may be coupled to the CAR-ID by a cyclooctyne, cyclopropene, aryl/alkyl azides, trans-cyclooctene, norborene, tetrazine, or a combination thereof. The one or more linkers may be coupled to the CAR-ID by a covalent bond, non-covalent bond, ionic bond, or a combination thereof. The one or more linkers may be coupled to the TID to form one or more switch intermediates of the Formula IIIA: L1-Y or Formula III: Y-L1, wherein Y is the TID and L1 is the linker. The one or more linkers may be coupled to the TID by an oxime. The one or more linkers may be coupled to the TID by a cyclooctyne, cyclopropene, aryl/alkyl azides, trans-cyclooctene, norborene, tetrazine, or a combination thereof. The one or more linkers may be coupled to the TID by a covalent bond, non-covalent bond, ionic bond, or a combination thereof.

The TID may comprise one or more amino acids. The one or more amino acids may comprise a natural amino acid. The linker may couple with one or more natural amino acids on the TID. The one or more amino acids may comprise one or more unnatural amino acids. The linker may couple with one or more unnatural amino acids on the TP. The linker may couple with an amino acid which is the product of site-specific mutagenesis. The linker may couple with a cysteine which is the product of site-specific mutagenesis. The linker (e.g., substituted maleimide) may couple with a cysteine which is the product of site-specific mutagenesis, as well as a native cysteine residue. Two linkers, each with complementary reactive functional groups, may couple with one another.

The one or more linkers may be a cleavable linker. The one or more linkers may be a non-cleavable linker. The one or more linkers may be a flexible linker. The one or more linkers may be an inflexible linker. The linker may be a bifunctional linker. A bifunctional linker may comprise a first functional group on one end and a second functional group on the second end. The bifunctional linker may be heterobifunctional linker. A heterobifunctional linker may comprise a first functional group on one end and a second functional group on the second end, wherein the first functional group and the second functional group are different. The bifunctional linker may be a homobifunctional linker. A homobifunctional linker may comprise a first functional group on one end and a second functional group on the second end, wherein the first functional group and the second functional group are the same.

The linker may comprise a chemical bond. The linker may comprise a functional group. The linker may comprise a polymer. The polymer may be a polyethylene glycol. The linker may comprise an amino acid.

The linker may comprise one or more functional groups. The linker may comprise two or more functional groups. The linker may comprise three or more functional groups. The linker may comprise four or more functional groups. The linker may comprise 5, 6, 7, 8, 9, 10 or more functional groups. The linker may be a bifunctional ethylene glycol linker.

The linker may comprise ethylene glycol. The linker may comprise about 1, about 2, about 3, about 4, about 5, about 6, about 7, about 8, about 9, about 10, about 11, about 12, about 13, about 14, about 15, about 16, about 17, about 18, about 19 or about 20 or more ethylene glycol subunits. The linker may comprise 4 or more ethylene glycol subunits. The linker may comprise 8 or more ethylene glycol subunits. The linker may comprise 10 or more ethylene glycol subunits. The linker may comprise 12 or more ethylene glycol subunits. The linker may comprise 15 or more ethylene glycol subunits. The linker may comprise 20 or more ethylene glycol subunits. The linker may comprise 25 or more ethylene glycol subunits. The linker may comprise 30 or more ethylene glycol subunits. The linker may comprise 35 or more ethylene glycol subunits.

The linker may comprise polyethylene glycol (PEG). The linker may comprise about 1, about 2, about 3, about 4, about 5, about 6, about 7, about 8, about 9, about 10, about 11, about 12, about 13, about 14, about 15, about 16, about 17, about 18, about 19 or about 20 or more polyethylene glycol (PEG) subunits. The linker may comprise 4 or more polyethylene glycol (PEG) subunits. The linker may comprise 8 or more polyethylene glycol (PEG) subunits. The linker may comprise 10 or more polyethylene glycol (PEG) subunits. The linker may comprise 12 or more polyethylene glycol (PEG) subunits. The linker may comprise 15 or more polyethylene glycol (PEG) subunits. The linker may comprise 20 or more polyethylene glycol (PEG) subunits. The linker may comprise 25 or more polyethylene glycol (PEG) subunits. The linker may comprise 30 or more polyethylene glycol (PEG) subunits. The linker may comprise 35 or more polyethylene glycol (PEG) subunits.

The linker may comprise a triazole. The triazole may be a 1,2,3-triazole. The triazole may be a 1,2,4-triazole.

The linker may comprise an aryl or a heteroaryl. The linker may comprise an aryl. The aryl may be phenyl. The phenyl may be disubstituted. The disubstituted phenyl may be 1,4-disubstituted phenyl. The disubstituted phenyl may be 1,3-disubstituted phenyl. The phenyl may be trisubstituted. The phenyl may be tetrasubstituted. Two of the substituents of the substituted phenyl may be NO₂. In some instances, the linker does not comprise a benzyl substituent.

The linker may comprise one or more polyethylene glycol (PEG) units. The linker may comprise multiple polyethylene glycol (PEG) units. The linker may comprise 2 or more polyethylene glycol (PEG) units. The linker may comprise 3 or more polyethylene glycol (PEG) units. The linker may comprise 4 or more polyethylene glycol (PEG) units. The linker may comprise 5 or more polyethylene glycol (PEG) units. The linker may comprise 6 or more polyethylene glycol (PEG) units. The linker may comprise 7 or more polyethylene glycol (PEG) units. The linker may comprise 8 or more polyethylene glycol (PEG) units. The linker may comprise 9 or more polyethylene glycol (PEG) units. The linker may comprise 10 or more polyethylene glycol (PEG) units. The linker may comprise 11 or more polyethylene glycol (PEG) units. The linker may comprise 12 or more polyethylene glycol (PEG) units. The linker may comprise 13 or more polyethylene glycol (PEG) units. The linker may comprise 14 or more polyethylene glycol (PEG) units.

The linker may comprise an amide on one end. The linker may comprise an amide on one end and an amine on the other end. The linker may comprise an amide on one end and a triazole on the other end.

The one or more linkers may comprise a 1,4-dicarboxylic moiety. The one or more linkers may comprise a 1,3-dinitro substituted phenyl moiety.

The one or more linkers may comprise one or more reactive functional groups. The reactive functional group may react with a complementary reactive functional group on a coupling partner. The reaction of the reactive functional group on the linker to a complementary reactive functional group on a coupling partner may occur prior to incorporation of the linker into the CAR-EC switch.

The linker may comprise at least one reactive functional group selected from alkoxy-amine, hydrazine, aryl/alkyl azide, alkyne, alkene, tetrazine, dichlorotriazine, tresylate, succinimidyl carbonate, benzotriazole carbonate, nitrophenyl carbonate, trichlorophenyl carbonate, carbonylimidazole, succinimidyl succinate, maleimide, vinylsulfone, haloacetamide, and disulfide. The alkene may be selected from norbornene, trans-cyclooctene, and cyclopropene. The linker may comprise at least one alkoxy amine. The linker may comprise at least one azide. The linker may comprise at least one cyclooctyne. The linker may comprise at least one tetrazine.

The one or more linkers may comprise an alkoxy-amine (or aminooxy) group, azide group and/or cyclooctyne group at one or more termini. The one or more linkers may comprise an alkoxy-amine at one terminus and an azide group at the other terminus. The one or more linkers may comprise an alkoxy-amine at one terminus and a cyclooctyne group at the other terminus. The alkoxy-amine may form a stable oxime with a ketone group on an amino acid. The alkoxy-amine may form a stable oxime with a ketone group on an unnatural amino acid. The ketone group may be on a *p*-acetyl phenylalanine (pAcF).

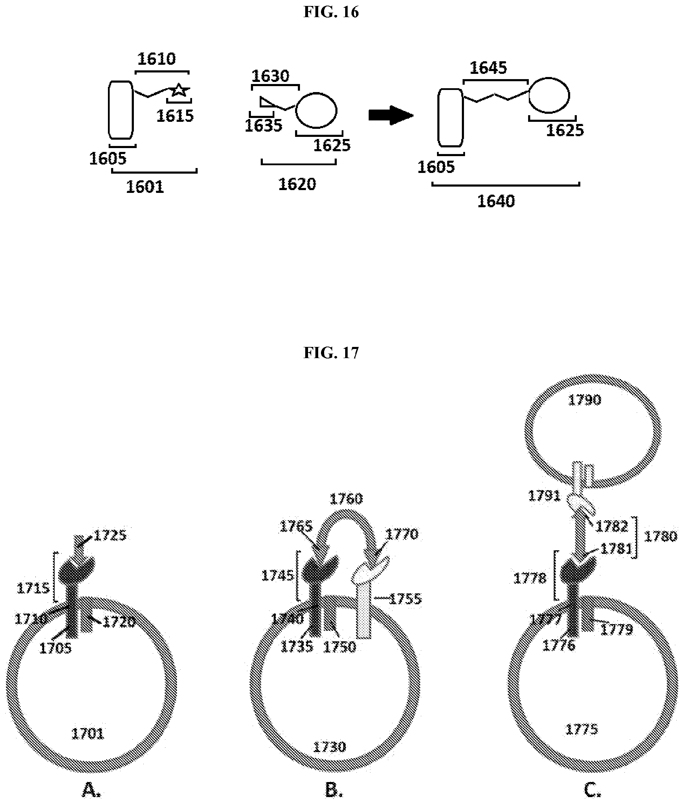

One or more linkers may be formed by reaction of reactive functional group on the CAR-ID with a complementary reactive functional group of a linker that is attached to the TID. One or more linkers may be formed by reaction of an amino acid or another reactive functional group on the TID with a complementary reactive functional group of a linker that is attached to the CAR-ID. One or more linkers may be formed by reaction of a linker that is attached to the CAR-ID with another linker that is attached to the TID. **FIG. 16** shows a schematic of producing a linker by reaction of reactive functional groups on two switch intermediates. As shown in **FIG. 16****,** a first switch intermediate (**1601**) comprising a CAR-ID (**1605**) and a first linker (**1610**) is contacted with a second switch intermediate (**1620**) comprising a TID (**1625**) and a second linker (**1630**)**.** The reactive functional group (**1615**) of the first linker (**1610**) reacts with the second functional group (**1635**) of the second linker (**1635**) to produce a new linker (**1645**). The reaction of the two switch intermediates (**1601, 1620**) results in the formation of a switch (**1640**) comprising the CAR-ID (**1605**) connected to the TID (**1625**) via the new linker (**1645**).

The linker may be the product of a bioorthogonal reaction. For example, amino acids that contain ketone, azide, alkyne, alkene, and tetrazine side chains can be genetically encoded in response to nonsense and frameshift codons. These side chains can act as chemical handles for bioorthogonal conjugation reactions (Kim et al., Curr Opin Chem Bio 17:412-419 (2013)). The linker may comprise an oxime, a tetrazole, a Diels Alder adduct, a hetero Diels Alder adduct, an aromatic substitution reaction product, a nucleophilic substitution reaction product, an ester, an amide, a carbamate, an ether, a thioether, or a Michael reaction product. The linker may be a cycloaddition product, a metathesis reaction product, a metal-mediated cross-coupling reaction product, a radical polymerization product, an oxidative coupling product, an acyl-transfer reaction product, or a photo click reaction product. The cycloaddition may be a Huisgen-cycloaddition. The cycloaddition may be a copper-free [3+2] Huisgen-cycloaddition. The cycloaddition may be a Diels-Alder reaction. The cycloaddition may be a hetero Diels-Alder reaction. The linker may be the product of an enzyme-mediated reaction. The linker may be a product of a transglutaminase-mediated reaction, non-limiting examples of which are described in Lin et al., J. Am. Chem. Soc. 128:4542-4543 (2006) and WO 2013/093809. The linker may comprise a disulfide bridge that connects two cysteine residues, such as ThioBridge™ technology by PolyTherics. The linker may comprise a maleimide bridge that connects two amino acid residues. The linker may comprise a maleimide bridge that connects two cysteine residues.

Two or more linkers may be linked. The two or more linkers may be linked through one or more copper-free reactions. The two or more linkers may be linked through one or more cycloadditions. The two or more linkers may be linked through one or more Huisgen-cycloadditions. The two or more linkers may be linked through one or more copper-free [3+2] Huisgen-cycloadditions. The two or more linkers may be linked through one or more copper-containing reactions. The two or more linkers may be linked through one or more Diels Alder reactions. The two or more linkers may be linked through one or more hetero Diels Alder reactions.

CAR-EC switches may be optimized by adjusting linker length. CAR-EC switches may comprise linkers of different lengths. Linkers may be relatively short. Linkers may be relatively long. The one or more linkers may be between about 1 angstroms (Å) to about 120 angstroms (Å) in length. The one or more linkers may be between about 5 angstroms (Å) to about 105 angstroms (Å) in length. The one or more linkers may be between about 10 angstroms (Å) to about 100 angstroms (Å) in length. The one or more linkers may be between about 10 angstroms (Å) to about 90 angstroms (Å) in length. The one or more linkers may be between about 10 angstroms (Å) to about 80 angstroms (Å) in length. The one or more linkers may be between about 10 angstroms (Å) to about 70 angstroms (Å) in length. The one or more linkers may be between about 15 angstroms (Å) to about 45 angstroms (Å) in length. The one or more linkers may be equal to or greater than about 4, 5, 6, 7, 8, 9, 10, 11, 12, 13, 14, 15, 16, 17, 18, 19, 20, 25, 27, 30 or more angstroms in length. The one or more linkers may be equal to or greater than about 10 angstroms in length. The one or more linkers may be equal to or greater than about 15 angstroms in length. The one or more linkers may be equal to or greater than about 20 angstroms in length. The one or more linkers may be equal to or less than about 110, 100, 90, 85, 80, 75, 70, 65, 60, 55, 50, 45, 43, 42, 41, 40, 39, 38, 37, 36, 35, 34, 33, 32, 31, 30 or fewer angstroms in length. The one or more linkers may be equal to or less than about 100 angstroms in length. The one or more linkers may be equal to or less than about 80 angstroms in length. The one or more linkers may be equal to or less than about 60 angstroms in length. The one or more linkers may be equal to or less than about 40 angstroms in length.

The total length of the linkers may be between about 1 angstroms (Å) to about 120 angstroms (Å). The total length of the linkers may be between about 5 angstroms (Å) to about 105 angstroms (Å). The total length of the linkers may be between about 10 angstroms (Å) to about 100 angstroms (Å). The total length of the linkers may be between about 10 angstroms (Å) to about 90 angstroms (Å). The total length of the linkers may be between about 10 angstroms (Å) to about 80 angstroms (Å). The total length of the linkers may be between about 10 angstroms (Å) to about 70 angstroms (Å). The total length of the linkers may be between about 15 angstroms to about 45 angstroms (Å). The total length of the linkers may be equal to or greater than about 4, 5, 6, 7, 8, 9, 10, 11, 12, 13, 14, 15, 16, 17, 18, 19, 20, 25, 27, 30 or more angstroms. The total length of the linkers may be equal to or greater than about 10 angstroms. The total length of the linkers may be equal to or greater than about 15 angstroms. The total length of the linkers may be equal to or greater than about 20 angstroms. The total length of the linkers may be equal to or less than about 110, 100, 90, 85, 80, 75, 70, 65, 60, 55, 50, 45, 43, 42, 41, 40, 39, 38, 37, 36, 35, 34, 33, 32, 31, 30 or fewer angstroms. The total length of the linkers may be equal to or less than about 100 angstroms. The total length of the linkers may be equal to or less than about 80 angstroms. The total length of the linkers may be equal to or less than about 60 angstroms. The total length of the linkers may be equal to or less than about 40 angstroms. The total length of the linkers may be equal to or less than about 25 (Å). The distance between the CAR-ID and the TID may be about 30 (Å).

Disclosed herein are compositions comprising a plurality of switches, wherein a switch of the plurality of switches comprises (a) a CAR-ID; (b) a TID; and (c) a linker, wherein at least about 60% of the switches of the plurality of switches are structurally homogeneous. At least about 60%, 61%, 62%, 63%, 64%, 65%, 66%, 67%, 68% or 69% of the switches of the plurality of switches may be structurally homogeneous. At least about 70%, 71%, 72%, 73%, 74%, 75%, 76%, 77%, 78% or 79% of the switches of the plurality of switches may be structurally homogeneous. At least about 80%, 81%, 82%, 83%, 84%, 85%, 86%, 87%, 88% or 89% of the switches of the plurality of switches may be structurally homogeneous. At least about 90%, 91%, 92%, 93%, 94%, 95%, 96%, 97%, 98% or 99% of the switches of the plurality of switches may be structurally homogeneous. Structurally homogeneous CAR-EC switches may be provided for by site-specifically linking the CAR-ID and the TID. The linker may be linked to a CAR-ID site-specifically. The linker may be linked to a TID site-specifically. A first site of the linker may be linked to a CAR-ID site-specifically and a second site of the linker may be linked to a TID site-specifically.

### Methods of Producing Switches and Switch Intermediates

Disclosed herein are methods of producing CAR-EC switches. Generally, the method comprises attaching a chimeric antigen receptor-interacting domain (CAR-ID) to a target interacting domain (TID). Alternatively, the method may comprise attaching a switch intermediate comprising a CAR-ID and a linker to a TID. The method may comprise attaching a switch intermediate comprising a TID and a linker to a CAR-ID. The method may comprise attaching a first switch intermediate comprising a CAR-ID and a first linker to a second switch comprising a TID and a second linker. Attachment of the CAR-ID to the TID may occur in a site-specific manner. Attachment in a site-specific manner may comprise attaching the CAR-ID to a predetermined site on the TID. Attachment in a site-specific manner may comprise attaching the TID to a predetermined site on the CAR-ID. Attachment of the CAR-ID to the TID may occur in a site-independent manner. Attachment in a site-independent manner may comprise attaching the CAR-ID to a random site on the TID. Attachment in a site-independent manner may comprise attaching the TID to a random site on the CAR-ID. The method may further comprise attaching one or more additional CAR-IDs to the TID. The method may further comprise attaching or more additional TIDs to the CAR-ID. The method may further comprise using one or more additional linkers to connect the TID to the CAR-ID. Attaching the CAR-ID to the TID may comprise conducting one or more chemical reactions.

The method of producing a switch may comprise linking a TID based on or derived from an antibody or antibody fragment to a CAR-ID or a switch intermediate comprising a CAR-ID to produce a CAR-EC switch comprising (a) the TID; (b) one or more linkers; and (c) the CAR-ID, The one or more linkers may link the TID to the CAR-ID. Linking the TID to the CAR-ID may occur in a site-specific manner. The CAR-ID may be attached to a predetermined site on the TID via the one or more linkers. The TID may be attached to a predetermined site on the CAR-ID via the one or more linkers.

Disclosed herein are methods of producing a switch of Formula I: X-L1-Y or Formula IA: Y-L1-X, wherein X is a chimeric antigen receptor-interacting domain (CAR-ID), Y is a target interacting domain (TID) and L1 is a linker. X may be a CAR binding small molecule and Y may be an antibody or antibody fragment. X may be a CAR binding small molecule that does not comprise a peptide and Y may be a peptide that does not comprise an antibody or antibody fragment. X may be a CAR binding small molecule that does not comprise a peptide and Y may be a targeting small molecule that does not comprise a peptide. The method may comprise conducting one or more reactions to attach the CAR-ID to a predetermined site in the TID. Conducting the one or more reactions to attach the CAR-ID to the TID may comprise mixing a plurality of CAR-IDs with a plurality of TIDs. The method may comprise attaching one end of the linker to the TID, followed by attachment of the other end of the linker to the CAR-ID. The method may comprise attaching one end of the linker to the CAR-ID, followed by attachment of the other end of the linker to the TID. Attachment of the linker to the TID may occur in a site-specific manner. The linker may be attached to a predetermined amino acid of the TID. The amino acid may be an unnatural amino acid. The linker may comprise a functional group that interacts with the amino acid. Attachment of the linker to the TID may occur in a site-independent manner. The linker may be randomly attached to the TID. The linker may comprise a functional group that reacts with a functional group in the TID. Attachment of the linker to the CAR-ID may occur in a site-specific manner. Attachment of the linker to the CAR-ID may occur in a site-independent manner. The linker may comprise a functional group that reacts with a function group in the CAR-ID. Conducting the one or more reactions to attach the CAR-ID to the TID may comprise conducting an oxime ligation.

Alternatively, or additionally, the method may comprise conducting a reaction to attach the linker or a precursor of the linker to the CAR-ID to produce a switch intermediate comprising the linker conjugated to the CAR-ID. The switch intermediate may have the Formula II: X-L1 or Formula IIA: L1-X, wherein X is the CAR-ID and L1 is the linker or precursor of the linker. The linker may be conjugated to the CAR-ID in a site-specific manner. The linker may be conjugated to the CAR-ID in a site-independent manner. Conducting the one or more reactions to attach the CAR-ID to the TID may comprise attaching the linker portion of the switch intermediate to the TID. Conducting the one or more reactions to attach the CAR-ID to the TID may comprise contacting a plurality of switch intermediates comprising the linker or linker precursor conjugated to the CAR-ID with a plurality of TIDs. Attachment of the linker portion of the switch intermediate to the TID may occur in a site-specific manner. The TID may comprise one or more unnatural amino acids. The linker portion of the switch may be attached to the TID via the one or more unnatural amino acids. Attachment of the linker portion of the switch intermediate may occur in a site-independent manner.

Alternatively, or additionally, the method may comprise conducting a reaction to attach the linker or a precursor of the linker to the TID to produce a switch intermediate comprising the linker or precursor of the linker conjugated to the TID. The switch intermediate may be of Formula III: Y-L1 or Formula IIIA: L1-Y, wherein Y is the TID and L1 is the linker or linker precursor. The linker may be conjugated to the TID in a site-specific manner. The linker may be conjugated to the TID in a site-independent manner. Conducting the one or more reactions to attach the CAR-ID to the TID may comprise attaching the linker portion of the switch intermediate to the CAR-ID. Conducting the one or more reactions to attach the CAR-ID to the TID may comprise contacting a plurality of switch intermediates comprising the linker or linker precursor conjugated to the TID with a plurality of CAR-IDs. Attachment of the linker portion of the switch intermediate to the CAR-ID may occur in a site-specific manner. Attachment of the linker portion of the switch intermediate may occur in a site-independent manner.

The method may comprise coupling one or more linkers to the TID to produce a switch intermediate of Formula III: Y-L1 or Formula IIIA: L1-Y, wherein Y is the TID and L1 is the linker; and conjugating the switch intermediate to the CAR-ID, thereby producing the CAR-EC switch. The switch intermediate may be conjugated to the CAR-ID in a site-specific manner. The switch intermediate may be conjugated to the CAR-ID in a site-independent manner. The method may further comprise incorporating one or more unnatural amino acids into the CAR-ID and/ or TID. The switch intermediate may be conjugated to the CAR-ID in a site-specific manner through the use of the unnatural amino acid.

The method may comprise coupling one or more linkers to the CAR-ID to produce a switch intermediate of Formula II: X-L1 or Formula IIA: L1-X, wherein X is the CAR-ID and L1 is the linker; and conjugating the switch intermediate to the TID, thereby producing the CAR-EC switch. The switch intermediate may be conjugated to the TID in a site-specific manner. The switch intermediate may be conjugated to the TID in a site-independent manner. The method may further comprise incorporating one or more unnatural amino acids into the CAR-ID and/ or TID. The switch intermediate may be conjugated to the TID in a site-specific manner through the use of the unnatural amino acid.

Conjugating the switch intermediate of Formula II: X-L1 or Formula IIA: L1-X, wherein X is the CAR-ID and L1, to the TID may comprise forming an oxime. Conjugating the switch intermediate of Formula III: Y-L1 or Formula IIIA: L1-Y, wherein Y is the TID and L1, to the CAR-ID may comprise forming an oxime. Forming an oxime may comprise conducting one or more reactions under acidic conditions. Forming an oxime may comprise conducting one or more reactions under slightly acidic conditions. Forming an oxime may comprise conducting one or more reactions under slightly neutral conditions.

A method of producing a switch may comprise (a) producing a target interacting domain (TID) comprising an unnatural amino acid; (b) attaching a first linker to the TID to produce a first switch intermediate comprising the TID and the first linker; (c) attaching a second switch intermediate comprising a chimeric antigen receptor-interacting domain (CAR-ID) and a second linker to the first switch intermediate, thereby producing the switch. The unnatural amino acid may be *p*-acetylphenalanine (*p*AcF). The TID may comprise a polypeptide based on or derived from an antibody or antibody fragment. The antibody may be selected from the group consisting of an anti-CD 19 antibody, an anti-CD22 antibody, an anti-CD20 antibody, an anti-EGFR antibody, an anti-EGFRvIII antibody, an anti-Her2 antibody, an anti-CSl antibody, an anti-BCMA antibody, an anti-CEA antibody, an anti-CLL-1 antibody and an anti-CD33 antibody. The antibody may be an anti-CD 19 antibody. The antibody may be an anti-EGFR antibody. The antibody may be an anti-CD20 antibody. The antibody may be an anti-HER2 antibody. The TID may comprise an antibody fragment. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56. The first linker may be a bifunctional linker. The linker may be a heterobifunctional linker. The linker may comprise one or more polyethylene glycol (PEG) subunits. The first linker may comprise cyclooctyne. The first linker may be a PEG-cyclooctyne linker. The linker may comprise an azide. The first linker may comprise triazole. The triazole may be 1,2,3-triazole. The triazole may be 1,2,4-triazole. The first linker may comprise an azide-PEG-aminoxy linker. The first linker may be attached to a ketone of the unnatural amino acid. The first linker may be attached to the TID via oxime ligation. The CAR-ID may comprise a small molecule. The CAR-ID may comprise FITC. The second linker may be a bifunctional linker. The linker may be a heterobifunctional linker. The linker may comprise one or more polyethylene glycol (PEG) subunits. The second linker may comprise cyclooctyne. The second linker may be a PEG-cyclooctyne linker. The linker may comprise an azide. The second linker may comprise triazole. The triazole may be 1,2,3-triazole. The triazole may be 1,2,4-triazole. The second linker may be a PEG-cyclooctyne linker. The second switch intermediate may be attached to the first switch intermediate via a click chemistry reaction. The second switch intermediate may be attached to the first switch intermediate through a cycloaddition reaction. The cycloaddition reaction may be a [3+2] cycloaddition reaction.

Conjugating the linker to the CAR-ID to produce the switch may comprise forming one or more bonds between the linker and the CAR-ID. Conjugating the linker to the TID to produce the switch may comprise forming one or more bonds between the linker and the TID. The one or more bonds may comprise an ionic bond, a covalent bond, a non-covalent bond or a combination thereof. Additional methods of conjugating the linker the CAR-ID and the TID may be performed as described in Roberts et al., Advanced Drug Delivery Reviews 54:459-476 (2002), which is included by reference in its entirety.

The CAR-ID may comprise any of the CAR-IDs disclosed herein. For example, the CAR-ID may comprise a small molecule. The CAR-ID may comprise FITC. The CAR-ID may be selected from the group consisting of DOTA, dinitrophenol, quinone, biotin, aniline, atrazine, an aniline-derivative, o-aminobenzoic acid, p-aminobenzoic acid, m-aminobenzoic acid, hydralazine, halothane, digoxigenin, benzene arsonate, lactose, trinitrophenol, biotin and derivatives thereof. The TID may comprise any of the TIDs disclosed herein. For example, the TID may comprise a small molecule. The TID may comprise 2-[3-(1,3-dicarboxypropyl)ureido] pentanedioic acid or a derivative thereof. The TID may comprise folate. The TID may be based on or derived from an antibody or antibody fragment. The antibody or antibody fragment may comprise anti-CD 19. The antibody or antibody fragment may be selected from the group consisting of anti-CD20, anti-CD22, anti-CD33, anti-BMSA, anti-CEA, anti-CLL1, anti-CS1, anti-EGFR, and anti-Her2. The linker may comprise any of the linkers disclosed herein. For example, the linker may comprise an aminooxy group, azide group cyclooctyne group, or a combination thereof at one or more termini. The linker may be a bifunctional linker. The linker may be a heterobifunctional linker. The linker may comprise one or more PEG subunits.

Disclosed herein are methods of producing a switch of Formula IV: X- L1-L2-Y, wherein in X is a CAR-ID, L1 is a first linker, L2 is a second linker and Y is a TID. The method may comprise (a) coupling L1 to X to produce a first switch intermediate of Formula II: X- L1; (b) coupling L2 to Y to produce a second switch intermediate of Formula V: L2-Y; and (c) linking the first switch intermediate of Formula II to the second switch intermediate of Formula: V, thereby producing the switch of Formula IV.

Disclosed herein are methods of producing a switch of Formula IVA: Y-L2-L1-X, wherein Y is a TID, L1 is a first linker, L2 is a second linker and X is a CAR-ID. The method may comprise (a) coupling L1 to X to produce a first switch intermediate of Formula IIA: L1-X; (b) coupling L2 to Y to produce a second switch intermediate of Formula VA: Y-L2; and (c) linking the first intermediate of Formula IIA to the second intermediate of Formula VA, thereby producing the CAR-EC switch of Formula IVA.

The methods may further comprise incorporating one or more unnatural amino acids into X and/or Y. The L1 may be coupled to X in a site-specific manner. The L1 may be coupled to X in a site-specific manner through the one or more unnatural amino acids. L2 may be coupled to Y in a site-specific manner. The L2 may be coupled to Y in a site-specific manner through the one or more unnatural amino acids. The method may further comprise modifying a nucleic acid encoding X to produce one or more amber codons in X. The method may further comprise modifying a nucleic acid encoding Y to produce one or more amber codons in Y.

Conjugating the linker to the CAR-ID to produce the first switch intermediate may comprise forming one or more bonds between the linker and the CAR-ID. Conjugating the linker to the TID to produce the second switch intermediate may comprise forming one or more bonds between the linker and the TID. The one or more bonds may comprise an ionic bond, a covalent bond, a non-covalent bond or a combination thereof. Additional methods of conjugating the linker the CAR-ID and the TID may be performed as described in Roberts et al., Advanced Drug Delivery Reviews 54:459-476 (2002), which is included by reference in its entirety.

Linking the first switch intermediate to the second switch intermediate may comprise a Huisgen-cycloaddition, a Diels-Halder reaction, a hetero Diels-Alder reaction or an enzyme-mediated reaction. Linking the first switch intermediate to the second switch intermediate may produce an oxime, a tetrazole, a Diels Alder adduct, a hetero Diels Alder adduct, an aromatic substitution reaction product, a nucleophilic substitution reaction product, an ester, an amide, a carbamate, an ether, a thioether, a Michael reaction product, cycloaddition product, a metathesis reaction product, a metal-mediated cross-coupling reaction product, a radical polymerization product, an oxidative coupling product, an acyl-transfer reaction product, or a photo click reaction product. Linking the first switch intermediate to the second switch intermediate may produce a disulfide bridge or a maleimide bridge.

L1 and/or L2 may comprise a linker selected from a bifunctional linker, a cleavable linker, a non-cleavable linker, an ethylene glycol linker, a bifunctional ethylene glycol linker, a flexible linker, or an inflexible linker. L1 and/or L2 may comprise a linker selected from the group comprising cyclooctyne, cyclopropene, aryl/alkyl azides, trans-cyclooctene, norborene, and tetrazines. A terminus of L1 and/or a terminus of L2 may comprise an alkoxy-amine. A terminus of L1 and/or a terminus of L2 may comprise an azide or cyclooctyne group. X may be coupled to L1 by a chemical group selected from a cyclooctyne, cyclopropene, aryl/alkyl azide, trans-cyclooctene, norborene, and tetrazine. Linking the first switch intermediate (X-L1 or L1-X) and second switch intermediate (Y-L2 or L2-Y) may comprise conducting one or more copper-free reactions. Linking the first switch intermediate (X-L1 or L1-X) and second switch intermediate (Y-L2 or L2-Y) may comprise conducting one or more copper-containing reactions. Linking the first switch intermediate (X-L1 or L1-X) and second switch intermediate (Y-L2 or L2-Y) may comprise one or more cycloadditions. Linking the first switch intermediate (X-L1 or L1-X) and second switch intermediate (Y-L2 or L2-Y) may comprise one or more Huisgen-cycloadditions. Linking the first switch intermediate (X-L1 or L1-X) and second switch intermediate (Y-L2 or L2-Y) may comprise one or more Diels Alder reactions. Linking the first switch intermediate (X-L1 or L1-X) and second switch intermediate (Y-L2 or L2-Y) may comprise one or more Hetero Diels Alder reaction.

The methods disclosed herein may comprise coupling one or more linkers to one or more TIDs, CAR-IDs or combinations thereof to produce one or more switch intermediates. The switch intermediate may comprise a TID attached to a linker (e.g., TID switch intermediate). The switch intermediate may comprise a CAR-ID attached to a linker (e.g., CAR-ID switch intermediates). The methods may comprise coupling a first linker to TID to produce a TID switch intermediate. The methods may comprise coupling a linker to a CAR-ID to produce a CAR-ID switch intermediate.

Coupling of the one or more linkers to the TID and the CAR-ID may occur simultaneously. Coupling of the one or more linkers to the TID and the CAR-ID may occur sequentially. Coupling of the one or more linkers to the TID and the CAR-ID may occur in a single reaction volume. Coupling of the one or more linkers to the TID and the CAR-ID may occur in two or more reaction volumes.

Coupling one or more linkers to the TID and/or the CAR-ID may comprise forming one or more oximes between the linker and the TID and/or the CAR-ID. Coupling one or more linkers to the TID and/or the CAR-ID may comprise forming one or more stable bonds between the linker and the TID and/or the CAR-ID. Coupling one or more linkers to the TID and/or the CAR-ID may comprise forming one or more covalent bonds between the linker and the TID and/or the CAR-ID. Coupling one or more linkers to the TID and/or the CAR-ID may comprise forming one or more non-covalent bonds between the linker and TID and/or the CAR-ID. Coupling one or more linkers to the TID and/or the CAR-ID may comprise forming one or more ionic bonds between the linker and the TID and/or the CAR-ID.

Coupling one or more linkers to the TID and/or the CAR-ID may comprise site specifically coupling one or more linkers to the TID and/or the CAR-ID. Site-specific coupling may comprise linking the one or more linkers to the unnatural amino acid of the TID and/or the CAR-ID. Linking the one or more linkers to the unnatural amino acid of the TID and/or the CAR-ID may comprise formation of an oxime. Linking the one or more linkers to the unnatural amino acid of the TID and/or the CAR-ID may comprise, by way of non-limiting example, reacting a hydroxylamine of the one or more linkers with an aldehyde or ketone of an amino acid. The amino acid may be an unnatural amino acid.

Conducting the one or more reactions to site-specifically link the CAR-ID to the TID, to site-specifically attach the linker or a precursor of the linker to the CAR-ID, to site-specifically attach the linker or a precursor of the linker to the TID, to site-specifically attach the CAR-ID switch intermediate to the TID, to site-specifically attach the TID switch intermediate to the CAR-ID or to site-specifically attach the TID switch intermediate to the CAR-ID switch intermediate may comprise conducting one or more reactions selected from a copper-free reaction, a cycloadditions, a Huisgen-cycloaddition, a copper-free [3+2] Huisgen-cycloaddition, a copper-containing reaction, a Diels Alder reactions, a hetero Diels Alder reaction, metathesis reaction, a metal-mediated cross-coupling reaction, a radical polymerization, an oxidative coupling, an acyl-transfer reaction, a photo click reaction, an enzyme-mediated reaction, a transglutaminase-mediated reaction.

The switches disclosed herein may comprise a CAR-ID comprising FITC or a derivative thereof. The method of producing such switches may comprise coupling a linker or precursor thereof, a switch intermediate comprising a TID (e.g., TID switch intermediate), or a TID to the CAR-ID. Coupling the linker or precursor thereof, the TID switch intermediate to the CAR-ID may comprise conjugation of an isothiocyanate of FITC to the linker or precursor thereof, TID switch intermediate or TID. The TID may be based on or derived from a polypeptide. The polypeptide may be an antibody or antibody fragment. Coupling a TID to the CAR-ID may comprise conjugating the isothiocyanate of FITC to an amino acid of the TID. The amino acid may be a lysine. The method may comprise coupling or more CAR-IDs to the TID. The method may comprise conjugating FITC from two or more CAR-IDs to two or more amino acids of the TID. The two or more amino acids may be lysine.

Producing a switch disclosed herein may comprise ester coupling. Ester coupling may comprise forming an amide bond between the CAR-ID and the TID. Ester coupling may comprise forming an amide bond between a switch intermediate and the TID. The switch intermediate may comprise a CAR-ID attached to a linker. The amide bond may be formed between the linker of the switch intermediate and the TID. The linker may be a NHS-ester linker. The amide bond may be formed between the linker of the switch intermediate and an amino acid of the TID. The CAR-ID may comprise a small molecule. The small molecule may be FITC. The TID may be based on or derived from a polypeptide. The polypeptide may be an antibody or antibody fragment. The TID may comprise a small molecule.

The method of producing a switch disclosed herein may comprise: (a) obtaining a switch intermediate comprising (i) a chimeric antigen receptor-interacting domain (CAR-ID); and (ii) a linker; and (b) contacting the switch intermediate with a target interacting domain (TID), thereby producing the switch. Contacting the switch intermediate with the TID may comprise performing an ester coupling reaction. The linker may comprise a NHS-ester linker. The TID may comprise one or more amino acids. Performing the ester coupling reaction may comprise forming an amide bond between the NHS-ester linker of the switch intermediate and the one or more amino acids of the TID. The method may further comprise producing a plurality of switches. Two or more switches of the plurality of switches may comprise two or more switch intermediates attached to two or more different amino acids of the TID. For example, a first switch intermediate may be attached to a lysine residue of a first TID and a second switch intermediate may be attached to a glycine residue of a second TID. Two or more switches of the plurality of switches may comprise two or more switch intermediates attached to the same amino acid of the TID. For example, the two or more switch intermediates may be attached to a lysine residue of a first and second TID. Two or more switches of the plurality of switches may comprise two or more switch intermediates attached to the same amino acid located at two or more different positions in the TID. For example, a first switch intermediate may be attached to lysine 10 of a first TID and the second switch intermediate may be attached to lysine 45 of a second TID. Two or more switches of the plurality of switches may comprise two or more switch intermediates attached to the same amino acid located at the same position in the TID. For example, a first switch intermediate may be attached to lysine 10 of a first TID and the second switch intermediate may be attached to lysine 10 of a second TID.

Methods of producing a switch disclosed herein may comprise using one or more unnatural amino acids. The method may comprise incorporating one or more unnatural amino acids into the CAR-ID. The CAR-ID may be based on or derived from a polypeptide that can interact with a chimeric antigen receptor on an effector cell. The polypeptide may be a non-antibody based polypeptide. Generally, a non-antibody based polypeptide is a polypeptide that does not comprise an antibody or antibody fragment. The unnatural amino acid may be incorporated into the non-antibody based polypeptide. The unnatural amino acid may replace an amino acid of the non-antibody based polypeptide. Alternatively, or additionally, the method may comprise incorporating one or more unnatural amino acids into the TID. The TID may be based on or derived from a polypeptide. The polypeptide may be an antibody. The polypeptide may be a non-antibody based polypeptide. The unnatural amino acid may be incorporated into the polypeptide. The unnatural amino acid may replace an amino acid of the polypeptide.

The method of producing the switch may further comprise modifying one or more amino acid residues in polypeptide from which the CAR-ID is based or derived. The method of producing the switch may comprise modifying one or more amino acid residues in polypeptide from which the TID is based or derived. Modifying the one or more amino acid residues may comprise mutating one or more nucleotides in the nucleotide sequence encoding the polypeptide. Mutating the one or more nucleotides in the nucleotide sequence encoding may comprise altering a codon encoding an amino acid to a nonsense codon.

Incorporating one or more unnatural amino acids into the polypeptide from which the CAR-ID is based or derived may comprise modifying one or more amino acid residues in the polypeptide to produce one or more amber codons in the antibody or antibody fragment. Incorporating one or more unnatural amino acids into the polypeptide from which the TID is based or derived may comprise modifying one or more amino acid residues in the polypeptide to produce one or more amber codons in the antibody or antibody fragment.

The one or more unnatural amino acids may be incorporated into the polypeptide in response to an amber codon. The one or more unnatural amino acids may be site-specifically incorporated into the polypeptide.

Incorporating one or more unnatural amino acids into the polypeptide from which the CAR-ID and the TID are based or derived may comprise use of one or more genetically encoded unnatural amino acids with orthogonal chemical reactivity relative to the canonical twenty amino acids to site-specifically modify the antibody, antibody fragment, or targeting peptide. Incorporating one or more unnatural amino acids may comprise the use of one or more tRNA synthetases. The tRNA synthetase may be an aminoacyl tRNA synthetase. The tRNA synthetase may be a mutant tRNA synthesis. Incorporating one or more unnatural amino acids may comprise a tRNA/tRNA synthetase pair. The tRNA/tRNA synthetase pair may comprise a tRNA/aminoacyl-tRNA synthetase pair. The tRNA/tRNA synthetase pair may comprise a tRNATyr/tyrosyl-tRNA synthetase pair. Incorporating the one or more unnatural amino acids may comprise use of an evolved tRNA/aminoacyl-tRNA synthetase pair to site-specifically incorporate one or more unnatural amino acids at defined sites in the polypeptide in response to one or more amber nonsense codon.

Additional methods for incorporating unnatural amino acids include, but are not limited to, methods disclosed in Chatterjee et al. (A Versatile Platform for Single- and Multiple-Unnatural Amino Acid Mutagenesis in Escherichia coli, Biochemistry, 2013), Kazane et al. (J Am Chem Soc, 135(1):340-6, 2013), Kim et al. (JAm Chem Soc, 134(24):9918-21, 2012), Johnson et al. (Nat Chem Biol, 7(11):779-86, 2011) and Hutchins et al. (J Mol Biol, 406(4):595-603,2011).

A method of producing a switch for activating a chimeric antigen receptor-effector cell (CAR-EC) may comprise (a) obtaining a target interacting domain (TID) comprising an unnatural amino acid; and (b) attaching a chimeric antigen receptor-interacting domain (CAR-ID) to the TID, thereby producing the switch. The CAR-ID may comprise a small molecule. The CAR-ID may be selected from the group consisting of DOTA, dinitrophenol, quinone, biotin, aniline, atrazine, an aniline-derivative, o-aminobenzoic acid, p-aminobenzoic acid, m-aminobenzoic acid, hydralazine, halothane, digoxigenin, benzene arsonate, lactose, trinitrophenol, biotin or a derivative thereof. The CAR-ID may comprise fluorescein isothiocyanate (FITC). The CAR-ID may comprise biotin. The CAR-ID may comprise dinitrophenol. The TID may comprise a small molecule. The TID may comprise 2-[3-(1,3-dicarboxypropyl)ureido] pentanedioic acid or a derivative thereof. The TID may comprise folate or a derivative thereof. The TID may comprise a polypeptide based on or derived from an antibody or antibody fragment. The antibody may be selected from the group consisting of an anti-CD19 antibody, an anti-CD22 antibody, an anti-CD20 antibody, an anti-EGFR antibody, an anti-EGFRvIII antibody, an anti-Her2 antibody, an anti-CSl antibody, an anti-BCMA antibody, an anti-CEA antibody, an anti-CLL-1 antibody and an anti-CD33 antibody. The antibody may be an anti-CD 19 antibody. The antibody may be an anti-EGFR antibody. The antibody may be an anti-CD20 antibody. The antibody may be an anti-HER2 antibody. The TID may comprise an antibody fragment. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56. The switch may further comprise a linker. The linker may be a bifunctional linker. The linker may be a heterobifunctional linker. The linker may be a homobifunctional linker. The linker may further comprise one or more polyethylene glycol subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The linker may comprise cyclooctyne at one end. The linker may be a PEG-cyclooctyne linker. The linker may comprise triazole. The triazole may be a 1,2,3-triazole or a 1,2,4-triazole. The linker may be a NHS-ester linker. The linker may be a TriA linker. The linker may be a NHS-ester linker.

Attaching the CAR-ID to the TID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the CAR-ID to the TID may comprise a copper free reaction. Attaching the CAR-ID to the TID may comprise one or more copper-containing reactions. Attaching the CAR-ID to the TID may comprise one or more Diels Alder reactions. Attaching the CAR-ID to the TID may comprise one or more hetero Diels Alder reactions. Attaching the CAR-ID to the TID may comprise one or more ester couplings. Attaching the CAR-ID to the TID may comprise one or more isothiocyanate couplings. Attaching the CAR-ID to the TID may comprise attaching the CAR-ID to an amino acid of TID. The amino acid may be an unnatural amino acid. Attaching the CAR-ID to the TID may comprise one or more bioorthogonal reactions. The CAR-ID may be attached to the TID in a site-specific manner. The CAR-ID may be attached to a predetermined site in the TID. The CAR-ID may be attached to the TID in a site-independent manner.

The method may further comprise attaching a first linker to the TID to produce first switch intermediate. Attaching the first linker to the TID may comprise one or cycloadditions. Attaching the first linker to the TID may comprise a copper free reaction. Attaching the first linker to the TID may comprise one or more copper-containing reactions. Attaching the first linker to the TID may comprise one or more Diels Alder reactions. Attaching the first linker to the TID may comprise one or more hetero Diels Alder reactions. Attaching the first linker to the TID may comprise one or more ester couplings. Attaching the first linker to the TID may comprise oxime ligation. Attaching the first linker to the TID may comprise forming one or more oximes between the first linker and the TID. Attaching the first linker to the TID may comprise forming one or more stable bonds between the first linker and the TID. Attaching the first linker to the TID may comprise forming one or more covalent bonds between the first linker and the TID. Attaching the first linker to the TID may comprise forming one or more non-covalent bonds between the first linker and the TID. Attaching the first linker to the TID may comprise forming one or more ionic bonds between the first linker and the TID. Attaching the first linker to the TID may comprise attaching the linker to an amino acid of TID. The amino acid may be an unnatural amino acid. Attaching the first linker to the TID may comprise one or more bioorthogonal reactions.

Attaching the CAR-ID to the TID may comprise attaching the first switch intermediate to the CAR-ID. Attaching the first switch intermediate to the CAR-ID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the first switch intermediate to the CAR-ID may comprise a copper free reaction. Attaching the first switch intermediate to the CAR-ID may comprise one or more copper-containing reactions. Attaching the first switch intermediate to the CAR-ID may comprise one or more Diels Alder reactions. Attaching the first switch intermediate to the CAR-ID may comprise one or more hetero Diels Alder reactions. Attaching the first switch intermediate to the CAR-ID may comprise one or more ester couplings. Attaching the first switch intermediate to the CAR-ID may comprise one or more isothiocyanate couplings.

The method may further comprise attaching a second linker to the CAR-ID to produce a second switch intermediate. Attaching the second linker to the CAR-ID may comprise one or cycloadditions. Attaching the second linker to the CAR-ID may comprise a copper free reaction. Attaching the second linker to the CAR-ID may comprise one or more copper-containing reactions. Attaching the second linker to the CAR-ID may comprise one or more Diels Alder reactions. Attaching the second linker to the CAR-ID may comprise one or more hetero Diels Alder reactions. Attaching the second linker to the CAR-ID may comprise one or more ester couplings. Attaching the second linker to the CAR-ID may comprise oxime ligation. Attaching the second linker to the CAR-ID may comprise forming one or more oximes between the second linker and the CAR-ID. Attaching the second linker to the CAR-ID may comprise forming one or more stable bonds between the second linker and the CAR-ID. Attaching the second linker to the CAR-ID may comprise forming one or more covalent bonds between the second linker and the CAR-ID. Attaching the second linker to the CAR-ID may comprise forming one or more non-covalent bonds between the second linker and the CAR-ID. Attaching the second linker to the CAR-ID may comprise forming one or more ionic bonds between the second linker and the CAR-ID.

Attaching the CAR-ID to the TID may comprise attaching the second switch intermediate to the TID. Attaching the second switch intermediate to the TID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the second switch intermediate to the TID may comprise a copper free reaction. Attaching the second switch intermediate to the TID may comprise one or more copper-containing reactions. Attaching the second switch intermediate to the TID may comprise one or more Diels Alder reactions. Attaching the second switch intermediate to the TID may comprise one or more hetero Diels Alder reactions. Attaching the second switch intermediate to the TID may comprise one or more ester couplings. Attaching the second switch intermediate to the TID may comprise one or more isothiocyanate couplings. Attaching the second switch intermediate to the TID may comprise attaching the linker to an amino acid of CAR-ID. The amino acid may be an unnatural amino acid. Attaching the second switch intermediate to the TID may comprise one or more bioorthogonal reactions.

Attaching the CAR-ID to the TID may comprise attaching the first switch intermediate to the second switch intermediate. Attaching the first switch intermediate to the second switch intermediate may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the first switch intermediate to the second switch intermediate may comprise a copper free reaction. Attaching the first switch intermediate to the second switch intermediate may comprise one or more copper-containing reactions. Attaching the first switch intermediate to the second switch intermediate may comprise one or more Diels Alder reactions. Attaching the first switch intermediate to the second switch intermediate may comprise one or more hetero Diels Alder reactions. Attaching the first switch intermediate to the second switch intermediate may comprise one or more ester couplings. Attaching the first switch intermediate to the second switch intermediate may comprise one or more isothiocyanate couplings.

A method of producing a switch for activating a chimeric antigen receptor-effector cell (CAR-EC) may comprise (a) contacting a chimeric antigen receptor-interacting domain (CAR-ID) with a target interacting domain (TID); and (b) producing the switch by attaching the CAR-ID to a predetermined site on the TID. The CAR-ID may comprise a small molecule. The CAR-ID may be selected from the group consisting of DOTA, dinitrophenol, quinone, biotin, aniline, atrazine, an aniline-derivative, o-aminobenzoic acid, p-aminobenzoic acid, m-aminobenzoic acid, hydralazine, halothane, digoxigenin, benzene arsonate, lactose, trinitrophenol, biotin or a derivative thereof. The CAR-ID may comprise fluorescein isothiocyanate (FITC). The CAR-ID may comprise biotin. The CAR-ID may comprise dinitrophenol. The TID may comprise a small molecule. The TID may comprise 2-[3-(1,3-dicarboxypropyl)ureido] pentanedioic acid or a derivative thereof. The TID may comprise folate or a derivative thereof. The TID may comprise a polypeptide based on or derived from an antibody or antibody fragment. The antibody may be selected from the group consisting of an anti-CD 19 antibody, an anti-CD22 antibody, an anti-CD20 antibody, an anti-EGFR antibody, an anti-EGFRvIII antibody, an anti-Her2 antibody, an anti-CS1 antibody, an anti-BCMA antibody, an anti-CEA antibody, an anti-CLL-1 antibody and an anti-CD33 antibody. The antibody may be an anti-CD 19 antibody. The antibody may be an anti-EGFR antibody. The antibody may be an anti-CD20 antibody. The antibody may be an anti-HER2 antibody. The TID may comprise an antibody fragment. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56. The switch may further comprise a linker. The linker may be a bifunctional linker. The linker may be a heterobifunctional linker. The linker may be a homobifunctional linker. The linker may further comprise one or more polyethylene glycol subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The linker may comprise cyclooctyne at one end. The linker may be a PEG-cyclooctyne linker. The linker may comprise triazole. The triazole may be a 1,2,3-triazole or a 1,2,4-triazole. The linker may be a NHS-ester linker. The linker may be a TriA linker. The linker may be a NHS-ester linker.

Attaching the CAR-ID to the TID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the CAR-ID to the TID may comprise a copper free reaction. Attaching the CAR-ID to the TID may comprise one or more copper-containing reactions. Attaching the CAR-ID to the TID may comprise one or more Diels Alder reactions. Attaching the CAR-ID to the TID may comprise one or more hetero Diels Alder reactions. Attaching the CAR-ID to the TID may comprise one or more ester couplings. Attaching the CAR-ID to the TID may comprise one or more isothiocyanate couplings. Attaching the CAR-ID to the TID may comprise attaching the CAR-ID to an amino acid of TID. The amino acid may be an unnatural amino acid. Attaching the CAR-ID to the TID may comprise one or more bioorthogonal reactions. The CAR-ID may be attached to the TID in a site-specific manner. The CAR-ID may be attached to a predetermined site in the TID. The CAR-ID may be attached to the TID in a site-independent manner.

The method may further comprise attaching a first linker to the TID to produce first switch intermediate. Attaching the first linker to the TID may comprise one or cycloadditions. Attaching the first linker to the TID may comprise a copper free reaction. Attaching the first linker to the TID may comprise one or more copper-containing reactions. Attaching the first linker to the TID may comprise one or more Diels Alder reactions. Attaching the first linker to the TID may comprise one or more hetero Diels Alder reactions. Attaching the first linker to the TID may comprise one or more ester couplings. Attaching the first linker to the TID may comprise oxime ligation. Attaching the first linker to the TID may comprise forming one or more oximes between the first linker and the TID. Attaching the first linker to the TID may comprise forming one or more stable bonds between the first linker and the TID. Attaching the first linker to the TID may comprise forming one or more covalent bonds between the first linker and the TID. Attaching the first linker to the TID may comprise forming one or more non-covalent bonds between the first linker and the TID. Attaching the first linker to the TID may comprise forming one or more ionic bonds between the first linker and the TID. Attaching the first linker to the TID may comprise attaching the linker to an amino acid of TID. The amino acid may be an unnatural amino acid. Attaching the first linker to the TID may comprise one or more bioorthogonal reactions.

Attaching the CAR-ID to the TID may comprise attaching the first switch intermediate to the CAR-ID. Attaching the first switch intermediate to the CAR-ID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the first switch intermediate to the CAR-ID may comprise a copper free reaction. Attaching the first switch intermediate to the CAR-ID may comprise one or more copper-containing reactions. Attaching the first switch intermediate to the CAR-ID may comprise one or more Diels Alder reactions. Attaching the first switch intermediate to the CAR-ID may comprise one or more hetero Diels Alder reactions. Attaching the first switch intermediate to the CAR-ID may comprise one or more ester couplings. Attaching the first switch intermediate to the CAR-ID may comprise one or more isothiocyanate couplings.

The method may further comprise attaching a second linker to the CAR-ID to produce a second switch intermediate. Attaching the second linker to the CAR-ID may comprise one or cycloadditions. Attaching the second linker to the CAR-ID may comprise a copper free reaction. Attaching the second linker to the CAR-ID may comprise one or more copper-containing reactions. Attaching the second linker to the CAR-ID may comprise one or more Diels Alder reactions. Attaching the second linker to the CAR-ID may comprise one or more hetero Diels Alder reactions. Attaching the second linker to the CAR-ID may comprise one or more ester couplings. Attaching the second linker to the CAR-ID may comprise oxime ligation. Attaching the second linker to the CAR-ID may comprise forming one or more oximes between the second linker and the CAR-ID. Attaching the second linker to the CAR-ID may comprise forming one or more stable bonds between the second linker and the CAR-ID. Attaching the second linker to the CAR-ID may comprise forming one or more covalent bonds between the second linker and the CAR-ID. Attaching the second linker to the CAR-ID may comprise forming one or more non-covalent bonds between the second linker and the CAR-ID. Attaching the second linker to the CAR-ID may comprise forming one or more ionic bonds between the second linker and the CAR-ID.

Attaching the CAR-ID to the TID may comprise attaching the second switch intermediate to the TID. Attaching the second switch intermediate to the TID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the second switch intermediate to the TID may comprise a copper free reaction. Attaching the second switch intermediate to the TID may comprise one or more copper-containing reactions. Attaching the second switch intermediate to the TID may comprise one or more Diels Alder reactions. Attaching the second switch intermediate to the TID may comprise one or more hetero Diels Alder reactions. Attaching the second switch intermediate to the TID may comprise one or more ester couplings. Attaching the second switch intermediate to the TID may comprise one or more isothiocyanate couplings. Attaching the second switch intermediate to the TID may comprise attaching the linker to an amino acid of CAR-ID. The amino acid may be an unnatural amino acid. Attaching the second switch intermediate to the TID may comprise one or more bioorthogonal reactions.

Attaching the CAR-ID to the TID may comprise attaching the first switch intermediate to the second switch intermediate. Attaching the first switch intermediate to the second switch intermediate may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the first switch intermediate to the second switch intermediate may comprise a copper free reaction. Attaching the first switch intermediate to the second switch intermediate may comprise one or more copper-containing reactions. Attaching the first switch intermediate to the second switch intermediate may comprise one or more Diels Alder reactions. Attaching the first switch intermediate to the second switch intermediate may comprise one or more hetero Diels Alder reactions. Attaching the first switch intermediate to the second switch intermediate may comprise one or more ester couplings. Attaching the first switch intermediate to the second switch intermediate may comprise one or more isothiocyanate couplings.

A method of producing a switch for activating a chimeric antigen receptor-effector cell (CAR-EC) may comprise (a) contacting a plurality of chimeric antigen receptor-interacting domains (CAR-IDs) with a plurality of target interacting domains (TIDs); and (b) attaching one or more CAR-IDs of the plurality of CAR-IDs to one or more TIDs of the plurality of TIDs, thereby producing a plurality of switches, wherein at least about 60% of the switches are structurally homologous. At least about 65%, 70%, 72%, 75%, 77%, 80%, 82%, 85%, 87%, 90%, 92%, 95%, 97%, 99% or 100% of the switches may be structurally homologous. At least about 70% of the switches may be structurally homologous. At least about 75% of the switches may be structurally homologous. At least about 80% of the switches may be structurally homologous. At least about 85% of the switches may be structurally homologous. At least about 90% of the switches may be structurally homologous. At least about 95% of the switches may be structurally homologous. The CAR-ID may comprise a small molecule. The CAR-ID may be selected from the group consisting of DOTA, dinitrophenol, quinone, biotin, aniline, atrazine, an aniline-derivative, o-aminobenzoic acid, p-aminobenzoic acid, m-aminobenzoic acid, hydralazine, halothane, digoxigenin, benzene arsonate, lactose, trinitrophenol, biotin or a derivative thereof. The CAR-ID may comprise fluorescein isothiocyanate (FITC). The CAR-ID may comprise biotin. The CAR-ID may comprise dinitrophenol. The TID may comprise a small molecule. The TID may comprise 2-[3-(1,3-dicarboxypropyl)ureido] pentanedioic acid or a derivative thereof. The TID may comprise folate or a derivative thereof. The TID may comprise a polypeptide based on or derived from an antibody or antibody fragment. The antibody may be selected from the group consisting of an anti-CD 19 antibody, an anti-CD22 antibody, an anti-CD20 antibody, an anti-EGFR antibody, an anti-EGFRvIII antibody, an anti-Her2 antibody, an anti-CS1 antibody, an anti-BCMA antibody, an anti-CEA antibody, an anti-CLL-1 antibody and an anti-CD33 antibody. The antibody may be an anti-CD 19 antibody. The antibody may be an anti-EGFR antibody. The antibody may be an anti-CD20 antibody. The antibody may be an anti-HER2 antibody. The TID may comprise an antibody fragment. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56. The switch may further comprise a linker. The linker may be a bifunctional linker. The linker may be a heterobifunctional linker. The linker may be a homobifunctional linker. The linker may further comprise one or more polyethylene glycol subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The linker may comprise cyclooctyne at one end. The linker may be a PEG-cyclooctyne linker. The linker may comprise triazole. The triazole may be a 1,2,3-triazole or a 1,2,4-triazole. The linker may be a NHS-ester linker. The linker may be a TriA linker. The linker may be a NHS-ester linker.

Attaching the CAR-ID to the TID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the CAR-ID to the TID may comprise a copper free reaction. Attaching the CAR-ID to the TID may comprise one or more copper-containing reactions. Attaching the CAR-ID to the TID may comprise one or more Diels Alder reactions. Attaching the CAR-ID to the TID may comprise one or more hetero Diels Alder reactions. Attaching the CAR-ID to the TID may comprise one or more ester couplings. Attaching the CAR-ID to the TID may comprise one or more isothiocyanate couplings. Attaching the CAR-ID to the TID may comprise attaching the CAR-ID to an amino acid of TID. The amino acid may be an unnatural amino acid. Attaching the CAR-ID to the TID may comprise one or more bioorthogonal reactions. The CAR-ID may be attached to the TID in a site-specific manner. The CAR-ID may be attached to a predetermined site in the TID. The CAR-ID may be attached to the TID in a site-independent manner.

The method may further comprise attaching a first linker to the TID to produce first switch intermediate. Attaching the first linker to the TID may comprise one or cycloadditions. Attaching the first linker to the TID may comprise a copper free reaction. Attaching the first linker to the TID may comprise one or more copper-containing reactions. Attaching the first linker to the TID may comprise one or more Diels Alder reactions. Attaching the first linker to the TID may comprise one or more hetero Diels Alder reactions. Attaching the first linker to the TID may comprise one or more ester couplings. Attaching the first linker to the TID may comprise oxime ligation. Attaching the first linker to the TID may comprise forming one or more oximes between the first linker and the TID. Attaching the first linker to the TID may comprise forming one or more stable bonds between the first linker and the TID. Attaching the first linker to the TID may comprise forming one or more covalent bonds between the first linker and the TID. Attaching the first linker to the TID may comprise forming one or more non-covalent bonds between the first linker and the TID. Attaching the first linker to the TID may comprise forming one or more ionic bonds between the first linker and the TID. Attaching the first linker to the TID may comprise attaching the linker to an amino acid of TID. The amino acid may be an unnatural amino acid. Attaching the first linker to the TID may comprise one or more bioorthogonal reactions.

Attaching the CAR-ID to the TID may comprise attaching the first switch intermediate to the CAR-ID. Attaching the first switch intermediate to the CAR-ID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the first switch intermediate to the CAR-ID may comprise a copper free reaction. Attaching the first switch intermediate to the CAR-ID may comprise one or more copper-containing reactions. Attaching the first switch intermediate to the CAR-ID may comprise one or more Diels Alder reactions. Attaching the first switch intermediate to the CAR-ID may comprise one or more hetero Diels Alder reactions. Attaching the first switch intermediate to the CAR-ID may comprise one or more ester couplings. Attaching the first switch intermediate to the CAR-ID may comprise one or more isothiocyanate couplings.

The method may further comprise attaching a second linker to the CAR-ID to produce a second switch intermediate. Attaching the second linker to the CAR-ID may comprise one or cycloadditions. Attaching the second linker to the CAR-ID may comprise a copper free reaction. Attaching the second linker to the CAR-ID may comprise one or more copper-containing reactions. Attaching the second linker to the CAR-ID may comprise one or more Diels Alder reactions. Attaching the second linker to the CAR-ID may comprise one or more hetero Diels Alder reactions. Attaching the second linker to the CAR-ID may comprise one or more ester couplings. Attaching the second linker to the CAR-ID may comprise oxime ligation. Attaching the second linker to the CAR-ID may comprise forming one or more oximes between the second linker and the CAR-ID. Attaching the second linker to the CAR-ID may comprise forming one or more stable bonds between the second linker and the CAR-ID. Attaching the second linker to the CAR-ID may comprise forming one or more covalent bonds between the second linker and the CAR-ID. Attaching the second linker to the CAR-ID may comprise forming one or more non-covalent bonds between the second linker and the CAR-ID. Attaching the second linker to the CAR-ID may comprise forming one or more ionic bonds between the second linker and the CAR-ID.

Attaching the CAR-ID to the TID may comprise attaching the second switch intermediate to the TID. Attaching the second switch intermediate to the TID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the second switch intermediate to the TID may comprise a copper free reaction. Attaching the second switch intermediate to the TID may comprise one or more copper-containing reactions. Attaching the second switch intermediate to the TID may comprise one or more Diels Alder reactions. Attaching the second switch intermediate to the TID may comprise one or more hetero Diels Alder reactions. Attaching the second switch intermediate to the TID may comprise one or more ester couplings. Attaching the second switch intermediate to the TID may comprise one or more isothiocyanate couplings. Attaching the second switch intermediate to the TID may comprise attaching the linker to an amino acid of CAR-ID. The amino acid may be an unnatural amino acid. Attaching the second switch intermediate to the TID may comprise one or more bioorthogonal reactions.

Attaching the CAR-ID to the TID may comprise attaching the first switch intermediate to the second switch intermediate. Attaching the first switch intermediate to the second switch intermediate may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the first switch intermediate to the second switch intermediate may comprise a copper free reaction. Attaching the first switch intermediate to the second switch intermediate may comprise one or more copper-containing reactions. Attaching the first switch intermediate to the second switch intermediate may comprise one or more Diels Alder reactions. Attaching the first switch intermediate to the second switch intermediate may comprise one or more hetero Diels Alder reactions. Attaching the first switch intermediate to the second switch intermediate may comprise one or more ester couplings. Attaching the first switch intermediate to the second switch intermediate may comprise one or more isothiocyanate couplings.

A method of producing a switch for activating a chimeric antigen receptor-effector cell (CAR-EC) may comprise (a) contacting a plurality of chimeric antigen receptor-interacting domains (CAR-IDs) with a plurality of target interacting domains (TIDs); and (b) attaching a CAR-ID of the plurality of CAR-IDs to a TID of the plurality of TIDs, thereby producing a plurality of switches, wherein the CAR-ID is attached to the same amino acid residue of the TID in at least about 60% of the switches. The CAR-ID may be attached to the same amino acid residue of the TID in at least about 62%, 65%, 67%, 70%, 72%, 75%, 77%, 80%, 82%, 85%, 87%, 90%, 92%, 95%, 97%, 99% or 100% of the switches. The CAR-ID may be attached to the same amino acid residue of the TID in at least about 70% of the switches. The CAR-ID may be attached to the same amino acid residue of the TID in at least about 75% of the switches. The CAR-ID may be attached to the same amino acid residue of the TID in at least about 80% of the switches. The CAR-ID may be attached to the same amino acid residue of the TID in at least about 85% of the switches. The CAR-ID may be attached to the same amino acid residue of the TID in at least about 90% of the switches.

The CAR-ID may comprise a small molecule. The CAR-ID may be selected from the group consisting of DOTA, dinitrophenol, quinone, biotin, aniline, atrazine, an aniline-derivative, o-aminobenzoic acid, p-aminobenzoic acid, m-aminobenzoic acid, hydralazine, halothane, digoxigenin, benzene arsonate, lactose, trinitrophenol, biotin or a derivative thereof. The CAR-ID may comprise fluorescein isothiocyanate (FITC). The CAR-ID may comprise biotin. The CAR-ID may comprise dinitrophenol. The TID may comprise a small molecule. The TID may comprise 2-[3-(1,3-dicarboxypropyl)ureido] pentanedioic acid or a derivative thereof. The TID may comprise folate or a derivative thereof. The TID may comprise a polypeptide based on or derived from an antibody or antibody fragment. The antibody may be selected from the group consisting of an anti-CD 19 antibody, an anti-CD22 antibody, an anti-CD20 antibody, an anti-EGFR antibody, an anti-EGFRvIII antibody, an anti-Her2 antibody, an anti-CS1 antibody, an anti-BCMA antibody, an anti-CEA antibody, an anti-CLL-1 antibody and an anti-CD33 antibody. The antibody may be an anti-CD 19 antibody. The antibody may be an anti-EGFR antibody. The antibody may be an anti-CD20 antibody. The antibody may be an anti-HER2 antibody. The TID may comprise an antibody fragment. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56. The switch may further comprise a linker. The linker may be a bifunctional linker. The linker may be a heterobifunctional linker. The linker may be a homobifunctional linker. The linker may further comprise one or more polyethylene glycol subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The linker may comprise cyclooctyne at one end. The linker may be a PEG-cyclooctyne linker. The linker may comprise triazole. The triazole may be a 1,2,3-triazole or a 1,2,4-triazole. The linker may be a NHS-ester linker. The linker may be a TriA linker. The linker may be a NHS-ester linker.

Attaching the CAR-ID to the TID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the CAR-ID to the TID may comprise a copper free reaction. Attaching the CAR-ID to the TID may comprise one or more copper-containing reactions. Attaching the CAR-ID to the TID may comprise one or more Diels Alder reactions. Attaching the CAR-ID to the TID may comprise one or more hetero Diels Alder reactions. Attaching the CAR-ID to the TID may comprise one or more ester couplings. Attaching the CAR-ID to the TID may comprise one or more isothiocyanate couplings. Attaching the CAR-ID to the TID may comprise attaching the CAR-ID to an amino acid of TID. The amino acid may be an unnatural amino acid. Attaching the CAR-ID to the TID may comprise one or more bioorthogonal reactions. The CAR-ID may be attached to the TID in a site-specific manner. The CAR-ID may be attached to a predetermined site in the TID. The CAR-ID may be attached to the TID in a site-independent manner.

The method may further comprise attaching a first linker to the TID to produce first switch intermediate. Attaching the first linker to the TID may comprise one or cycloadditions. Attaching the first linker to the TID may comprise a copper free reaction. Attaching the first linker to the TID may comprise one or more copper-containing reactions. Attaching the first linker to the TID may comprise one or more Diels Alder reactions. Attaching the first linker to the TID may comprise one or more hetero Diels Alder reactions. Attaching the first linker to the TID may comprise one or more ester couplings. Attaching the first linker to the TID may comprise oxime ligation. Attaching the first linker to the TID may comprise forming one or more oximes between the first linker and the TID. Attaching the first linker to the TID may comprise forming one or more stable bonds between the first linker and the TID. Attaching the first linker to the TID may comprise forming one or more covalent bonds between the first linker and the TID. Attaching the first linker to the TID may comprise forming one or more non-covalent bonds between the first linker and the TID. Attaching the first linker to the TID may comprise forming one or more ionic bonds between the first linker and the TID. Attaching the first linker to the TID may comprise attaching the linker to an amino acid of TID. The amino acid may be an unnatural amino acid. Attaching the first linker to the TID may comprise one or more bioorthogonal reactions.

Attaching the CAR-ID to the TID may comprise attaching the first switch intermediate to the CAR-ID. Attaching the first switch intermediate to the CAR-ID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the first switch intermediate to the CAR-ID may comprise a copper free reaction. Attaching the first switch intermediate to the CAR-ID may comprise one or more copper-containing reactions. Attaching the first switch intermediate to the CAR-ID may comprise one or more Diels Alder reactions. Attaching the first switch intermediate to the CAR-ID may comprise one or more hetero Diels Alder reactions. Attaching the first switch intermediate to the CAR-ID may comprise one or more ester couplings. Attaching the first switch intermediate to the CAR-ID may comprise one or more isothiocyanate couplings.

The method may further comprise attaching a second linker to the CAR-ID to produce a second switch intermediate. Attaching the second linker to the CAR-ID may comprise one or cycloadditions. Attaching the second linker to the CAR-ID may comprise a copper free reaction. Attaching the second linker to the CAR-ID may comprise one or more copper-containing reactions. Attaching the second linker to the CAR-ID may comprise one or more Diels Alder reactions. Attaching the second linker to the CAR-ID may comprise one or more hetero Diels Alder reactions. Attaching the second linker to the CAR-ID may comprise one or more ester couplings. Attaching the second linker to the CAR-ID may comprise oxime ligation. Attaching the second linker to the CAR-ID may comprise forming one or more oximes between the second linker and the CAR-ID. Attaching the second linker to the CAR-ID may comprise forming one or more stable bonds between the second linker and the CAR-ID. Attaching the second linker to the CAR-ID may comprise forming one or more covalent bonds between the second linker and the CAR-ID. Attaching the second linker to the CAR-ID may comprise forming one or more non-covalent bonds between the second linker and the CAR-ID. Attaching the second linker to the CAR-ID may comprise forming one or more ionic bonds between the second linker and the CAR-ID.

Attaching the CAR-ID to the TID may comprise attaching the second switch intermediate to the TID. Attaching the second switch intermediate to the TID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the second switch intermediate to the TID may comprise a copper free reaction. Attaching the second switch intermediate to the TID may comprise one or more copper-containing reactions. Attaching the second switch intermediate to the TID may comprise one or more Diels Alder reactions. Attaching the second switch intermediate to the TID may comprise one or more hetero Diels Alder reactions. Attaching the second switch intermediate to the TID may comprise one or more ester couplings. Attaching the second switch intermediate to the TID may comprise one or more isothiocyanate couplings. Attaching the second switch intermediate to the TID may comprise attaching the linker to an amino acid of CAR-ID. The amino acid may be an unnatural amino acid. Attaching the second switch intermediate to the TID may comprise one or more bioorthogonal reactions.

Attaching the CAR-ID to the TID may comprise attaching the first switch intermediate to the second switch intermediate. Attaching the first switch intermediate to the second switch intermediate may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the first switch intermediate to the second switch intermediate may comprise a copper free reaction. Attaching the first switch intermediate to the second switch intermediate may comprise one or more copper-containing reactions. Attaching the first switch intermediate to the second switch intermediate may comprise one or more Diels Alder reactions. Attaching the first switch intermediate to the second switch intermediate may comprise one or more hetero Diels Alder reactions. Attaching the first switch intermediate to the second switch intermediate may comprise one or more ester couplings. Attaching the first switch intermediate to the second switch intermediate may comprise one or more isothiocyanate couplings.

A method of producing a switch for activating a chimeric antigen receptor-effector cell (CAR-EC) may comprise (a) contacting a chimeric antigen receptor-interacting domain (CAR-ID) with a target interacting domain (TID); and (b) attaching the CAR-ID to the TID by oxime conjugation, Huisgen cycloaddition, or Diels Alder reaction, thereby producing the switch. The CAR-ID may comprise a small molecule. The CAR-ID may be selected from the group consisting of DOTA, dinitrophenol, quinone, biotin, aniline, atrazine, an aniline-derivative, o-aminobenzoic acid, p-aminobenzoic acid, m-aminobenzoic acid, hydralazine, halothane, digoxigenin, benzene arsonate, lactose, trinitrophenol, biotin or a derivative thereof. The CAR-ID may comprise fluorescein isothiocyanate (FITC). The CAR-ID may comprise biotin. The CAR-ID may comprise dinitrophenol. The TID may comprise a small molecule. The TID may comprise 2-[3-(1,3-dicarboxypropyl)ureido] pentanedioic acid or a derivative thereof. The TID may comprise folate or a derivative thereof. The TID may comprise a polypeptide based on or derived from an antibody or antibody fragment. The antibody may be selected from the group consisting of an anti-CD 19 antibody, an anti-CD22 antibody, an anti-CD20 antibody, an anti-EGFR antibody, an anti-EGFRvIII antibody, an anti-Her2 antibody, an anti-CSl antibody, an anti-BCMA antibody, an anti-CEA antibody, an anti-CLL-1 antibody and an anti-CD33 antibody. The antibody may be an anti-CD 19 antibody. The antibody may be an anti-EGFR antibody. The antibody may be an anti-CD20 antibody. The antibody may be an anti-HER2 antibody. The TID may comprise an antibody fragment. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56. The switch may further comprise a linker. The linker may be a bifunctional linker. The linker may be a heterobifunctional linker. The linker may be a homobifunctional linker. The linker may further comprise one or more polyethylene glycol subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The linker may comprise cyclooctyne at one end. The linker may be a PEG-cyclooctyne linker. The linker may comprise triazole. The triazole may be a 1,2,3-triazole or a 1,2,4-triazole. The linker may be a NHS-ester linker. The linker may be a TriA linker. The linker may be a NHS-ester linker.

Attaching the CAR-ID to the TID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the CAR-ID to the TID may comprise a copper free reaction. Attaching the CAR-ID to the TID may comprise one or more copper-containing reactions. Attaching the CAR-ID to the TID may comprise one or more Diels Alder reactions. Attaching the CAR-ID to the TID may comprise one or more hetero Diels Alder reactions. Attaching the CAR-ID to the TID may comprise one or more ester couplings. Attaching the CAR-ID to the TID may comprise one or more isothiocyanate couplings. Attaching the CAR-ID to the TID may comprise attaching the CAR-ID to an amino acid of TID. The amino acid may be an unnatural amino acid. Attaching the CAR-ID to the TID may comprise one or more bioorthogonal reactions. The CAR-ID may be attached to the TID in a site-specific manner. The CAR-ID may be attached to a predetermined site in the TID. The CAR-ID may be attached to the TID in a site-independent manner.

The method may further comprise attaching a first linker to the TID to produce first switch intermediate. Attaching the first linker to the TID may comprise one or cycloadditions. Attaching the first linker to the TID may comprise a copper free reaction. Attaching the first linker to the TID may comprise one or more copper-containing reactions. Attaching the first linker to the TID may comprise one or more Diels Alder reactions. Attaching the first linker to the TID may comprise one or more hetero Diels Alder reactions. Attaching the first linker to the TID may comprise one or more ester couplings. Attaching the first linker to the TID may comprise oxime ligation. Attaching the first linker to the TID may comprise forming one or more oximes between the first linker and the TID. Attaching the first linker to the TID may comprise forming one or more stable bonds between the first linker and the TID. Attaching the first linker to the TID may comprise forming one or more covalent bonds between the first linker and the TID. Attaching the first linker to the TID may comprise forming one or more non-covalent bonds between the first linker and the TID. Attaching the first linker to the TID may comprise forming one or more ionic bonds between the first linker and the TID. Attaching the first linker to the TID may comprise attaching the linker to an amino acid of TID. The amino acid may be an unnatural amino acid. Attaching the first linker to the TID may comprise one or more bioorthogonal reactions.

Attaching the CAR-ID to the TID may comprise attaching the first switch intermediate to the CAR-ID. Attaching the first switch intermediate to the CAR-ID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the first switch intermediate to the CAR-ID may comprise a copper free reaction. Attaching the first switch intermediate to the CAR-ID may comprise one or more copper-containing reactions. Attaching the first switch intermediate to the CAR-ID may comprise one or more Diels Alder reactions. Attaching the first switch intermediate to the CAR-ID may comprise one or more hetero Diels Alder reactions. Attaching the first switch intermediate to the CAR-ID may comprise one or more ester couplings. Attaching the first switch intermediate to the CAR-ID may comprise one or more isothiocyanate couplings.

The method may further comprise attaching a second linker to the CAR-ID to produce a second switch intermediate. Attaching the second linker to the CAR-ID may comprise one or cycloadditions. Attaching the second linker to the CAR-ID may comprise a copper free reaction. Attaching the second linker to the CAR-ID may comprise one or more copper-containing reactions. Attaching the second linker to the CAR-ID may comprise one or more Diels Alder reactions. Attaching the second linker to the CAR-ID may comprise one or more hetero Diels Alder reactions. Attaching the second linker to the CAR-ID may comprise one or more ester couplings. Attaching the second linker to the CAR-ID may comprise oxime ligation. Attaching the second linker to the CAR-ID may comprise forming one or more oximes between the second linker and the CAR-ID. Attaching the second linker to the CAR-ID may comprise forming one or more stable bonds between the second linker and the CAR-ID. Attaching the second linker to the CAR-ID may comprise forming one or more covalent bonds between the second linker and the CAR-ID. Attaching the second linker to the CAR-ID may comprise forming one or more non-covalent bonds between the second linker and the CAR-ID. Attaching the second linker to the CAR-ID may comprise forming one or more ionic bonds between the second linker and the CAR-ID.

Attaching the CAR-ID to the TID may comprise attaching the second switch intermediate to the TID. Attaching the second switch intermediate to the TID may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the second switch intermediate to the TID may comprise a copper free reaction. Attaching the second switch intermediate to the TID may comprise one or more copper-containing reactions. Attaching the second switch intermediate to the TID may comprise one or more Diels Alder reactions. Attaching the second switch intermediate to the TID may comprise one or more hetero Diels Alder reactions. Attaching the second switch intermediate to the TID may comprise one or more ester couplings. Attaching the second switch intermediate to the TID may comprise one or more isothiocyanate couplings. Attaching the second switch intermediate to the TID may comprise attaching the linker to an amino acid of CAR-ID. The amino acid may be an unnatural amino acid. Attaching the second switch intermediate to the TID may comprise one or more bioorthogonal reactions.

Attaching the CAR-ID to the TID may comprise attaching the first switch intermediate to the second switch intermediate. Attaching the first switch intermediate to the second switch intermediate may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Attaching the first switch intermediate to the second switch intermediate may comprise a copper free reaction. Attaching the first switch intermediate to the second switch intermediate may comprise one or more copper-containing reactions. Attaching the first switch intermediate to the second switch intermediate may comprise one or more Diels Alder reactions. Attaching the first switch intermediate to the second switch intermediate may comprise one or more hetero Diels Alder reactions. Attaching the first switch intermediate to the second switch intermediate may comprise one or more ester couplings. Attaching the first switch intermediate to the second switch intermediate may comprise one or more isothiocyanate couplings.

A method of producing a switch intermediate for activating a chimeric antigen receptor-effector cell (CAR-EC) may comprise (b) contacting a target interacting domain (TID) with a linker, the linker comprising an aminooxy group, azide group and/or cyclooctyne group at one or more termini; and (c) attaching the linker to the TID. The TID may comprise a polypeptide based on or derived from an antibody or antibody fragment. The antibody may be selected from the group consisting of an anti-CD 19 antibody, an anti-CD22 antibody, an anti-CD20 antibody, an anti-EGFR antibody, an anti-EGFRvIII antibody, an anti-Her2 antibody, an anti-CS1 antibody, an anti-BCMA antibody, an anti-CEA antibody, an anti-CLL-1 antibody and an anti-CD33 antibody. The antibody may be an anti-CD 19 antibody. The antibody may be an anti-EGFR antibody. The antibody may be an anti-CD20 antibody. The antibody may be an anti-HER2 antibody. The TID may comprise an antibody fragment. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56. The TID may comprise one or more unnatural amino acids. Attaching the linker to the TID may comprise forming an oxime. The oxime may be formed between a ketone of an amino acid on the TID and the aminooxy group on the linker. The oxime may be formed between an alkoxy-amine group of the linker and a ketone of the unnatural amino acid. The oxime may be formed between an alkoxy-amine group of the linker and a ketone of the unnatural amino acid, wherein the unnatural amino acid is p-acetylphenylalanine (pAcF). The linker may be a bifunctional linker. The linker may be a heterobifunctional linker. The linker may be a homobifunctional linker. The linker may comprise an aminooxy group, azide group cyclooctyne group, or a combination thereof at one or more termini. The linker may comprise one or more polyethylene glycol subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The linker may comprise cyclooctyne. The linker may be a PEG-cyclooctyne linker. The linker may comprise triazole. The triazole may be a 1,2,3-triazole or a 1,2,4-triazole. The linker may be a NHS-ester linker. The linker may be a TriA linker. The linker may be a NHS-ester linker.

Attaching the linker to the TID may comprise one or cycloadditions. Attaching the linker to the TID may comprise a copper free reaction. Attaching the linker to the TID may comprise one or more copper-containing reactions. Attaching the linker to the TID may comprise one or more Diels Alder reactions. Attaching the linker to the TID may comprise one or more hetero Diels Alder reactions. Attaching the linker to the TID may comprise one or more ester couplings. Attaching the linker to the TID may comprise oxime ligation. Attaching the linker to the TID may comprise forming one or more oximes between the linker and the TID. Attaching the linker to the TID may comprise forming one or more stable bonds between the linker and the TID. Attaching the linker to the TID may comprise forming one or more covalent bonds between the linker and the TID. Attaching the linker to the TID may comprise forming one or more non-covalent bonds between the linker and the TID. Attaching the linker to the TID may comprise forming one or more ionic bonds between the linker and the TID. Attaching the linker to the TID may comprise attaching the linker to an amino acid of TID. The amino acid may be an unnatural amino acid. Attaching the linker to the TID may comprise one or more bioorthogonal reactions.

A method of producing a switch intermediate for activating a chimeric antigen receptor-effector cell (CAR-EC) may comprise (a) contacting a chimeric antigen receptor-interacting domain (CAR-ID) with a linker, the linker comprising an aminooxy group, azide group and/or cyclooctyne group at one or more termini; and (b) attaching the linker to the CAR-ID. The CAR-ID may comprise a small molecule. The CAR-ID may be selected from the group consisting of DOTA, dinitrophenol, quinone, biotin, aniline, atrazine, an aniline-derivative, o-aminobenzoic acid, p-aminobenzoic acid, m-aminobenzoic acid, hydralazine, halothane, digoxigenin, benzene arsonate, lactose, trinitrophenol, biotin or a derivative thereof. The CAR-ID may comprise fluorescein isothiocyanate (FITC). The CAR-ID may comprise biotin. The CAR-ID may comprise dinitrophenol. The linker may be a bifunctional linker. The linker may be a heterobifunctional linker. The linker may be a homobifunctional linker. The linker may further comprise one or more polyethylene glycol subunits. The linker may comprise at least four PEG subunits. The linker may comprise at least 10 PEG subunits. The linker may comprise at least 20 PEG subunits. The linker may comprise at least 30 PEG subunits. The linker may comprise an azide at one end. The linker may comprise an aminooxy at one end. The linker may be an azide-PEG-aminooxy linker. The linker may comprise cyclooctyne at one end. The linker may be a PEG-cyclooctyne linker. The linker may be attached to the CAR-ID by oxime ligation.

Attaching the linker to the CAR-ID may comprise one or cycloadditions. Attaching the linker to the CAR-ID may comprise a copper free reaction. Attaching the linker to the CAR-ID may comprise one or more copper-containing reactions. Attaching the linker to the CAR-ID may comprise one or more Diels Alder reactions. Attaching the linker to the CAR-ID may comprise one or more hetero Diels Alder reactions. Attaching the linker to the CAR-ID may comprise one or more ester couplings. Attaching the linker to the CAR-ID may comprise oxime ligation. Attaching the linker to the CAR-ID may comprise forming one or more oximes between the linker and the CAR-ID. Attaching the linker to the CAR-ID may comprise forming one or more stable bonds between the linker and the CAR-ID. Attaching the linker to the CAR-ID may comprise forming one or more covalent bonds between the linker and the CAR-ID. Attaching the linker to the CAR-ID may comprise forming one or more non-covalent bonds between the linker and the CAR-ID. Attaching the linker to the CAR-ID may comprise forming one or more ionic bonds between the linker and the CAR-ID. Attaching the linker to the CAR-ID may comprise attaching the linker to an amino acid of CAR-ID. The amino acid may be an unnatural amino acid. Attaching the linker to the CAR-ID may comprise one or more bioorthogonal reactions.

A method of producing a switch of Formula IV: X- L1-L2-Y or Formula IVA: Y-L2-L1-X may comprise (a) coupling L1 to X to produce a first switch intermediate of Formula IIA: L1-X, wherein (i) X comprises a chimeric antigen receptor-interacting domain (CAR-ID) that interacts with a chimeric antigen receptor on an effector cell; and (ii) L1 comprises a first linker before being coupled to X; (b) coupling L2 to Y to produce a second switch intermediate of Formula VA: Y-L2, wherein (i) Y comprises a target interacting domain (TID) that interacts with a surface molecule on a target cell; and (ii) L2 comprises a second linker before being coupled to X; and (c) linking the first switch intermediate to the second switch intermediate, thereby producing the switch of Formula IV or IVA. The CAR-ID may comprise a small molecule. The CAR-ID may be selected from the group consisting of DOTA, dinitrophenol, quinone, biotin, aniline, atrazine, an aniline-derivative, o-aminobenzoic acid, p-aminobenzoic acid, m-aminobenzoic acid, hydralazine, halothane, digoxigenin, benzene arsonate, lactose, trinitrophenol, biotin or a derivative thereof. The CAR-ID may comprise fluorescein isothiocyanate (FITC). The CAR-ID may comprise biotin. The CAR-ID may comprise dinitrophenol.

The first linker may be a bifunctional linker. The first linker may be a heterobifunctional linker. The first linker may be a homobifunctional linker. The first linker may further comprise one or more polyethylene glycol subunits. The first linker may comprise at least four PEG subunits. The first linker may comprise at least 10 PEG subunits. The first linker may comprise at least 20 PEG subunits. The first linker may comprise at least 30 PEG subunits. The first linker may comprise an azide at one end. The first linker may comprise an aminooxy at one end. The first linker may be an azide-PEG-aminooxy linker. The first linker may comprise cyclooctyne at one end. The first linker may be a PEG-cyclooctyne linker.

The TID may comprise a small molecule. The TID may comprise 2-[3-(1,3-dicarboxypropyl)ureido] pentanedioic acid or a derivative thereof. The TID may comprise folate or a derivative thereof. The TID may comprise a polypeptide based on or derived from an antibody or antibody fragment. The antibody may be selected from the group consisting of an anti-CD19 antibody, an anti-CD22 antibody, an anti-CD20 antibody, an anti-EGFR antibody, an anti-EGFRvIII antibody, an anti-Her2 antibody, an anti-CS1 antibody, an anti-BCMA antibody, an anti-CEA antibody, an anti-CLL-1 antibody and an anti-CD33 antibody. The antibody may be an anti-CD 19 antibody. The antibody may be an anti-EGFR antibody. The antibody may be an anti-CD20 antibody. The antibody may be an anti-HER2 antibody. The TID may comprise an antibody fragment. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56.

The second linker may be a bifunctional linker. The second linker may be a heterobifunctional linker. The second linker may be a homobifunctional linker. The second linker may further comprise one or more polyethylene glycol subunits. The second linker may comprise at least four PEG subunits. The second linker may comprise at least 10 PEG subunits. The second linker may comprise at least 20 PEG subunits. The second linker may comprise at least 30 PEG subunits. The second linker may comprise an azide at one end. The second linker may comprise an aminooxy at one end. The second linker may be an azide-PEG-aminooxy linker. The second linker may comprise cyclooctyne at one end. The second linker may be a PEG-cyclooctyne linker.

The method may further comprise incorporating one or more unnatural amino acids into X. Coupling L1 to X may comprise attaching L1 to an unnatural amino acid in X. Coupling L1 to X may comprise site-specific attachment. Coupling L1 to X may comprise attaching L1 to X in a predetermined site. Coupling L1 to X may comprise oxime ligation. Coupling L1 to X may comprise ester coupling.

The method may further comprise incorporating one or more unnatural amino acids into Y. Coupling L2 to Y may comprise attaching L2 to an unnatural amino acid in Y. Coupling L2 to Y may comprise site-specific attachment. Coupling L2 to Y may comprise attaching L2 to Y in a predetermined site. Coupling L2 to Y may comprise oxime ligation. Coupling L2 to Y may comprise ester coupling.

Coupling the first switch intermediate to the second switch intermediate may comprise one or cycloadditions. The one or more cycloadditions may comprise a Huisgen cycloaddition. The one or more cycloadditions may comprise a [3+2] cycloaddition. The one or more cycloadditions may comprise a [3+2] Huisgen cycloaddition. The one or more cycloadditions may comprise a copper-free cycloaddition. Coupling the first switch intermediate to the second switch intermediate may comprise a copper free reaction. Coupling the first switch intermediate to the second switch intermediate may comprise one or more copper-containing reactions. Attaching the CAR-ID to the TID may comprise one or more Diels Alder reactions. Coupling the first switch intermediate to the second switch intermediate may comprise one or more hetero Diels Alder reactions. Coupling the first switch intermediate to the second switch intermediate may comprise one or more ester couplings. Coupling the first switch intermediate to the second switch intermediate may comprise one or more isothiocyanate couplings. Coupling the first switch intermediate to the second switch intermediate may comprise attaching the CAR-ID to an amino acid of TID. The amino acid may be an unnatural amino acid. Coupling the first switch intermediate to the second switch intermediate may comprise one or more bioorthogonal reactions. The first switch intermediate may be attached to the second switch intermediate in a site-specific manner. The first switch intermediate may be attached to a predetermined site in the second switch intermediate. The first switch intermediate may be attached to the second switch intermediate in a site-independent manner.

Attaching the first linker to the CAR-ID may comprise one or cycloadditions. Attaching the first linker to the CAR-ID may comprise a copper free reaction. Attaching the first linker to the CAR-ID may comprise one or more copper-containing reactions. Attaching the first linker to the CAR-ID may comprise one or more Diels Alder reactions. Attaching the first linker to the CAR-ID may comprise one or more hetero Diels Alder reactions. Attaching the first linker to the CAR-ID may comprise one or more ester couplings. Attaching the first linker to the CAR-ID may comprise oxime ligation. Attaching the first linker to the CAR-ID may comprise forming one or more oximes between the first linker and the CAR-ID. Attaching the first linker to the CAR-ID may comprise forming one or more stable bonds between the first linker and the CAR-ID. Attaching the first linker to the CAR-ID may comprise forming one or more covalent bonds between the first linker and the CAR-ID. Attaching the first linker to the CAR-ID may comprise forming one or more non-covalent bonds between the first linker and the CAR-ID. Attaching the first linker to the CAR-ID may comprise forming one or more ionic bonds between the first linker and the CAR-ID. Attaching the first linker to the CAR-ID may comprise attaching the linker to an amino acid of CAR-ID. The amino acid may be an unnatural amino acid. Attaching the first linker to the CAR-ID may comprise one or more bioorthogonal reactions.

Attaching the second linker to the TID may comprise one or cycloadditions. Attaching the second linker to the TID may comprise a copper free reaction. Attaching the second linker to the TID may comprise one or more copper-containing reactions. Attaching the second linker to the TID may comprise one or more Diels Alder reactions. Attaching the second linker to the TID may comprise one or more hetero Diels Alder reactions. Attaching the second linker to the TID may comprise one or more ester couplings. Attaching the second linker to the TID may comprise oxime ligation. Attaching the second linker to the TID may comprise forming one or more oximes between the second linker and the TID. Attaching the second linker to the TID may comprise forming one or more stable bonds between the second linker and the TID. Attaching the second linker to the TID may comprise forming one or more covalent bonds between the second linker and the TID. Attaching the second linker to the TID may comprise forming one or more non-covalent bonds between the second linker and the TID. Attaching the second linker to the TID may comprise forming one or more ionic bonds between the second linker and the TID. Attaching the second linker to the TID may comprise attaching the linker to an amino acid of TID. The amino acid may be an unnatural amino acid. Attaching the second linker to the TID may comprise one or more bioorthogonal reactions.

The method of producing the switches disclosed herein may further comprise purifying the switches. The method may further comprise purifying the TID, CAR-ID, linker, or any combinatino thereof. The methods may further comprise purifying one or more switch intermediates. The TID may comprise a polypeptide. The polypeptide may comprise an antibody or antibody fragment. The switch intermediate may comprise a TID and a linker. Purifying the switch and switch intermediate may comprise removing excess linkers, excess TIDs, and excess CAR-IDs. The excess linkers, TIDs, and CAR-IDs may be removed simultaneously. The excess linkers, TIDs, and CAR-IDs may be removed sequentially. Removal of the TIDs and CAR-IDs comprising polypeptides may comprise treating a solution comprising the switch, switch intermediate, linkers, CAR-IDs, and/or TIDs with one or more proteases. Purifying the switch, switch intermediate, linkers, CAR-IDs, and/or TIDs may comprise column purification. Purifying the switch, switch intermediate, linkers, CAR-IDs, and/or TIDs may comprise use of one or more concentrator columns, electrophoresis, filtration, centrifugation, chromatography or a combination thereof. Chromatography may comprise size-exclusion chromatography. Additional chromatography methods include, but are not limited to, hydrophobic interaction chromatography, ion exchange chromatography, affinity chromatography, metal binding, immunoaffinity chromatography, and high performance liquid chromatography or high pressure liquid chromatography. Electrophoresis may comprise denaturing electrophoresis or non-denaturing electrophoresis.

The switch, switch intermediate, linkers, CAR-IDs, and/or TIDs may comprise one or more purifying tags or purifying molecules. The linkers may comprise one or more tags. The tags may be used to purify the switch, switch intermediate, linkers, CAR-IDs, and/or TIDs. Examples of tags include, but are not limited to, polyhistidine, FLAG® tag, HA, c-myc, V5, chitin binding protein (CBP), maltose binding protein (MBP), and glutathione-S-transferase (GST). The tag may be a non-peptide tag. The tag may be biotin. The one or more tags may be cleaved by one or more proteases.

The methods may further comprise lyophilization or ultracentrifugation of the CAR-BPs, CAR-IDs, CAR-IDs, TCs, TIDs and intermediates thereof.

The purity of the switch, switch intermediate, linkers, CAR-IDs, and/or TIDs may be equal to or greater than 50%, 55%, 60%, 65%, 70%, 75%, 80%, 85%, 90%, 91%, 92%, 93%, 94%, 95%, 96%, 97%, 98%, 99% or more. The purity of the switch, switch intermediate, linkers, CAR-IDs, and/or TIDs may be equal to or greater than 85%. The purity of the switch, switch intermediate, linkers, CAR-IDs, and/or TIDs may be equal to or greater than 90%. The purity of the switch, switch intermediate, linkers, CAR-IDs, and/or TIDs may be equal to or greater than 95%. The purity of the CAR-BPs, CAR-IDs, CAR-IDs, TCs, TIDs and intermediates thereof may be equal to or greater than 97%.

The homogeneity of the switches or switch intermediates disclosed herein may be equal to or greater than 50%, 55%, 60%, 65%, 70%, 75%, 80%, 85%, 90%, 91%, 92%, 93%, 94%, 95%, 96%, 97%, 98%, 99% or more. The homogeneity of the switches or switch intermediates may be equal to or greater than 85%. The homogeneity of the switches or switch intermediates may be equal to or greater than 90%. The homogeneity of the switches or switch intermediates may be equal to or greater than 95%. The homogeneity of the switches or switch intermediates may be equal to or greater than 97%. The homogeneity may refer to a structural homogeneity. The homogeneity may be a structural homogeneity prior to administering the cell to a subject. The homogeneity may be a structural homogeneity prior to modifications to the CAR-EC switch by cellular activities (methylation, acetylation, glycosylation, etc.). These high percentages of homogeneity may provide a more predictable effect of the CAR-EC switch. These high percentages of homogeneity may provide for less off-target effects of the CAR-EC switch, when combined with a CAR-EC to treat a condition in a subject.

The methods of producing CAR-EC switches disclosed herein may comprise producing a plurality of switches with a purity of equal to or greater than 50%, 55%, 60%, 65%, 70%, 75%, 80%, 85%, 90%, 91%, 92%, 93%, 94%, 95%, 96%, 97%, 98%, 99% or more. The purity of the plurality of switches may be equal to or greater than 85%. The purity of the plurality of switches may be equal to or greater than 90%. The plurality of switches may be equal to or greater than 95%. The purity of the plurality of switches may be equal to or greater than 97%. Purity may be assessed by methods known in the art (e.g. color, water content, pyrogen content, impurity content, etc.). The purity of a plurality of CAR-EC switches comprising a CAR-ID comprising FITC and a TID comprising a polypeptide produced by oxime conjugation, Huisgen cycloaddition, or Diels Alder reaction may be greater than 70%, 75%, 80%, 82%, 85%, 87%, 90%, 92%, 95%, 97%, or 99%. The purity of a plurality of CAR-EC switches comprising a CAR-ID comprising FITC and a TID comprising a polypeptide produced by oxime conjugation, Huisgen cycloaddition, or Diels Alder reaction may be greater than 85%. The purity of a plurality of CAR-EC switches comprising a CAR-ID comprising FITC and a TID comprising a polypeptide produced by oxime conjugation, Huisgen cycloaddition, or Diels Alder reaction may be greater than 90%. The purity of a plurality of CAR-EC switches comprising a CAR-ID comprising FITC and a TID comprising a polypeptide produced by oxime conjugation, Huisgen cycloaddition, or Diels Alder reaction may be greater than 95%. The purity of a plurality of switches comprising a CAR-ID comprising FITC and a TID comprising a small molecule produced by chemical synthesis may be purity greater than 70%, 75%, 80%, 82%, 85%, 87%, 90%, 92%, 95%, 97%, or 99%. The purity of a plurality of switches comprising a CAR-ID comprising FITC and a TID comprising a small molecule produced by chemical synthesis may be purity greater than 80%. The purity of a plurality of switches comprising a CAR-ID comprising FITC and a TID comprising a small molecule produced by chemical synthesis may be purity greater than 85%. The purity of a plurality of switches comprising a CAR-ID comprising FITC and a TID comprising a small molecule produced by chemical synthesis may be purity greater than 90%. The purity of a plurality of switches comprising a CAR-ID comprising FITC and a TID comprising a small molecule produced by chemical synthesis may be purity greater than 95%. The purity of a plurality of switches comprising a CAR-ID comprising FITC and a TID comprising a small molecule produced by chemical synthesis may be purity greater than 98%.

### Chimeric Antigen Receptor Effector Cells (CAR-EC)

The methods, platforms and kits disclosed herein may comprise one or more chimeric antigen receptor effector cells (CAR-EC) or uses thereof. The methods, platforms or kits comprise two or more CAR-ECs or uses thereof. The methods, platforms or kits may comprise a plurality of CAR-ECs or uses thereof. At least two of the plurality of CAR-ECs may be of the same cell type. At least two of the plurality of CAR-ECs may be of the same cell lineage. At least two of the plurality of CAR-ECs may be of a different cell type. At least two of the plurality of CAR-ECs may be of different cell lineages. At least two of the plurality of CAR-ECs may comprise identical CARs. At least two of the plurality of CAR-ECs may comprise two or more different CARs. At least two of the plurality of CAR-ECs may comprise two or more similar CARs.

A CAR-EC may comprise an effector cell that expresses a CAR. The effector cell may be a T cell. The effector cell may be a cell of a T cell lineage. The effector cell may be a mature T cell. The effector cell may be a precursor T cell. The effector cell may be a cytotoxic T cell. The effector cell may be a naive T cell. The effector cell may be a memory stem cell T cell (T_{MSC}). The effector cell may be a central memory T cell (T_{CM}). The effector cell may be an effector T cell (TE). The effector cell may be a CD4+ T cell. The T cell may be a CD8+ T cell. The effector cell may be a CD4+ and CD8+ cell. The effector cell may be an αβ T cell. The effector cell may be a γδ T cell. The effector cell may be a natural killer T cell. The effector cell may be a helper T cell. The T cell may overexpress FoxP3.

The effector cell may be an effector cell that has an effect on a target or target cell when brought into proximity of the target or target cell. The effector cell may be a cell that has a cytotoxic effect on a target or target cell when brought into proximity of the target or target cell. The effector cell may be an immune cell. The effector cell may be selected from a B cell, a monocyte, a thrombocyte, a leukocyte, a neutrophil, an eosinophil, a basophil, a macrophage, or a lymphocyte. The effector cell may be a lymphocyte. The effector cell may be a macrophage. The effector cell may be a phagocytic cell. The effector cell may be an effector B cell. The effector cell may be a natural killer cell. The effector cell may be a cell derived from a subject to be treated with a CAR-EC switch or switch intermediate disclosed herein.

The CAR-EC may comprise one or more polynucleotides selected from SEQ ID NOs: 1-4. The CAR-EC may comprise a polynucleotide that is at least about 5%, 10%, 15%, 20%, 25%, 30%, 35%, 40%, 45%, 50%, 55%, 60%, 65%, 70%, 75%, 80%, 85%, 90% or 95% identical to one or more polynucleotides selected from SEQ ID NOs: 1-4. The CAR-EC may comprise a polynucleotide that is at least about 70% identical to one or more polynucleotides selected from SEQ ID NOs: 1-4. The CAR-EC may express a polypeptide encoded by one or more polynucleotides selected from SEQ ID NOs: 1-4. The CAR-EC may express a polypeptide encoded by a polynucleotide that is at least about 5%, 10%, 15%, 20%, 25%, 30%, 35%, 40%, 45%, 50%, 55%, 60%, 65%, 70%, 75%, 80%, 85%, 90% or 95% identical to one or more polynucleotides selected from SEQ ID NOs: 1-4. The polynucleotide may be constitutively expressed. The polynucleotide may be conditionally expressed. For example, in order to activate expression of the polynucleotide in the CAR-EC, a drug is administered to the CAR-EC. Alternatively, in order to terminate expression of the polynucleotide in the CAR-EC, a drug is administered to the CAR-EC.

Disclosed herein are methods of producing one or more CAR-ECs. The method may comprise (a) infecting an effector cell with one or more viruses comprising one or more polynucleotides selected from SEQ ID NOs: 1-4; and (b) culturing the effector cell in culture media, thereby producing the CAR-EC. The method may further comprise administering one or more reagents to the culture media to stimulate expression of the polynucleotides. The method may further comprise administering one or more reagents to the culture media to enrich for the CAR-EC. For example, the polynucleotide may further comprise a drug-selectable marker that can be used to enrich for effector cells that have been infected with the virus. The method may further comprise purifying the CAR-ECs. Purifying the CAR-ECs may comprise using a cell sorter. For example, the polynucleotide may further comprise a detectable marker (e.g., a fluorescent protein) that can be used to purify the effector cells that have been infected with the virus. Purifying the CAR-ECs may comprise positive selection. For example, a mixture comprising the effector cells and the CAR-ECs may be passed through a column comprising a molecule that can interact with the CAR of the CAR-ECs. Thus, CAR-ECs may be bound by the column and the effector cells that have not be successfully infected are released in the eluate. The column may be washed several times. The CAR-ECs may be eluted from the column. Purifying the CAR-ECs may comprise negative selection. For example, expression of the CAR in infected effector cells may result in down-regulation of a surface molecule. A mixture comprising the effector cells and the CAR-ECs may be passed through a column comprising a molecule that can interact with the down-regulated surface molecule. Thus, effector cells that have not been infected may be bound by the column and the effector cells that express the CAR are released in the eluate. The virus may be a lentivirus. The virus may be an adenovirus. The virus may be a retrovirus. The virus may be an adeno-associated virus. The virus may be a self-complementary adeno-associated virus (scAAV). The virus may be a modified human immunodeficiency (HIV) virus. The virus may be a modified herpes simplex virus (HSV) virus.

The method of producing a CAR-EC may comprise (a) transfecting an effector cell with one or more vectors comprising one or more polynucleotides selected from SEQ ID NOs: 1-4; and (b) culturing the effector cell in culture media, thereby producing the CAR-EC. The method may further comprise administering one or more reagents to the culture media to stimulate expression of the polynucleotides. The method may further comprise administering one or more reagents to the culture media to enrich for the CAR-EC. For example, the polynucleotide may further comprise a drug-selectable marker that can be used to enrich for effector cells that have been transfected. The method may further comprise purifying the CAR-ECs. Purifying the CAR-ECs may comprise using a cell sorter. For example, the polynucleotide may further comprise a detectable marker (e.g., a fluorescent protein) that can be used to purify the effector cells that have been transfected. Purifying the CAR-ECs may comprise positive selection. For example, a mixture comprising the effector cells and the CAR-ECs may be passed through a column comprising a molecule that can interact with the CAR of the CAR-ECs. Thus, CAR-ECs may be bound by the column and the effector cells that have not be successfully transfected are released in the eluate. The column may be washed several times. The CAR-ECs may be eluted from the column. Purifying the CAR-ECs may comprise negative selection. For example, expression of the CAR in transfected effector cells may result in down-regulation of a surface molecule. A mixture comprising the effector cells and the CAR-ECs may be passed through a column comprising a molecule that can interact with the down-regulated surface molecule. Thus, effector cells that have not been transfected may be bound by the column and the effector cells that express the CAR are released in the eluate.

### Chimeric Antigen Receptor (CAR)

The switches disclosed herein may interact with a chimeric antigen receptor (CAR) on a CAR-EC, thereby regulating the activities of the CAR-EC. Generally, the interaction of the CAR-ID with the CAR may result in the activation of an immune response by the cell. The CAR may comprise an extracellular domain, a transmembrane domain and an intracellular domain. The extracellular domain may interact with the CAR-ID of the CAR-EC switch. The extracellular domain may comprise at least a portion of an antibody. In some instances, the antibody is not a full-length antibody. The extracelluar domain may comprise at least a portion of an immunoglobulin or fragment thereof. The immunoglobulin or fragment thereof may be selected from a group comprising IgA1, IgA2, IgD, IgM, IgE, IgG1, IgG2, IgG3, IgG4, scFv, di-scFv, bi-scFv and Fab, Fc, F(ab')₂, pFc', a nanobody, an affibody, a DARPin, a diabody, a camelid, an engineered T cell receptor, or a monobody. The immunoglobulin may comprise IgG4.

The antibody may have a binding affinity of about 0.01 pM, about 0.02 pM, about 0.03 pM, about 0.04 pM, 0.05 pM, about 0.06 pM, about 0.07 pM, about 0.08 pM, about 0.09 pM, about 0.1 pM, about 0.2 pM, 0.3 pM, about 0.4 pM, about 0.5 pM, about 0.6 pM, about 0.7 pM, about 0.8 pM, about 0.9 pM or about 1 pM, about 2 pM, about 3 pM, about 4 pM, about 5 pM, about 6 pM, about 7 pM, about 8 pM, about 9 pM, about 10 pM, about 0.01 nM, about 0.02 nM, about 0.03 nM, about 0.04 nM, about 0.05 nM, about 0.06 nM, about 0.07 nM, about 0.08 nM, about 0.09 nM, about 0.1 nM, about 0.2 nM, about 0.3 nM, about 0.4 nM, about 0.5 nM, about 0.6 nM, about 0.7 nM, about 0.8 nM, about 0.9 nM, about 1 nM, about 2 nM, about 3 nM, about 4 nM, about 5 nM, about 6 nM, about 7 nM, about 8 nM, about 9 nM, about 10 nM, about 12nM, about 14 nM, about 16 nM, about 18 nM, about 20 nM, about 22 nM, about 24 nM, about 26 nM, about 28 nM or about 30 nM. The extracellular domain may comprise at least a portion of a single chain variable fragment (scFv). The extracellular domain may comprise avidin or a fragment thereof. The extracellular domain may not comprise avidin or fragment thereof. The antibody may comprise an anti-FITC antibody or fragment thereof. The anti-FITC antibody may be an anti-FITC scFv. The anti-FITC scFv may be selected from 4-4-20, 4D5Flu, 4M5.3 and FITC-E2. The anti-FITC scFv may be encoded by a sequence selected from SEQ ID NOs: 1-4.

The antibody to FITC or fragment thereof may have a binding affinity for FITC less than 0.1 pM. The antibody to FITC or fragment thereof may have a binding affinity for FITC between about 0.1 pM and about 1 pM. The antibody to FITC or fragment thereof may have a binding affinity for FITC between about 1 pM and about 10 pM. The antibody to FITC or fragment thereof may have a binding affinity for FITC of about 10 pM, about 20 pM, about 30 pM, about 40 pM, about 50 pM, about 60 pM, about 70 pM, about 80 pM, about 90 pM or about 100 pM. The antibody to FITC or fragment thereof may have a binding affinity for FITC of about 100 pM, about 200 pM, about 300 pM, about 400 pM, about 500 pM, about 600 pM, about 700 pM, about 800 pM, about 900 pM or about 1 nM. The antibody to FITC or fragment thereof may have a binding affinity for FITC of about 1 nM, about 2 nM, about 3 nM, about 4 nM, about 5 nM, about 6 nM, about 7 nM, about 8 nM, about 9 nM or about 10 nM. The antibody to FITC or fragment thereof may have a binding affinity for FITC of about 10 nM, about 15 nM, about 20 nM, about 25 nM, about 30 nM, about 35 nM, about 40 nM, about 45 nM or about 50 nM. The antibody to FITC or fragment thereof may have a binding affinity for FITC greater than 50 nM. The antibody to FITC may comprise an anti-FITC scFv or fragment thereof. The anti-FITC scFv may be selected from a group comprising 4-4-20, 4D5Flu, 4M5.3 and FITC-E2. The binding affinity of 4-4-20 may be about 0.2 nM. The binding affinity of 4D5Flu may be about 20 nM. The binding affinity of 4M5.3 may be about 0.3 pM. The binding affinity of FITC-E2 may be about 0.3 nM.

The transmembrane domain and/or the intracellular domain may comprise at least a portion of a cytoplasmic signaling domain. The intracellular domain may comprise at least a portion of a signaling molecule selected from the group comprising CD3ξ, CD28, and 4-1BB. The intracellular domain may comprise an Fc receptor or a portion thereof. The Fc receptor or portion thereof may be CD16 or a portion thereof. The signaling molecule may comprise CD3ξ. The signaling molecule may comprise CD28. The signaling molecule may comprise 4-1BB. The intracellular domain may comprise at least a portion of CD3ξ. The intracellular domain may comprise at least a portion of CD28, The intracellular domain may comprise at least a portion of 4-1BB, The intracellular domain may comprise at least a portion of OX-40, The intracellular domain may comprise at least a portion of CD30, The intracellular domain may comprise at least a portion of CD40, The intracellular domain may comprise at least a portion of CD2. The intracellular domain may comprise at least a portion of CD27. The intracellular domain may comprise at least a portion of PD-1. The intracellular domain may comprise at least a portion of ICOS. The intracellular domain may comprise at least a portion of lymphocyte function-associated antigen-1 (LFA-1). The intracellular domain may comprise at least a portion of CD7. The intracellular domain may comprise at least a portion of LIGHT. The intracellular domain may comprise at least a portion of NKG2C. The intracellular domain may comprise at least a portion of B7-H3. The intracellular domain may comprise at least a portion of a cytoplasmic signaling domain from one or more signaling molecules. The intracellular domain may comprise at least a portion of two or more cytoplasmic signaling domains. The two or more cytoplasmic signaling domains may be from two or more different signaling molecules. The intracellular domain may comprise at least a portion of three or more cytoplasmic signaling domains. The intracellular domain may comprise at least a portion of four or more cytoplasmic signaling domains. The intracellular domain may comprise at least a portion of a ligand that binds to one or more signaling molecules. The intracellular domain may comprise at least a portion of a ligand that binds to CD83.

### CAR-EC platform

Disclosed herein are chimeric antigen receptor effector cell (CAR-EC) platforms comprising an effector cell expressing a chimeric antigen receptor (CAR); and a chimeric antigen receptor effector cell (CAR-EC) switch disclosed herein. The CAR-EC switch may be homogenous. The CAR-EC switch may be structurally homogenous. The CAR-ID may be selected from a CAR binding small molecule (CAR-ID) and a CAR binding component (CAR-ID) disclosed herein. The TID may be selected from a target interacting domain and a TID disclosed herein.

The CAR-EC platform may comprise a plurality of CAR-EC switches, wherein at least one of the CAR-EC switches is disclosed herein. The plurality of CAR-EC switches may comprise two or more CAR-EC switches. The plurality of CAR-EC switches may comprise 1, 2, 3, 4, 5, 6, 7, 8, 9, 10, 11, 12, 13, 14, 15, 16, 17, 18, 19, 20 or more CAR-EC switches. The plurality of CAR-EC switches may comprise more than 20, more than 25, more than 30, more than 35, more than 40, more than 45 or more than 50 CAR-EC switches. The two or more CAR-EC switches may be selected from one or more CAR-EC switches disclosed herein or a combination thereof.

The antibodies or peptides disclosed herein may comprise one or more unnatural amino acids. The TID may comprise one or more unnatural amino acids. The CAR-ID and the TID may comprise one or more unnatural amino acids. The CAR-ID and the TID may be linked through the one or more unnatural amino acids. The CAR-ID and the TID may be linked through 2 unnatural amino acids. The CAR-ID and the TID may be linked through 3 unnatural amino acids. One or more unnatural amino acids may replace one or more amino acid residues of the TID. The one or more unnatural amino acids may replace any amino acid of the peptides or antibodies disclosed herein.

The CAR-EC platforms disclosed herein may further comprise two or more CAR-EC switches. Two or more CAR-EC switches may comprise two or more identical CAR-IDs. Two or more CAR-EC switches may comprise two or more different CAR-IDs. Two or more CAR-EC switches may comprise two or more similar CAR-IDs. The two or more CAR-IDs may comprise similar amino acid sequences. The amino acid sequences of the two or more CAR-IDs may be about 99%, about 98%, about 97%, about 96%, about 95%, about 92%, about 90%, about 85%, about 80%, about 75%, about 70%, about 65%, about 60%, about 55%, about 50%, about 45%, about 40%, about 35%, about 30%, about 25%, about 20%, about 15%, about 10%, about 5% or about 2% identical. The amino acid sequences of the two or more CAR-IDs may be about 75% identical.

Two or more CAR-EC switches may comprise two or more different TIDs. Two or more CAR-EC switches may comprise two or more identical TIDs. The two or more CAR-EC switches may comprise two or more similar TIDs. The two or more TIDs may comprise similar amino acid sequences. The amino acid sequences of the two or more TIDs may be about 99%, about 98%, about 97%, about 96%, about 95%, about 92%, about 90%, about 85%, about 80%, about 75%, about 70%, about 65%, about 60%, about 55%, about 50%, about 45%, about 40%, about 35%, about 30%, about 25%, about 20%, about 15%, about 10%, about 5% or about 2% identical. The amino acid sequences of the two or more TIDs may be about 75% identical.

### CAR-EC Targets

The switches disclosed herein may direct one or more chimeric antigen receptor-effector cells (CAR-ECs) to one or more targets. Generally, binding of the CAR-EC and the target to the CAR-EC switch brings the target into proximity with the CAR-EC that is sufficiently close for an activity of the CAR-EC to have an effect on the target. For example, when the CAR-EC and the target are attached to the CAR-EC switch, the CAR-EC may release cytokines that bind to cytokine receptors on the target. The switches disclosed herein may direct one or more chimeric antigen receptor-effector cells (CAR-ECs) to two or more targets. The switches disclosed herein may direct one or more chimeric antigen receptor-effector cells (CAR-ECs) to three or more targets. The switches disclosed herein may direct four or more chimeric antigen receptor-effector cells (CAR-ECs) to one or more targets. The switches disclosed herein may direct five or more chimeric antigen receptor-effector cells (CAR-ECs) to one or more targets. The switches disclosed herein may direct 6, 7, 8, 9, 10, 11, 12, 13, 14, 15, 16, 17, 18, 19, 20 or more chimeric antigen receptor-effector cells (CAR-ECs) to one or more targets. The two or more targets may be the same. The two or more targets may be of the same cell type. The two or more targets may be of the same cell lineage. The two or more targets may be different. The two or more targets may be of different cell types. The two or more targets may be of different cell lineages. At least two of the three or more targets may be the same. At least two of the three or more targets may be of the same cell type. At least two of the three or more targets may be of the same cell lineage. At least two of the three or more targets may be different. At least two of the three or more targets may be of different cell types. At least two of the three or more targets may be of different cell lineages.

The target may be a cell. The target may be a fragment of a cell. The target may be one or more cells. The target may comprise a cell from a subject suffering from a disease or condition. The target may comprise an infected cell. The target may comprise a pathogenically infected cell. The target may comprise a diseased cell. The target may be a cancer cell. The target may be a genetically modified cell. The target may be one or more dividing cells. The target may comprise cell that is foreign to a subject. The target may come from an invading organism (e.g. yeast, worm, bacteria, fungus). The target may be a pathogen. The target may be bacteria. The target may be a virus or a portion thereof.

The target may be selected from a stem cell, a cancer stem cell, a pluripotent cell, a hematopoietic stem cell or an endothelial progenitor cell. The target may be derived from a tissue. The tissue may be selected from brain, esophagus, breast, colon, lung, glia, ovary, uterus, testes, prostate, gastrointestinal tract, bladder, liver, thymus, bone and skin. The target may be derived from one or more endocrine glands. Alternatively, or additionally, the target may be derived from one or more endocrine glands. The endocrine gland may be a lymph gland, pituitary gland, thyroid gland, parathyroid gland, pancreas, gonad or pineal gland.

The target may comprise a cancerous cell. The cancerous cell may be derived from a tissue. The tissue may be selected from brain, esophagus, breast, colon, lung, glia, ovary, uterus, testes, prostate, gastrointestinal tract, bladder, liver, thymus and skin. The cancerous cell may be derived from bone. The cancerous cell may be derived from blood. The cancerous cell may be derived from a B cell, a T cell, a monocyte, a thrombocyte, a leukocyte, a neutrophil, an eosinophil, a basophil, a lymphocyte, a hematopoietic stem cell or an endothelial cell progenitor. The cancerous cell be derived from a CD19-positive B lymphocyte. The cancerous cell may be derived from a stem cell. The cancerous cell may be derived from a pluripotent cell. The cancerous cell may be derived from one or more endocrine glands. The endocrine gland may be a lymph gland, pituitary gland, thyroid gland, parathyroid gland, pancreas, gonad or pineal gland.

The target may be a CD19-positive cell. The target may be a CD19-positive B lymphocyte. The target may be a Her2-positive cell. The Her2-positive cell may be a Her2-positive breast cancer cell. The target may be a BCMA-positive cell. The target may be a BCMA-positive multiple myeloma cell. The target may be a CS1-positive cell. The CS1-positive cell may be a multiple myeloma cell. The target may be a EGFRvIII-positive cell. The target may be a EGFRvIII-positive glioblastoma cell. The target may be a CD20-positive cell. The target may be a PSMA-positive cell. The target may be a PSMA-positive prostate cancer cell. The target may be a folate receptor-positive cell. The target may be a folate receptor-positive ovarian cancer cell

### Surface Molecules on CAR-EC Targets

The target interacting domain (TID) of the switches disclosed herein may bind to or interact with a molecule on a target. The TID may bind an antigen, a protein, a peptide, or a biomolecule on a target. The protein may be an enzyme. The enzyme may have enzymatic activity. A biomolecule, by non-limiting example, may be selected from a fiber, a biopolymer (e.g. collagen), a glycan, a glycolipid, a proteoglycan, a lipid, a sterol, a carbohydrate, a nucleic acid and a cellular fragment. The antigen may be at least a portion of a surface antigen or a cell surface marker on a cell. The antigen may be a receptor or a co-receptor on a cell.

The TID may bind to an antigen on a target. The antigen may be a surface antigen on a target. The antigen may be selected from a protein, a lipid, a glycolipid, a carbohydrate, a polysaccharide, a nucleic acid or a combination thereof. The antigen may comprise parts (e.g., coats, capsules, cell walls, flagella, fimbrae, and toxins) of bacteria, viruses, and other microorganisms. The antigen may comprise a glycan. The antigen may comprise a lipid. The antigen may comprise a glycolipid. The antigen may comprise a carbohydrate. The antigen may comprise a protein. The antigen may comprise a modification. The modification, by non-limiting example, may be a phosphorylation, an acetylation, a myristoylation, a palmitoylation or a methylation. The antigen may comprise a prostate specific membrane antigen.

The antigen may evoke the production of one or more antibodies. The antigen may refer to a molecule or molecular fragment that may be bound by a major histocompatibility complex (MHC) and presented to a T-cell receptor. The term "antigen" may also refer to an immunogen. The immunogen may provoke an adaptive immune response if injected on its own into a subject. The immunogen may induce an immune response by itself. The antigen may be a superantigen, T-dependent antigen or a T-independent antigen.

The antigen may be an exogenous antigen or endogenous antigen. Exogenous antigens are typically antigens that have entered the body from the outside, for example by inhalation, ingestion, or injection. Some antigens may start out as exogenous antigens, and later become endogenous (for example, intracellular viruses). Intracellular antigens may be released back into circulation upon the destruction of the infected cell, again. Endogenous antigens may be antigens that have been generated within previously-normal cells as a result of normal cell metabolism, or because of viral or intracellular bacterial infection.

The antigen may also include autoantigens. An autoantigen may be a normal protein or complex of proteins (and sometimes DNA or RNA) that is recognized by the immune system of patients suffering from a specific autoimmune disease. These antigens should, under normal conditions, not be the target of the immune system, but, due to mainly genetic and environmental factors, the normal immunological tolerance for such an antigen has been lost in these patients.

The antigen may include a tumor antigen. Tumor antigens or neoantigens may be antigens that are presented by MHC I or MHC II molecules on the surface of tumor cells. These antigens may sometimes be presented by tumor cells and never by the normal ones. In this case, they are called tumor-specific antigens (TSAs) and, in general, result from a tumor-specific mutation. More common are antigens that are presented by tumor cells and normal cells, and they are called tumor-associated antigens (TAAs). Cytotoxic T lymphocytes that recognize these antigens may be able to destroy the tumor cells before they proliferate or metastasize. Tumor antigens may also be on the surface of the tumor in the form of, for example, a mutated receptor, in which case they may be recognized by B cells.

The antigen may be a receptor. The receptor may be an extracellular receptor. The receptor may be a cell surface receptor. The receptor may bind a hormone, a neurotransmitter, a cytokine, a growth factor or a cell recognition molecule. The receptor may be a transmembrane receptor. The receptor may be an enzyme-linked receptor.

The TID may bind a surface molecule. The surface molecule, by non-limiting example, may be selected from an antigen, a receptor, a co-receptor, a trans-membrane protein or a cell marker on the target. The cell surface protein may be selected from a cholecystokinin B receptor, a gonadotropin-releasing hormone receptor, a somatostatin receptor 2, an avb3 integrin, a gastrin-releasing peptide receptor, a neurokinin 1 receptor, a melanocortin 1 receptor, a neurotensin receptor, neuropeptide Y receptor and C-type lectin like molecule 1.

The TID may bind a target that is at least about 5%, about 10 %, about 15%, about 20%, about 25%, about 30%, about 35%, about 40%, about 45%, about 50%, about 55%, about 60%, about 65%, about 70%, about 75%, about 80%, about 85%, about 90%, about 95%, about 98% or about 100% homologous to prostate specific membrane antigen (PSMA). The TID may bind a target that is at least about 50% homologous to prostate specific membrane antigen (PSMA). The TID may bind a target that is at least about 70% homologous to prostate specific membrane antigen (PSMA). The TID may bind a target that is at least about 85% homologous to prostate specific membrane antigen (PSMA). The TID may comprise a prostate specific membrane antigen (PSMA) ligand, activator, binding molecule or a derivative thereof. The TID may comprise a prostate specific membrane antigen (PSMA) inhibitor, or a derivative thereof. PSMA may also be referred to as glutamate carboxypeptidase II and N-acetyl-L-aspartyl-L-glutamate peptidase I. The PSMA inhibitor may be 2-[3-(1,3-dicarboxypropy)ureidol] pentanedioic acid or a derivative thereof.

The TID may bind a target that is at least about 5%, about 10 %, about 15%, about 20%, about 25%, about 30%, about 35%, about 40%, about 45%, about 50%, about 55%, about 60%, about 65%, about 70%, about 75%, about 80%, about 85%, about 90%, about 95%, about 98% or about 100% homologous to a folate receptor. The TID may bind a target that is at least about 50% homologous to a folate receptor. The TID may bind a target that is at least about 70% homologous to a folate receptor. The TID may bind a target that is at least about 85% homologous to a folate receptor. The TID may be selected from a folate receptor ligand, a folate receptor inhibitor, a folate receptor activator, a folate receptor binding molecule or a derivative thereof. The TID may comprise folate or a derivative thereof. The TID may consist essentially of folate or a derivative thereof.

The receptor may be a G-protein couple receptor (GPCR). The GPCR receptor may be GNRH receptor, endothelin receptor, Smoothened, Frizzled, CXCR4, CCR5, CXCR1 , CXCR2, CCR7, CCK2 receptor, SIP receptor, a protease activated receptor (PAR) or portion thereof. The receptor may be an ion channel linked receptor. The ion channel linked receptor may be a voltage gated potassium channel, a voltage gated calcium channel, a transient receptor potential channel, a melastatin-like transient receptor potential channel (e.g. TRPM1, TRPM7, TRPM8), a vallinoid channel (e.g. TRPV6) or an epithelial sodium channel.

The TID may interact with a receptor on a target. The receptor may be a growth factor receptor. The growth factor receptor may be epidermal growth factor receptor, fibroblast growth factor receptor, platelet derived growth factor receptor, nerve growth factor receptor, transforming growth factor receptor, bone morphogenic protein growth factor receptor, hepatocyte growth factor receptor, vascular endothelial growth factor receptor, stem cell factor receptor, insulin growth factor receptor, somatomedin receptor or erythropoietin receptor. The receptor may be a hormone receptor. The receptor may be an insulin receptor. The receptor may be an eicosanoid receptor, a prostaglandin receptor, an estrogen receptor, a follicle stimulating hormone receptor, a progesterone receptor, a growth hormone receptor or a gonadotropin-releasing hormone receptor. The receptor may be an adrenergic receptor. The receptor may be an integrin. The receptor may be an Eph receptor.

The receptor may be an immune receptor. The immune receptor may be a pattern recognition receptor, a toll-like receptor, a NOD like receptor, a killer activated receptor, a killer inhibitor receptor, an Fc receptor, a B cell receptor, a complement receptor, a chemokines receptor or a cytokine receptor. The cytokine receptor may be an interleukin receptor, an interferon receptor, a transforming growth factor receptor, a tumor necrosis factor receptor or a colony stimulating factor receptor.

The receptor may be a receptor kinase. The receptor kinase may be a tyrosine kinase receptor. The receptor kinase may be a serine kinase receptor. The receptor kinase may be a threonine kinase receptor. The receptor kinase may activate a Ras, a Raf, PI3K, PKA, PKC, AKT, AMPK or a phospholipase. The receptor kinase may activate a MAPK/ERK signaling pathway. The receptor kinase may activate Jak, Stat or Smad.

The TID may interact with a surface molecule that comprises a glycolipid. The TID may interact with a surface molecule that comprises a carbohydrate. The TID may interact with a surface molecule that comprises a cluster of differentiation (CD) protein. The TID may interact with a surface molecule that is selected from the group consisting of CD34, CD31, CD117, CD45, CD11b, CD15, CD24, CD114, CD182, CD14, CD11a, CD91, CD16, CD3, CD4, CD25, Foxp3, CD8, CD38, CD61, CD56, CD30, CD13 and CD33.

### Kits, Vectors and Polynucleotides

Disclosed herein are kits comprising one or more CAR-EC switches disclosed herein. The kit may further comprise two or more CAR-EC switches. The kit may comprise three CAR-EC switches. The kit may comprise about 3, 4, 5, 6, 7, 8, 9, 10, 12, 15, 20, 24, 30, 35, 48, 50, 55, 60, 65, 70, 75, 80, 85, 90, 96, 100, 120, 150, 200, 300, 384, 400, 500, 600, 700, 800, 900 or 1000 CAR-EC switches. A kit comprising two or more CAR-EC switches may comprise two or more identical unnatural amino acids. The two or more CAR-EC switches may comprise two or more different unnatural amino acids. The two or more CAR-EC switches may comprise two or more similar unnatural amino acids. The two or more CAR-EC switches may comprise two or more unnatural amino acids at different sites. The two or more CAR-EC switches may comprise two or more unnatural amino acids at contiguous sites. The kit may be employed for biological research. The kit may be used for diagnosing a disease or a condition. The kit may be used for treating a disease or condition. The kit may further comprise one or more effector cells. The effector cell may be a T cell. The effector cell may express one or more CARs. The kit may further comprise a polynucleotide encoding an antibody/peptide of a CAR. The kit may further comprise a vector comprising a polynucleotide encoding an antibody/peptide of a CAR. The CAR may be selected from any of the CARs disclosed herein.

Antibodies and/or peptides of CAR-EC switches disclosed herein may be encoded by one or more polynucleotides selected from SEQ ID NOs: 5-9. CARs or portions thereof may be encoded by a polynucleotide at least about 5%, 10%, 15%, 20%, 25%, 30%, 35%, 40%, 45%, 50%, 55%, 60%, 65%, 70%, 75%, 80%, 85%, 90% or 95% identical to one or more polynucleotides selected from SEQ ID NOs: 5-9. Antibodies and/or peptides of CAR-EC switches disclosed herein may be encoded by a polynucleotide may be at least about 70% identical to one or more polynucleotides selected from SEQ ID NOs: 5-9. Disclosed herein are vectors comprising one or more polynucleotides selected from SEQ ID NOs: 5-9.

### Therapeutic Use

The chimeric antigen receptor-effector cells (CAR-EC) switches and switch intermediates may be used in the treatment of a disease or condition in a subject in need thereof. The switches or the switch intermediates disclosed herein may be used in the manufacture of a medicament for the treatment of a disease. The methods of treating a disease or condition in a subject in need thereof may comprise administering any of the switches disclosed herein to the subject. The switch may comprise a chimeric antigen receptor-interacting domain (CAR-ID) and a target interacting domain (TID). The switch may further comprise one or more additional CAR-IDs. The switch may comprise a chimeric antigen receptor-interacting domain (CAR-ID) comprising a small molecule. The CAR-ID may comprise FITC. The CAR-IDs may comprise one or more unnatural amino acids. The switch may further comprise one or more TIDs. The switch may comprise a target interacting domain (TID) comprising a polypeptide that is based on or derived from an antibody. The antibody may be an anti-CD 19 antibody. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56. The TID may comprise a polypeptide comprising an unnatural amino acid. The CAR-ID and the TID may be attached in a site-specific manner. Site-specific attachment of the CAR-ID to the TID may occur through the unnatural amino acid. The CAR-ID and the TID may be attached in a site-independent manner. The switch may comprise a TID comprising a small molecule. The TID may comprise 2-[3-(1,3-dicarboxypropyl)ureido] pentanedioic acid or a derivative thereof. The TID may comprise folate. The switch may further comprise a linker. The switch may further comprise one or more additional linkers. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. Alternatively, or additionally, the method may further comprise administering a chimeric antigen receptor effector cell (CAR-EC). The CAR-EC may express a chimeric antigen receptor (CAR). The CAR may be encoded by a nucleotide sequence of any one of SEQ ID NOs: 1-4. The CAR may comprise an external domain that is based on or derived from an antibody. The external domain of the CAR may be based on or derived from an anti-FITC antibody. The external domain of the CAR may be based on or derived from an anti-FITC scFv. The switch and the CAR-EC may be administered simultaneously. The switch and the CAR-EC may be administered sequentially.

The method of treating a disease or condition in a subject in need thereof may comprise administering one or more switch intermediates to the subject. The switch intermediate may comprise a CAR-ID and a linker, wherein the linker comprises a reactive functional group. The switch intermediate may comprise a TID and a linker. The switch intermediate may comprise a TID and a linker, wherein the linker comprises a reactive functional group. The switch intermediate may comprise a CAR-ID comprising a reactive functional group. The switch intermediate may comprise a TID comprising a reactive functional group. A first switch intermediate comprising a CAR-ID may be combined with a second switch intermediate comprising a TID. A first switch intermediate comprising a CAR-ID and a linker may be combined with a second switch intermediate comprising a TID. A first switch intermediate comprising a CAR-ID may be combined with a second switch intermediate comprising a TID and a linker. A first switch intermediate comprising a CAR-ID and a first linker may be combined with a second switch intermediate comprising a TID and a second linker. The first switch intermediate may comprise a first reactive functional group. The second switch intermediate may comprise a second reactive functional group. The first reactive functional group of the first switch and second reactive functional group of the second switch may interact with each other. The CAR-ID may comprise a small molecule. The CAR-ID may comprise FITC. The CAR-ID may comprise one or more unnatural amino acids. The TID may comprise a polypeptide that is based on or derived from an antibody. The antibody may be an anti-CD 19 antibody. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56. The TID may comprise a polypeptide comprising an unnatural amino acid. The TID may comprise a small molecule. The TID may comprise 2-[3-(1,3-dicarboxypropyl)ureido] pentanedioic acid or a derivative thereof. The TID may comprise folate. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole.

The methods may comprise administering one or more CAR-EC switches. The methods may comprise administering about 1, 2, 3, 4, 5, 6, 7, 8, 9, 10, 12, 15, 20, 24, 30, 35, 48, 50, 55, 60, 65, 70, 75, 80, 85, 90, 96, 100, 120, 150, 200, 300, 384, 400, 500, 600, 700, 800, 900, 1000 or more CAR-EC switches. The methods may comprise administering two or more CAR-EC switches. The two or more CAR-EC switches may comprise the same CAR-ID. The two more CAR-EC switches may comprise the same TIDs. The two or more CAR-EC switches may comprise the same linkers. The two or more CAR-EC switches may comprise one or more different CAR-IDs. The two more CAR-EC switches may comprise one or more different TIDs. The two or more CAR-EC switches may comprise one or more different linkers. The two or more CAR-EC switches may comprise one or more identical unnatural amino acids. The two or more CAR-EC switches may comprise one or more different unnatural amino acids. The two or more CAR-EC switches may comprise one or more similar unnatural amino acids. The two or more CAR-EC switches may comprise one or more unnatural amino acids at the same site. For example, a first TID of a first CAR-EC switch may comprise an unnatural amino acid at position 309 and a second TID of a second CAR-EC switch may comprise an unnatural amino acid at position 309. The two or more CAR-EC switches may comprise one or more unnatural amino acids at different sites. For example, a first TID of a first CAR-EC switch may comprise an unnatural amino acid at position 110 and a second TID of a second CAR-EC switch may comprise an unnatural amino acid at position 205. The two or more CAR-EC switches may comprise one or more unnatural amino acids at contiguous sites. For example, a first TID of a first CAR-EC switch may comprise an unnatural amino acid at position 202 and a second TID of a second CAR-EC switch may comprise an unnatural amino acid at position 203. The unnatural amino acids may be within the TIDs. The unnatural amino acids may be within the CAR-IDs.

Disclosed herein are methods of treating a disease or condition in a subject in need thereof, the method comprising administering a chimeric antigen receptor effector cell (CAR-EC) switch disclosed herein to the subject, wherein the CAR-EC switch comprises: a chimeric antigen receptor-binding small molecule (CAR-ID) that binds a chimeric antigen receptor (CAR) on a CAR-EC; and a target interacting domain (TID) that binds a surface molecule on a target and that comprises an unnatural amino acid. The CAR-ID and TID may be site-specifically linked through the unnatural amino acid. The CAR-ID, by non-limiting example, may be FITC. The TID, by non-limiting example, may be selected from an anti-CD 19 antibody, an anti-CD22 antibody, an anti-CD20 antibody, an anti-EGFR antibody, an anti-EGFRvIII antibody, an anti-Her2 antibody, an anti-CSl antibody, an anti-BCMA antibody, an anti-CEA antibody, an anti-CLL-1 antibody and an anti-CD33 antibody. The TID may comprise an antibody fragment. The CAR-EC switch may further comprise a linker. The linker may connect the CAR-ID to the TID. The linker may be connected to the unnatural amino acid within the TID. The linker may be attached to the TID by oxime ligation. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. Alternatively, or additionally, the method may further comprise administering a chimeric antigen receptor effector cell (CAR-EC). The CAR-EC may express a chimeric antigen receptor (CAR). The CAR may be encoded by a nucleotide sequence of any one of SEQ ID NOs: 1-4. The CAR may comprise an external domain that is based on or derived from an antibody. The external domain of the CAR may be based on or derived from an anti-FITC antibody. The external domain of the CAR may be based on or derived from an anti-FITC scFv. The switch and the CAR-EC may be administered simultaneously. The switch and the CAR-EC may be administered sequentially. The disease or condition may be cancer. The cancer may be prostate cancer. The cancer may be breast cancer. The cancer may be leukemia.

Disclosed herein are methods of treating a disease or condition in a subject in need thereof, the method comprising administering a chimeric antigen receptor effector cell (CAR-EC) switch to the subject, wherein the CAR-EC switch comprises: a chimeric antigen receptor-interacting domain (CAR-ID); and a target interacting domain (TID), wherein the CAR-ID and the TID do not comprise two or more amino acids linked by an amide bond. The CAR-ID may be a small molecule. The CAR-ID, by non-limiting example may be FITC. The TID may be a small molecule. The TID may be folate or a derivative thereof. The TID may interact with a surface molecule on a target that is at least 50% homologous to a prostate specific membrane antigen (PSMA). The TID may be 2-[3-(1,3-dicarboxypropyl)ureido] pentanedioic acid or a derivative thereof. The CAR-EC switch may further comprise a linker. The linker may connect the CAR-ID to the TID. The linker may be connected to the unnatural amino acid within the TID. The linker may be attached to the TID by oxime ligation. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. Alternatively, or additionally, the method may further comprise administering a chimeric antigen receptor effector cell (CAR-EC). The CAR-EC may express a chimeric antigen receptor (CAR). The CAR may be encoded by a nucleotide sequence of any one of SEQ ID NOs: 1-4. The CAR may comprise an external domain that is based on or derived from an antibody. The external domain of the CAR may be based on or derived from an anti-FITC antibody. The external domain of the CAR may be based on or derived from an anti-FITC scFv. The switch and the CAR-EC may be administered simultaneously. The switch and the CAR-EC may be administered sequentially. The disease or condition may be cancer.

The switches or the switch intermediates disclosed herein may be used in the manufacture of a medicament for the treatment of a disease. The switch may comprise a chimeric antigen receptor-interacting domain (CAR-ID) and a targeting interacting domain (TID). The switch may further comprise one or more linkers. The switch may further comprise one or more additional CAR-IDs. The switch may further comprise one or more TIDs. The CAR-IDs may comprise one or more unnatural amino acids. The TIDs may comprise one or more unnatural amino acids. The CAR-ID and the TID may be attached in a site-specific manner. Site-specific attachment of the CAR-ID to the TID may occur through the unnatural amino acid. The CAR-ID and the TID may be attached in a site-independent manner. The switch intermediate may comprise a CAR-ID and a linker. The switch intermediate may comprise a CAR-ID and a linker, wherein the linker comprises a reactive functional group. The switch intermediate may comprise a TID and a linker. The switch intermediate may comprise a TID and a linker, wherein the linker comprises a reactive functional group. The switch intermediate may comprise a CAR-ID comprising a reactive functional group. The switch intermediate may comprise a TID comprising a reactive functional group. A first switch intermediate comprising a CAR-ID may be combined with a second switch intermediate comprising a TID. A first switch intermediate comprising a CAR-ID and a linker may be combined with a second switch intermediate comprising a TID. A first switch intermediate comprising a CAR-ID may be combined with a second switch intermediate comprising a TID and a linker. A first switch intermediate comprising a CAR-ID and a first linker may be combined with a second switch intermediate comprising a TID and a second linker. The first switch intermediate may comprise a first reactive functional group. The second switch intermediate may comprise a second reactive functional group. The first reactive functional group of the first switch and second reactive functional group of the second switch may interact with each other.

Disclosed herein is use of a switch comprising a chimeric antigen receptor-interacting domain (CAR-ID) that interacts with a chimeric antigen receptor (CAR) on an effector cell; and a target interacting domain (TID) comprising an unnatural amino acid, wherein the TID interacts with a surface molecule on a target to treat a disease or condition in a subject in need thereof. The CAR-ID and the TID may be attached. The CAR-ID and the TID may be attached in a site-specific manner. Site-specific attachment of the CAR-ID to the TID may comprise attaching the CAR-ID to the unnatural amino acid in the TID. The switch may further comprise a linker. The CAR-ID may comprise a small molecule. The CAR-ID may comprise FITC. The CAR-ID may comprise one or more unnatural amino acids. The TID may comprise a polypeptide that is based on or derived from an antibody. The antibody may be selected from the group consisting of an anti-CD19 antibody, an anti-CD22 antibody, an anti-CD20 antibody, an anti-EGFR antibody, an anti-EGFRvIII antibody, an anti-Her2 antibody, an anti-CSl antibody, an anti-BCMA antibody, an anti-CEA antibody, an anti-CLL-1 antibody and an anti-CD33 antibody. The antibody may be an anti-CD 19 antibody. The antibody may be an anti-EGFR antibody. The antibody may be an anti-CD20 antibody. The antibody may be an anti-HER2 antibody. The TID may comprise an antibody fragment. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56. The TID may comprise a polypeptide comprising an unnatural amino acid. The TID may comprise a small molecule. The TID may comprise 2-[3-(1,3-dicarboxypropyl)ureido] pentanedioic acid or a derivative thereof. The TID may comprise folate. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. The disease may be cancer. The cancer may be prostate cancer. The cancer may be breast cancer. The cancer may be leukemia.

Disclosed herein is use of a switch comprising a chimeric antigen receptor-interacting domain (CAR-ID) that interacts with a chimeric antigen receptor (CAR) on an effector cell; and a target interacting domain (TID) comprising an unnatural amino acid, wherein the TID interacts with a surface molecule on a target in the manufacture of a medicament for the treatment of a disease. The CAR-ID and the TID may be attached. The CAR-ID and the TID may be attached in a site-specific manner. Site-specific attachment of the CAR-ID to the TID may comprise attaching the CAR-ID to the unnatural amino acid in the TID. The switch may further comprise a linker. The CAR-ID may comprise a small molecule. The CAR-ID may comprise FITC. The CAR-ID may comprise one or more unnatural amino acids. The TID may comprise a polypeptide that is based on or derived from an antibody. The antibody may be selected from the group consisting of an anti-CD 19 antibody, an anti-CD22 antibody, an anti-CD20 antibody, an anti-EGFR antibody, an anti-EGFRvIII antibody, an anti-Her2 antibody, an anti-CSl antibody, an anti-BCMA antibody, an anti-CEA antibody, an anti-CLL-1 antibody and an anti-CD33 antibody. The antibody may be an anti-CD 19 antibody. The antibody may be an anti-EGFR antibody. The antibody may be an anti-CD20 antibody. The antibody may be an anti-HER2 antibody. The TID may comprise an antibody fragment. The antibody may comprise an amino acid sequence of any one of SEQ ID NOs: 10-17. The antibody may be encoded by a nucleotide sequence of any one of SEQ ID NOs: 5-9. The TID may comprise a polypeptide that is based on or derived from any one of SEQ ID NOs: 18-56. The TID may comprise a polypeptide comprising an unnatural amino acid. The TID may comprise a small molecule. The TID may comprise 2-[3-(1,3-dicarboxypropyl)ureido] pentanedioic acid or a derivative thereof. The TID may comprise folate. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. The disease may be cancer. The cancer may be prostate cancer. The cancer may be breast cancer. The cancer may be leukemia.

Further disclosed herein is use of a switch comprising (a) a chimeric antigen receptor-interacting domain (CAR-ID) comprising FITC or derivative thereof; and (b) a target interacting domain (TID) that interacts with a surface molecule on a target for the treatment of a disease or a condition in a subject. The TID may comprise a polypeptide. The polypeptide may be based on or derived from an antibody or antibody fragment. The antibody may be selected from the group consisting of an anti-CD 19 antibody, an anti-CD22 antibody, an anti-CD20 antibody, an anti-EGFR antibody, an anti-EGFRvIII antibody, an anti-Her2 antibody, an anti-CSl antibody, an anti-BCMA antibody, an anti-CEA antibody, an anti-CLL-1 antibody and an anti-CD33 antibody. The antibody may be an anti-CD 19 antibody. The TID may comprise an antibody fragment. The TID may comprise a polypeptide comprising an unnatural amino acid. The CAR-ID may be attached to the TID. The CAR-ID may be site-specifically attached to the TID. The CAR-ID may be site-specifically attached to the TID via the unnatural amino acid. The CAR-ID may be attached to the TID in a site-independent manner. The switch may further comprise a linker. The linker may attach the CAR-ID to the TID. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. The TID may comprise interact with a surface molecule on a target. The surface molecule may be CD19. The CD19 may be on a B-cell. The CD19 may be on a plasma cell. The CD19 may be on a plasma B-cell. The CD19 may be on a plasmocyte. The CD19 may be on an effector B-cell. The disease or condition may be cancer. The cancer may be multiple myeloma.

Further disclosed herein is use of a switch comprising (a) a chimeric antigen receptor-interacting domain (CAR-ID) comprising FITC or derivative thereof; and (b) a target interacting domain (TID) that interacts with a surface molecule on a target for the treatment of a disease or a condition in a subject. The TID may comprise a polypeptide. The polypeptide may be based on or derived from an antibody or antibody fragment. The antibody may be selected from the group consisting of an anti-CD 19 antibody, an anti-CD22 antibody, an anti-CD20 antibody, an anti-EGFR antibody, an anti-EGFRvIII antibody, an anti-Her2 antibody, an anti-CSl antibody, an anti-BCMA antibody, an anti-CEA antibody, an anti-CLL-1 antibody and an anti-CD33 antibody. The antibody may be an anti-HER2 antibody. The TID may comprise an antibody fragment. The TID may comprise a polypeptide comprising an unnatural amino acid. The CAR-ID may be attached to the TID. The CAR-ID may be site-specifically attached to the TID. The CAR-ID may be site-specifically attached to the TID via the unnatural amino acid. The CAR-ID may be attached to the TID in a site-independent manner. The switch may further comprise a linker. The linker may attach the CAR-ID to the TID. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. The TID may comprise interact with a surface molecule on a target. The surface molecule may be Her2. The Her2 may be on a cancer cell. The Her2 may be on a breast cancer cell. The Her2 may be on a cell that originates from a breast tissue. The target may be a cell that expresses Her2. The target may be a cell that overexpresses Her2. The disease or condition may be cancer. The cancer may be breast cancer.

Further disclosed herein is use of a switch comprising (a) a chimeric antigen receptor-interacting domain (CAR-ID) comprising FITC or derivative thereof; and (b) a target interacting domain (TID) that interacts with a surface molecule on a target for the treatment of a disease or a condition in a subject. The TID may comprise a polypeptide. The polypeptide may be based on or derived from an antibody or antibody fragment. The antibody may be selected from the group consisting of an anti-CD 19 antibody, an anti-CD22 antibody, an anti-CD20 antibody, an anti-EGFR antibody, an anti-EGFRvIII antibody, an anti-Her2 antibody, an anti-CSl antibody, an anti-BCMA antibody, an anti-CEA antibody, an anti-CLL-1 antibody and an anti-CD33 antibody. The antibody may be an anti-CS1 antibody. The TID may comprise an antibody fragment. The TID may comprise a polypeptide comprising an unnatural amino acid. The CAR-ID may be attached to the TID. The CAR-ID may be site-specifically attached to the TID. The CAR-ID may be site-specifically attached to the TID via the unnatural amino acid. The CAR-ID may be attached to the TID in a site-independent manner. The switch may further comprise a linker. The linker may attach the CAR-ID to the TID. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. The TID may comprise interact with a surface molecule on a target. The surface molecule may be CS1. The CS1 may be on a B-cell. The CS1 may be on a plasma cell. The CS1 may be on a plasma B-cell. The CS1 may be on a plasmocyte. The CS1 may be on an effector B-cell. The disease or condition may be cancer. The cancer may be multiple myeloma.

Further disclosed herein is use of a switch comprising (a) a chimeric antigen receptor-interacting domain (CAR-ID) comprising FITC or derivative thereof; and (b) a target interacting domain (TID) that interacts with a surface molecule on a target for the treatment of a disease or a condition in a subject. The TID may comprise a polypeptide. The polypeptide may be based on or derived from an antibody or antibody fragment. The antibody may be selected from the group consisting of an anti-CD 19 antibody, an anti-CD22 antibody, an anti-CD20 antibody, an anti-EGFR antibody, an anti-EGFRvIII antibody, an anti-Her2 antibody, an anti-CSl antibody, an anti-BCMA antibody, an anti-CEA antibody, an anti-CLL-1 antibody and an anti-CD33 antibody. The antibody may be an anti-BCMA antibody. The TID may comprise an antibody fragment. The TID may comprise a polypeptide comprising an unnatural amino acid. The CAR-ID may be attached to the TID. The CAR-ID may be site-specifically attached to the TID. The CAR-ID may be site-specifically attached to the TID via the unnatural amino acid. The CAR-ID may be attached to the TID in a site-independent manner. The switch may further comprise a linker. The linker may attach the CAR-ID to the TID. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. The TID may comprise interact with a surface molecule on a target. The surface molecule may be BCMA. The BCMA may be on a B-cell. The BCMA may be on a plasma cell. The BCMA may be on a plasma B-cell. The BCMA may be on a plasmocyte. The BCMA may be on an effector B-cell. The disease or condition may be cancer. The cancer may be multiple myeloma.

Further disclosed herein is use of a switch comprising (a) a chimeric antigen receptor-interacting domain (CAR-ID) comprising FITC or derivative thereof; and (b) a target interacting domain (TID) that interacts with a surface molecule on a target for the treatment of a disease or a condition in a subject. The TID may comprise a polypeptide. The polypeptide may be based on or derived from an antibody or antibody fragment. The antibody may be selected from the group consisting of an anti-CD 19 antibody, an anti-CD22 antibody, an anti-CD20 antibody, an anti-EGFR antibody, an anti-EGFRvIII antibody, an anti-Her2 antibody, an anti-CSl antibody, an anti-BCMA antibody, an anti-CEA antibody, an anti-CLL-1 antibody and an anti-CD33 antibody. The antibody may be an anti-EGFRvIII antibody. The TID may comprise an antibody fragment. The TID may comprise a polypeptide comprising an unnatural amino acid. The CAR-ID may be attached to the TID. The CAR-ID may be site-specifically attached to the TID. The CAR-ID may be site-specifically attached to the TID via the unnatural amino acid. The CAR-ID may be attached to the TID in a site-independent manner. The switch may further comprise a linker. The linker may attach the CAR-ID to the TID. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. The TID may comprise interact with a surface molecule on a target. The surface molecule may be a receptor. The receptor may be a growth factor receptor. The growth factor receptor may be an EGFR. The EGFR may be an EGFRvIII. The surface molecule may be on a cell from a glioma. The surface molecule may be on a cell from a glioblastom. The surface molecule may be on a glial cell. The surface molecule may be on an ependymal cell, astrocyte or oligondendrocyte. The disease or condition may be cancer. The cancer may be a brain tumor. The cancer may be glioma. The cancer may be a glioblastoma.

Disclosed herein is use of a switch comprising (a) a chimeric antigen receptor-interacting domain (CAR-ID); and (b) a target interacting domain (TID), wherein the CAR-ID and the TID do not comprise two or more amino acids linked by an amide bond to treat a disease or condition in a subject in need thereof. The CAR-ID may comprise a small molecule. The CAR-ID may comprise FITC. The TID may comprise a small molecule. The TID may comprise 2-[3-(1,3-dicarboxypropyl)ureido] pentanedioic acid or a derivative thereof. The TID may comprise folate. The switch may further comprise a linker. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. The CAR-ID may be attached to the TID. The CAR-ID may be attached to the TID in a site-specific manner. The CAR-ID may be attached to a predetermined site on the TID. The CAR-ID may be attached to the TID by conjugation of an isothiocyanate of FITC to the TID. The CAR-ID may be attached to the TID by ester coupling. The linker may attach the CAR-ID to the TID. The linker may attach the CAR-ID to the TID by a click chemistry reaction. The linker may be attached to the CAR-ID. The linker may be chemically attached to the CAR-ID. The linker may be attached to the CAR-ID by an oxime ligation. The CAR-ID may be attached to the linker by conjugation of an isothiocyanate of FITC to the linker. The CAR-ID may be attached to the linker by ester coupling. The linker may be attached to the TID. The linker may be chemically attached to the TID. The linker may be attached to the TID by an oxime ligation. The disease or condition may be cancer. The cancer may be prostate cancer. The cancer may be ovarian cancer.

Disclosed herein is use of a small molecule CAR-EC switch comprising (a) a chimeric antigen receptor-interacting domain (CAR-ID); and (b) a target interacting domain (TID), wherein the CAR-ID and the TID do not comprise two or more amino acids linked by an amide bond in the manufacture of a medicament for the treatment of a disease. The CAR-ID may comprise a small molecule. The CAR-ID may comprise FITC. The TID may comprise a small molecule. The TID may comprise 2-[3-(1,3-dicarboxypropyl)ureido] pentanedioic acid or a derivative thereof. The TID may comprise folate. The switch may further comprise a linker. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. The CAR-ID may be attached to the TID. The CAR-ID may be attached to the TID in a site-specific manner. The CAR-ID may be attached to a predetermined site on the TID. The CAR-ID may be attached to the TID by conjugation of an isothiocyanate of FITC to the TID. The CAR-ID may be attached to the TID by ester coupling. The linker may attach the CAR-ID to the TID. The linker may attach the CAR-ID to the TID by a click chemistry reaction. The linker may be attached to the CAR-ID. The linker may be chemically attached to the CAR-ID. The linker may be attached to the CAR-ID by an oxime ligation. The CAR-ID may be attached to the linker by conjugation of an isothiocyanate of FITC to the linker. The CAR-ID may be attached to the linker by ester coupling. The linker may be attached to the TID. The linker may be chemically attached to the TID. The linker may be attached to the TID by an oxime ligation. The disease or condition may be cancer. The cancer may be prostate cancer. The cancer may be ovarian cancer.

Further disclosed herein is use of a switch comprising (a) a chimeric antigen receptor-interacting domain (CAR-ID) comprising FITC; and (b) a target interacting domain (TID) comprising 2-[3-(1,3-dicarboxypropyl)ureido] pentanedioic acid or a derivative thereof to treat a disease or condition. The disease or condition may be cancer. The cancer may be prostate cancer. The switch may further comprise a linker. linker. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. The CAR-ID may be attached to the TID. The CAR-ID may be attached to the TID in a site-specific manner. The CAR-ID may be attached to a predetermined site on the TID. The CAR-ID may be attached to the TID by conjugation of an isothiocyanate of FITC to the TID. The CAR-ID may be attached to the TID by ester coupling. The linker may attach the CAR-ID to the TID. The linker may attach the CAR-ID to the TID by a click chemistry reaction. The linker may be attached to the CAR-ID. The linker may be chemically attached to the CAR-ID. The linker may be attached to the CAR-ID by an oxime ligation. The CAR-ID may be attached to the linker by conjugation of an isothiocyanate of FITC to the linker. The CAR-ID may be attached to the linker by ester coupling. The linker may be attached to the TID. The linker may be chemically attached to the TID. The linker may be attached to the TID by an oxime ligation.

Disclosed herein is use of a switch comprising (a) a chimeric antigen receptor-interacting domain (CAR-ID) comprising FITC; and (b) a target interacting domain (TID) comprising folate or a derivative thereof to treat a disease or condition. linker. The linker may be a bifunctional linker. The linker may be a heterobifunctional linker. The linker may comprise one or more polyethylene glycol (PEG) subunits. The linker may comprise cyclooctyne. The linker may be a PEG-cyclooctyne linker. The linker may comprise an azide. The linker may comprise triazole. The triazole may be 1,2,3-triazole. The triazole may be 1,2,4-triazole. The CAR-ID may be attached to the TID. The CAR-ID may be attached to the TID in a site-specific manner. The CAR-ID may be attached to a predetermined site on the TID. The CAR-ID may be attached to the TID by conjugation of an isothiocyanate of FITC to the TID. The CAR-ID may be attached to the TID by ester coupling. The linker may attach the CAR-ID to the TID. The linker may attach the CAR-ID to the TID by a click chemistry reaction. The linker may be attached to the CAR-ID. The linker may be chemically attached to the CAR-ID. The linker may be attached to the CAR-ID by an oxime ligation. The CAR-ID may be attached to the linker by conjugation of an isothiocyanate of FITC to the linker. The CAR-ID may be attached to the linker by ester coupling. The linker may be attached to the TID. The linker may be chemically attached to the TID. The linker may be attached to the TID by an oxime ligation. The disease or condition may be cancer. The cancer may be ovarian cancer.

The methods disclosed herein may comprise administering one or more effector cells. The one or more effector cells may comprise any one of the chimeric antigen receptor effector cells (CAR-ECs) disclosed herein. The one or more effector cells may comprise a polynucleotide encoding a chimeric antigen receptor (CAR). The CAR may be encoded by a nucleotide sequence of any one of SEQ ID NOs: 1-4. The CAR may comprise an external domain that is based on or derived from an antibody. The external domain of the CAR may be based on or derived from an anti-FITC antibody. The external domain of the CAR may be based on or derived from an anti-FITC scFv. The switch and the CAR-EC may be administered simultaneously. The switch and the CAR-EC may be administered sequentially. The methods may comprise administering a first effector cell and a second effector cell. The first effector cell and the second effector cell may be different. The first effector cell and the second effector cell may be the same. The first effector cell and the second effector cell may be of the same cell type. The first effector cell and the second effector cell may be of the same cell lineage. The first effector cell may comprise a CAR having a first extracellular domain that binds a first CAR-ID of a first CAR-EC switch and the second effector cell may comprise a CAR having a second extracellular domain that binds a second CAR-ID of a second CAR-EC switch. The first CAR-ID and the second CAR-ID may be different. The first CAR-ID and the second CAR-ID may be the same. The first CAR-IEC switch and the second CAR-EC switch may be different. The first CAR-EC switch and the second CAR-EC switch may comprise different TIDs. The first CAR-EC switch and the second CAR-EC switch may be the same. The first CAR-EC switch and the second CAR-EC switch may comprise the same TID.

The CAR-EC switch may have a therapeutic effect because it brings an effector cell in proximity of a target cell. The therapeutic effect on the intended indication of the CAR-EC switch may be due to the CAR-EC switch recruiting an effector cell to the target cell. The therapeutic effect on the intended indication of the CAR-EC switch may be wholly due to the CAR-EC switch recruiting an effector cell to the target cell. The therapeutic effect on the intended indication of the CAR-EC switch may be predominantly due to the CAR-EC switch recruiting an effector cell to the target cell.

The therapeutic effect of the intended indication may be due to the CAR-EC switch recruiting a protein, peptide or biomolecule to the target cell. The therapeutic effect of the intended indication may be wholly due to the CAR-EC switch recruiting a protein, peptide or biomolecule to the target cell. The therapeutic effect on the intended indication may be at least partially due to the CAR-EC switch recruiting a protein, peptide or biomolecule to the target cell.

Administering the CAR-EC switch may not have any therapeutic effect without further administering an effector cell. The CAR-EC switch may not have a significant, desirable and/or intended therapeutic effect without further administering an effector cell. The CAR-EC switch may not have any therapeutic effect towards an intended indication of the CAR-EC platform without further administering an effector cell. A portion or component of the CAR-EC switch (e.g. TID) may not have a therapeutic effect towards the intended indication of the CAR-EC switch without being conjugated to a second portion or component of the CAR-EC switch (e.g. CAR-ID). The dose of a portion or component of the CAR-EC switch (e.g. CAR-ID, TID) when administered as part of the CAR-EC platform to provide a therapeutic effect may not have a therapeutic effect when the portion or component of the CAR-EC switch is administered alone at that dose. The portion or component of the CAR-EC switch may not be intended to have any therapeutic effect besides recruiting the effector cell to the target cell. The portion or component of the CAR-EC switch may have a therapeutic effect on the target cell, wherein the therapeutic effect is negligible relative to the therapeutic effect of the CAR-EC switch recruiting the effector cell, protein, peptide or biomolecule to the target cell. The portion or component of the CAR-EC switch may have a therapeutic effect on the target cell, wherein the therapeutic effect is less than the therapeutic effect of recruiting the effector cell, to the target cell. The binding of the portion or component of the CAR-EC switch to the target cell may induce an unintentional response from the target cell. The binding of the portion or component of the CAR-EC switch to the target cell may induce an unintentional therapeutic effect in addition to the therapeutic effect of recruiting the effector cell, protein, peptide or biomolecule to the target cell.

Disclosed herein are platforms, kits and methods for treating a disease or condition in a subject. The subject may be suffering from a disease or condition. The subject may be suffering from more than one disease or condition. The subject may be suffering from chronic lymphocytic leukemia. The subject may be suffering from acute lymphoblastic leukemia. The subject may be an animal. The subject may be a mammal. The mammal may be a human, a chimpanzee, a gorilla, a monkey, a bovine, a horse, a donkey, a mule, a dog, a cat, a pig, a rabbit, a goat, a sheep, a rat, a hamster, a guinea pig or a mouse. The subject may be a bird or a chicken. The subject may be a human. The subject may be an adult. The subject may be a child. The child may be suffering from acute lymphoblastic leukemia. The subject may be less than 6 months old. The subject may be at least about 1 year old, about 2 years old, about 3 years old, about 4 years old, about 5 years old, about 6 years old, about 7 years old, about 8 years old, about 9 years old, about 10 years old, about 11 years old, about 12 years old, about 13 years old, about 14 years old, or about 15 years old. The subject may be at least about 16 years old, about 17 years, or about 18 years old. The subject may be at least about 19 years old, about 20 years old, or about 25 years old. The subject may be at least about 30 years old, about 35 years old, about 40 years old, about 45 years old, about 50 years old, about 55 years old. The subject may be at least about 60 years old, about 65 years old, about 70 years old, about 75 years old, about 80 years old, or about 85 years old. The subject may be about 90 years old, about 95 years old, about 100 years old or about 105 years old. The subject may be greater than 65 years old. The subject may be less than 30 years old.

The switches and switch intermediates disclosed herein may be used to treat one or more diseases or conditions in a subject in need thereof. The disease or condition may be a cancer, a pathogenic infection, autoimmune disease, inflammatory disease, or genetic disorder.

In some instances, the one or more diseases comprises a cancer. The cancer may comprise a recurrent and/or refractory cancer. The cancer may be an acute cancer. The cancer may be a chronic cancer. The cancer may be a recurrent cancer. The cancer may be a refractory cancer accelerated. The cancer may be in remission. The cancer may be a stage I, stage II, stage III, or stage IV cancer. The cancer may be a juvenile cancer. The cancer may be an adult cancer. Examples of cancers include, but are not limited to, sarcomas, carcinomas, lymphomas or leukemias. The disease or condition may be a cell proliferative disorder. The cell proliferative disorder may be selected from a solid tumor, a lymphoma, a leukemia and a liposarcoma. The cancer may be selected from myelogenous leukemia, lymphoblastic leukemia, myeloid leukemia, an acute myeloid leukemia, myelomonocytic leukemia, neutrophilic leukemia, myelodysplastic syndrome, B-cell lymphoma, burkitt lymphoma, large cell lymphoma, mixed cell lymphoma, follicular lymphoma, mantle cell lymphoma, hodgkin lymphoma, recurrent small lymphocytic lymphoma, hairy cell leukemia, multiple myeloma, basophilic leukemia, eosinophilic leukemia, megakaryoblastic leukemia, monoblastic leukemia, monocytic leukemia, erythroleukemia, erythroid leukemia and hepatocellular carcinoma. The cancer may comprise a hematological malignancy. The hematological malignancy may comprise a B cell malignancy. The cancer may comprise a chronic lymphocytic leukemia. The cancer may comprise an acute lymphoblastic leukemia. The cancer may comprise a CD19-positive Burkitt's lymphoma.

The cancer may comprise a neuroendocrine cancer. The cancer may comprise a pancreatic cancer. The cancer may comprise an exocrine pancreatic cancer. The cancer may comprise a thyroid cancer. The thyroid cancer may comprise a medullary thyroid cancer.

The cancer may comprise a prostate cancer. The prostate cancer may be a PSMA-positive prostate cancer. PSMA expression may be highly upregulated and restricted to cancer cells in some or all stages of the prostate cancer. The cancer may be hormone-refractory prostate cancer.

The cancer may comprise an epithelial cancer. The cancer may comprise a breast cancer. The cancer may comprise an endometrial cancer. The cancer may comprise an ovarian cancer. The ovarian cancer may comprise a stromal ovarian cancer. The cancer may comprise a cervical cancer.

The cancer may comprise a skin cancer. The skin cancer may comprise a neo-angiogenic skin cancer. The skin cancer may comprise a melanoma.

The cancer may comprise a kidney cancer.

The cancer may comprise a lung cancer. The lung cancer may comprise a small cell lung cancer. The lung cancer may comprise a non-small cell lung cancer.

The cancer may comprise a colorectal cancer. The cancer may comprise a gastric cancer. The cancer may comprise a colon cancer.

The cancer may comprise a brain cancer. The brain cancer may comprise a brain tumor. The cancer may comprise a glioblastoma. The cancer may comprise an astrocytoma.

The cancer may comprise a blood cancer. The blood cancer may comprise a leukemia. The leukemia may comprise a myeloid leukemia. The cancer may comprise a lymphoma. The lymphoma may comprise a non-Hodgkin's lymphoma.

The cancer may comprise a sarcoma. Generally, sarcomas are cancers of the bone, cartilage, fat, muscle, blood vessels, or other connective or supportive tissue. Sarcomas include, but are not limited to, bone cancer, fibrosarcoma, chondrosarcoma, Ewing's sarcoma, malignant hemangioendothelioma, malignant schwannoma, bilateral vestibular schwannoma, osteosarcoma, soft tissue sarcomas (e.g. alveolar soft part sarcoma, angiosarcoma, cystosarcoma phylloides, dermatofibrosarcoma, desmoid tumor, epithelioid sarcoma, extraskeletal osteosarcoma, fibrosarcoma, hemangiopericytoma, hemangiosarcoma, Kaposi's sarcoma, leiomyosarcoma, liposarcoma, lymphangiosarcoma, lymphosarcoma, malignant fibrous histiocytoma, neurofibrosarcoma, rhabdomyosarcoma, and synovial sarcoma). The sarcoma may comprise an Ewing's sarcoma.

The cancer may be a carcinoma. Generally, carcinomas are cancers that begin in the epithelial cells, which are cells that cover the surface of the body, produce hormones, and make up glands. By way of non-limiting example, carcinomas include breast cancer, pancreatic cancer, lung cancer, colon cancer, colorectal cancer, rectal cancer, kidney cancer, bladder cancer, stomach cancer, prostate cancer, liver cancer, ovarian cancer, brain cancer, vaginal cancer, vulvar cancer, uterine cancer, oral cancer, penile cancer, testicular cancer, esophageal cancer, skin cancer, cancer of the fallopian tubes, head and neck cancer, gastrointestinal stromal cancer, adenocarcinoma, cutaneous or intraocular melanoma, cancer of the anal region, cancer of the small intestine, cancer of the endocrine system, cancer of the thyroid gland, cancer of the parathyroid gland, cancer of the adrenal gland, cancer of the urethra, cancer of the renal pelvis, cancer of the ureter, cancer of the endometrium, cancer of the cervix, cancer of the pituitary gland, neoplasms of the central nervous system (CNS), primary CNS lymphoma, brain stem glioma, and spinal axis tumors. In some instances, the cancer is a skin cancer, such as a basal cell carcinoma, squamous, melanoma, nonmelanoma, or actinic (solar) keratosis.

In some instances, the cancer is a lung cancer. Lung cancer may start in the airways that branch off the trachea to supply the lungs (bronchi) or the small air sacs of the lung (the alveoli). Lung cancers include non-small cell lung carcinoma (NSCLC), small cell lung carcinoma, and mesotheliomia. Examples of NSCLC include squamous cell carcinoma, adenocarcinoma, and large cell carcinoma. The mesothelioma may be a cancerous tumor of the lining of the lung and chest cavity (pleura) or lining of the abdomen (peritoneum). The mesothelioma may be due to asbestos exposure. The cancer may be a brain cancer, such as a glioblastoma.

Alternatively, the cancer may be a central nervous system (CNS) tumor. CNS tumors may be classified as gliomas or nongliomas. The glioma may be malignant glioma, high grade glioma, diffuse intrinsic pontine glioma. Examples of gliomas include astrocytomas, oligodendrogliomas (or mixtures of oligodendroglioma and astocytoma elements), and ependymomas. Astrocytomas include, but are not limited to, low-grade astrocytomas, anaplastic astrocytomas, glioblastoma multiforme, pilocytic astrocytoma, pleomorphic xanthoastrocytoma, and subependymal giant cell astrocytoma. Oligodendrogliomas include low-grade oligodendrogliomas (or oligoastrocytomas) and anaplastic oligodendriogliomas. Nongliomas include meningiomas, pituitary adenomas, primary CNS lymphomas, and medulloblastomas. In some instances, the cancer is a meningioma.

The leukemia may be an acute lymphocytic leukemia, acute myelocytic leukemia, chronic lymphocytic leukemia, or chronic myelocytic leukemia. Additional types of leukemias include hairy cell leukemia, chronic myelomonocytic leukemia, and juvenile myelomonocytic leukemia.

Lymphomas are cancers of the lymphocytes and may develop from either B or T lymphocytes. The two major types of lymphoma are Hodgkin's lymphoma, previously known as Hodgkin's disease, and non-Hodgkin's lymphoma. Hodgkin's lymphoma is marked by the presence of the Reed-Sternberg cell. Non-Hodgkin's lymphomas are all lymphomas which are not Hodgkin's lymphoma. Non-Hodgkin lymphomas may be indolent lymphomas and aggressive lymphomas. Non-Hodgkin's lymphomas include, but are not limited to, diffuse large B cell lymphoma, follicular lymphoma, mucosa-associated lymphatic tissue lymphoma (MALT), small cell lymphocytic lymphoma, mantle cell lymphoma, Burkitt's lymphoma, mediastinal large B cell lymphoma, Waldenstrom macroglobulinemia, nodal marginal zone B cell lymphoma (NMZL), splenic marginal zone lymphoma (SMZL), extranodal marginal zone B cell lymphoma, intravascular large B cell lymphoma, primary effusion lymphoma, and lymphomatoid granulomatosis.

The cancer may comprise a solid tumor. The cancer may comprise a sarcoma. The cancer may be selected from a group consisting of a bladder cancer, a breast cancer, a colon cancer, a rectal cancer, an endometrial cancer, a kidney cancer, a lung cancer, melanoma, a myeloma, a thyroid cancer, a pancreatic cancer, a glioma, a malignant glioma of the brain, a glioblastoma, an ovarian cancer, a prostate cancer and a PSMA-positive prostate cancer. The cancer may have non-uniform antigen expression. The cancer may have modulated antigen expression. The antigen may be a surface antigen. The cancer may not comprise a myeloma. The cancer may not comprise a melanoma. The cancer may not comprise a colon cancer. The cancer may be acute lymphoblastic leukemia (ALL). The cancer may be relapsed ALL. The cancer may be refractory ALL. The cancer may be relapsed, refractory ALL. The cancer may be chronic lymphocytic leukemia (CLL). The cancer may be relapsed CLL. The cancer may be refractory CLL. The cancer may be relapsed, refractory CLL.

The cancer may comprise a breast cancer. The breast cancer may be triple positive breast cancer (estrogen receptor, progesterone receptor and Her2 positive). The breast cancer may be triple negative breast cancer (estrogen receptor, progesterone receptor and Her2 negative). The breast cancer may be estrogen receptor positive. The breast cancer may be estrogen receptor negative. The breast cancer may be progesterone receptor positive. The breast cancer may be progesterone receptor negative. The breast cancer may comprise a Her2 negative breast cancer. The breast cancer may comprise a low-expressing Her2 breast cancer. The breast cancer may comprise a Her2 positive breast cancer. Cell lines expressing Her2 have been well-characterized for antigen density, reflecting clinical immunohistochemistry characterization which classifies malignancies as 0 (<20,000 Her2 antigens per cell), 1+ (100,000 Her2 antigens per cell), 2+

(500,000 Her2 antigens per cell), and 3+ (>2,000,000 Her2 antigens per cell). The present invention provides for methods of treating breast cancers of these classifications. The breast cancer may comprise a breast cancer classified as Her2 0. The breast cancer may comprise a breast cancer classified as Her2 1+. The breast cancer may comprise a breast cancer classified as Her2 2+. The breast cancer may comprise a breast cancer classified as a Her2 3+.

The disease or condition may be a pathogenic infection. Pathogenic infections may be caused by one or more pathogens. In some instances, the pathogen is a bacterium, fungi, virus, or protozoan.

Exemplary pathogens include but are not limited to: Bordetella, Borrelia, Brucella, Campylobacter, Chlamydia, Chlamydophila, Clostridium, Corynebacterium, Enterococcus, Escherichia, Francisella, Haemophilus, Helicobacter, Legionella, Leptospira, Listeria, Mycobacterium, Mycoplasma, Neisseria, Pseudomonas, Rickettsia, Salmonella, Shigella, Staphylococcus, Streptococcus, Treponema, Vibrio, or Yersinia. In some cases, the disease or condition caused by the pathogen is tuberculosis and the heterogeneous sample comprises foreign molecules derived from the bacterium Mycobacterium tuberculosis and molecules derived from the subject. In some instances, the disease or condition is caused by a bacterium is tuberculosis, pneumonia, which may be caused by bacteria such as Streptococcus and Pseudomonas, a foodborne illness, which may be caused by bacteria such as Shigella, Campylobacter and Salmonella, and an infection such as tetanus, typhoid fever, diphtheria, syphilis and leprosy. The disease or condition may be bacterial vaginosis, a disease of the vagina caused by an imbalance of naturally occurring bacterial flora. Alternatively, the disease or condition is bacterial meningitis, bacterial inflammation of the meninges (e.g., the protective membranes covering the brain and spinal cord). Other diseases or conditions caused by bacteria include, but are not limited to, bacterial pneumonia, a urinary tract infection, bacterial gastroenteritis, and bacterial skin infection. Examples of bacterial skin infections include, but are not limited to, impetigo which may be caused by Staphylococcus aureus or Streptococcus pyogenes; erysipelas which may be caused by a streptococcus bacterial infection of the deep epidermis with lymphatic spread; and cellulitis which may be caused by normal skin flora or by exogenous bacteria.

The pathogen may be a fungus, such as, *Candida, Aspergillus, Cryptococcus, Histoplasma, Pneumocystis,* and *Stachybotrys.* Examples of diseases or conditions caused by a fungus include, but are not limited to, jock itch, yeast infection, ringworm, and athlete's foot.

The pathogen may be a virus. Examples of viruses include, but are not limited to, adenovirus, coxsackievirus, Epstein-Barr virus, Hepatitis virus (e.g., Hepatitis A, B, and C), herpes simplex virus (type 1 and 2), cytomegalovirus, herpes virus, HIV, influenza virus, measles virus, mumps virus, papillomavirus, parainfluenza virus, poliovirus, respiratory syncytial virus, rubella virus, and varicella-zoster virus. Examples of diseases or conditions caused by viruses include, but are not limited to, cold, flu, hepatitis, AIDS, chicken pox, rubella, mumps, measles, warts, and poliomyelitis.

The pathogen may be a protozoan, such as Acanthamoeba (e.g., A. astronyxis, A. castellanii, A. culbertsoni, A. hatchetti, A. polyphaga, A. rhysodes, A. healyi, A. divionensis), Brachiola (e.g., B connori, B. vesicularum), Cryptosporidium (e.g., C. parvum), Cyclospora (e.g., C. cayetanensis), Encephalitozoon (e.g., E. cuniculi, E. hellem, E. intestinalis), Entamoeba (e.g., E. histolytica), Enterocytozoon (e.g., E. bieneusi), Giardia (e.g., G. lamblia), Isospora (e.g, I. belli), Microsporidium (e.g., M. africanum, M. ceylonensis), Naegleria (e.g., N. fowleri), Nosema (e.g., N. algerae, N. ocularum), Pleistophora, Trachipleistophora (e.g., T. anthropophthera, T. hominis), and Vittaforma (e.g., V. corneae).

The disease or condition may be an autoimmune disease or autoimmune related disease. An autoimmune disorder may be a malfunction of the body's immune system that causes the body to attack its own tissues. Examples of autoimmune diseases and autoimmune related diseases include, but are not limited to, Addison's disease, alopecia areata, ankylosing spondylitis, antiphospholipid syndrome (APS), autoimmune aplastic anemia, autoimmune hemolytic anemia, autoimmune hepatitis, autoimmune myocarditis, Behcet's disease, celiac sprue, Crohn's disease, dermatomyositis, eosinophilic fasciitis, erythema nodosum, giant cell arteritis (temporal arteritis), Goodpasture's syndrome, Graves' disease, Hashimoto's disease, idiopathic thrombocytopenic purpura (ITP), IgA nephropathy, juvenile arthritis, diabetes, juvenile diabetes, Kawasaki syndrome, Lambert-Eaton syndrome, lupus (SLE), mixed connective tissue disease (MCTD), multiple sclerosis, myasthenia gravis, pemphigus, polyarteritis nodosa, type I, II, & III autoimmune polyglandular syndromes, polymyalgia rheumatica, polymyositis, psoriasis, psoriatic arthritis, Reiter's syndrome, relapsing polychondritis, rheumatoid arthritis, sarcoidosis, scleroderma, Sjogren's syndrome, sperm & testicular autoimmunity, stiff person syndrome, Takayasu's arteritis, temporal arteritis/giant cell arteritis, ulcerative colitis, uveitis, vasculitis, vitiligo, and Wegener's granulomatosis.

The disease or condition may be an inflammatory disease. Examples of inflammatory diseases include, but are not limited to, alveolitis, amyloidosis, angiitis, ankylosing spondylitis, avascular necrosis, Basedow's disease, Bell's palsy, bursitis, carpal tunnel syndrome, celiac disease, cholangitis, chondromalacia patella, chronic active hepatitis, chronic fatigue syndrome, Cogan's syndrome, congenital hip dysplasia, costochondritis, Crohn's Disease, cystic fibrosis, De Quervain's tendinitis, diabetes associated arthritis, diffuse idiopathic skeletal hyperostosis, discoid lupus, Ehlers-Danlos syndrome, familial mediterranean fever, fascitis, fibrositis/fibromyalgia, frozen shoulder, ganglion cysts, giant cell arteritis, gout, Graves' Disease, HIV-associated rheumatic disease syndromes, hyperparathyroid associated arthritis, infectious arthritis, inflammatory bowel syndrome/ irritable bowel syndrome, juvenile rheumatoid arthritis, lyme disease, Marfan's Syndrome, Mikulicz's Disease, mixed connective tissue disease, multiple sclerosis, myofascial pain syndrome, osteoarthritis, osteomalacia, osteoporosis and corticosteroid-induced osteoporosis, Paget's Disease, palindromic rheumatism, Parkinson's Disease, Plummer's Disease, polymyalgia rheumatica, polymyositis, pseudogout, psoriatic arthritis, Raynaud's Phenomenon/Syndrome, Reiter's Syndrome, rheumatic fever, rheumatoid arthritis, sarcoidosis, sciatica (lumbar radiculopathy), scleroderma, scurvy, sickle cell arthritis, Sjogren's Syndrome, spinal stenosis, spondyloisthesis, Still's Disease, systemic lupus erythematosis, Takayasu's (Pulseless) Disease, Tendinitis, tennis elbow/golf elbow, thyroid associated arthritis, trigger finger, ulcerative colitis, Wegener's Granulomatosis, and Whipple's Disease.

Methods of treatment disclosed herein may comprise off-target activity as measured by cytokine levels. The method may reduce the off-target activity, as measured by cytokine levels, when compared to other CAR-EC therapies. The method may reduce the off-target activity as measured by interferon gamma levels. Other off-target activities that may be reduced include toxic lymphophenia, fatal cytolysis of solid tumor targets and chronic hypogammaglobulinemia for hematological targets. Methods of treatment and compositions disclosed herein may be used to treat a cancer comprising CD19-mediated B cell aplasia. The methods and compositions may minimize the CD19-mediated B cell aplasia. The method may avoid long-term B-cell aplasia.

The CAR-EC platforms, methods and compositions disclosed herein may be used to treat a heterogeneous tumor or a heterogeneous blood cell malignancy in a subject in need thereof. The "pan-B cell" marker CD20 is the most prevalently targeted antigen for B cell neoplasms and the FDA-approved antibody rituximab is a vital component in the treatment of many leukemias and lymphomas. However, resistance mechanisms related to modulation of CD20 antigen expression occurs in a significant number of patients. It is clear that targeting with either CD 19 or CD20 antigen alone is insufficient for a curative therapy. The methods disclosed herein provide for construction and administration of two or more switches with different specificities (e.g. an anti-CD19 antibody CAR-EC switch and an anti-CD20 antibody CAR-EC switch). The methods disclosed herein provide for construction and administration of two or more switches with different specificities (e.g. an anti-CD19 antibody CAR-EC switch and an anti-CD22 antibody CAR-EC switch). A heterogeneous tumor or heterogeneous blood cell malignancy may be treated with a first antibody CAR-EC switch and a second antibody CAR-EC switch, wherein the CAR-EC switch binds two different targets. One or more CAR-EC switches may be administered sequentially or simultaneously.

The CAR-EC switch may be administered with one or more additional therapeutic agents. The one or more additional therapeutic agents may be selected from a group consisting of an immunotherapy, a chemotherapy and a steroid. The one or more additional therapeutic agents may be a chemotherapy drug. The chemotherapy drug may be an alkylating agent, an antimetabolite, an anthracycline, a topoisomerase inhibitor, a mitotic inhibitor, a corticosteroid or a differentiating agent. The chemotherapy drug may be selected from actinomycin-D, bleomycin, altretamine, bortezomib, busulfan, carboplatin, capecitabine, carmustine, chlorambucil, cisplatin, cladribine, clofarabine, cyclophosphamide, cytarabine, dacarbazine, daunorubicin, docetaxel, doxorubicin, epirubicin, etoposide, estramustine, floxuridine, fludarabine, fluorouracil, gemcitbine (Gemzar), hydroxyurea, idarubicin, ifosfamide, irinotecan (Camptosar), ixabepilone, L-asparaginase, lomustine, mechlorethamine, melphalan, 6-mercaptopurine, methotrexate, mitomycin-C, paclitaxel (Taxol), pemetrexed, pentostatin, streptozocin, temozolomide, teniposide, thioguanine, thiotepa, topotecan (Hycamtin), vincristine, vinblastine, vinorelbine, retinoids, tretinoin (ATRA or Atralin®), bexarotene (Targretin®) and arsenic trioxide (Arsenox®). The chemotherapy may be administered as a pill to swallow, as an injection into the muscle or fat tissue, intravenously, topically or directly into a body cavity.

The one or more additional therapeutic agents may comprise an angiogenesis inhibitor. The angiogenesis inhibitor may be selected from bevacizumab, itraconazole, carboxyamidotriazole, TNP-470, CM101, IFN alpha, IL-12, platelet factor 4, suramin, SU5416, thrombospondin, a VEGFR antagonist, an angiostatic steroid with heparin, CAR-ECilage-derived angiogenesis inhibitory factor, matrix metalloprotease inhibitors, angiostatin, endostatin, sorafenib, sunitinib, pazopanib, everolimus, 2-methoxyestradiol, tecogalan, tetrathiomolybdate, thalidomide, prolactin, αvβ₃ inhibitor, linomide, tasquinimod, soluble VEGFR-1, soluble NRP-1, angiopoietin 2, vasostatin, calreticulin, TIMP, CDAI, Meth-1, Meth-2, interferon-alpha, interferon-beta, interferon-gamma, CXCL10, IL-4, IL-12, IL-18, prothrombin, antithrombin III fragment, prolactin, VEGI, SPARC, osteopontin, maspin, canstatin, proliferin-related protein and restin.

The one or more additional therapeutic agents may comprise a hormone therapy. The hormone therapy may be selected from an anti-estrogen (e.g. fulvestrant (Faslodex®), tamoxifen, toremifene (Fareston®)); an aromatase inhibitor (e.g. anastrozole (Arimidex®), exemestane (Aromasin®), letrozole (Femara®)); a progestin (e.g. megestrol acetate (Megace®)); an estrogen; an anti-androgen (e.g. bicalutamide (Casodex®), flutamide (Eulexin®), nilutamide (Nilandron®)); a gonadotropin-releasing hormone (GnRH) or luteinizing hormone-releasing hormone (LHRH) agonist or analog (e.g. leuprolide (Lupron®), goserelin (Zoladex®)).

The one or more additional therapeutic agents may comprise a steroid. The steroid may be a corticosteroid. The steroid may be cortisol or a derivative thereof. The steroid may be selected from prednisone, methylprednisolone (Solumedrol®) or dexamethasone.

The CAR-EC switch may be administered with one or more additional therapies. The one or more additional therapies may comprise laser therapy. The one or more additional therapies may comprise radiation therapy. The one or more additional therapies may comprise surgery.

### CAR-EC Regulators

In some instances, it may be desirable to remove the inactive chimeric antigen receptor-effector cells (CAR-ECs) from a subject or to down regulate the expression of the chimeric antigen receptor (CAR) by the CAR-EC. For instance, a CAR-EC may elicit an immune response from the subject and it may be desirable to down regulate the expression of the CAR to eliminate the immune response. Alternatively, it may be desirable to trigger cell death of the CAR-EC. Disclosed herein are methods of regulating CAR-ECs in a subject, the method comprising administering a CAR-EC regulator. Unlike the switches disclosed herein which activate the CAR-EC and direct the activity of the CAR-EC to a target, the CAR-EC regulator may interact with a surface molecule on a CAR-EC to induce down regulation of the expression of the CAR or to induce programmed cell death of the CAR-EC. The CAR-EC may comprise a polynucleotide that encodes for the CAR. The expression of the CAR may controlled by a promoter. The promoter may be a regulatable promoter. For example, expression of the CAR may be controlled by a Tet promoter. In the absence of tetracycline or a derivative thereof (e.g., doxycycline), the CAR-EC may express the CAR. In the presence of tetracycline or derivative thereof, expression of the CAR is inhibited. Thus, the CAR-EC regulator may comprise a molecule (e.g., tetracycline, doxycycline) that inhibits the expression of the CAR. The expression of the CAR may be controlled by a chimeric regulatable system. The CAR-EC regulator may comprise a transactivator that can interact with specific sequences that have been engineered into the polynucleotide encoding the CAR. Chimeric systems may be regulated by tetracycline, progesterone antagonist RU486, insect hormone ecdysone, or rapamycin (FK506). These drugs or hormones (or their analogs) may act on modulator transactivators composed of natural or mutant ligand binding domains and intrinsic or extrinsic DNA binding and transcriptional activation domains. (Agha-Mohammadi and Lotze, Regulatable systems: applications in gene therapy and replicating viruses, J Clin Invest., 105(9):1177-1183, 2000).

The CAR-EC regulator may comprise a molecule that interacts with the CAR of the CAR-EC. The CAR-EC regulator may compete with the CAR-EC switch for the CAR on the CAR-EC. The CAR-EC regulator may comprise a small molecule. The CAR-EC regulator may comprise a peptide. The peptide may be an antibody or antibody fragment.

The CAR-EC regulator may further comprise a drug or a toxin. The drug or toxin may be conjugated to the portion of the CAR-EC regulator that interacts with the CAR of the CAR-EC. The drug or toxin may be selected from maytasine (e.g. DM1, DM4), monomethylauristatin E, monomethylauristatin F, Ki-4.dgA, dolastatin 10, calicheamicin, SN-38, duocarmycin, irinotecan, ricin, saporin, gelonin, poke weed antiviral protein, *pseudomonas aeruginosa* exotoxin A or diphtheria toxin. The toxin may comprise a poison, a bacterial toxin (e.g. bacterial toxins causing tetanus, diphtheria), a plant toxin or animal toxin. The toxin may be a snake venom. The toxin may comprise vinblastine. The toxin may comprise auristatin. The toxin may be contained in a liposome membrane-coated vesicle. The antibody may be attached to the liposome membrane-coated vesicle.

**FIG. 17A-C** show schematics of exemplary CAR regulator-CAR-EC interactions. As shown in **FIG. 17A**, a chimeric antigen receptor effector cell (CAR-EC) (**1701**) may comprise a chimeric antigen receptor (**1704**) and a costimulatory molecule (**1720**). The CAR (**1704**) may comprise an external domain (**1715**), a transmembrane domain (**1710**) and an internal domain (**1705**). The CAR-EC regulator (**1725**) may interact with the external domain (**1715**) of the CAR (**1704**). The attachment of the CAR-EC regulator (**1725**) to the CAR (**1704**) may induce apoptosis of CAR-EC. The attachment of the CAR-EC regulator (**1725**) to the CAR (**1704**) may induce activation-induced cell death of CAR-EC. The attachment of the CAR-EC regulator (**1725**) to the CAR (**1704**) may induce autophagy of CAR-EC. The attachment of the CAR-EC regulator (**1725**) to the CAR (**1704**) may induce down regulation of the CAR. The attachment of the CAR-EC regulator (**1725**) to the CAR (**1704**) may prevent the CAR-EC switch from attaching to the CAR.

As shown in **FIG**. **17B**, a chimeric antigen receptor effector cell (CAR-EC) (**1730**) may comprise a chimeric antigen receptor (CAR) (**1731**), a costimulatory molecule (**1750**) and a surface molecule (**1755**). The CAR (**1731**) may comprise an external domain (**1745**), a transmembrane domain (**1740**) and an internal domain (**1735**). The CAR-EC regulator (**1760**) may comprise a first end (**1765**) that interacts with the external domain (**1715**) of the CAR (**1731**) and a second end (**1770**) that interacts with the surface molecule (**1755**) on the CAR-EC. The attachment of the one end of the CAR-EC regulator (**1760**) to the CAR (**1731**) and the surface molecule (**1755**) of the CAR-EC (**1730**) may induce apoptosis of CAR-EC. The attachment of the one end of the CAR-EC regulator (**1760**) to the CAR (**1731**) and the surface molecule (**1755**) of the CAR-EC (**1730**) may induce activation-induced cell death of CAR-EC. The attachment of the one end of the CAR-EC regulator (**1760**) to the CAR (**1731**) and the surface molecule (**1755**) of the CAR-EC (**1730**) may induce autophagy of CAR-EC. The attachment of the one end of the CAR-EC regulator (**1760**) to the CAR (**1731**) and the surface molecule (**1755**) of the CAR-EC (**1730**) may induce down regulation of the CAR. The attachment of one end of the CAR-EC regulator (**1765**) to the CAR (**1731**) may prevent the CAR-EC switch from attaching to the CAR.

As shown in **FIG. 17C**, a chimeric antigen receptor effector cell (CAR-EC) (**1775**) may comprise a chimeric antigen receptor (1774) and a costimulatory molecule (**1779**). The CAR (**1774**) may comprise an external domain (**1778**), a transmembrane domain (**1777**) and an internal domain (**1776**). The CAR-EC regulator (**1780**) may comprise a first region (**1781**) that interacts with the external domain (**1778**) of the CAR (**1774**) on the effector cell. The CAR-EC regulator (**1780**) may further comprise a second region (**1782**) that interacts with a surface molecule (**1791**) on another cell (**1790**). The cell (**1790**) may secrete cytokines or other molecules that can interact with the CAR-EC. The interaction of the cytokines or other molecules with the CAR-EC may induce apoptosis of CAR-EC. The interaction of the cytokines or other molecules with the CAR-EC may induce activation-induced cell death of CAR-EC. The interaction of the cytokines or other molecules with the CAR-EC may induce autophagy of CAR-EC. The interaction of the cytokines or other molecules with the CAR-EC may induce down regulation of the CAR. The attachment of one end of the CAR-EC regulator (**1781**) to the CAR (**1774**) may prevent the CAR-EC switch from attaching to the CAR.

The CAR-EC may comprise an effector cell that is modified to express a surface molecule that can interact with the CAR-EC regulator. The surface molecule on the CAR-EC may be a viral protein or fragment thereof. Alternatively or additionally, the effector cell expresses a viral protein or fragment thereof that is not a cell surface marker. The effector cell expressing a viral protein or fragment thereof may be targeted with a drug. Wherein the effector cell comprises a viral protein or fragment thereof, the drug may be selected from a group comprising abacavir, acyclovir, adefovir, amantadine, amprenavir, ampligen, arbidol, atazanavir, atripla, balavir, boceprevirertet, cidofovir, combivir, darunavir, delavirdine, didanosine, docosanol, edoxudine, efavirenz, emtricitabine, enfuvirtide, entecavir, an entry inhibitor, famciclovir, a fixed dose combination antiretroviral drug, fomivirsen, fosamprenavir, foscarnet, fosfonet, a fusion inhibitor, ganciclovir, ibacitabine, imunovir, idoxuridine, imiquimod, indinavir, inosine, integrase inhibitor, interferon type III, interferon type II, interferon type I, interferon, lamivudine, lopinavir, loviride, maraviroc, moroxydine, methisazone, nelfinavir, nevirapine, nexavir, nucleoside analogue, oseltamivir, peginterferon alfa-2a, penciclovir, peramivir, pleconaril, podophyllotoxin, protease inhibiro, raltegravir, a reverse transcriptase inhibitor, ribavirin, rimantadine, ritonavir, pyramidine, saquinavir, sofosbuvir, stavudine, a synergistic enhancer retroviral durg, tea tree oil, telaprevir, tenofovir, tenofovir disoproxil, tipranavir, trifluridine, trizivir, tromantadine, truvada, valaciclovir, vicriviroc, vidarabine, viramidine, zacitabine, zanamivir or zidovudine. The drug may be ganciclovir. The drug may be acyclovir.

### Pharmaceutical Compositions

The switches, switch intermediates and CAR-ECs disclosed herein may be used in the formulation of one or more compositions. The compositions may be pharmaceutical compositions. The compositions comprising one or more switches, switch intermediates and/or CAR-ECs may further comprise one or more pharmaceutically acceptable salts, excipients or vehicles. Pharmaceutically acceptable salts, excipients, or vehicles include, but are not limited to, carriers, excipients, diluents, antioxidants, preservatives, coloring, flavoring and diluting agents, emulsifying agents, suspending agents, solvents, fillers, bulking agents, buffers, delivery vehicles, tonicity agents, cosolvents, wetting agents, complexing agents, buffering agents, antimicrobials, and surfactants. Neutral buffered saline or saline mixed with serum albumin are exemplary appropriate carriers.

The compositions may include antioxidants such as ascorbic acid; low molecular weight polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, pluronics, or polyethylene glycol (PEG). Also by way of example, suitable tonicity enhancing agents include alkali metal halides (preferably sodium or potassium chloride), mannitol, sorbitol, and the like. Suitable preservatives include benzalkonium chloride, thimerosal, phenethyl alcohol, methylparaben, propylparaben, chlorhexidine, sorbic acid and the like. Hydrogen peroxide also may be used as preservative. Suitable cosolvents include glycerin, propylene glycol, and PEG. Suitable complexing agents include caffeine, polyvinylpyrrolidone, beta-cyclodextrin or hydroxy-propyl-beta-cyclodextrin. Suitable surfactants or wetting agents include sorbitan esters, polysorbates such as polysorbate 80, tromethamine, lecithin, cholesterol, tyloxapal, and the like. The buffers may be conventional buffers such as acetate, borate, citrate, phosphate, bicarbonate, or Tris-HCl. Acetate buffer may be about pH 4-5.5, and Tris buffer may be about pH 7-8.5. Additional pharmaceutical agents are set forth in Remington's Pharmaceutical Sciences, 18th Edition, A. R. Gennaro, ed., Mack Publishing Company, 1990.

The composition may be in liquid form, lyophilized form or freeze-dried form. The composition may include one or more lyoprotectants, excipients, surfactants, high molecular weight structural additives and/or bulking agents (see, for example, U.S. Patent Nos. 6,685,940, 6,566,329, and 6,372,716). In one embodiment, a lyoprotectant is included, which is a nonreducing sugar such as sucrose, lactose or trehalose. The amount of lyoprotectant generally included is such that, upon reconstitution, the resulting formulation will be isotonic, although hypertonic or slightly hypotonic formulations also may be suitable. In addition, the amount of lyoprotectant should be sufficient to prevent an unacceptable amount of degradation and/or aggregation of the protein upon lyophilization. Exemplary lyoprotectant concentrations for sugars (*e.g*., sucrose, lactose, trehalose) in the pre-lyophilized formulation are from about 10 mM to about 400 mM. In another embodiment, a surfactant is included, such as for example, nonionic surfactants and ionic surfactants such as polysorbates (*e.g*., polysorbate 20, polysorbate 80); poloxamers (*e.g*., poloxamer 188); poly(ethylene glycol) phenyl ethers (*e.g*., Triton); sodium dodecyl sulfate (SDS); sodium laurel sulfate; sodium octyl glycoside; lauryl-, myristyl-, linoleyl-, or stearyl-sulfobetaine; lauryl-, myristyl-, linoleyl-or stearyl-sarcosine; linoleyl, myristyl-, or cetyl-betaine; lauroamidopropyl-, cocamidopropyl-, linoleamidopropyl-, myristamidopropyl-, palmidopropyl-, or isostearamidopropyl-betaine (*e.g*., lauroamidopropyl); myristamidopropyl-, palmidopropyl-, or isostearamidopropyl-dimethylamine; sodium methyl cocoyl-, or disodium methyl ofeyl-taurate; and the MONAQUAT™. series (Mona Industries, Inc., Paterson, N.J.), polyethyl glycol, polypropyl glycol, and copolymers of ethylene and propylene glycol (*e.g*., Pluronics, PF68 etc). Exemplary amounts of surfactant that may be present in the pre-lyophilized formulation are from about 0.001-0.5%. High molecular weight structural additives (*e.g*., fillers, binders) may include for example, acacia, albumin, alginic acid, calcium phosphate (dibasic), cellulose, carboxymethylcellulose, carboxymethylcellulose sodium, hydroxyethylcellulose, hydroxypropylcellulose, hydroxypropylmethylcellulose, microcrystalline cellulose, dextran, dextrin, dextrates, sucrose, tylose, pregelatinized starch, calcium sulfate, amylose, glycine, bentonite, maltose, sorbitol, ethylcellulose, disodium hydrogen phosphate, disodium phosphate, disodium pyrosulfite, polyvinyl alcohol, gelatin, glucose, guar gum, liquid glucose, compressible sugar, magnesium aluminum silicate, maltodextrin, polyethylene oxide, polymethacrylates, povidone, sodium alginate, tragacanth microcrystalline cellulose, starch, and zein. Exemplary concentrations of high molecular weight structural additives are from 0.1% to 10% by weight. In other embodiments, a bulking agent (*e.g*., mannitol, glycine) may be included.

Compositions may be suitable for parenteral administration. Exemplary compositions are suitable for injection or infusion into an animal by any route available to the skilled worker, such as intraarticular, subcutaneous, intravenous, intramuscular, intraperitoneal, intracerebral (intraparenchymal), intracerebroventricular, intramuscular, intraocular, intraarterial, or intralesional routes. A parenteral formulation typically will be a sterile, pyrogen-free, isotonic aqueous solution, optionally containing pharmaceutically acceptable preservatives.

Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringers' dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives may also be present, such as, for example, anti-microbials, anti-oxidants, chelating agents, inert gases and the like. See generally, Remington's Pharmaceutical Science, 16th Ed., Mack Eds., 1980.

Pharmaceutical compositions described herein may be formulated for controlled or sustained delivery in a manner that provides local concentration of the product (*e.g*., bolus, depot effect) and/or increased stability or half-life in a particular local environment. The compositions may comprise the formulation of CAR-EC switches, polypeptides, nucleic acids, or vectors disclosed herein with particulate preparations of polymeric compounds such as polylactic acid, polyglycolic acid, *etc*., as well as agents such as a biodegradable matrix, injectable microspheres, microcapsular particles, microcapsules, bioerodible particles beads, liposomes, and implantable delivery devices that provide for the controlled or sustained release of the active agent which then may be delivered as a depot injection. Techniques for formulating such sustained-or controlled-delivery means are known and a variety of polymers have been developed and used for the controlled release and delivery of drugs. Such polymers are typically biodegradable and biocompatible. Polymer hydrogels, including those formed by complexation of enantiomeric polymer or polypeptide segments, and hydrogels with temperature or pH sensitive properties, may be desirable for providing drug depot effect because of the mild and aqueous conditions involved in trapping bioactive protein agents (*e.g*., antibodies comprising an ultralong CDR3). See, for example, the description of controlled release porous polymeric microparticles for the delivery of pharmaceutical compositions in WO 93/15722. Suitable materials for this purpose include polylactides (see, *e.g*., U.S. Patent No. 3,773,919), polymers of poly-(a-hydroxycarboxylic acids), such as poly-D-(-)-3-hydroxybutyric acid (EP 133,988A), copolymers of L-glutamic acid and gamma ethyl-L-glutamate (Sidman et al., Biopolymers, 22: 547-556 (1983)), poly(2-hydroxyethyl-methacrylate) (Langer et al., J. Biomed. Mater. Res., 15: 167-277 (1981), and Langer, Chem. Tech., 12: 98-105 (1982)), ethylene vinyl acetate, or poly-D(-)-3-hydroxybutyric acid. Other biodegradable polymers include poly(lactones), poly(acetals), poly(orthoesters), and poly(orthocarbonates). Sustained-release compositions also may include liposomes, which may be prepared by any of several methods known in the art (see, *e.g*., Eppstein et al., Proc. Natl. Acad. Sci. USA, 82: 3688-92 (1985)). The carrier itself, or its degradation products, should be nontoxic in the target tissue and should not further aggravate the condition. This may be determined by routine screening in animal models of the target disorder or, if such models are unavailable, in normal animals. Microencapsulation of recombinant proteins for sustained release has been performed successfully with human growth hormone (rhGH), interferon-(rhIFN-), interleukin-2, and MN rgp120. Johnson et al., Nat. Med., 2:795-799 (1996); Yasuda, Biomed. Ther., 27:1221-1223 (1993); Hora et al., Bio/Technology. 8:755-758 (1990); Cleland, "Design and Production of Single Immunization Vaccines Using Polylactide Polyglycolide Microsphere Systems," in Vaccine Design: The Subunit and Adjuvant Approach, Powell and Newman, eds, (Plenum Press: New York, 1995), pp. 439-462; WO 97/03692, WO 96/40072, WO 96/07399; and U.S. Patent No. 5,654,010. The sustained-release formulations of these proteins were developed using poly-lactic-coglycolic acid (PLGA) polymer due to its biocompatibility and wide range of biodegradable properties. The degradation products of PLGA, lactic and glycolic acids may be cleared quickly within the human body. Moreover, the degradability of this polymer may be depending on its molecular weight and composition. Lewis, "Controlled release of bioactive agents from lactide/glycolide polymer," in: M. Chasin and R. Langer (Eds.), Biodegradable Polymers as Drug Delivery Systems (Marcel Dekker: New York, 1990), pp. 1-41. Additional examples of sustained release compositions include, for example, EP 58,481A, U.S. Patent No. 3,887,699, EP 158,277A, Canadian Patent No. 1176565, U. Sidman et al., Biopolymers 22, 547 [1983], R. Langer et al., Chem. Tech. 12, 98 [1982], Sinha et al., J. Control. Release 90, 261 [2003], Zhu et al., Nat. Biotechnol. 18, 24 [2000], and Dai et al., Colloids Surf B Biointerfaces 41, 117 [2005].

Bioadhesive polymers are also contemplated for use in or with compositions of the present disclosure. Bioadhesives are synthetic and naturally occurring materials able to adhere to biological substrates for extended time periods. For example, Carbopol and polycarbophil are both synthetic cross-linked derivatives of poly(acrylic acid). Bioadhesive delivery systems based on naturally occurring substances include for example hyaluronic acid, also known as hyaluronan. Hyaluronic acid is a naturally occurring mucopolysaccharide consisting of residues of D-glucuronic and N-acetyl-D-glucosamine. Hyaluronic acid is found in the extracellular tissue matrix of vertebrates, including in connective tissues, as well as in synovial fluid and in the vitreous and aqueous humor of the eye. Esterified derivatives of hyaluronic acid have been used to produce microspheres for use in delivery that are biocompatible and biodegradable (see, for example, Cortivo et al., Biomaterials (1991) 12:727-730; EP 517,565; WO 96/29998; Illum et al., J. Controlled Rel. (1994) 29:133-141).

Both biodegradable and non-biodegradable polymeric matrices may be used to deliver compositions of the present disclosure, and such polymeric matrices may comprise natural or synthetic polymers. Biodegradable matrices are preferred. The period of time over which release occurs is based on selection of the polymer. Typically, release over a period ranging from between a few hours and three to twelve months is most desirable. Exemplary synthetic polymers which may be used to form the biodegradable delivery system include: polymers of lactic acid and glycolic acid, polyamides, polycarbonates, polyalkylenes, polyalkylene glycols, polyalkylene oxides, polyalkylene terepthalates, polyvinyl alcohols, polyvinyl ethers, polyvinyl esters, poly-vinyl halides, polyvinylpyrrolidone, polyglycolides, polysiloxanes, polyanhydrides, polyurethanes and co-polymers thereof, poly(butic acid), poly(valeric acid), alkyl cellulose, hydroxyalkyl celluloses, cellulose ethers, cellulose esters, nitro celluloses, polymers of acrylic and methacrylic esters, methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxylethyl cellulose, cellulose triacetate, cellulose sulphate sodium salt, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene, poly(ethylene glycol), poly(ethylene oxide), poly(ethylene terephthalate), poly(vinyl alcohols), polyvinyl acetate, poly vinyl chloride, polystyrene and polyvinylpyrrolidone. Exemplary natural polymers include alginate and other polysaccharides including dextran and cellulose, collagen, chemical derivatives thereof (substitutions, additions of chemical groups, for example, alkyl, alkylene, hydroxylations, oxidations, and other modifications routinely made by those skilled in the art), albumin and other hydrophilic proteins, zein and other prolamines and hydrophobic proteins, copolymers and mixtures thereof. In general, these materials degrade either by enzymatic hydrolysis or exposure to water *in vivo,* by surface or bulk erosion. The polymer optionally is in the form of a hydrogel (see, for example, WO 04/009664, WO 05/087201, Sawhney, et al., Macromolecules, 1993, 26, 581-587) that can absorb up to about 90% of its weight in water and further, optionally is cross-linked with multivalent ions or other polymers.

Delivery systems also include non-polymer systems that are lipids including sterols such as cholesterol, cholesterol esters and fatty acids or neutral fats such as mono-di-and triglycerides; hydrogel release systems; silastic systems; peptide based systems; wax coatings; compressed tablets using conventional binders and excipients; partially fused implants; and the like. Specific examples include, but are not limited to: (a) erosional systems in which the product is contained in a form within a matrix such as those described in U.S. Patent Nos. 4,452,775, 4,675,189 and 5,736,152 and (b) diffusional systems in which a product permeates at a controlled rate from a polymer such as described in U.S. Patent Nos. 3,854,480, 5,133,974 and 5,407,686. Liposomes containing the product may be prepared by methods known methods, such as for example (DE 3,218,121; Epstein et al., Proc. Natl. Acad. Sci. USA, 82: 3688-3692 (1985); Hwang et al., Proc. Natl. Acad. Sci. USA, 77: 4030-4034 (1980); EP 52,322; EP 36,676; EP 88,046; EP 143,949; EP 142,641; JP 83-118008; U.S. Patent Nos. 4,485,045 and 4,544,545; and EP 102,324).

Alternatively or additionally, the compositions may be administered locally via implantation into the affected area of a membrane, sponge, or other appropriate material on to which a CAR-EC switch disclosed herein has been absorbed or encapsulated. Where an implantation device is used, the device may be implanted into any suitable tissue or organ, and delivery of a CAR-EC switch, nucleic acid, or vector disclosed herein may be directly through the device via bolus, or via continuous administration, or via catheter using continuous infusion.

A pharmaceutical composition comprising a CAR-EC switch disclosed herein may be formulated for inhalation, such as for example, as a dry powder. Inhalation solutions also may be formulated in a liquefied propellant for aerosol delivery. In yet another formulation, solutions may be nebulized. Additional pharmaceutical composition for pulmonary administration include, those described, for example, in WO 94/20069, which discloses pulmonary delivery of chemically modified proteins. For pulmonary delivery, the particle size should be suitable for delivery to the distal lung. For example, the particle size may be from 1 µm to 5 µm; however, larger particles may be used, for example, if each particle is fairly porous.

Certain formulations containing CAR-EC switches disclosed herein may be administered orally. Formulations administered in this fashion may be formulated with or without those carriers customarily used in the compounding of solid dosage forms such as tablets and capsules. For example, a capsule may be designed to release the active portion of the formulation at the point in the gastrointestinal tract when bioavailability is maximized and pre-systemic degradation is minimized. Additional agents may be included to facilitate absorption of a selective binding agent. Diluents, flavorings, low melting point waxes, vegetable oils, lubricants, suspending agents, tablet disintegrating agents, and binders also may be employed.

Another preparation may involve an effective quantity of a CAR-EC switch disclosed herein in a mixture with non-toxic excipients which are suitable for the manufacture of tablets. By dissolving the tablets in sterile water, or another appropriate vehicle, solutions may be prepared in unit dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Suitable and/or preferred pharmaceutical formulations may be determined in view of the present disclosure and general knowledge of formulation technology, depending upon the intended route of administration, delivery format, and desired dosage. Regardless of the manner of administration, an effective dose may be calculated according to patient body weight, body surface area, or organ size. Further refinement of the calculations for determining the appropriate dosage for treatment involving each of the formulations described herein are routinely made in the art and is within the ambit of tasks routinely performed in the art. Appropriate dosages may be ascertained through use of appropriate dose-response data.

### EXAMPLES

The following illustrative examples are representative of embodiments of the applications, systems, and methods described herein and are not meant to be limiting in any way.

### Example 1 - CAR-T Cytotoxicity of a site-specifically linked FITC-anti-CD19 antibody CAR-T switch platform

Anti-FITC scFv's 4-4-20 (4-4-20), 4D5Flu (4D5Flu), 4M5.3 (4M5.3), and FITC-E2 (FITC-E2) (Table 1) were subcloned into a lentiviral vector containing a second generation CAR (from C-terminus: CD3ζ, 4-1BB, CD8 transmembrane domain, FITC scFv). T cells were transduced with the resulting lentiviral vector.

**Table 1. FITC Antibodies**

| **scFv** | **Scaffold** | **Kd** | **Notes** |
|---|---|---|---|
| 4-4-20 | Murine | 0.23 nM | Generated from mouse hybridoma, and matured by framework transplantation and error prone DNA shuffling |
| 4D5Flu | Chimeric | 22 nM | CDRs of 4-4-20 transplanted to the human framework of 4D5 |
| 4M5.3 | Murine | 0.3 pM | 4-4-20 mutagenized by error-prone DNA shuffling: extremely high affinity |
| FITC-E2 | Human | 0.3 nM | Selected by phage display from a naive human scFv library |

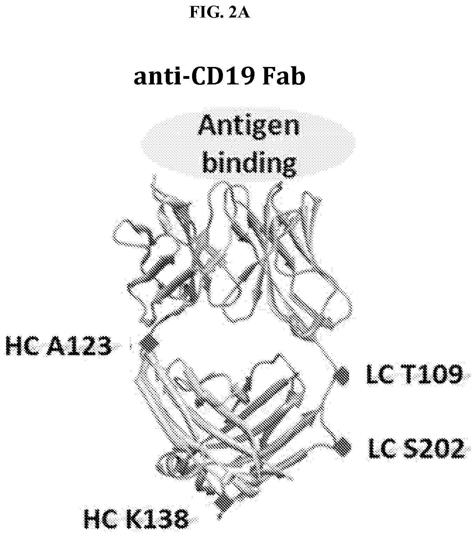

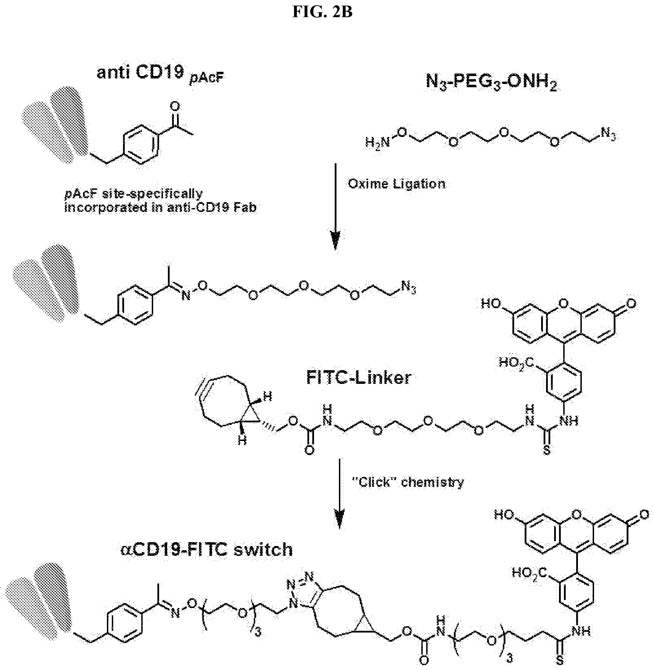

CAR-T switch production was accomplished using site-specific modification of an anti-CD19 Fab with the unnatural amino acid *p*-acetylphenylalanine (*p*AcF). *p*AcF was incorporated at two independent sites (heavy chain, HC K136; or light chain, LC S202, **FIG. 2A**) distal to the binding interface of the anti-CD 19 Fab. The ketone of *p*AcF was then used as a chemical handle to site-specifically modify the Fab with a heterobifunctional azide -PEG-aminooxy linker (N₃-TEG-ONH2) via an oxime ligation (**FIG. 2B**). The oxime ligation is highly stable under physiological conditions. A FITC modified with a PEG-cyclooctyne linker was then "clicked" to the protein through a [3+2] cycloaddition reaction.

To assess the activity and specificity of the switchable CAR-T platform, the cytotoxicity of isolated human effector cells transduced with lenti-virus harboring one of the four FITC-based chimeric antigen receptors (transduction efficiencies were 40-60%) was assayed. Cytolytic activity of CAR-Ts was dependent on the anti-CD 19-FITC switch against CD19 positive multiple myeloma cells (IM-9, EC₅₀ = 3-12 pM) and CD19 positive acute lymphoblastic leukemia (ALL) cells (RS4;11) while having very low activity against CD19 negative cells (K526, EC₅₀ n.d.) (**FIG. 3A-B**), (Table 2). Unexpectedly, cytolysis was independent of FITC scFv affinity in these assays. These results were reproducible against CD19-positive Burkitt's lymphoma (Daudi) and CD-19 positive IM-9 cells. Notably, results indicate the switch produced via random conjugation of anti-CD 19-FITC (average FITC to antibody ratio = 2:1) was less efficacious than switches created by site-specific conjugation to either LC S202*p*AcF, HC K136*p*AcF, or both (**FIG. 3C**). Additionally, nonspecifically conjugated switches lead to higher background cytolysis of CD19-negative K562 cells (than site-specifically conjugated switches) at concentrations of 1 nM or higher. Nonspecifically conjugated switches also demonstrated batch variability in cytotoxicity assays. Thus site-specific switches have demonstrated higher efficacy and lower off-target in preliminary results, encouraging their further investigation.

### Cytotoxicity Assay

Transduced human effector cells (~ 50% transduction efficiency) were co-incubated with target cells at 10:1 (total T cells to target cells) ratio for 24hr in the presence of varying concentrations of FITC "switches." Redirected lysis of target cells was determined by CytoTox96 nonradioactive cytotoxicity assay kit (Promega, Madison, WI) according to manufacturer's protocol. The percentage of cytotoxicity was calculated by: % cytotoxicity = (Absorbance experimental average - Absorbance spontaneous average)/(absorbance max killing average - Absorbance spontaneous average) x 100.

**Table 2.**

| RS4;11 | %Cytotoxicity | | | |
|---|---|---|---|---|
| Concentration (pM) | 4-4-20 | 4D5Flu | 4M5.3 | FITC-E2 |
| 1 | 4.736842 | 6.952663 | 7.608696 | 15.92837 |
| 10 | 15.89474 | 20.46351 | 33.69565 | 36.19227 |
| 100 | 37.68421 | 39.00394 | 53.36439 | 51.46088 |
| 1000 | 45.05263 | 51.13412 | 62.57764 | 54.38266 |
| 10000 | 43.78947 | 53.59961 | 65.68323 | 55.04241 |
| | | | | |

| IM-9 | %Cytotoxicity | | | |
|---|---|---|---|---|
| Concentration (pM) | 4-4-20 | 4D5Flu | 4M5.3 | FITC-E2 |
| 1 | 0 | 0 | 16.92913 | 6.829268 |
| 10 | 43.08943 | 68.19923 | 60.62992 | 65.36585 |
| 100 | 77.64227 | 73.18008 | 88.97638 | 68.78049 |
| 1000 | 69.51219 | 77.0115 | 87.00787 | 86.82927 |
| 10000 | 63.00813 | 75.09579 | 79.52756 | 81.95122 |
| | | | | |

| K562 | %Cytotoxicity | | | |
|---|---|---|---|---|
| Concentration (pM) | 4-4-20 | 4D5Flu | 4M5.3 | FITC-E2 |
| 1 | 5.214724 | 0 | 0.3948667 | 0 |
| 10 | 0 | 0 | 1.48075 | 0 |
| 100 | 0.9202454 | 0 | 4.047384 | 0 |
| 1000 | 1.533742 | 0 | 0 | 17.75599 |
| 10000 | 13.59918 | 30.28105 | 13.6229 | 15.35948 |

### Example 2 - Cytotoxicity of a site-specifically linked FITC-anti-Her2 antibody CAR-T switch

CAR-T activity against SKBR3 Her2-positive breast cancer cells using an anti-Her2 switch based on the Trastuzumab (Herceptin) antibody was assayed. The Fab switch was randomly conjugated or conjugated at LC S202*p*AcF with FITC in similar fashion to the FITC-anti -CD 19 antibody CAR-T switch that was randomly conjugated. Results demonstrated this FITC-anti -Her2 antibody CAR-T switch with FITC conjugated at LC S202*p*AcF was effective at controlling the activity against Her2-positive cells (EC₅₀ = 8-20 nM) (**FIG. 3D****,** Table 3). Nonspecifically conjugated switches also demonstrated batch variability in cytotoxicity assays.

**Table 3. FITC switch activity by conjugation method**

| SKBR3 | %Cytotoxicity | | | | |
|---|---|---|---|---|---|
| Concentration (pM) | aHer2 met azide | aHer2 wt NHS (0.5) | aHer2 wt NHS (1) | aHer2 wt iso (0.5) | aHer2 wt iso (1) |
| 0.01 | 1.973684 | 0.5695688 | 1.72117 | 0.5967605 | 3.948577 |
| 0.1 | 9.12966 | 14.533 | 7.825433 | 0.0372995 | 0 |
| 1 | 41.97158 | 60.69239 | 24.68021 | 27.04215 | 0 |
| 10 | 77.65542 | 73.0256 | 60.19564 | 72.69675 | 35.50652 |
| 100 | 87.67318 | 84.06059 | 78.85629 | 75.53152 | 54.46339 |
| 1000 | 95.7016 | 85.28669 | 92.85177 | 89.10854 | 72.81846 |
| 10000 | 91.08348 | 87.23404 | 91.94884 | 83.28982 | 74.42327 |
| | | | | | |

| MDA MB231 | %Cytotoxicity | | | | |
|---|---|---|---|---|---|
| Concentration (pM) | aHer2 met azide | aHer2 wt NHS (0.5) | aHer2 wt NHS (1) | aHer2 wt iso (0.5) | aHer2 wt iso (1) |
| 0.01 | 0 | 3.824247 | 1.72117 | 0.5967605 | 3.948577 |
| 0.1 | 0 | 0 | 0 | 0 | 0 |
| 1 | 0.4157044 | 0 | 0 | 0 | 0 |
| 10 | 9.745958 | 0 | 0.877193 | 3.500691 | 0 |
| 100 | 47.99076 | 34.01163 | 36.88828 | 39.88945 | 20.78413 |
| 1000 | 48.72979 | 46.89922 | 46.49123 | 45.87748 | 37.12801 |
| 10000 | 45.86605 | 43.70155 | 43.53647 | 41.08706 | 36.65564 |
| | | | | | |

| MDA MB468 | %Cytotoxicity | | | | |
|---|---|---|---|---|---|
| Concentration (pM) | aHer2 met azide | aHer2 wt NHS (0.5) | aHer2 wt NHS (1) | aHer2 wt iso (0.5) | aHer2 wt iso (1) |
| 0.01 | 0 | 0 | 0 | 0 | 0 |
| 0.1 | 0 | 0 | 0.6703911 | 0 | 0 |
| 1 | -5.84989 | 0 | 0 | 0 | 0 |
| 10 | 0 | 0 | 0 | 0 | 0 |
| 100 | 0 | 0 | 0 | 2.99667 | 30.60296 |
| 1000 | 0 | 0 | 14.07821 | 36.73696 | 39.70421 |
| 10000 | 2.538631 | 8.604651 | 21.45251 | 36.29301 | 40.84187 |

### Example 3 - Efficacies of FITC-anti-Her2 CAR-T switches with varying affinities for respective CARs.

Cell lines expressing Her2 have been well-characterized for antigen density, reflecting clinical immunohistochemistry characterization which classifies malignancies as 0 (<20,000 antigens per cell), 1+ (100,000 /cell), 2+ (500,000 /cell), and 3+ (>2,000,000 /cell). The availability of quantitatively characterized cell lines for Her2 makes it an ideal system to study the effect of CAR-T scFv affinity on antigen density. Building on preliminary data which demonstrated the efficacy of a Her2 switch against SKBR3 cells (Her2, 3+) the activities of various Her2 switches against breast cancer lines MDA MB453 (Her2, 2+), MDA MB231 (Her2, 1+), and MDA MB468 (Her2, negative, 0) were tested. Because there are no effective therapies for unresectable Her2 1+ or 2+ breast cancer (trastuzumab is only prescribed to 3+ patients) clinical application of this CAR-T may present a significant treatment option for thousands of women with unresectable breast cancer. In addition, clinical trials with Her2 CAR-T cells have demonstrated these therapies are potentially toxic at high doses. The use of a switch to titer on or off a CAR-T response may be particularly advantageous in this context.

**Table 4. FITC switch activity by target antigen density**

| SKBR3 | %Cytotoxicity | | | |
|---|---|---|---|---|
| Concentration (pM) | 4-4-20 | 4D5Flu | 4M5.3 | FITC-E2 |
| 1 | 4.736842 | 6.952663 | 7.608696 | 15.92837 |
| 10 | 15.89474 | 20.46351 | 33.69565 | 36.19227 |
| 100 | 37.68421 | 39.00394 | 53.36439 | 51.46088 |
| 1000 | 45.05263 | 51.13412 | 62.57764 | 54.38266 |
| 10000 | 43.78947 | 53.59961 | 65.68323 | 55.04241 |
| | | | | |

| MDA MB231 | %Cytotoxicity | | | |
|---|---|---|---|---|
| Concentration (pM) | 4-4-20 | 4D5Flu | 4M5.3 | FITC-E2 |
| 1 | 0 | 0 | 0 | 0 |
| 10 | 3.395062 | 8.403806 | 25.14029 | 12.71137 |
| 100 | 32.65432 | 34.93658 | 55.66779 | 49.79592 |
| 1000 | 36.97531 | 47.51586 | 57.68799 | 50.37901 |
| 10000 | 30.4321 | 51.32135 | 59.48373 | 57.95918 |
| | | | | |

| MDA MB468 | %Cytotoxicity | | | |
|---|---|---|---|---|
| Concentration (pM) | 4-4-20 | 4D5Flu | 4M5.3 | FITC-E2 |
| 1 | 0 | 0 | 0 | 0 |
| 10 | 0 | 0 | 0 | 0 |
| 100 | 0 | 0 | 0 | 0 |
| 1000 | 0 | 0 | 0 | 0 |
| 10000 | 0 | 0 | 0 | 0 |

### Example 4. Biophysical characteristics of an optimal switch

A thorough understanding of the structure activity relationships in the pseudo-immunological synapse is critical to advancing switchable CAR-ECs to clinical candidacy. Because site-specific conjugation methodology is used to create CAR-EC switches disclosed herein, biophysical characteristics of the switch can be assessed that could not otherwise be explored using traditional nonspecific conjugation methods (e.g. geometry, length, valency, and antibody format of the switch and their effects on CAR-EC activation).

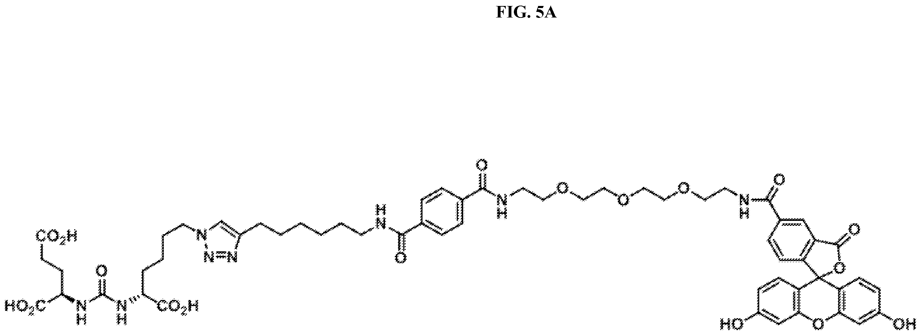

While the current two step method of conjugation (oxime ligation followed by click chemistry, **FIG. 2**) was convenient for linker optimization, one-step click conjugation is desirable for scalable production. Towards this end, the unnatural amino acid *p-*azidophenylalanine (*p*AzF) is incorporated to serve as a proteinogenic click substrate for one-step attachment of the FITC-linker (**FIG. 5A**). To explore the size of the switch using this conjugation strategy the length of the PEG linker is varied from 4 PEG subunits (16 Å) to 32 PEG subunits (112 Å) and assessed for the switch's ability to mediate cytotoxicity. Linkers that are too short interfere with binding, while excessively long linkers decrease efficacy by increasing the distance between the CAR-T and target cell.

To further study the effect of site-specific conjugation on switchable CAR-ECs, *p*AzF is incorporated at LC T109 or HC A123 which are 30 Å or more from the original sites and"turn" the geometry of the switch to perpendicular to the immunological synapse. In addition, these sites are closer to the antigen binding interface of the Fab and should bring the CAR-T closer to its target. Additional mutations are explored throughout the Fab.

Effects of valency were tested by incorporating the *p*AzF residue at multiple sites in the protein simultaneously to create a multivalent substrate. Preliminary results indicated that conjugation of 2 FITC molecules to both LC S202 and HC K136 was equally as efficacious as conjugation to one site (**FIG. 3C**).

### Example 5. Evaluation of the CAR-T system in xenograft and syngeneic mouse models, and with heterogeneous tumors.

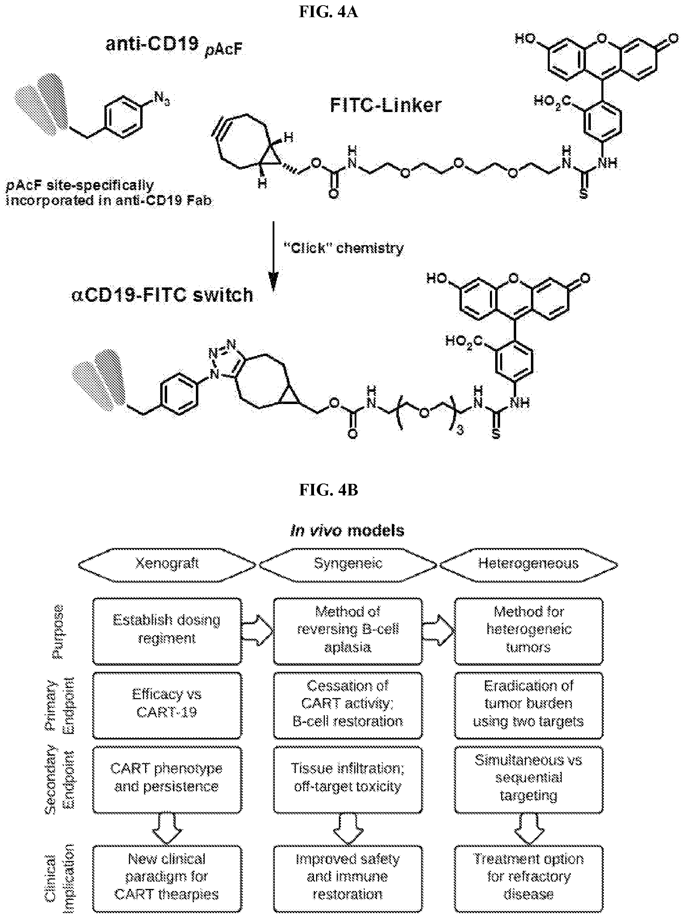

After optimization of switches and off-target reactivity *in vitro,* the efficacy of the CAR-T platform is analyzed in three mouse models: xenograft, syngeneic, and heterogeneous (see flowchart depicted in **FIG. 4B**). Optimal FITC- CAR-T configurations are performed in *in vivo* studies. Because serum half-life of the switch is likely to have a large impact on the ability to regulate activity *in vivo,* the PK of Fab, and IgG switches were first evaluated. It will be important to balance serum half-life with the ability to safely titer on a persistent T cell response. Fab's may be an ideal (t_{1/2} = 3-4 hr) antibody format for efficacy and control of CAR-ECs. Modification of the antibody with FITC is not expected to significantly alter PK. Integrity of the FITC conjugates in PK studies is confirmed by sandwich ELISA and high resolution mass spectroscopy. In addition to intravenous administration, subcutaneous bioavailability is also assessed.

### Xenograft

To evaluate efficacy, mouse xenograft models are used to compare these switchable platforms to previously developed by CAR-T switch platforms. Towards this end, RS4; 11, NALM-6, Raji or other CD19 positive cell lines are used to establish tumor models in non-obese diabetic-severe combined immunodeficiency (NOD-SCID-_{γ}^{-/-}, NSG) mice. FITC-CAR-ECs are delivered IV Dose-range finding is carried out for the FITC anti-CD 19 switch, and is compared to a wild type CD19 Fab control. Efficacy is judged based on tumor burden and overall survival. Mice are monitored for 90 days with weekly blood draws to monitor proliferation of CAR-ECs in peripheral blood. Detailed immunophenotypic characterization of CAR-ECs focus on effector, memory, senescent (terminally differentiated), or anergized phenotypes defined according to standard phenotypic parameters using multi-channel flow cytometry. This is particularly relevant to determine if *in vivo* development of switchable CAR-ECs is biased towards a particular T cell compartment which might affect clinical persistence of the cells compared with CAR-T-19.

Efficacy of the Fab and IgG based switches are delivered at appropriate dosages per observed PK data and compared. IgG may be most efficacious in this model for its long residence time *in vivo.* Further exploration on this idea is carried out in the syngeneic model.

Primary patient-derived ALL or CLL samples are obtained and for generating xenograft models in NSG mice. Primary samples are characterized for CD19 expression by flow cytometry. Leukemia is established in mice for 2-3 weeks prior to administration of therapy. Efficacy versus CAR-T-19 is judged by monitoring CD19⁺ ALL blast counts in peripheral blood. In the event that leukemia is not controlled or eliminated, proliferated blasts are immunophenotyped (specifically looking for loss of CD19 antigen expression, *vida infra* for further study). Persistence of CAR-ECs is also monitored (although the latter is not expected to differ substantially from RS4;11-based xenografts).

### Syngeneic

Switchable CAR-ECs are being tested for the ability to reverse B cell aplasia in an immunocompetent B cell lymphoma mouse model. To create a murine surrogate CAR-T, the engineered FITC-based chimeric receptor is cloned to a Moloney murine leukemia-based retroviral vector for transduction into murine splenocytes. The murine-derived signaling domains CD28 and CD3z are used. The anti-human CD19 antibody does not cross-react with mouse CD19; therefore, the rat anti-mouse CD19 hybridoma 1D3 is obtained (from ATCC) and variable regions sequenced. This sequence is cloned into an expression vector for unnatural amino acid incorporation to create the switch and is cloned into a chimeric antigen receptor to create a CAR-T-19 mouse surrogate.

After optimization of transduction and assessment of efficacy *in vitro,* the Myc5-CD19 cell line is used to establish B cell lymphoma in wild type C57BL/6 mice. CAR-ECs and switches are administered with dosing schedules based on xenograft studies and *in vitro* assays with surrogate system. Of particular interest in this model is to compare Fab, and IgG based switches on the rate of Myc5-CD19 disappearance and B cell ablation. As with xenografts studies, CAR-T proliferation is monitored and immunophenotypic characterization is carried out *ex vivo.* After eradication of lymphoma cells, switch administration is halted and the reproliferation of B cells in peripheral blood is monitored. Both the surrogate CAR-T-19 and the surrogate switchable CAR-T are expected to enable long-term remission, but only the switchable platform enables repopulation of B cells. CAR-T infiltration to major organs is monitored via histology on predefined cohorts and cellular analysis is carried out to 180 days post-therapy. Long-term persistence of CAR-ECs in the absence of stimulation is followed.

### CAR-EC Switches for targeting heterogeneous tumors

A second switch based on the anti-CD20 antibody rituximab to sequentially or simultaneously target different antigens in the same patient using a single adoptively transferred CAR-T to combat ALL relapse attributed to a CD19 escape variant during CAR-T-19 therapy.

An anti-CD20 switch is created in analogous fashion to the anti-CD 19 switch using the optimal characteristics determined in Example 3. A CAR-T-20 based on rituximab is constructed for comparison. Efficacy is tested *in vitro* against CD20-positive IM-9 and Daudi cells lines. To create a heterogeneous B-cell lymphoblast, the chronic myelogenous leukemia-derived K562 cell line (which is negative for CD20 and CD19) is stably transduced with the CD19 antigen using a lentiviral vector. Single cell clones are obtained via flow-sorting to obtain a population with homogenous CD19 expression. This cell line is then transduced with CD20 and sorted by high (CD20^{hi}) or low (CD20^{low}) level of antigen expression. The activation and cytotoxicity of the switchable CAR-T on mixtures of CD19⁺CD20⁻ and CD19⁺CD20^{hi} or CD19⁺CD20^{low} are assessed *in vitro* using the CD19 and CD20 switches (simultaneous or sequential administration). The method provides an opportunity to study the lowest percentage of CD20^{hi} or CD20^{low} cells in a population that are necessary to stimulate the CAR-T with the rituximab switch. This system is then tested in a xenograft mouse model. A mixture of CD19⁺CD20⁻ and CD19⁺CD20⁺ are used to establish the xenograft. Alternatively, primary patient derived ALL samples are used for this experiment if found to be heterogeneous for CD19 or CD20 expression in our initial xenograft study. Switchable CAR-ECs with the anti-CD20 switch are administered to eliminate the CD19⁺CD20⁺ population and allow outgrowth of CD19⁺CD20⁻ cells. To demonstrate the feasibility of retargeting the same CAR-T, the anti-CD 19 switch is subsequently dosed and growth of remaining xenograft monitored. Tumors are evaluated for antigen expression in cohorts of sacrificed mice or in primary blasts. Simultaneous targeting is also assessed. Treatment is compared with CAR-T-19, CAR-T-20, or both simultaneously. This methodology may offer a significant advantage against the propensity for relapse in the clinic while avoiding persistent loss of B cells.

### Example 6. Small molecule switches

### PBMCs and Cell lines

Human PBMCs were freshly isolated from whole blood of healthy donors by centrifugation with Ficoll-Paque PLUS (GE healthcare, Piscataway, NJ) density gradient medium according to manufacturer's protocol. Cells were immediately activated with Dynabeads Human effector-Activator CD3/CD28 (Life Technologies, Carlsbad, CA) at 3:1 (bead to cell) ratio in AIM-V media (Life Technologies) containing 5% human AB serum (Valley Biomedical, Winchester, VA). Lentiviral production was carried out using HEK293FT cell lines. Viral supernatant was used directly to transduce activated PBMCs via spinoculation in the presence of protamine sulfate and recombinant human IL2 (R&D systems, Minneapolis, MN). C4-2, KB, and A549 cell lines were maintained in RPMI-1640, Eagles's Minimum Essential Media, and F-12K medium supplemented with 10% fetal bovine serum, respectively (all from Life Technologies).

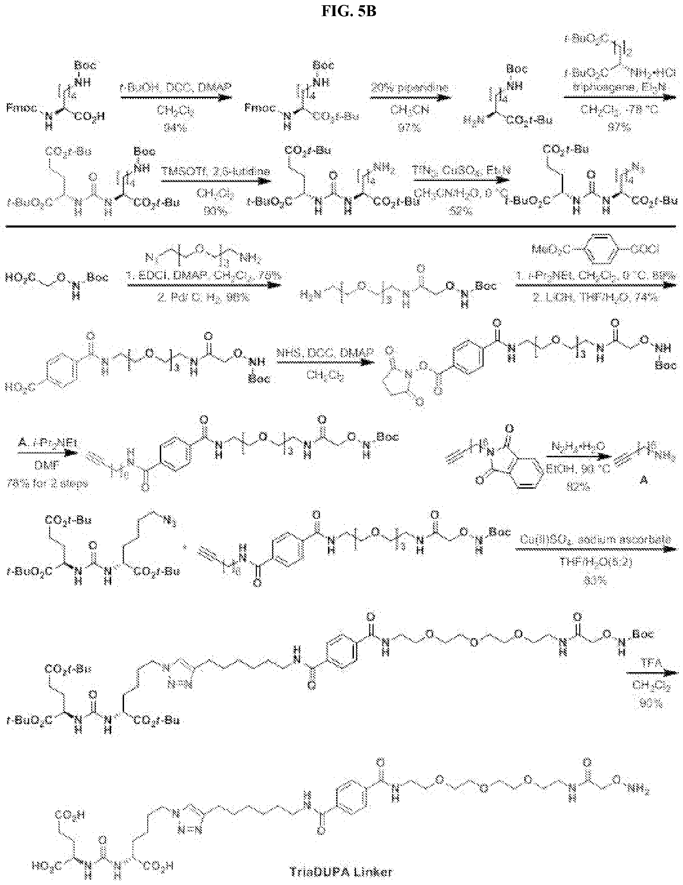

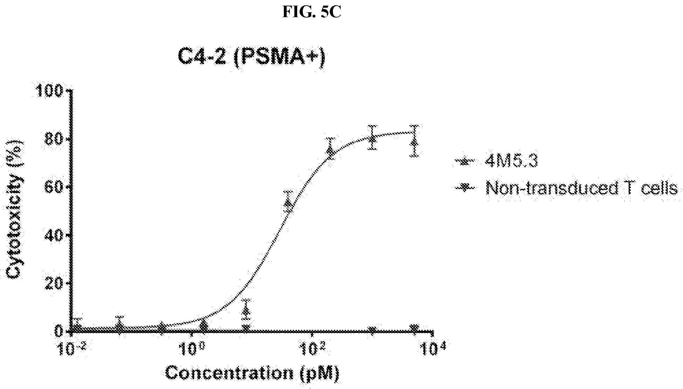

**FIG. 5A** shows the structure of P-TriA-FITC. **FIG. 5B** depicts a scheme for synthesis of the TriA-2-[3-(1,3-dicarboxypropyl)ureido] pentanedioic acid linker to be conjugated to FITC. **FIG. 5C** depicts the results from a 24hr cytotoxicity assay of anti-FITC-CAR T cells targeting C4-2 (PSMA+) prostate cancer cells with serial dilutions of P-TriA-FITC. Each concentration was carried out in triplicate. Results are shown as average percentage of cytotoxicity. Numerical values for the graph depicted in **FIG. 5C** are shown in Table 5.

| Table 5. | | | | | | |
|---|---|---|---|---|---|---|
| Concentration (pM) | 4M5.3 | | | Non-transduced T cells | | |
| 0.000000 | 4.662171 | 4.215603 | 5.510651 | 0.176394 | 0.346090 | 1.203501 |
| 0.000512 | 3.992319 | 3.358192 | 4.688965 | 1.417854 | 0.846247 | 1.051668 |
| 0.002560 | 3.661859 | 2.491850 | 2.206047 | 0.265708 | 0.846247 | 0.140669 |
| 0.012800 | 3.554682 | 1.303979 | 1.205734 | 1.632206 | 0.149600 | 0.855178 |
| 0.064000 | 2.840173 | 2.447193 | 4.572858 | 0.506855 | -0.814990 | 0.881972 |
| 0.320000 | 2.223909 | 2.545438 | 2.965212 | 0.194257 | 0.551512 | -0.189790 |
| 1.600000 | 4.340642 | 3.652927 | 3.956594 | -0.341620 | 0.551512 | 1.212432 |
| 8.000000 | 9.172509 | 10.762290 | 7.582727 | 1.408922 | 0.462198 | 0.551512 |
| 40.000000 | 52.561070 | 55.812080 | 53.561380 | -1.109720 | -0.582770 | 1.176707 |
| 200.000000 | 77.220560 | 76.532850 | 73.915960 | -0.529180 | 0.506855 | -0.422010 |
| 1000.000000 | 82.624030 | 79.944630 | 78.908590 | 0.024561 | 0.096012 | 0.042424 |
| 5000.000000 | 82.043500 | 77.452780 | 78.006520 | 1.051668 | -0.913230 | 0.113875 |

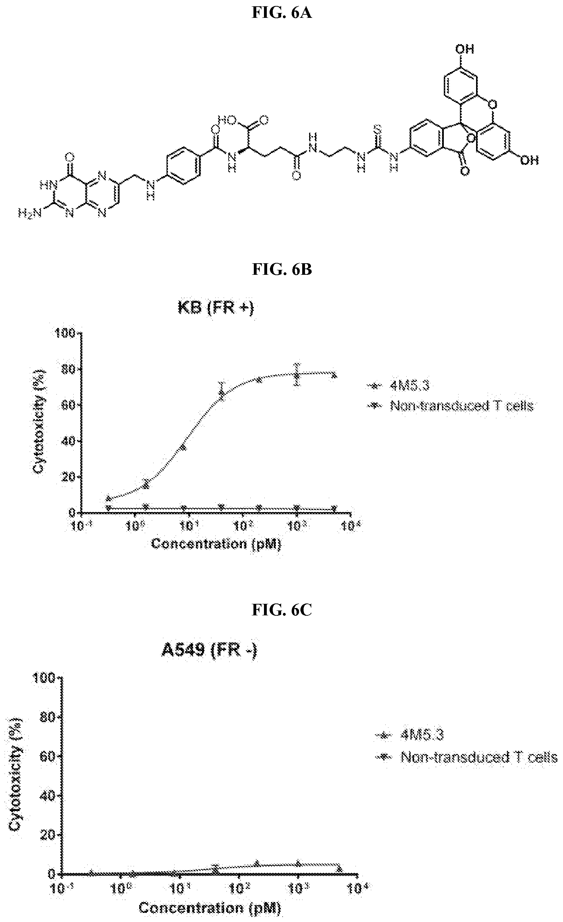

**FIG. 6A** shows the structure of Folate-FITC. **FIG. 6B-6C** depicts the results from an in vitro cytotoxicity assay of anti-FITC-CAR-Ts co-incubated with KB (FR+) (see **FIG. 6B**) or A549 (FR-) (see **FIG. 6C**) target cells in the presence of varying concentrations Folate-FITC. Each concentration was carried out in triplicates. Results are shown as average percentage of cytotoxicity. Numerical values for the graph depicted in **FIG. 6B** and **6C** are shown in Tables 6 and 7, respectively.

**Table 6. Cytotoxicity of FITC-folate switch in FR+ cells**

| KB | %cytotoxicity | |
|---|---|---|
| Concentration (pM) | 4M5.3 | non-transduced T cells |
| 0 | 5.777075 | 1.843410667 |
| 0.32 | 8.299503 | 2.267117 |
| 1.6 | 16.50075 | 3.005248333 |
| 8 | 37.10323 | 2.186593667 |
| 40 | 68.10538 | 2.880628667 |
| 200 | 74.5856 | 2.324634 |
| 1000 | 77.07735 | 2.380233333 |
| 5000 | 76.95082 | 2.042802 |

**Table 7. Cytotoxicity of FITC-folate switch in FR- cells**

| A549 | %cytotoxicity | |
|---|---|---|
| Concentration (pM) | 4M5.3 | non-transduced T cells |
| 0 | 0.483774 | 0.212992333 |
| 0.32 | 0.957294667 | -0.985726667 |
| 1.6 | 0.526652 | -1.021143333 |
| 8 | 0.765276333 | -1.052836667 |
| 40 | 2.554960333 | -1.51517 |
| 200 | 5.776392333 | -0.959626667 |
| 1000 | 5.873333 | -1.157236667 |
| 5000 | 3.145929 | -1.613976667 |

### Example 7. CAR and CAR-T switch construction

The CARs were constructed as follows:
LV-EFla-4-4-20-BBZ (SEQ ID NO: 1) was designed to target fluorescein isothiocyanate (FITC). The scFv 4-4-20 with 20nM affinity was generated from mouse hybridoma, and matured by framework transplantation or error prone DNA shuffling. It was constructed with the light chain preceding the heavy chain and a (GGGGS)₆, (SEQ ID NO. 57, wherein n=6) linker from reference Plückthun A. Improving in vivo folding and stability of a single-chain Fv antibody fragment by loop grafting. Protein Eng. 1997 Aug;10(8):959-66.
LV-EFla-4D5Flu-BBZ (SEQ ID NO: 2) was designed to target Fluorescein isothiocyanate (FITC). The scFv 4D5Flu with 20nM affinity was generated by transplanting the CDRs of 4-4-20 to the framework of humanized Her2/neu scFv 4D5. It was constructed with the light chain preceding the heavy chain and a (GGGGS)₆, (SEQ ID NO. 57, wherein n=6) linker from reference Jung S, Plückthun A. Improving in vivo folding and stability of a single-chain Fv antibody fragment by loop grafting. Protein Eng. 1997 Aug;10(8):959-66.
LV-EFla-4M5.3-BBZ (SEQ ID NO: 3) was designed to target Fluorescein isothiocyanate (FITC). The scFv 4M5.3 with affinity of 0.3pM was generated from its parental scFv 4-4-20 mutagenized by error-prone DNA shuffling. It was constructed with the light chain preceding the heavy chain and a 25 amino acid linker (SSADDAKKDAAKKDDAKKDDAKKDG), (SEQ ID NO. 58) from reference Boder ET, Midelfort KS, Wittrup KD. Directed evolution of antibody fragments with monovalent femtomolar antigen-binding affinity. Proc Natl Acad Sci U S A. 2000 Sep 26;97(20):10701-5.
LV-EFla-FITC-E2-BBZ (SEQ ID NO: 4) was designed to target Fluorescein isothiocyanate (FITC). The scFv FITC-E2 with affinity of 0.3nM was selected from a naive human scFv library by using phage display. It was constructed with the heavy chain preceding the light chain and a (GGGGS)₃, (SEQ ID NO. 57, wherein n=3) linker from reference Vaughan J, Williams AJ, Pritchard K, Osbourn JK, Pope AR, Earnshaw JC, McCafferty J, Hodits RA, Wilton J, Johnson KS. Human antibodies with sub-nanomolar affinities isolated from a large non-immunized phage display library. Nat Biotechnol. 1996 Mar;14(3):309-14.

The target interacting domains (TIDs) of the CAR-T switch were constructed as followed:
pBAD-CD19wt (SEQ ID NO: 5) comprises a Fab that targets the mouse anti-human CD19 receptor, found on the surface of B-cells, and the related malignances. The sequence was constructed by inserting mouse anti-human CD19 VH and VL domain into the human CHI and CL frame. The sequence was from Milone MC, Fish JD, Carpenito C, Carroll RG, Binder GK, Teachey D, Samanta M, Lakhal M, Gloss B, Danet-Desnoyers G, Campana D, Riley JL, Grupp SA, June CH. Chimeric receptors containing CD 137 signal transduction domains mediate enhanced survival of T cells and increased antileukemic efficacy in vivo. Mol Ther. 2009 Aug;17(8):1453-64. The sequence was derived from the anti-CD 19 antibody FMC63. The sequence was cloned into the pBAD vector.
pBAD-CD19 LS202X mt (SEQ ID NO: 6) comprises a Fab that targets the mouse anti-human CD 19 receptor, found on the surface of B-cells, and the related malignances. The sequence was constructed by inserting mouse anti-human CD19 VH and VL domain into the human CHI and CL frame. There is one Amber mutant site in Serine 202 of the light chain. The Amber mutant site allows for the incorporation of an unnatural amino acid.
pBAD-CD19 HK136X mt (SEQ ID NO: 7) comprises a Fab that targets the mouse anti-human CD 19 receptor, found on the surface of B-cells, and the related malignances. The sequence was constructed by inserting mouse anti-human CD19 VH and VL domain into the human CHI and CL frame. There is one Amber mutant sites in Lysine 136 of the heavy chain. The Amber mutant site allows for the incorporation of an unnatural amino acid.
pBAD-CD19 LS202/HK136X mt (SEQ ID NO: 8) comprises a Fab that targets the mouse anti-human CD19 receptor, found on the surface of B-cells, and the related malignances. The sequence was constructed by inserting mouse anti-human CD19 VH and VL domain into the human CHI and CL frame. There is two Amber mutant sites in Serine 202 of the light chain and Lysine 136 of the heavy chain. The Amber mutant sites allow for the incorporation of unnatural amino acids.

### Example 8. Cytotoxicity of FITC-αCS1 antibody CAR-T switch

Cytotoxicity of a FITC-αCS1 antibody CAR-T switch was assayed in MM.1S cell line. Variable regions from the anti-CS1 clone HuLuc63 (SEQ ID NOs: 10 or 11) was cloned to human constant regions in a Fab format and expressed from a pBAD vector in E coli cells. Fab was purified by protein G and conjugated non-specifically with FITC using NHS-ester activated linker-FITC molecules. Conjugations yielded antibody to FITC ratios which ranged from 0.5 to 1.5. To assess cytotoxicity the anti-FITC CAR-T derived from the FITC-E2 scFv was used at a 10:1 ratio against target cells with varying concentrations of the conjugated anti-CS1-FITC switch.

**Table 8. Cytotoxicity of FITC-αCS1 antibody switch in MM.1S (CS1⁺CD19⁻) cell line**

| | %Cytotoxicity | |
|---|---|---|
| Concentration (pM) | αCS1-FITC switch | αCD19-FITC switch |
| 10 | 97.29295 | 4.996922 |
| 1 | 81.90135 | 8.273115 |
| 0.1 | 54.43238 | 4.841375 |
| 0.01 | 5.488979 | -0.8360615 |
| 0.001 | 1.685213 | -3.69422 |
| 0.0001 | 1.669164 | 6.05658 |
| 0.00001 | 3.715493 | 9.459152 |

### Example 9. Cytotoxicity of FITC-αBCMA antibody CAR-T switch

Cytotoxicity of a FITC- αBCMA antibody CAR-T switch was assayed in RPMI 8226 (BCMA⁺) cell line. Variable regions from the anti-BCMA clone BCMA98 (SEQ ID NOs: 14 or 15) was cloned to human constant regions in a Fab format and expressed from a pBAD vector in E coli cells. Fab was purified by protein G and conjugated non-specifically with FITC using NHS-ester activated linker-FITC molecules. Conjugations yielded antibody to FITC ratios which ranged from 0.5 to 1.5. To assess cytotoxicity the anti-FITC CAR-T derived from the FITC-E2 scFv was used at a 10:1 ratio against target cells with varying concentrations of the conjugated anti-BCMA-FITC switch.

**Table 9. Cytotoxicity of FITC-αBCMA antibody switch in RPMI 8226 (BCMA⁺) cell line**

| | %Cytotoxicity |
|---|---|
| Concentration (pM) | αBCMA-FITC switch |
| 10 | 57.57705 |
| 1 | 48.45045 |
| 0.1 | 24.6988 |
| 0.01 | 0.7577045 |
| 0.001 | -3.643465 |
| 0.0001 | -2.776545 |
| 0.00001 | -1.573434 |

### Example 10. Cytotoxicity of FITC-α EGFRvIII antibody CAR-T switch

Cytotoxicity of FITC-EGFRvIII antibody switch was assayed in U87MGAEGFR (EGFRvIII⁺) cell line and A549 (EGFRvIII^{low}) cell line Variable regions from the anti-EGFRvIII clone hu806 (SEQ ID NOs: 12 and 13) was cloned to human constant regions in an Fab format and expressed from a pBAD vector in E coli cells. Fab was purified by protein G and conjugated non-specifically with FITC using NHS-ester activated linker-FITC molecules. Conjugations yielded antibody to FITC ratios which ranged from 0.5 to 1.5. To assess cytotoxicity the anti-FITC CAR-T derived from the FITC-E2 scFv was used at a 10:1 ratio against target cells with varying concentrations of the conjugated anti-EGFRvIII-FITC switch.

**Table 10. Cytotoxicity of FITC-EGFRvIII antibody switch in U87MGΔEGFR (EGFRvIII⁺) cell line and A549 (EGFRvIII^{low}) cell line**

| | %Cytotoxicity | |
|---|---|---|
| Concentration (pM) | A549 (EGFRlow) | U87MGDEGFR (EGFRvIII+) |
| 10 | 1.6 | 34.5 |
| 1 | 0.7 | 31.4 |
| 0.1 | -0.5 | 24.1 |
| 0.01 | -0.1 | 9.7 |
| 0.001 | 0 | 1 |
| 0.0001 | 1.2 | 3 |
| 0.00001 | 1.2 | 1.5 |

### Example 11: Optimization of FITC conjugation site in anti-CD19 Fab

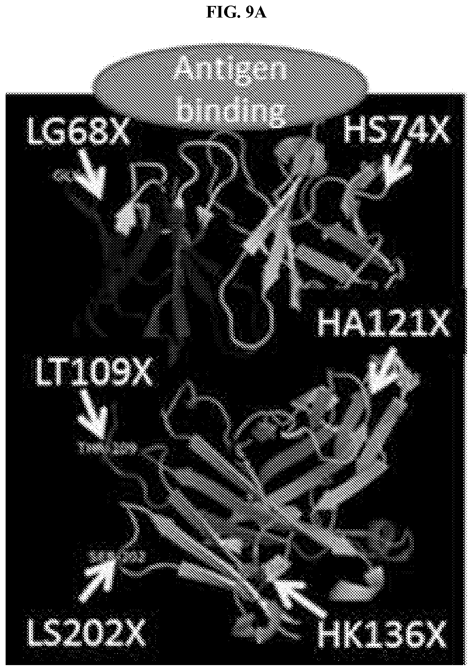

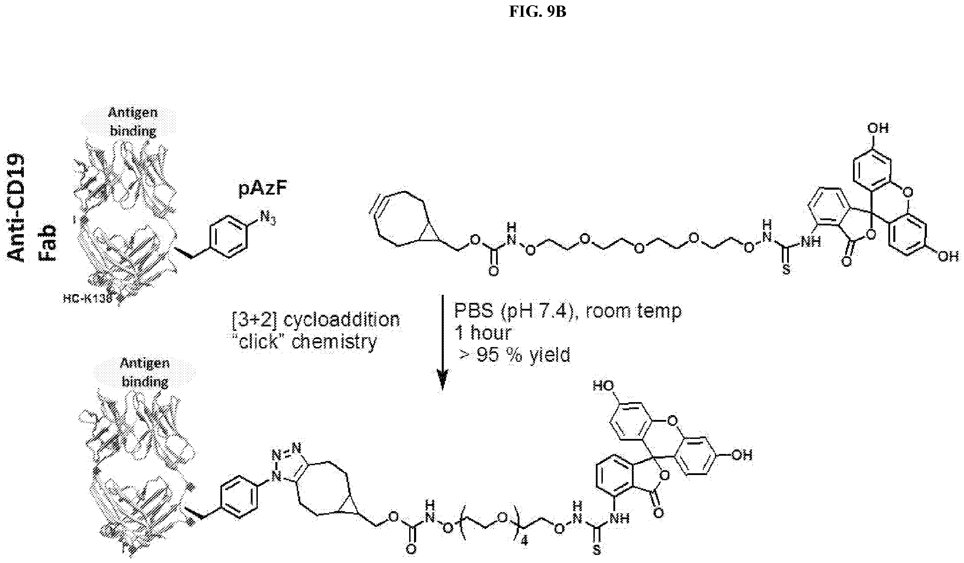

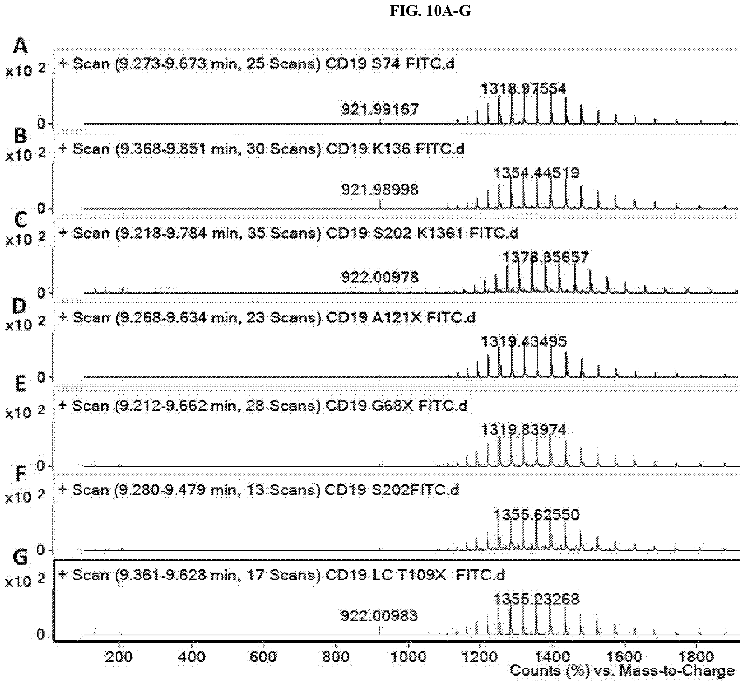

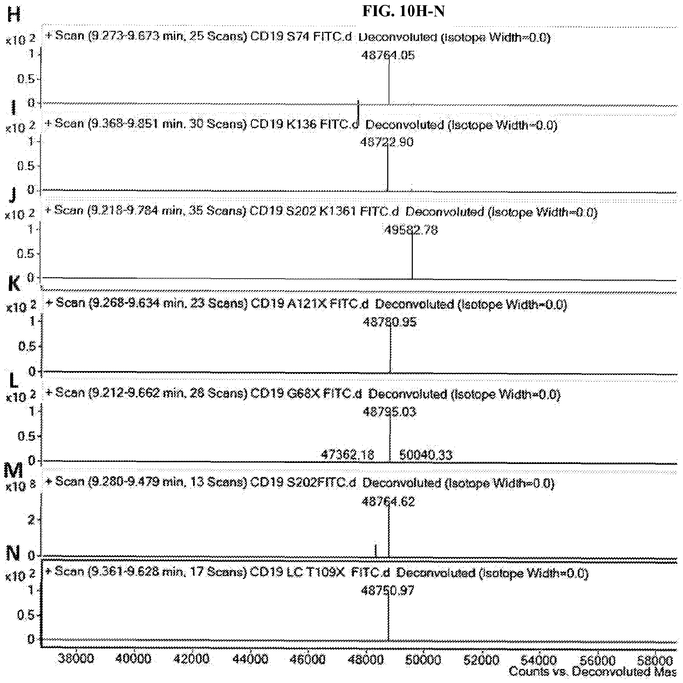

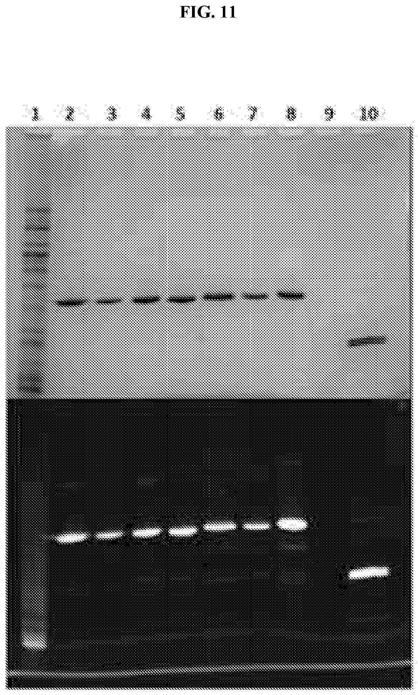

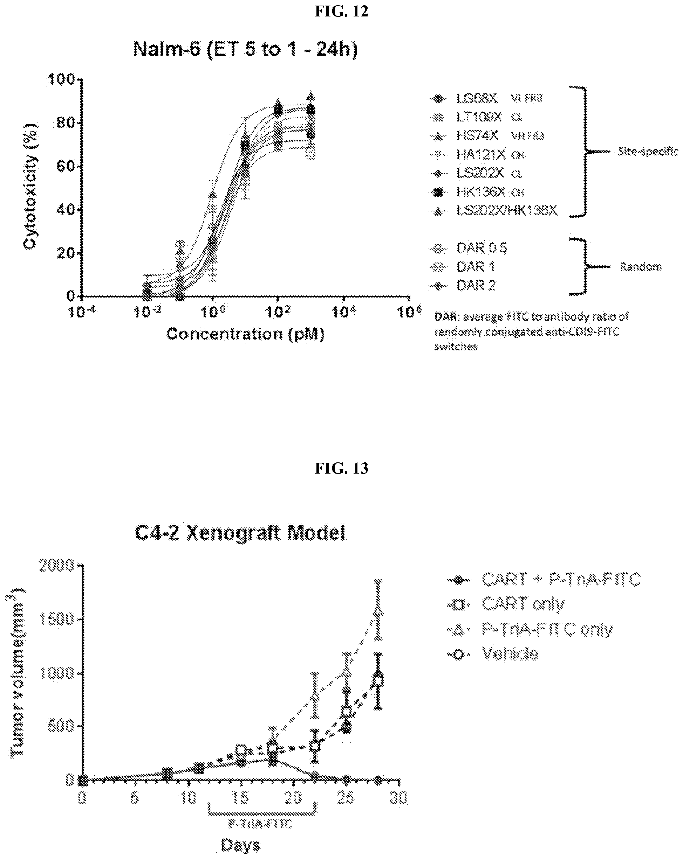

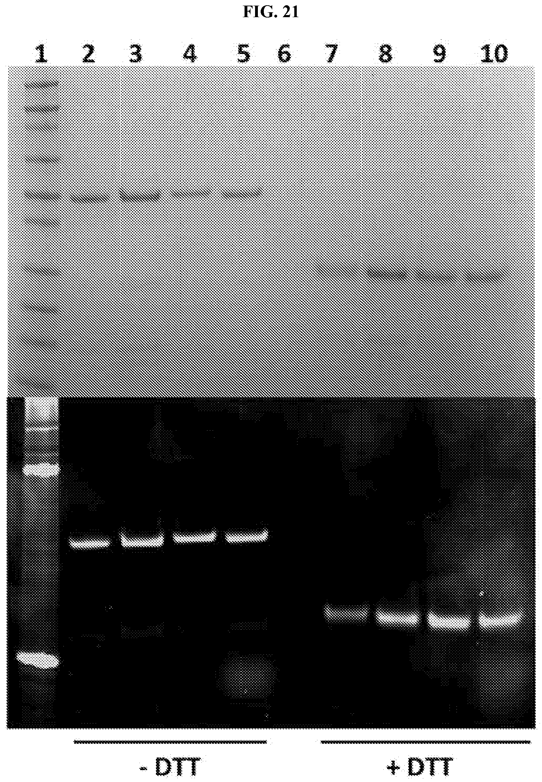

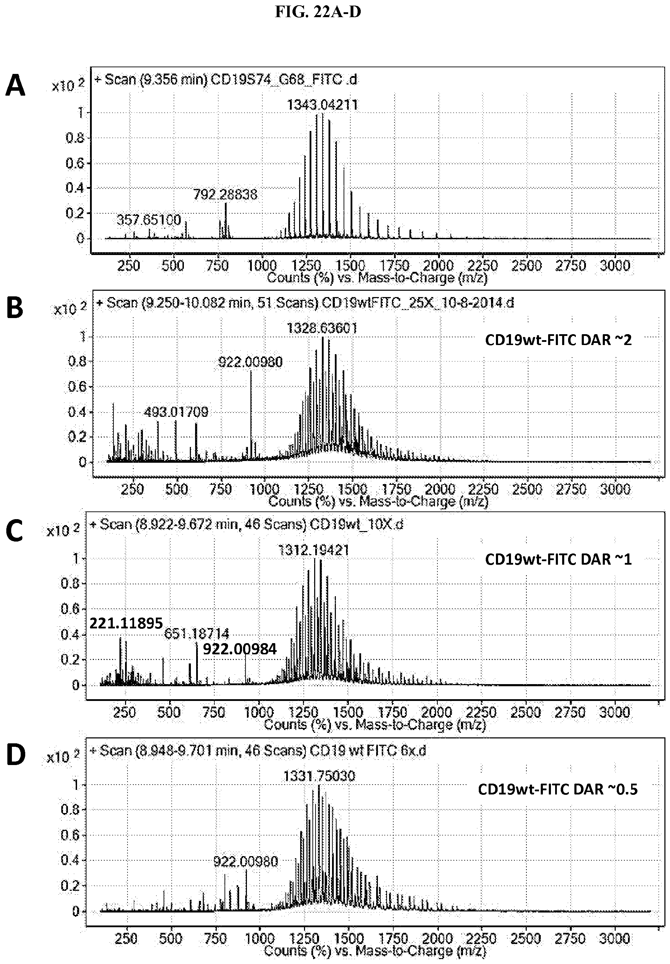

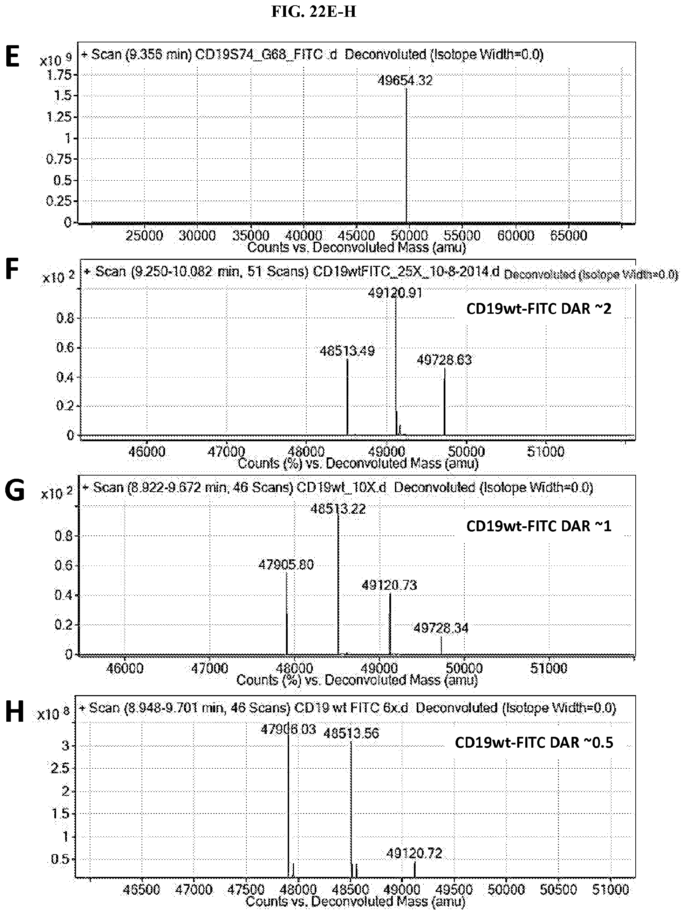

Although the anti-FITC-CAR-T cells disclosed herein are composed of similar signaling domains as the conventional CAR-T cells, the activation of the switchable CAR-T cells relies upon the formation of the ternary complex (e.g., target cell, antibody switch, and CAR-T cell) in comparison to the binary complex (e.g., target cell and CAR-T cell) observed with conventional CAR-T cells. Therefore, each component of the ternary complex is studied to determine the optimal parameters for the universal CAR-EC platform. In order to study the effect of the conjugation site, several anti-CD 19 Fab mutants were expressed, in which *p*AzF was incorporated at different positions on the light chain (LC) and heavy chain (HC) of anti-CD 19 Fab (**FIG. 9A**). The *p*AzF was incorporated at positions serine 202 (LCS202), glycine 68 (LCG68) and threonine 109 (LCT109) of the light chain of the anti-CD 19 Fab. The *p*AzF was incorporated at positions serine 74 (HCS74), alanine 121 (HC121), lysine 136 (HCK136) of the heavy chain of the anti-CD 19 Fab. Double mutants were also constructed by incorporating *p*AzF into serine 202 of the light chain and lysine 136 of the heavy chain (LCS202/HCK136) and by incorporating *p*AzF into serine 74 of the heavy chain and glycine 68 of the light chain (HCS74/LCG68). Briefly, an orthogonal amber suppressor tRNA and aminoacyl-tRNA synthetase (aaRS) pair were coexpressed in *E.coli* with Fab genes containing a TAG codon at different positions, and cultures were allowed to grow and incorporate pAzF at introduced TAG codons. Following periplasmic lysis of *E.coli*, the Fabs were purified by protein G affinity purification. The molecular weight and site-specific incorporation of pAzF in Fabs was verified by SDS-PAGE gel and ESI-MS. The mutant Fabs containing pAzF at different sites were then site-specifically modified with the cyclooctyne-FITC linker in a single step reaction (**FIG. 9B**). The final antibody conjugates were further purified by size-exclusion chromatography and characterized by SDS-PAGE (**FIG. 11** **and** **FIG. 21**) and ESI-MS **(****FIG. 10A-N** and **FIG. 22A****H).** As shown in **FIG. 11**, Lane 1 is the protein standard, Lane 2 is LCG68, Lane 3 is LCT109, Lane 4 is HCS74, Lane 5 is HCA121, Lane 6 is LCS202, Lane 7 is HCK136, Lane 8 is LCS202/HCK136, Lane 9 is blank and Lane 10 is LCS202/HCK136 treated with DTT. As shown in **FIG. 21**, Lane 1 is the protein standard, Lane 2 is LCS202 (no DTT), Lane 3 is HCS74 (no DTT), Lane 4 is HCS74/LCG68 (no DTT), Lane 5 is WT:DAR ∼1 (no DTT), Lane 6 is blank, Lane 7 is LCS202 (with DTT), Lane 8 is HCS74 (with DTT), Lane 9 is HCS74/LCG68 (with DTT), and Lane 10 is WT:DAR ∼1 (with DTT). **FIG. 10A** shows the ESI-MS scan for HCS74. **FIG. 10B** shows the ESI-MS scan for HCK136. **FIG. 10C** shows the ESI-MS scan for LCS202/HCK136. **FIG. 10D** shows the ESI-MS scan for HCA121. **FIG. 10E** shows the ESI-MS scan for LCG68. **FIG. 10F** shows the ESI-MS scan for LCS202. **FIG. 10G** shows the ESI-MS scan for LCT109. **FIG. 10H** shows the deconvoluted ESI-MS scan for HCS74. **FIG. 10I** shows the deconvoluted ESI-MS scan for HCK136. **FIG. 10J** shows the deconvoluted ESI-MS scan for LCS202/HCK136. **FIG. 10K** shows the deconvoluted ESI-MS scan for HCA121. **FIG. 10L** shows the deconvoluted ESI-MS scan for LCG68. **FIG. 10M** shows the deconvoluted ESI-MS scan for LCS202. **FIG. 10N** shows the deconvoluted ESI-MS scan for LCT109. **FIG. 22A** shows the ESI-MS scan for HCS74/LCG68. **FIG. 22B** shows the ESI-MS scan for wild-type anti-CD 19-FITC DAR∼2. **FIG. 22C** shows the ESI-MS scan for wild-type anti-CD 19-FITC DAR∼1. **FIG. 22D** shows the ESI-MS scan for wild-type anti-CD 19-FITC DAR∼0.5. **FIG. 22E** shows the deconvoluted ESI-MS scan for HCS74/LCG68. **FIG. 22F** shows the deconvoluted ESI-MS scan for wild-type anti-CD 19-FITC DAR∼2. **FIG. 22G** shows the deconvoluted ESI-MS scan for wild-type anti-CD19-FITC DAR∼1. **FIG. 22H** shows the deconvoluted ESI-MS scan for wild-type anti-CD 19-FITC DAR∼0.5. Upon completion of site-specific conjugation to a FITC molecule, *in vitro* cytolytic activity of anti-FITC (FITC-E2) CAR-T cells in the presence of different concentrations of switch molecules was evaluated using CD19+ B-cell leukemia line NALM-6 as target cells. Briefly, anti-FITC-CAR T cells (FITC-E2) and NALM6 (CD19+) cells were co-cultured at effector to target ratio of 5 to 1 with serial dilutions of various anti-CD19Fab-FITC conjugated switch molecules. Cytotoxicity was measured by flow cytometry. As shown in **FIG. 12** and Tables 11 and 12, most switch molecules demonstrated potent *in vitro* efficacy at picomolar ranges. Notably, two conjugation sites located in the variable region of the antibody, LG68 and HS74, demonstrated superior activity compared to other positions. Moreover, the dual conjugate HS74/LG68X anti-CD 19-FITC switch demonstrated the most significant improvement in efficacy, resulting in sub-picomolar (EC₅₀ = 0.07 pM) activity in our assay settings. Thus, these results demonstrate the importance of determining the ideal conjugation site of each Fab in the development of an optimal switch molecule.

**Table 11. Cytotoxicity EC50 of anti-CD 19 Fab-FITC CAR-T switches and anti-FITC-CAR T cells (FITC-E2) towards NALM6 (CD19+) cells.**

| Mutation | LG68X | LT109X | HS74X | HA121X | LS202X | HK136X | LS202X/ HS74X | Random/ DAR-0.5 | Random/ DAR-1 | Random/ DAR-2 |
|---|---|---|---|---|---|---|---|---|---|---|
| EC50 [pM] | 3.53 | 2.823 | 0.97 | 3.39 | 3.80 | 2.43 | 2.53 | 2.60 | 2.19 | 1.46 |

**Table 12. Cytotoxicity of various concentrations of anti-CD 19 Fab-FITC CAR-T switches and anti-FITC-CAR T cells (FITC-E2) towards NALM6 (CD19+) cells.**

| | % cytotoxicity | | | | | | | | | |
|---|---|---|---|---|---|---|---|---|---|---|
| Conc(pM) | aCD19-FITC LG68X | aCD19-FITC LT108X | aCD19-FITC HA121X | aCD19-FITC HA121X | aCD19-FITC LS202X | aCD19-FITC HK136X | aCD19-FITC LS202X/ HK136X | aCD19-FITC Random DAR (0.5) | aCD19-FITC Random DAR (1.0) | aCD19-FITC Random DAR(2.0) |
| 0.01 | 0 | 0 | 0 | 4.408353 | 0.465116 | 1.502146 | 0 | 0 | 0 | 4.98576 |
| 0.1 | 3.846154 | 7.537155 | 21.42857 | 0 | 8.139535 | 0 | 14.94737 | 24.18953 | 0 | 0 |
| 1 | 17.78846 | 17.19746 | 47.55102 | 18.79351 | 25.5814 | 25.1073 | 26.94737 | 24.93766 | 23.0137 | 31.9088 |
| 10 | 56.73077 | 66.66667 | 74.69388 | 64.03713 | 60.23256 | 69.95709 | 60 | 67.0823 | 53.1507 | 60.9687 |
| 100 | 78.125 | 76.22081 | 89.59184 | 78.42227 | 83.95349 | 86.2661 | 76.63158 | 71.82045 | 72.0548 | 69.5158 |
| 100 0 | 74.51923 | 77.91932 | 92.85715 | 84.45476 | 87.67442 | 86.05151 | 77.05263 | 81.79551 | 66.5754 | 74.9289 |

### Example 12. In vivo efficacy of FITC-CART and P-TriA-FITC

Pharmacokinetic (PK) studies of the PSMA targeting small molecule switch, P-TriA-FITC in mice revealed that the compound has modest intravenous (IV) half-life (t_{1/2} = 1.08 ± 0.14 hr). In brief, subcutaneous C4-2 (PSMA+) tumors were established in non-obese diabetic-severe combined immunodeficiency (NOD-SCID-γ-/-, NSG) mice. Upon the formation of palpable tumors (> 200mm³ in length or width of tumor), FITC-CART cells (FITC-E2) were adoptively transferred by intraperitoneal injections and daily doses of P-TriA-FITC at 1mg/kg were administered intravenously for 10 consecutive days. Upon completion of switch treatment, tumors had regressed in mice that had received FITC-CART cells and P-TriA-FITC whereas tumor growth continued in mice that received either CART cells or switch only, or CART cells with daily doses of vehicle (PBS), see **FIG. 13**. This *in vivo* efficacy study indicates that the FITC-CART cells in combination with P-TriA-FITC switches is highly effective in targeting PSMA-positive tumors. Additional studies may involve characterization and optimization to demonstrate *in vivo* dose-titratability of the switchable CAR-T cells.

### Example 13. Evaluation of anti-FITC CAR-EC and FITC-anti-CD19 switch in the treatment of B-cell lymphomas

Purpose: This study evaluates the efficacy and safety of a chimeric antigen receptor-effector cell (CAR-EC) comprising an effector cell expressing a chimeric antigen receptor (CAR) and a switch in the treatment of B cell lymphomas. The CAR comprises an external region comprising an anti-FITC scFv. The switch comprises a chimeric antigen receptor-interacting domain (CAR-ID) comprising FITC and a target interacting domain comprising an anti-CD 19 scFv.

| Condition | Intervention | Phase |
|---|---|---|
| B-cell lymphomas | Genetic: anti-FITC CAR T cells and FITC-anti-CD19 scFv switch | Phase I, Phase II |

Study Type: Interventional
Study Design: Endpoint Classification: Safety/Efficacy Study
Intervention Model: Single Group Assignment
Masking: Open Label
Primary Purpose: Treatment

Primary Outcome Measures:
Number of patients with adverse events. [Time Frame: 2 years.]
[Designated as safety issue: Yes]
Determine the toxicity profile of the CAR T cells with Common Toxicity Criteria for Adverse Effects (CTCAE) version 4.0.

Secondary Outcome Measures:
Survival time of Anti-FITC CAR T cells in vivo. [Time Frame: 2 years.]
[Designated as safety issue: No] Measure the survival of CAR T cells transduced with the anti-FITC lentiviral vector.
Response rates to the CAR Tcells. [Time Frame: 2 years.]
[Designated as safety issue: No] Describe the response rates of patients treated with
4th generation CAR T cells, including partial remission (PR), complete remission (CR), stable disease (SD) and progressive disease (PD).

Survival time of the patients. [Time Frame: 2 years.] [Designated as safety issue: No] Evaluate the survival time of the patients treated with the 4th generation CAR T cells, including progression free survival (PFS) and overall survival (OS).

| | |
|---|---|
| Estimated Enrollment: | 20 |
| Study Start Date: | TBD |
| Estimated Study Completion Date: | TBD |
| Estimated Primary Completion Date: | TBD (Final data collection date for primary outcome measure) |

| **Arms** | **Assigned Interventions** |
|---|---|
| Experimental: CAR T cells Autologous anti-FITC CAR T cells | Genetic: Anti-FITC CAR T cells Autologous 4th generation withdrawable lentiviral-transduced anti-FITC CAR T cells |

### Detailed Description:

CD19 single chain antibody-based chimeric antigen receptor (CAR)-engineered T cells have demonstrated great clinical potential in treating chronic and acute B cell leukemias. B cell lymphomas, similar to B cell leukemias, express CD19 surface molecules, and the majority of the B cell lymphoma patients cannot be cured by standard chemo-radiotherapy. CD19 CAR-based adoptive T cell therapy is associated with an unwanted adverse effect, the loss of CD19 B cells, which results in humoral immune deficiency. This study will evaluate a novel anti-FITC CAR-T cells and FITC-anti-CD19 scFv switch for both efficacy and safety in lymphoma patients. Patients receiving the anti-FITC CAR-T cells and the FITC-anti-CD19 scFv switches will be closely monitored for infusion response, tumor eradication effect, longevity of the CAR T cells, and the recovery of B cell functions after withdrawal of the CAR T cells and/or the FITC-anti-CD 19 scFv switch.

### Eligibility

| | |
|---|---|
| Ages Eligible for Study: | 18 Years and older |
| Genders Eligible for Study: | Both |
| Accepts Healthy Volunteers: | No |

### Criteria

Inclusion Criteria:
- Relapsed or refractory CD19(+) B cell lymphoma patients proved by immuno-histochemistry (IHC) or Flow-cytometry.
- Not eligible for autologous stem-cell transplantation (ASCT) or relapsed after ASCT.
- Eastern Cooperative Oncology Group (ECOG) performance status of 0-2.
- Age≥18.
- Pulse oximetry of > 90% on room air.
- Adequate hepatic function, defined as alanine transaminase (ALT) <3 x upper limit of normal (ULN), aspartate aminotransferase (AST) <3 x ULN; serum bilirubin and alkaline phosphatase <2 x ULN.
- Adequate renal function, defined as serum creatinine <2.0mg/dl.
- Adequate heart function with LVEF≥50%
- Hb≥80g/L
- Measurable disease can be identified.
- Life expectancy ≥3 months.
- Sexually active patients must be willing to utilize one of the more effective birth control methods during the study and for 1 year after the study is concluded. The male partner should use a condom.
- Patients must sign an informed consent.

Exclusion Criteria:
- Uncontrolled active infection.
- Active infection with hepatitis B virus (HBV), hepatitis C virus (HCV).
- HIV positive
- Pregnant or lactating.
- Currently enrolled in another clinical trial.
- Concurrent use of systemic steroids.

While preferred embodiments of the present invention have been shown and described herein, it will be obvious to those skilled in the art that such embodiments are provided by way of example only.

| **Table 13. CAR -Nucleotide Sequence** | | |
|---|---|---|
| **NAME** | **SEQ ID** | **SEQUENCE** |
| LV-EF1a-4-4-20-BBZ | 1 | caggtggcacttttcggggaaatgtgcgcggaacccctatttgtttatttttctaaatacattcaaatatgtatccgctcatga gacaataaccctgataaatgcttcaataatattgaaaaaggaagagtatgagtattcaacatttccgtgtcgcccttattcc cttttttgcggcattttgccttcctgtttttgctcacccagaaacgctggtgaaagtaaaagatgctgaagatcagttgggtg cacgagtgggttacatcgaactggatctcaacagcggtaagatccttgagagttttcgccccgaagaacgttttccaatg |
| | | atgagcacttttaaagttctgctatgtggcgcggtattatcccgtattgacgccgggcaagagcaactcggtcgccgcat acactattctcagaatgacttggttgagtactcaccagtcacagaaaagcatcttacggatggcatgacagtaagagaat tatgcagtgctgccataaccatgagtgataacactgcggccaacttacttctgacaacgatcggaggaccgaaggagc taaccgcttttttgcacaacatgggggatcatgtaactcgccttgatcgttgggaaccggagctgaatgaagccatacca aacgacgagcgtgacaccacgatgcctgtagcaatggcaacaacgttgcgcaaactattaactggcgaactacttact ctagcttcccggcaacaattaatagactggatggaggcggataaagttgcaggaccacttctgcgctcggcccttccgg ctggctggtttattgctgataaatctggagccggtgagcgtgggtctcgcggtatcattgcagcactggggccagatggt aagccctcccgtatcgtagttatctacacgacggggagtcaggcaactatggatgaacgaaatagacagatcgctgag ataggtgcctcactgattaagcattggtaactgtcagaccaagtttactcatatatactttagattgatttaaaacttcattttta atttaaaaggatctaggtgaagatcctttttgataatctcatgaccaaaatcccttaacgtgagttttcgttccactgagcgtc agaccccgtagaaaagatcaaaggatcttcttgagatcctttttttctgcgcgtaatctgctgcttgcaaacaaaaaaacc accgctaccagcggtggtttgtttgccggatcaagagctaccaactctttttccgaaggtaactggcttcagcagagcgc agataccaaatactgtccttctagtgtagccgtagttaggccaccacttcaagaactctgtagcaccgcctacatacctcg ctctgctaatcctgttaccagtggctgctgccagtggcgataagtcgtgtcttaccgggttggactcaagacgatagttac cggataaggcgcagcggtcgggctgaacggggggttcgtgcacacagcccagcttggagcgaacgacctacaccg aactgagatacctacagcgtgagctatgagaaagcgccacgcttcccgaagggagaaaggcggacaggtatccggt aagcggcagggtcggaacaggagagcgcacgagggagcttccagggggaaacgcctggtatctttatagtcctgtc gggtttcgccacctctgacttgagcgtcgatttttgtgatgctcgtcaggggggcggagcctatggaaaaacgccagca acgcggcctttttacggttcctggccttttgctggccttttgctcacatgttctttcctgcgttatcccctgattctgtggataa ccgtattaccgcctttgagtgagctgataccgctcgccgcagccgaacgaccgagcgcagcgagtcagtgagcgag gaagcggaagagcgcccaatacgcaaaccgcctctccccgcgcgttggccgattcattaatgcagctggcacgaca ggtttcccgactggaaagcgggcagtgagcgcaacgcaattaatgtgagttagctcactcattaggcaccccaggcttt acactttatgcttccggctcgtatgttgtgtggaattgtgagcggataacaatttcacacaggaaacagctatgaccatgat tacgccaagcgcgcaattaaccctcactaaagggaacaaaagctggagctgcaagcttaatgtagtcttatgcaatact cttgtagtcttgcaacatggtaacgatgagttagcaacatgccttacaaggagagaaaaagcaccgtgcatgccgattg gtggaagtaaggtggtacgatcgtgccttattaggaaggcaacagacgggtctgacatggattggacgaaccactgaa ttgccgcattgcagagatattgtatttaagtgcctagctcgatacaataaacgggtctctctggttagaccagatctgagcc tgggagctctctggctaactagggaacccactgcttaagcctcaataaagcttgccttgagtgcttcaagtagtgtgtgcc cgtctgttgtgtgactctggtaactagagatccctcagacccttttagtcagtgtggaaaatctctagcagtggcgcccga acagggacctgaaagcgaaagggaaaccagagctctctcgacgcaggactcggcttgctgaagcgcgcacggcaa gaggcgaggggcggcgactggtgagtacgccaaaaattttgactagcggaggctagaaggagagagatgggtgcg agagcgtcagtattaagcgggggagaattagatcgcgatgggaaaaaattcggttaaggccagggggaaagaaaaa atataaattaaaacatatagtatgggcaagcagggagctagaacgattcgcagttaatcctggcctgttagaaacatcag aaggctgtagacaaatactgggacagctacaaccatcccttcagacaggatcagaagaacttagatcattatataataca gtagcaaccctctattgtgtgcatcaaaggatagagataaaagacaccaaggaagctttagacaagatagaggaagag caaaacaaaagtaagaccaccgcacagcaagcggccgctgatcttcagacctggaggaggagatatgagggacaat tggagaagtgaattatataaatataaagtagtaaaaattgaaccattaggagtagcacccaccaaggcaaagagaaga gtggtgcagagagaaaaaagagcagtgggaataggagctttgttccttgggttcttgggagcagcaggaagcactatg ggcgcagcctcaatgacgctgacggtacaggccagacaattattgtctggtatagtgcagcagcagaacaatttgctga gggctattgaggcgcaacagcatctgttgcaactcacagtctggggcatcaagcagctccaggcaagaatcctggctg tggaaagatacctaaaggatcaacagctcctggggatttggggttgctctggaaaactcatttgcaccactgctgtgcctt ggaatgctagttggagtaataaatctctggaacagattggaatcacacgacctggatggagtgggacagagaaattaa caattacacaagcttaatacactccttaattgaagaatcgcaaaaccagcaagaaaagaatgaacaagaattattggaat tagataaatgggcaagtttgtggaattggtttaacataacaaattggctgtggtatataaaattattcataatgatagtagga ggcttggtaggtttaagaatagtttttgctgtactttctatagtgaatagagttaggcagggatattcaccattatcgtttcag acccacctcccaaccccgaggggacccgacaggcccgaaggaatagaagaagaaggtggagagagagacagag acagatccattcgattagtgaacggatctcgacggttaacttttaaaagaaaaggggggattggggggtacagtgcag gggaaagaatagtagacataatagcaacagacatacaaactaaagaattacaaaaacaaattacaaaaattcaaaattt atcgagctttgcaaagatggataaagttttaaacagagaggaatctttgcagctaatggaccttctaggtcttgaaaggag tgcctcgtgaggctccggtgcccgtcagtgggcagagcgcacatcgcccacagtccccgagaagttggggggagg |
| | | ggtcggcaattgaaccggtgcctagagaaggtggcgcggggtaaactgggaaagtgatgtcgtgtactggctccgcc tttttcccgagggtgggggagaaccgtatataagtgcagtagtcgccgtgaacgttctttttcgcaacgggtttgccgcc agaacacaggtaagtgccgtgtgtggttcccgcgggcctggcctctttacgggttatggcccttgcgtgccttgaattac ttccacctggctgcagtacgtgattcttgatcccgagcttcgggttggaagtgggtgggagagttcgaggccttgcgctt aaggagccccttcgcctcgtgcttgagttgaggcctggcctgggcgctggggccgccgcgtgcgaatctggtggcac cttcgcgcctgtctcgctgctttcgataagtctctagccatttaaaatttttgatgacctgctgcgacgctttttttctggcaag atagtcttgtaaatgcgggccaagatctgcacactggtatttcggtttttggggccgcgggcggcgacggggcccgtg cgtcccagcgcacatgttcggcgaggcggggcctgcgagcgcggccaccgagaatcggacgggggtagtctcaag ctggccggcctgctctggtgcctggcctcgcgccgccgtgtatcgccccgccctgggcggcaaggctggcccggtc ggcaccagttgcgtgagcggaaagatggccgcttcccggccctgctgcagggagctcaaaatggaggacgcggcg ctcgggagagcgggcgggtgagtcacccacacaaaggaaaagggcctttccgtcctcagccgtcgcttcatgtgact ccacggagtaccgggcgccgtccaggcacctcgattagttctcgagcttttggagtacgtcgtctttaggttggggggga ggggttttatgcgatggagtttccccacactgagtgggtggagactgaagttaggccagcttggcacttgatgtaattctc cttggaatttgccctttttgagtttggatcttggttcattctcaagcctcagacagtggttcaaagtttttttcttccatttcaggt gtcgtgaggaattcggtaccgcggccgcccggggatccatggccttaccagtgaccgccttgctcctgccgctggcct tgctgctccacgccgccaggccggatgttgttatgacccagaccccgctgtccctgcccgtttccctgggtgaccaggc gagtatctcttgccgttcctctcagtccctggttcacagtcagggtaacacctatctgcgctggtatctgcaaaaaccagg ccagagccctaaagtgctgatttataaggtttcaaatcggtttagcggcgtcccggatcgcttctctgggagtggatcag ggaccgactttacactgaaaattagccgcgtggaagcagaggatctgggcgtgtacttttgcagccagtccactcatgt gccgtggaccttcggcggtgggacaaaactggaaattaagcgtgcaggaggcggtgggagcggaggcggtgggtc cggaggcggtgggtctggaggcggtgggagtggaggcggtgggtcaggcggtggtgggagcgaagtgaaactgg atgagacaggaggaggtctggttcagccaggtcggcccatgaagctgtcctgtgtggcctctggctttaccttctccga ctattggatgaactgggtccgtcagtctccggaaaaaggtctggagtgggtggcgcagattcggaacaagccctacaa ctacgaaacttactactctgatagtgttaaaggccgcttcaccatcagtcgtgatgactcaaaaagcagcgtttacctgca aatgaacaatctgcgtgtcgaggacatgggcatctattactgcacaggctcctactatgggatggattattgggggcag gggacttcagttacagtttcctcaaccacgacgccagcgccgcgaccaccaacaccggcgcccaccatcgcgtcgca gcccctgtccctgcgcccagaggcgtgccggccagcggcgggggggcgcagtgcacacgagggggctggacttcg cctgtgatatctacatctgggcgcccttggccgggacttgtggggtccttctcctgtcactggttatcaccctttactgcaa acggggcagaaagaaactcctgtatatattcaaacaaccatttatgagaccagtacaaactactcaagaggaagatggc tgtagctgccgatttccagaagaagaagaaggaggatgtgaactgagagtgaagttcagcaggagcgcagacgccc ccgcgtacaagcagggccagaaccagctctataacgagctcaatctaggacgaagagaggagtacgatgttttggac aagagacgtggccgggaccctgagatggggggaaagccgagaaggaagaaccctcaggaaggcctgtacaatga actgcagaaagataagatggcggaggcctacagtgagattgggatgaaaggcgagcgccggaggggcaaggggc acgatggcctttaccagggtctcagtacagccaccaaggacacctacgacgcccttcacatgcaggccctgccccctc gctaagtcgacaatcaacctctggattacaaaatttgtgaaagattgactggtattcttaactatgttgctccttttacgctat gtggatacgctgctttaatgcctttgtatcatgctattgcttcccgtatggctttcattttctcctccttgtataaatcctggttgc tgtctctttatgaggagttgtggcccgttgtcaggcaacgtggcgtggtgtgcactgtgtttgctgacgcaacccccactg gttggggcattgccaccacctgtcagctcctttccgggactttcgctttccccctccctattgccacggcggaactcatcg ccgcctgccttgcccgctgctggacaggggctcggctgttgggcactgacaattccgtggtgttgtcggggaagctga cgtcctttccatggctgctcgcctgtgttgccacctggattctgcgcgggacgtccttctgctacgtcccttcggccctca atccagcggaccttccttcccgcggcctgctgccggctctgcggcctcttccgcgtcttcgccttcgccctcagacgagt cggatctccctttgggccgcctccccgcctggaattcgagctcggtacctttaagaccaatgacttacaaggcagctgta gatcttagccactttttaaaagaaaaggggggactggaagggctaattcactcccaacgaagacaagatctgctttttgc ttgtactgggtctctctggttagaccagatctgagcctgggagctctctggctaactagggaacccactgcttaagcctca ataaagcttgccttgagtgcttcaagtagtgtgtgcccgtctgttgtgtgactctggtaactagagatccctcagaccctttt agtcagtgtggaaaatctctagcagtagtagttcatgtcatcttattattcagtatttataacttgcaaagaaatgaatatcag agagtgagaggaacttgtttattgcagcttataatggttacaaataaagcaatagcatcacaaatttcacaaataaagcatt tttttcactgcattctagttgtggtttgtccaaactcatcaatgtatcttatcatgtctggctctagctatcccgcccctaactcc gcccagttccgcccattctccgccccatggctgactaattttttttatttatgcagaggccgaggccgcctcggcctctga gctattccagaagtagtgaggaggcttttttggaggcctaggcttttgcgtcgagacgtacccaattcgccctatagtgag |
| | | tcgtattacgcgcgctcactggccgtcgttttacaacgtcgtgactgggaaaaccctggcgttacccaacttaatcgcctt gcagcacatccccctttcgccagctggcgtaatagcgaagaggcccgcaccgatcgcccttcccaacagttgcgcag cctgaatggcgaatggcgcgacgcgccctgtagcggcgcattaagcgcggcgggtgtggtggttacgcgcagcgtg accgctacacttgccagcgccctagcgcccgctcctttcgctttcttcccttcctttctcgccacgttcgccggctttcccc gtcaagctctaaatcgggggctccctttagggttccgatttagtgctttacggcacctcgaccccaaaaaacttgattagg gtgatggttcacgtagtgggccatcgccctgatagacggtttttcgccctttgacgttggagtccacgttctttaatagtgg actcttgttccaaactggaacaacactcaaccctatctcggtctattcttttgatttataagggattttgccgatttcggcctat tggttaaaaaatgagctgatttaacaaaaatttaacgcgaattttaacaaaatattaacgtttacaatttcc |
| LV-EF1a-4D5Flu-BBZ | 2 | caggtggcacttttcggggaaatgtgcgcggaacccctatttgtttatttttctaaatacattcaaatatgtatccgctcatga gacaataaccctgataaatgcttcaataatattgaaaaaggaagagtatgagtattcaacatttccgtgtcgcccttattcc cttttttgcggcattttgccttcctgtttttgctcacccagaaacgctggtgaaagtaaaagatgctgaagatcagttgggtg cacgagtgggttacatcgaactggatctcaacagcggtaagatccttgagagttttcgccccgaagaacgttttccaatg atgagcacttttaaagttctgctatgtggcgcggtattatcccgtattgacgccgggcaagagcaactcggtcgccgcat acactattctcagaatgacttggttgagtactcaccagtcacagaaaagcatcttacggatggcatgacagtaagagaat tatgcagtgctgccataaccatgagtgataacactgcggccaacttacttctgacaacgatcggaggaccgaaggagc taaccgcttttttgcacaacatgggggatcatgtaactcgccttgatcgttgggaaccggagctgaatgaagccatacca aacgacgagcgtgacaccacgatgcctgtagcaatggcaacaacgttgcgcaaactattaactggcgaactacttact ctagcttcccggcaacaattaatagactggatggaggcggataaagttgcaggaccacttctgcgctcggcccttccgg ctggctggtttattgctgataaatctggagccggtgagcgtgggtctcgcggtatcattgcagcactggggccagatggt aagccctcccgtatcgtagttatctacacgacggggagtcaggcaactatggatgaacgaaatagacagatcgctgag ataggtgcctcactgattaagcattggtaactgtcagaccaagtttactcatatatactttagattgatttaaaacttcattttta atttaaaaggatctaggtgaagatcctttttgataatctcatgaccaaaatcccttaacgtgagttttcgttccactgagcgtc agaccccgtagaaaagatcaaaggatcttcttgagatcctttttttctgcgcgtaatctgctgcttgcaaacaaaaaaacc accgctaccagcggtggtttgtttgccggatcaagagctaccaactctttttccgaaggtaactggcttcagcagagcgc agataccaaatactgtccttctagtgtagccgtagttaggccaccacttcaagaactctgtagcaccgcctacatacctcg ctctgctaatcctgttaccagtggctgctgccagtggcgataagtcgtgtcttaccgggttggactcaagacgatagttac cggataaggcgcagcggtcgggctgaacggggggttcgtgcacacagcccagcttggagcgaacgacctacaccg aactgagatacctacagcgtgagctatgagaaagcgccacgcttcccgaagggagaaaggcggacaggtatccggt aagcggcagggtcggaacaggagagcgcacgagggagcttccagggggaaacgcctggtatctttatagtcctgtc gggtttcgccacctctgacttgagcgtcgatttttgtgatgctcgtcaggggggcggagcctatggaaaaacgccagca acgcggcctttttacggttcctggccttttgctggccttttgctcacatgttctttcctgcgttatcccctgattctgtggataa ccgtattaccgcctttgagtgagctgataccgctcgccgcagccgaacgaccgagcgcagcgagtcagtgagcgag gaagcggaagagcgcccaatacgcaaaccgcctctccccgcgcgttggccgattcattaatgcagctggcacgaca ggtttcccgactggaaagcgggcagtgagcgcaacgcaattaatgtgagttagctcactcattaggcaccccaggcttt acactttatgcttccggctcgtatgttgtgtggaattgtgagcggataacaatttcacacaggaaacagctatgaccatgat tacgccaagcgcgcaattaaccctcactaaagggaacaaaagctggagctgcaagcttaatgtagtcttatgcaatact cttgtagtcttgcaacatggtaacgatgagttagcaacatgccttacaaggagagaaaaagcaccgtgcatgccgattg gtggaagtaaggtggtacgatcgtgccttattaggaaggcaacagacgggtctgacatggattggacgaaccactgaa ttgccgcattgcagagatattgtatttaagtgcctagctcgatacaataaacgggtctctctggttagaccagatctgagcc tgggagctctctggctaactagggaacccactgcttaagcctcaataaagcttgccttgagtgcttcaagtagtgtgtgcc cgtctgttgtgtgactctggtaactagagatccctcagacccttttagtcagtgtggaaaatctctagcagtggcgcccga acagggacctgaaagcgaaagggaaaccagagctctctcgacgcaggactcggcttgctgaagcgcgcacggcaa gaggcgaggggcggcgactggtgagtacgccaaaaattttgactagcggaggctagaaggagagagatgggtgcg agagcgtcagtattaagcgggggagaattagatcgcgatgggaaaaaattcggttaaggccagggggaaagaaaaa atataaattaaaacatatagtatgggcaagcagggagctagaacgattcgcagttaatcctggcctgttagaaacatcag aaggctgtagacaaatactgggacagctacaaccatcccttcagacaggatcagaagaacttagatcattatataataca gtagcaaccctctattgtgtgcatcaaaggatagagataaaagacaccaaggaagctttagacaagatagaggaagag caaaacaaaagtaagaccaccgcacagcaagcggccgctgatcttcagacctggaggaggagatatgagggacaat tggagaagtgaattatataaatataaagtagtaaaaattgaaccattaggagtagcacccaccaaggcaaagagaaga |
| | | gtggtgcagagagaaaaaagagcagtgggaataggagctttgttccttgggttcttgggagcagcaggaagcactatg ggcgcagcctcaatgacgctgacggtacaggccagacaattattgtctggtatagtgcagcagcagaacaatttgctga gggctattgaggcgcaacagcatctgttgcaactcacagtctggggcatcaagcagctccaggcaagaatcctggctg tggaaagatacctaaaggatcaacagctcctggggatttggggttgctctggaaaactcatttgcaccactgctgtgcctt ggaatgctagttggagtaataaatctctggaacagattggaatcacacgacctggatggagtgggacagagaaattaa caattacacaagcttaatacactccttaattgaagaatcgcaaaaccagcaagaaaagaatgaacaagaattattggaat tagataaatgggcaagtttgtggaattggtttaacataacaaattggctgtggtatataaaattattcataatgatagtagga ggcttggtaggtttaagaatagtttttgctgtactttctatagtgaatagagttaggcagggatattcaccattatcgtttcag acccacctcccaaccccgaggggacccgacaggcccgaaggaatagaagaagaaggtggagagagagacagag acagatccattcgattagtgaacggatctcgacggttaacttttaaaagaaaaggggggattggggggtacagtgcag gggaaagaatagtagacataatagcaacagacatacaaactaaagaattacaaaaacaaattacaaaaattcaaaatttt atcgagctttgcaaagatggataaagttttaaacagagaggaatctttgcagctaatggaccttctaggtcttgaaaggag tgcctcgtgaggctccggtgcccgtcagtgggcagagcgcacatcgcccacagtccccgagaagttggggggagg ggtcggcaattgaaccggtgcctagagaaggtggcgcggggtaaactgggaaagtgatgtcgtgtactggctccgcc tttttcccgagggtgggggagaaccgtatataagtgcagtagtcgccgtgaacgttctttttcgcaacgggtttgccgcc agaacacaggtaagtgccgtgtgtggttcccgcgggcctggcctctttacgggttatggcccttgcgtgccttgaattac ttccacctggctgcagtacgtgattcttgatcccgagcttcgggttggaagtgggtgggagagttcgaggccttgcgctt aaggagccccttcgcctcgtgcttgagttgaggcctggcctgggcgctggggccgccgcgtgcgaatctggtggcac cttcgcgcctgtctcgctgctttcgataagtctctagccatttaaaatttttgatgacctgctgcgacgctttttttctggcaag atagtcttgtaaatgcgggccaagatctgcacactggtatttcggtttttggggccgcgggcggcgacggggcccgtg cgtcccagcgcacatgttcggcgaggcggggcctgcgagcgcggccaccgagaatcggacgggggtagtctcaag ctggccggcctgctctggtgcctggcctcgcgccgccgtgtatcgccccgccctgggcggcaaggctggcccggtc ggcaccagttgcgtgagcggaaagatggccgcttcccggccctgctgcagggagctcaaaatggaggacgcggcg ctcgggagagcgggcgggtgagtcacccacacaaaggaaaagggcctttccgtcctcagccgtcgcttcatgtgact ccacggagtaccgggcgccgtccaggcacctcgattagttctcgagcttttggagtacgtcgtctttaggttggggggga ggggttttatgcgatggagtttccccacactgagtgggtggagactgaagttaggccagcttggcacttgatgtaattctc cttggaatttgccctttttgagtttggatcttggttcattctcaagcctcagacagtggttcaaagtttttttcttccatttcaggt gtcgtgaggaattcggtaccgcggccgcccggggatccatggccttaccagtgaccgccttgctcctgccgctggcct tgctgctccacgccgccaggccggacattcagatgactcagagcccgtcttccctgtccgcttcagtgggtgaccgtgt taccattacctgccgtgcttctcagagcctggtgcactcccagggtaacacctatctgcggtggtatcagcagaaaccg ggcaaggcccccaaagttctgatttataaggtctctaatcggtttagtggcgttccgtcacgcttctcaggtagcgggtcc ggaacagattttaccctgacaattagcagcctgcaaccggaagacttcgcaacctattactgccagcagtctacccatgt gccctggacatttggccagggtactaaggttgagctgaaacgtgctggaggtggaggaagcggaggtggaggcagc ggcggtgggggatctggcggtgggggaagtggcggtgggggatcaggcggtgggggaagcgaagtccagctggt ggagagtggaggtggactggtgcagccaggaggcagcctgcgtctgtcctgtgccgcgtctggctttaccttcagcga ttactggatgaactgggtccgtcaggcaccaggaaagggcctggaatgggtggcgcagattcggaacaagccttaca actacgagacttactacgcggatagcgtgaagggtcgcttcaccatcagtcgtgacacatcaaagaacactgtttacct gcaaatgaacagcctgcgtgcagaagataccgctgtctattactgcacaggctcttattacggcatggactactggggc cagggtactctggtgaccgtttctagtaccacgacgccagcgccgcgaccaccaacaccggcgcccaccatcgcgtc gcagcccctgtccctgcgcccagaggcgtgccggccagcggcgggggggcgcagtgcacacgagggggctggact tcgcctgtgatatctacatctgggcgcccttggccgggacttgtggggtccttctcctgtcactggttatcaccctttactg caaacggggcagaaagaaactcctgtatatattcaaacaaccatttatgagaccagtacaaactactcaagaggaagat ggctgtagctgccgatttccagaagaagaagaaggaggatgtgaactgagagtgaagttcagcaggagcgcagacg cccccgcgtacaagcagggccagaaccagctctataacgagctcaatctaggacgaagagaggagtacgatgttttg gacaagagacgtggccgggaccctgagatggggggaaagccgagaaggaagaaccctcaggaaggcctgtacaa tgaactgcagaaagataagatggcggaggcctacagtgagattgggatgaaaggcgagcgccggaggggcaagg ggcacgatggcctttaccagggtctcagtacagccaccaaggacacctacgacgcccttcacatgcaggccctgccc cctcgctaagtcgacaatcaacctctggattacaaaatttgtgaaagattgactggtattcttaactatgttgctccttttacg ctatgtggatacgctgctttaatgcctttgtatcatgctattgcttcccgtatggctttcattttctcctccttgtataaatcctgg ttgctgtctctttatgaggagttgtggcccgttgtcaggcaacgtggcgtggtgtgcactgtgtttgctgacgcaacccccc |
| | | actggttggggcattgccaccacctgtcagctcctttccgggactttcgctttccccctccctattgccacggcggaactc atcgccgcctgccttgcccgctgctggacaggggctcggctgttgggcactgacaattccgtggtgttgtcggggaag ctgacgtcctttccatggctgctcgcctgtgttgccacctggattctgcgcgggacgtccttctgctacgtcccttcggcc ctcaatccagcggaccttccttcccgcggcctgctgccggctctgcggcctcttccgcgtcttcgccttcgccctcagac gagtcggatctccctttgggccgcctccccgcctggaattcgagctcggtacctttaagaccaatgacttacaaggcag ctgtagatcttagccactttttaaaagaaaaggggggactggaagggctaattcactcccaacgaagacaagatctgctt tttgcttgtactgggtctctctggttagaccagatctgagcctgggagctctctggctaactagggaacccactgcttaag cctcaataaagcttgccttgagtgcttcaagtagtgtgtgcccgtctgttgtgtgactctggtaactagagatccctcagac ccttttagtcagtgtggaaaatctctagcagtagtagttcatgtcatcttattattcagtatttataacttgcaaagaaatgaat atcagagagtgagaggaacttgtttattgcagcttataatggttacaaataaagcaatagcatcacaaatttcacaaataa agcatttttttcactgcattctagttgtggtttgtccaaactcatcaatgtatcttatcatgtctggctctagctatcccgcccct aactccgcccagttccgcccattctccgccccatggctgactaattttttttatttatgcagaggccgaggccgcctcggc ctctgagctattccagaagtagtgaggaggcttttttggaggcctaggcttttgcgtcgagacgtacccaattcgccctat agtgagtcgtattacgcgcgctcactggccgtcgttttacaacgtcgtgactgggaaaaccctggcgttacccaacttaa tcgccttgcagcacatccccctttcgccagctggcgtaatagcgaagaggcccgcaccgatcgcccttcccaacagtt gcgcagcctgaatggcgaatggcgcgacgcgccctgtagcggcgcattaagcgcggcgggtgtggtggttacgcg cagcgtgaccgctacacttgccagcgccctagcgcccgctcctttcgctttcttcccttcctttctcgccacgttcgccgg ctttccccgtcaagctctaaatcgggggctccctttagggttccgatttagtgctttacggcacctcgaccccaaaaaactt gattagggtgatggttcacgtagtgggccatcgccctgatagacggtttttcgccctttgacgttggagtccacgttcttta atagtggactcttgttccaaactggaacaacactcaaccctatctcggtctattcttttgatttataagggattttgccgatttc ggcctattggttaaaaaatgagctgatttaacaaaaatttaacgcgaattttaacaaaatattaacgtttacaatttcc |
| LV-EF1a-4M5.3-BBZ | 3 | caggtggcacttttcggggaaatgtgcgcggaacccctatttgtttatttttctaaatacattcaaatatgtatccgctcatga gacaataaccctgataaatgcttcaataatattgaaaaaggaagagtatgagtattcaacatttccgtgtcgcccttattcc cttttttgcggcattttgccttcctgtttttgctcacccagaaacgctggtgaaagtaaaagatgctgaagatcagttgggtg cacgagtgggttacatcgaactggatctcaacagcggtaagatccttgagagttttcgccccgaagaacgttttccaatg atgagcacttttaaagttctgctatgtggcgcggtattatcccgtattgacgccgggcaagagcaactcggtcgccgcat acactattctcagaatgacttggttgagtactcaccagtcacagaaaagcatcttacggatggcatgacagtaagagaat tatgcagtgctgccataaccatgagtgataacactgcggccaacttacttctgacaacgatcggaggaccgaaggagc taaccgcttttttgcacaacatgggggatcatgtaactcgccttgatcgttgggaaccggagctgaatgaagccatacca aacgacgagcgtgacaccacgatgcctgtagcaatggcaacaacgttgcgcaaactattaactggcgaactacttact ctagcttcccggcaacaattaatagactggatggaggcggataaagttgcaggaccacttctgcgctcggcccttccgg ctggctggtttattgctgataaatctggagccggtgagcgtgggtctcgcggtatcattgcagcactggggccagatggt aagccctcccgtatcgtagttatctacacgacggggagtcaggcaactatggatgaacgaaatagacagatcgctgag ataggtgcctcactgattaagcattggtaactgtcagaccaagtttactcatatatactttagattgatttaaaacttcattttta atttaaaaggatctaggtgaagatcctttttgataatctcatgaccaaaatcccttaacgtgagttttcgttccactgagcgtc agaccccgtagaaaagatcaaaggatcttcttgagatcctttttttctgcgcgtaatctgctgcttgcaaacaaaaaaacc accgctaccagcggtggtttgtttgccggatcaagagctaccaactctttttccgaaggtaactggcttcagcagagcgc agataccaaatactgtccttctagtgtagccgtagttaggccaccacttcaagaactctgtagcaccgcctacatacctcg ctctgctaatcctgttaccagtggctgctgccagtggcgataagtcgtgtcttaccgggttggactcaagacgatagttac cggataaggcgcagcggtcgggctgaacggggggttcgtgcacacagcccagcttggagcgaacgacctacaccg aactgagatacctacagcgtgagctatgagaaagcgccacgcttcccgaagggagaaaggcggacaggtatccggt aagcggcagggtcggaacaggagagcgcacgagggagcttccagggggaaacgcctggtatctttatagtcctgtc gggtttcgccacctctgacttgagcgtcgatttttgtgatgctcgtcaggggggcggagcctatggaaaaacgccagca acgcggcctttttacggttcctggccttttgctggccttttgctcacatgttctttcctgcgttatcccctgattctgtggataa ccgtattaccgcctttgagtgagctgataccgctcgccgcagccgaacgaccgagcgcagcgagtcagtgagcgag gaagcggaagagcgcccaatacgcaaaccgcctctccccgcgcgttggccgattcattaatgcagctggcacgaca ggtttcccgactggaaagcgggcagtgagcgcaacgcaattaatgtgagttagctcactcattaggcaccccaggcttt acactttatgcttccggctcgtatgttgtgtggaattgtgagcggataacaatttcacacaggaaacagctatgaccatgat tacgccaagcgcgcaattaaccctcactaaagggaacaaaagctggagctgcaagcttaatgtagtcttatgcaatact cttgtagtcttgcaacatggtaacgatgagttagcaacatgccttacaaggagagaaaaagcaccgtgcatgccgattg |
| | | gtggaagtaaggtggtacgatcgtgccttattaggaaggcaacagacgggtctgacatggattggacgaaccactgaa ttgccgcattgcagagatattgtatttaagtgcctagctcgatacaataaacgggtctctctggttagaccagatctgagcc tgggagctctctggctaactagggaacccactgcttaagcctcaataaagcttgccttgagtgcttcaagtagtgtgtgcc cgtctgttgtgtgactctggtaactagagatccctcagacccttttagtcagtgtggaaaatctctagcagtggcgcccga acagggacctgaaagcgaaagggaaaccagagctctctcgacgcaggactcggcttgctgaagcgcgcacggcaa gaggcgaggggcggcgactggtgagtacgccaaaaattttgactagcggaggctagaaggagagagatgggtgcg agagcgtcagtattaagcgggggagaattagatcgcgatgggaaaaaattcggttaaggccagggggaaagaaaaa atataaattaaaacatatagtatgggcaagcagggagctagaacgattcgcagttaatcctggcctgttagaaacatcag aaggctgtagacaaatactgggacagctacaaccatcccttcagacaggatcagaagaacttagatcattatataataca gtagcaaccctctattgtgtgcatcaaaggatagagataaaagacaccaaggaagctttagacaagatagaggaagag caaaacaaaagtaagaccaccgcacagcaagcggccgctgatcttcagacctggaggaggagatatgagggacaat tggagaagtgaattatataaatataaagtagtaaaaattgaaccattaggagtagcacccaccaaggcaaagagaaga gtggtgcagagagaaaaaagagcagtgggaataggagctttgttccttgggttcttgggagcagcaggaagcactatg ggcgcagcctcaatgacgctgacggtacaggccagacaattattgtctggtatagtgcagcagcagaacaatttgctga gggctattgaggcgcaacagcatctgttgcaactcacagtctggggcatcaagcagctccaggcaagaatcctggctg tggaaagatacctaaaggatcaacagctcctggggatttggggttgctctggaaaactcatttgcaccactgctgtgcctt ggaatgctagttggagtaataaatctctggaacagattggaatcacacgacctggatggagtgggacagagaaattaa caattacacaagcttaatacactccttaattgaagaatcgcaaaaccagcaagaaaagaatgaacaagaattattggaat tagataaatgggcaagtttgtggaattggtttaacataacaaattggctgtggtatataaaattattcataatgatagtagga ggcttggtaggtttaagaatagtttttgctgtactttctatagtgaatagagttaggcagggatattcaccattatcgtttcag acccacctcccaaccccgaggggacccgacaggcccgaaggaatagaagaagaaggtggagagagagacagag acagatccattcgattagtgaacggatctcgacggttaacttttaaaagaaaaggggggattggggggtacagtgcag gggaaagaatagtagacataatagcaacagacatacaaactaaagaattacaaaaacaaattacaaaaattcaaaattt atcgagctttgcaaagatggataaagttttaaacagagaggaatctttgcagctaatggaccttctaggtcttgaaaggag tgcctcgtgaggctccggtgcccgtcagtgggcagagcgcacatcgcccacagtccccgagaagttggggggagg ggtcggcaattgaaccggtgcctagagaaggtggcgcggggtaaactgggaaagtgatgtcgtgtactggctccgcc tttttcccgagggtgggggagaaccgtatataagtgcagtagtcgccgtgaacgttctttttcgcaacgggtttgccgcc agaacacaggtaagtgccgtgtgtggttcccgcgggcctggcctctttacgggttatggcccttgcgtgccttgaattac ttccacctggctgcagtacgtgattcttgatcccgagcttcgggttggaagtgggtgggagagttcgaggccttgcgctt aaggagccccttcgcctcgtgcttgagttgaggcctggcctgggcgctggggccgccgcgtgcgaatctggtggcac cttcgcgcctgtctcgctgctttcgataagtctctagccatttaaaatttttgatgacctgctgcgacgctttttttctggcaag atagtcttgtaaatgcgggccaagatctgcacactggtatttcggtttttggggccgcgggcggcgacggggcccgtg cgtcccagcgcacatgttcggcgaggcggggcctgcgagcgcggccaccgagaatcggacgggggtagtctcaag ctggccggcctgctctggtgcctggcctcgcgccgccgtgtatcgccccgccctgggcggcaaggctggcccggtc ggcaccagttgcgtgagcggaaagatggccgcttcccggccctgctgcagggagctcaaaatggaggacgcggcg ctcgggagagcgggcgggtgagtcacccacacaaaggaaaagggcctttccgtcctcagccgtcgcttcatgtgact ccacggagtaccgggcgccgtccaggcacctcgattagttctcgagcttttggagtacgtcgtctttaggttggggggga ggggttttatgcgatggagtttccccacactgagtgggtggagactgaagttaggccagcttggcacttgatgtaattctc cttggaatttgccctttttgagtttggatcttggttcattctcaagcctcagacagtggttcaaagtttttttcttccatttcaggt gtcgtgaggaattcggtaccgcggccgcccggggatccatggccttaccagtgaccgccttgctcctgccgctggcct tgctgctccacgccgccaggccggatgttgttatgacacagaccccgctgagtctgcccgtctccctgggcgaccagg cctccatttcttgccgtagttcacagtccctggtgcactctaacggaaatacctatctgcgctggtacctgcagaaaccgg gccagagccccaaagtcctgatctataaggtgtccaaccgggtgtctggtgttccggatcgcttttcagggagcggcag cggcaccgacttcacactgaagatcaatcgtgttgaagcagaggatctgggcgtctacttttgctctcagagtacacatg tgccatggactttcggcggtgggaccaaactggaaattaagagctccgcagatgacgctaaaaaggacgccgcgaaa aaggatgacgccaaaaaggatgacgcgaaaaaggatggaggcgtcaaactggacgagaccggtgggggactggt gcagccgggcggtgcaatgaagctgagttgtgtgacttcaggttttaccttcgggcactattggatgaactgggttcgtc agagtccagaaaaaggtctggagtgggtcgctcagtttcggaacaagccttacaactacgaaacatactactcagatag cgttaaagggcgcttcactattagccgtgatgactccaagtctagtgtgtacctgcagatgaacaatctgcgggttgagg acacaggcatctattactgcacaggggcgtcctatggtatggagtatctggggcagggaacaagcgtcaccgtctcaa |
| | | ccacgacgccagcgccgcgaccaccaacaccggcgcccaccatcgcgtcgcagcccctgtccctgcgcccagag gcgtgccggccagcggcgggggcgcagtgcacacgagggggctggacttcgcctgtgatatctacatctgggcg cccttggccgggacttgtggggtccttctcctgtcactggttatcaccctttactgcaaacggggcagaaagaaactcct gtatatattcaaacaaccatttatgagaccagtacaaactactcaagaggaagatggctgtagctgccgatttccagaag aagaagaaggaggatgtgaactgagagtgaagttcagcaggagcgcagacgcccccgcgtacaagcagggccag aaccagctctataacgagctcaatctaggacgaagagaggagtacgatgttttggacaagagacgtggccgggaccc tgagatggggggaaagccgagaaggaagaaccctcaggaaggcctgtacaatgaactgcagaaagataagatggc ggaggcctacagtgagattgggatgaaaggcgagcgccggaggggcaaggggcacgatggcctttaccagggtct cagtacagccaccaaggacacctacgacgcccttcacatgcaggccctgccccctcgctaagtcgacaatcaacctct ggattacaaaatttgtgaaagattgactggtattcttaactatgttgctccttttacgctatgtggatacgctgctttaatgcctt tgtatcatgctattgcttcccgtatggctttcattttctcctccttgtataaatcctggttgctgtctctttatgaggagttgtggc ccgttgtcaggcaacgtggcgtggtgtgcactgtgtttgctgacgcaacccccactggttggggcattgccaccacctg tcagctcctttccgggactttcgctttccccctccctattgccacggcggaactcatcgccgcctgccttgcccgctgctg gacaggggctcggctgttgggcactgacaattccgtggtgttgtcggggaagctgacgtcctttccatggctgctcgcc tgtgttgccacctggattctgcgcgggacgtccttctgctacgtcccttcggccctcaatccagcggaccttccttcccgc ggcctgctgccggctctgcggcctcttccgcgtcttcgccttcgccctcagacgagtcggatctccctttgggccgcctc cccgcctggaattcgagctcggtacctttaagaccaatgacttacaaggcagctgtagatcttagccactttttaaaagaa aaggggggactggaagggctaattcactcccaacgaagacaagatctgctttttgcttgtactgggtctctctggttaga ccagatctgagcctgggagctctctggctaactagggaacccactgcttaagcctcaataaagcttgccttgagtgcttc aagtagtgtgtgcccgtctgttgtgtgactctggtaactagagatccctcagacccttttagtcagtgtggaaaatctctag cagtagtagttcatgtcatcttattattcagtatttataacttgcaaagaaatgaatatcagagagtgagaggaacttgtttatt gcagcttataatggttacaaataaagcaatagcatcacaaatttcacaaataaagcatttttttcactgcattctagttgtggt ttgtccaaactcatcaatgtatcttatcatgtctggctctagctatcccgcccctaactccgcccagttccgcccattctccg ccccatggctgactaattttttttatttatgcagaggccgaggccgcctcggcctctgagctattccagaagtagtgagga ggcttttttggaggcctaggcttttgcgtcgagacgtacccaattcgccctatagtgagtcgtattacgcgcgctcactgg ccgtcgttttacaacgtcgtgactgggaaaaccctggcgttacccaacttaatcgccttgcagcacatccccctttcgcca gctggcgtaatagcgaagaggcccgcaccgatcgcccttcccaacagttgcgcagcctgaatggcgaatggcgcga cgcgccctgtagcggcgcattaagcgcggcgggtgtggtggttacgcgcagcgtgaccgctacacttgccagcgcc ctagcgcccgctcctttcgctttcttcccttcctttctcgccacgttcgccggctttccccgtcaagctctaaatcgggggct ccctttagggttccgatttagtgctttacggcacctcgaccccaaaaaacttgattagggtgatggttcacgtagtgggcc atcgccctgatagacggtttttcgccctttgacgttggagtccacgttctttaatagtggactcttgttccaaactggaacaa cactcaaccctatctcggtctattcttttgatttataagggattttgccgatttcggcctattggttaaaaaatgagctgattta acaaaaatttaacgcgaattttaacaaaatattaacgtttacaatttcc |
| LV-EF1a-FITC(E2) -BBZ | 4 | caggtggcacttttcggggaaatgtgcgcggaacccctatttgtttatttttctaaatacattcaaatatgtatccgctcatga gacaataaccctgataaatgcttcaataatattgaaaaaggaagagtatgagtattcaacatttccgtgtcgcccttattcc cttttttgcggcattttgccttcctgtttttgctcacccagaaacgctggtgaaagtaaaagatgctgaagatcagttgggtg cacgagtgggttacatcgaactggatctcaacagcggtaagatccttgagagttttcgccccgaagaacgttttccaatg atgagcacttttaaagttctgctatgtggcgcggtattatcccgtattgacgccgggcaagagcaactcggtcgccgcat acactattctcagaatgacttggttgagtactcaccagtcacagaaaagcatcttacggatggcatgacagtaagagaat tatgcagtgctgccataaccatgagtgataacactgcggccaacttacttctgacaacgatcggaggaccgaaggagc taaccgcttttttgcacaacatgggggatcatgtaactcgccttgatcgttgggaaccggagctgaatgaagccatacca aacgacgagcgtgacaccacgatgcctgtagcaatggcaacaacgttgcgcaaactattaactggcgaactacttact ctagcttcccggcaacaattaatagactggatggaggcggataaagttgcaggaccacttctgcgctcggcccttccgg ctggctggtttattgctgataaatctggagccggtgagcgtgggtctcgcggtatcattgcagcactggggccagatggt aagccctcccgtatcgtagttatctacacgacggggagtcaggcaactatggatgaacgaaatagacagatcgctgag ataggtgcctcactgattaagcattggtaactgtcagaccaagtttactcatatatactttagattgatttaaaacttcattttta atttaaaaggatctaggtgaagatcctttttgataatctcatgaccaaaatcccttaacgtgagttttcgttccactgagcgtc agaccccgtagaaaagatcaaaggatcttcttgagatcctttttttctgcgcgtaatctgctgcttgcaaacaaaaaaacc accgctaccagcggtggtttgtttgccggatcaagagctaccaactctttttccgaaggtaactggcttcagcagagcgc agataccaaatactgtccttctagtgtagccgtagttaggccaccacttcaagaactctgtagcaccgcctacatacctcg |
| | | ctctgctaatcctgttaccagtggctgctgccagtggcgataagtcgtgtcttaccgggttggactcaagacgatagttac cggataaggcgcagcggtcgggctgaacggggggttcgtgcacacagcccagcttggagcgaacgacctacaccg aactgagatacctacagcgtgagctatgagaaagcgccacgcttcccgaagggagaaaggcggacaggtatccggt aagcggcagggtcggaacaggagagcgcacgagggagcttccagggggaaacgcctggtatctttatagtcctgtc gggtttcgccacctctgacttgagcgtcgatttttgtgatgctcgtcaggggggcggagcctatggaaaaacgccagca acgcggcctttttacggttcctggccttttgctggccttttgctcacatgttctttcctgcgttatcccctgattctgtggataa ccgtattaccgcctttgagtgagctgataccgctcgccgcagccgaacgaccgagcgcagcgagtcagtgagcgag gaagcggaagagcgcccaatacgcaaaccgcctctccccgcgcgttggccgattcattaatgcagctggcacgaca ggtttcccgactggaaagcgggcagtgagcgcaacgcaattaatgtgagttagctcactcattaggcaccccaggcttt acactttatgcttccggctcgtatgttgtgtggaattgtgagcggataacaatttcacacaggaaacagctatgaccatgat tacgccaagcgcgcaattaaccctcactaaagggaacaaaagctggagctgcaagcttaatgtagtcttatgcaatact cttgtagtcttgcaacatggtaacgatgagttagcaacatgccttacaaggagagaaaaagcaccgtgcatgccgattg gtggaagtaaggtggtacgatcgtgccttattaggaaggcaacagacgggtctgacatggattggacgaaccactgaa ttgccgcattgcagagatattgtatttaagtgcctagctcgatacaataaacgggtctctctggttagaccagatctgagcc tgggagctctctggctaactagggaacccactgcttaagcctcaataaagcttgccttgagtgcttcaagtagtgtgtgcc cgtctgttgtgtgactctggtaactagagatccctcagacccttttagtcagtgtggaaaatctctagcagtggcgcccga acagggacctgaaagcgaaagggaaaccagagctctctcgacgcaggactcggcttgctgaagcgcgcacggcaa gaggcgaggggcggcgactggtgagtacgccaaaaattttgactagcggaggctagaaggagagagatgggtgcg agagcgtcagtattaagcgggggagaattagatcgcgatgggaaaaaattcggttaaggccagggggaaagaaaaa atataaattaaaacatatagtatgggcaagcagggagctagaacgattcgcagttaatcctggcctgttagaaacatcag aaggctgtagacaaatactgggacagctacaaccatcccttcagacaggatcagaagaacttagatcattatataataca gtagcaaccctctattgtgtgcatcaaaggatagagataaaagacaccaaggaagctttagacaagatagaggaagag caaaacaaaagtaagaccaccgcacagcaagcggccgctgatcttcagacctggaggaggagatatgagggacaat tggagaagtgaattatataaatataaagtagtaaaaattgaaccattaggagtagcacccaccaaggcaaagagaaga gtggtgcagagagaaaaaagagcagtgggaataggagctttgttccttgggttcttgggagcagcaggaagcactatg ggcgcagcctcaatgacgctgacggtacaggccagacaattattgtctggtatagtgcagcagcagaacaatttgctga gggctattgaggcgcaacagcatctgttgcaactcacagtctggggcatcaagcagctccaggcaagaatcctggctg tggaaagatacctaaaggatcaacagctcctggggatttggggttgctctggaaaactcatttgcaccactgctgtgcctt ggaatgctagttggagtaataaatctctggaacagattggaatcacacgacctggatggagtgggacagagaaattaa caattacacaagcttaatacactccttaattgaagaatcgcaaaaccagcaagaaaagaatgaacaagaattattggaat tagataaatgggcaagtttgtggaattggtttaacataacaaattggctgtggtatataaaattattcataatgatagtagga ggcttggtaggtttaagaatagtttttgctgtactttctatagtgaatagagttaggcagggatattcaccattatcgtttcag acccacctcccaaccccgaggggacccgacaggcccgaaggaatagaagaagaaggtggagagagagacagag acagatccattcgattagtgaacggatctcgacggttaacttttaaaagaaaaggggggattggggggtacagtgcag gggaaagaatagtagacataatagcaacagacatacaaactaaagaattacaaaaacaaattacaaaaattcaaaattt atcgagctttgcaaagatggataaagttttaaacagagaggaatctttgcagctaatggaccttctaggtcttgaaaggag tgcctCGTGAGGCTCCGGTGCCCGTCAGTGGGCAGAGCGCACATCGCCCA CAGTCCCCGAGAAGTTGGGGGGAGGGGTCGGCAATTGAACCGGTGCCT AGAGAAGGTGGCGCGGGGTAAACTGGGAAAGTGATGTCGTGTACTGGC TCCGCCTTTTTCCCGAGGGTGGGGGAGAACCGTATATAAGTGCAGTAGT CGCCGTGAACGTTCTTTTTCGCAACGGGTTTGCCGCCAGAACACAGGTA AGTGCCGTGTGTGGTTCCCGCGGGCCTGGCCTCTTTACGGGTTATGGCC CTTGCGTGCCTTGAATTACTTCCACCTGGCTGCAGTACGTGATTCTTGAT CCCGAGCTTCGGGTTGGAAGTGGGTGGGAGAGTTCGAGGCCTTGCGCTT AAGGAGCCCCTTCGCCTCGTGCTTGAGTTGAGGCCTGGCCTGGGCGCTG GGGCCGCCGCGTGCGAATCTGGTGGCACCTTCGCGCCTGTCTCGCTGCT TTCGATAAGTCTCTAGCCATTTAAAATTTTTGATGACCTGCTGCGACGCT TTTTTTCTGGCAAGATAGTCTTGTAAATGCGGGCCAAGATCTGCACACT GGTATTTCGGTTTTTGGGGCCGCGGGCGGCGACGGGGCCCGTGCGTCCC AGCGCACATGTTCGGCGAGGCGGGGCCTGCGAGCGCGGCCACCGAGAA |
| | | TCGGACGGGGTAGTCTCAAGCTGGCCGGCCTGCTCTGGTGCCTGGCCT CGCGCCGCCGTGTATCGCCCCGCCCTGGGCGGCAAGGCTGGCCCGGTC GGCACCAGTTGCGTGAGCGGAAAGATGGCCGCTTCCCGGCCCTGCTGC AGGGAGCTCAAAATGGAGGACGCGGCGCTCGGGAGAGCGGGCGGGTG AGTCACCCACACAAAGGAAAAGGGCCTTTCCGTCCTCAGCCGTCGCTTC ATGTGACTCCACGGAGTACCGGGCGCCGTCCAGGCACCTCGATTAGTTC TCGAGCTTTTGGAGTACGTCGTCTTTAGGTTGGGGGGAGGGGTTTTATG CGATGGAGTTTCCCCACACTGAGTGGGTGGAGACTGAAGTTAGGCCAG CTTGGCACTTGATGTAATTCTCCTTGGAATTTGCCCTTTTTGAGTTTGGA TCTTGGTTCATTCTCAAGCCTCAGACAGTGGTTCAAAGTTTTTTTCTTCC ATTTCAGGTGTCGTGAggaattcggtaccgcggccgcccggggatccatggccttaccagtgaccgcc ttgctcctgccgctggccttgctgctccacgccgccaggccgCAGGTTCAGCTGGTTGAGAGCG GAGGCAATCTGGTTCAGCCCGGTGGTAGTCTGCGTCTGTCTTGTGCGGC GTCAGGGTTCACTTTCGGTAGTTTTTCAATGAGCTGGGTCCGTCAGGCA CCAGGCGGTGGGCTGGAATGGGTGGCAGGTCTGTCTGCACGTAGCTCC CTGACCCACTATGCAGATAGTGTTAAAGGGCGGTTCACAATTTCACGCG ACAACGCTAAGAATAGCGTCTACCTGCAAATGAACTCCCTGCGGGTCG AGGATACCGCAGTGTATTACTGCGCTCGCCGTTCTTATGACTCTAGTGG ATACTGGGGCCATTTTTATAGCTACATGGATGTGTGGGGACAGGGCACT CTGGTGACCGTTTCCGGAGGCGGTGGGTCTGGAGGCGGTGGGAGTGGA GGCGGTGGGTCAAGCGTTCTGACCCAGCCGTCCTCTGTCAGCGCCGCGC CAGGCCAGAAAGTGACAATTTCCTGTTCTGGAAGTACTTCAAACATCGG CAACAATTATGTTTCCTGGTATCAGCAGCACCCGGGCAAAGCGCCCAA GCTGATGATTTATGATGTGTCTAAACGTCCAAGTGGTGTTCCTGACCGG TTCAGCGGTTCCAAGTCTGGGAATAGTGCCTCACTGGACATCTCAGGCC TGCAAAGCGAAGATGAGGCGGACTATTACTGCGCAGCTTGGGATGACA GCCTGTCCGAATTTCTGTTCGGCACCGGGACAAAGCTGACCGTGCTGGG Caccacgacgccagcgccgcgaccaccaacaccggcgcccaccatcgcgtcgcagcccctgtccctgcgcccag aggcgtgccggccagcggcggggggcgcagtgcacacgagggggctggacttcgcctgtgatatctacatctggg cgcccttggccgggacttgtggggtccttctcctgtcactggttatcaccctttactgcaaacggggcagaaagaaactc ctgtatatattcaaacaaccatttatgagaccagtacaaactactcaagaggaagatggctgtagctgccgatttccagaa gaagaagaaggaggatgtgaactgagagtgaagttcagcaggagcgcagacgcccccgcgtacaagcagggcca gaaccagctctataacgagctcaatctaggacgaagagaggagtacgatgttttggacaagagacgtggccgggacc ctgagatggggggaaagccgagaaggaagaaccctcaggaaggcctgtacaatgaactgcagaaagataagatgg cggaggcctacagtgagattgggatgaaaggcgagcgccggaggggcaaggggcacgatggcctttaccagggtc tcagtacagccaccaaggacacctacgacgcccttcacatgcaggccctgccccctcgctaagtcgacaatcaacctc tggattacaaaatttgtgaaagattgactggtattcttaactatgttgctccttttacgctatgtggatacgctgctttaatgcct ttgtatcatgctattgcttcccgtatggctttcattttctcctccttgtataaatcctggttgctgtctctttatgaggagttgtgg cccgttgtcaggcaacgtggcgtggtgtgcactgtgtttgctgacgcaacccccactggttggggcattgccaccacct gtcagctcctttccgggactttcgctttccccctccctattgccacggcggaactcatcgccgcctgccttgcccgctgct ggacaggggctcggctgttgggcactgacaattccgtggtgttgtcggggaagctgacgtcctttccatggctgctcgc ctgtgttgccacctggattctgcgcgggacgtccttctgctacgtcccttcggccctcaatccagcggaccttccttcccg cggcctgctgccggctctgcggcctcttccgcgtcttcgccttcgccctcagacgagtcggatctccctttgggccgcct ccccgcctggaattcgagctcggtacctttaagaccaatgacttacaaggcagctgtagatcttagccactttttaaaaga aaaggggggactggaagggctaattcactcccaacgaagacaagatctgctttttgcttgtactgggtctctctggttag accagatctgagcctgggagctctctggctaactagggaacccactgcttaagcctcaataaagcttgccttgagtgctt caagtagtgtgtgcccgtctgttgtgtgactctggtaactagagatccctcagacccttttagtcagtgtggaaaatctcta gcagtagtagttcatgtcatcttattattcagtatttataacttgcaaagaaatgaatatcagagagtgagaggaacttgttta ttgcagcttataatggttacaaataaagcaatagcatcacaaatttcacaaataaagcatttttttcactgcattctagttgtg gtttgtccaaactcatcaatgtatcttatcatgtctggctctagctatcccgcccctaactccgcccagttccgcccattctc |
| | | cgccccatggctgactaattttttttatttatgcagaggccgaggccgcctcggcctctgagctattccagaagtagtgag gaggcttttttggaggcctaggcttttgcgtcgagacgtacccaattcgccctatagtgagtcgtattacgcgcgctcact ggccgtcgttttacaacgtcgtgactgggaaaaccctggcgttacccaacttaatcgccttgcagcacatccccctttcg ccagctggcgtaatagcgaagaggcccgcaccgatcgcccttcccaacagttgcgcagcctgaatggcgaatggcg cgacgcgccctgtagcggcgcattaagcgcggcgggtgtggtggttacgcgcagcgtgaccgctacacttgccagc gccctagcgcccgctcctttcgctttcttcccttcctttctcgccacgttcgccggctttccccgtcaagctctaaatcggg ggctccctttagggttccgatttagtgctttacggcacctcgaccccaaaaaacttgattagggtgatggttcacgtagtg ggccatcgccctgatagacggtttttcgccctttgacgttggagtccacgttctttaatagtggactcttgttccaaactgga acaacactcaaccctatctcggtctattcttttgatttataagggattttgccgatttcggcctattggttaaaaaatgagctg atttaacaaaaatttaacgcgaattttaacaaaatattaacgtttacaatttcc |

| **Table 14. CAR-EC switch target interacting domain (antibodies) -Nucleotide Sequence** | | |
|---|---|---|
| **NAME** | **SEQ ID** | **SEQUENCE** |
| pBAD-CD19wt | 5 | aagaaaccaattgtccatattgcatcagacattgccgtcactgcgtcttttactggctcttctcgctaaccaaaccggtaac cctgattatttgcacggagtcacactttgctatgccatagcatttttatccataagattagcggatcctacctgacgcttttat cgcaactctctactgtttctccatacccgtttttttgggctagaaataattttgtttaactttaagaaggagaatacatcaacta gtacgcaagttcacgtaaaaagggtatctagaggttgaggtgattttatgaaaaagaatatcgcatttcttcttgctagcat gttcgtttttctattgctacaaacgcatacgctgacatccagatgacacagactacatcctccctgtctgcctctctggga gacagagtcaccatcagttgcagggcaagtcaggacattagtaaatatttaaattggtatcagcagaaaccagatggaa ctgttaaactcctgatctaccatacatcaagattacactcaggagtcccatcaaggttcagtggcagtgggtctggaaca gattattctctcaccattagcaacctggagcaagaagatattgccacttacttttgccaacagggtaatacgcttccgtaca cgttcggaggggggaccaagcttgagatcaaacgaactgtggctgcaccatctgtcttcatcttcccgccatctgatga gcagttgaaatctggaactgcctctgtcgtgtgcctgctgaataacttctatcccagagaggccaaagtacagtggaag gtggataacgccctccaatcgggtaactcccaggagagtgtcacagagcaggacagcaaggacagcacctacagcc tcagcagcaccctgacgctgagcaaagcagactacgagaaacacaaagtctacgcctgcgaagtcacccatcaggg cctgtcctcgcccgtcacaaagagcttcaacaggggagagtgttaagctggggatcctctagaggttgaggtgattttat gaaaaagaatatcgcatttcttcttgcatctatgttcgtttttctattgctacaaacgcgtacgctgaggtgaaactgcagg agtcaggacctggcctggtggcgccctcacagagcctgtccgtcacatgcactgtctcaggggtctcattacccgacta tggtgtaagctggattcgccagcctccacgaaagggtctggagtggctgggagtaatatggggtagtgaaaccacata ctataattcagctctcaaatccagactgaccatcatcaaggacaactccaagagccaagttttcttaaaaatgaacagtct gcaaactgatgacacagccatttactactgtgccaaacattattactacggtggtagctatgctatggactactggggcca aggaacctcagtcaccgtctcctcagcctccaccaagggcccatcggtcttccccctggcaccctcctccaagagcac ctctgggggcacagcggccctgggctgcctggtcaaggactacttccccgaaccggtgacggtgtcgtggaactcag gcgccctgaccagcggcgtgcacaccttcccggctgtcctacagtcctcaggactctactccctcagcagcgtggtga ctgtgccctctagcagcttgggcacccagacctacatctgcaacgtgaatcacaagcccagcaacaccaaggtggac aagaaagttgagcccaaatcttgtgacaaaactcacacataataagtcgaccgatgcccttgagagccttcaacccagt cagctccttccggtgggcgcggggcatgactatcgtcgccgcacttatgactgtcttctttatcatgcaactcgtaggaca ggtgccaaacggtctccagcttggctgttttggcggatgagagaagattttcagcctgatacagattaaatcagaacgca gaagcggtctgataaaacagaatttgcctggcggcagtagcgcggtggtcccacctgaccccatgccgaactcagaa gtgaaacgccgtagcgccgatggtagtgtggggtctccccatgcgagagtagggaactgccaggcatcaaataaaac gaaaggctcagtcgaaagactgggcctttcgttttatctgttgtttgtcggtgaacgctctcctgagtaggacaaatccgc cgggagcggatttgaacgttgcgaagcaacggcccggagggtggcgggcaggacgcccgccataaactgccagg catcaaattaagcagaaggccatcctgacggatggcctttttgcgtttctacaaactctttttgtttatttttctaaatacattca aatatgtatccgctcatgagacaataaccctgataaatgcttcaataatattgaaaaaggaagagtatgagtattcaacatt tccgtgtcgcccttattcccttttttgcggcattttgccttcctgtttttgctcacccagaaacgctggtgaaagtaaaagatg ctgaagatcagttgggtgcacgagtgggttacatcgaactggatctcaacagcggtaagatccttgagagttttcgcccc gaagaacgttttccaatgatgagcacttttaaagttctgctatgtggcgcggtattatcccgtgttgacgccgggcaagag caactcggtcgccgcatacactattctcagaatgacttggttgagtactcaccagtcacagaaaagcatcttacggatgg catgacagtaagagaattatgcagtgctgccataaccatgagtgataacactgcggccaacttacttctgacaacgatcg gaggaccgaaggagctaaccgcttttttgcacaacatgggggatcatgtaactcgccttgatcgttgggaaccggagct |
| | | gaatgaagccataccaaacgacgagcgtgacaccacgatgcctgtagcaatggcaacaacgttgcgcaaactattaa ctggcgaactacttactctagcttcccggcaacaattaatagactggatggaggcggataaagttgcaggaccacttctg cgctcggcccttccggctggctggtttattgctgataaatctggagccggtgagcgtgggtctcgcggtatcattgcagc actggggccagatggtaagccctcccgtatcgtagttatctacacgacggggagtcaggcaactatggatgaacgaaa tagacagatcgctgagataggtgcctcactgattaagcattggtaactgtcagaccaagtttactcatatatactttagattg atttaaaacttcatttttaatttaaaaggatctaggtgaagatcctttttgataatctcatgaccaaaatcccttaacgtgagttt tcgttccactgagcgtcagaccccgtagaaaagatcaaaggatcttcttgagatcctttttttctgcgcgtaatctgctgctt gcaaacaaaaaaaccaccgctaccagcggtggtttgtttgccggatcaagagctaccaactctttttccgaaggtaactg gcttcagcagagcgcagataccaaatactgtccttctagtgtagccgtagttaggccaccacttcaagaactctgtagca ccgcctacatacctcgctctgctaatcctgttaccagtggctgctgccagtggcgataagtcgtgtcttaccgggttggac tcaagacgatagttaccggataaggcgcagcggtcgggctgaacggggggttcgtgcacacagcccagcttggagc gaacgacctacaccgaactgagatacctacagcgtgagctatgagaaagcgccacgcttcccgaagggagaaaggc ggacaggtatccggtaagcggcagggtcggaacaggagagcgcacgagggagcttccagggggaaacgcctggt atctttatagtcctgtcgggtttcgccacctctgacttgagcgtcgatttttgtgatgctcgtcaggggggcggagcctatg gaaaaacgccagcaacgcggcctttttacggttcctggccttttgctggccttttgctcacatgttctttcctgcgttatccc ctgattctgtggataaccgtattaccgcctttgagtgagctgataccgctcgccgcagccgaacgaccgagcgcagcg agtcagtgagcgaggaagcggaagagcgcctgatgcggtattttctccttacgcatctgtgcggtatttcacaccgcata tggtgcactctcagtacaatctgctctgatgccgcatagttaagccagtatacactccgctatcgctacgtgactgggtca tggctgcgccccgacacccgccaacacccgctgacgcgccctgacgggcttgtctgctcccggcatccgcttacaga caagctgtgaccgtctccgggagctgcatgtgtcagaggttttcaccgtcatcaccgaaacgcgcgaggcagcagatc aattcgcgcgcgaaggcgaagcggcatgcataatgtgcctgtcaaatggacgaagcagggattctgcaaaccctatg ctactccgtcaagccgtcaattgtctgattcgttaccaattatgacaacttgacggctacatcattcactttttcttcacaacc ggcacggaactcgctcgggctggccccggtgcattttttaaatacccgcgagaaatagagttgatcgtcaaaaccaac attgcgaccgacggtggcgataggcatccgggtggtgctcaaaagcagcttcgcctggctgatacgttggtcctcgcg ccagcttaagacgctaatccctaactgctggcggaaaagatgtgacagacgcgacggcgacaagcaaacatgctgtg cgacgctggcgatatcaaaattgctgtctgccaggtgatcgctgatgtactgacaagcctcgcgtacccgattatccatc ggtggatggagcgactcgttaatcgcttccatgcgccgcagtaacaattgctcaagcagatttatcgccagcagctccg aatagcgcccttccccttgcccggcgttaatgatttgcccaaacaggtcgctgaaatgcggctggtgcgcttcatccgg gcgaaagaaccccgtattggcaaatattgacggccagttaagccattcatgccagtaggcgcgcggacgaaagtaaa cccactggtgataccattcgcgagcctccggatgacgaccgtagtgatgaatctctcctggcgggaacagcaaaatat cacccggtcggcaaacaaattctcgtccctgatttttcaccaccccctgaccgcgaatggtgagattgagaatataacctt tcattcccagcggtcggtcgataaaaaaatcgagataaccgttggcctcaatcggcgttaaacccgccaccagatggg cattaaacgagtatcccggcagcaggggatcattttgcgcttcagccatacttttcatactcccgccattcagag |
| pBAD-CD19 LS202X mt | 6 | aagaaaccaattgtccatattgcatcagacattgccgtcactgcgtcttttactggctcttctcgctaaccaaaccggtaac cctgattatttgcacggagtcacactttgctatgccatagcatttttatccataagattagcggatcctacctgacgctttttat cgcaactctctactgtttctccatacccgtttttttgggctagaaataattttgtttaactttaagaaggagaatacatcaacta gtacgcaagttcacgtaaaaagggtatctagaggttgaggtgattttatgaaaaagaatatcgcatttcttcttgctagcat gttcgtttttctattgctacaaacgcatacgctgacatccagatgacacagactacatcctccctgtctgcctctctggga gacagagtcaccatcagttgcagggcaagtcaggacattagtaaatatttaaattggtatcagcagaaaccagatggaa ctgttaaactcctgatctaccatacatcaagattacactcaggagtcccatcaaggttcagtggcagtgggtctggaaca gattattctctcaccattagcaacctggagcaagaagatattgccacttacttttgccaacagggtaatacgcttccgtaca cgttcggaggggggaccaagcttgagatcaaacgaactgtggctgcaccatctgtcttcatcttcccgccatctgatga gcagttgaaatctggaactgcctctgtcgtgtgcctgctgaataacttctatcccagagaggccaaagtacagtggaag gtggataacgccctccaatcgggtaactcccaggagagtgtcacagagcaggacagcaaggacagcacctacagcc tcagcagcaccctgacgctgagcaaagcagactacgagaaacacaaagtctacgcctgcgaagtcacccatcaggg cctgTAGtcgcccgtcacaaagagcttcaacaggggagagtgttaagctggggatcctctagaggttgaggtgattt tatgaaaaagaatatcgcatttcttcttgcatctatgttcgtttttctattgctacaaacgcgtacgctgaggtgaaactgca ggagtcaggacctggcctggtggcgccctcacagagcctgtccgtcacatgcactgtctcaggggtctcattacccga ctatggtgtaagctggattcgccagcctccacgaaagggtctggagtggctgggagtaatatggggtagtgaaaccac atactataattcagctctcaaatccagactgaccatcatcaaggacaactccaagagccaagttttcttaaaaatgaacag |
| | | tctgcaaactgatgacacagccatttactactgtgccaaacattattactacggtggtagctatgctatggactactgggg ccaaggaacctcagtcaccgtctcctcagcctccaccaagggcccatcggtcttccccctggcaccctcctccaagag cacctctgggggcacagcggccctgggctgcctggtcaaggactacttccccgaaccggtgacggtgtcgtggaact caggcgccctgaccagcggcgtgcacaccttcccggctgtcctacagtcctcaggactctactccctcagcagcgtgg tgactgtgccctctagcagcttgggcacccagacctacatctgcaacgtgaatcacaagcccagcaacaccaaggtgg acaagaaagttgagcccaaatcttgtgacaaaactcacacataataagtcgaccgatgcccttgagagccttcaaccca gtcagctccttccggtgggcgcggggcatgactatcgtcgccgcacttatgactgtcttctttatcatgcaactcgtagga caggtgccaaacggtctccagcttggctgttttggcggatgagagaagattttcagcctgatacagattaaatcagaacg cagaagcggtctgataaaacagaatttgcctggcggcagtagcgcggtggtcccacctgaccccatgccgaactcag aagtgaaacgccgtagcgccgatggtagtgtggggtctccccatgcgagagtagggaactgccaggcatcaaataaa acgaaaggctcagtcgaaagactgggcctttcgttttatctgttgtttgtcggtgaacgctctcctgagtaggacaaatcc gccgggagcggatttgaacgttgcgaagcaacggcccggagggtggcgggcaggacgcccgccataaactgcca ggcatcaaattaagcagaaggccatcctgacggatggcctttttgcgtttctacaaactctttttgtttatttttctaaatacatt caaatatgtatccgctcatgagacaataaccctgataaatgcttcaataatattgaaaaaggaagagtatgagtattcaac atttccgtgtcgcccttattcccttttttgcggcattttgccttcctgtttttgctcacccagaaacgctggtgaaagtaaaaga tgctgaagatcagttgggtgcacgagtgggttacatcgaactggatctcaacagcggtaagatccttgagagttttcgcc ccgaagaacgttttccaatgatgagcacttttaaagttctgctatgtggcgcggtattatcccgtgttgacgccgggcaag agcaactcggtcgccgcatacactattctcagaatgacttggttgagtactcaccagtcacagaaaagcatcttacggat ggcatgacagtaagagaattatgcagtgctgccataaccatgagtgataacactgcggccaacttacttctgacaacga tcggaggaccgaaggagctaaccgcttttttgcacaacatgggggatcatgtaactcgccttgatcgttgggaaccgga gctgaatgaagccataccaaacgacgagcgtgacaccacgatgcctgtagcaatggcaacaacgttgcgcaaactatt aactggcgaactacttactctagcttcccggcaacaattaatagactggatggaggcggataaagttgcaggaccactt ctgcgctcggcccttccggctggctggtttattgctgataaatctggagccggtgagcgtgggtctcgcggtatcattgc agcactggggccagatggtaagccctcccgtatcgtagttatctacacgacggggagtcaggcaactatggatgaacg aaatagacagatcgctgagataggtgcctcactgattaagcattggtaactgtcagaccaagtttactcatatatactttag attgatttaaaacttcatttttaatttaaaaggatctaggtgaagatcctttttgataatctcatgaccaaaatcccttaacgtga gttttcgttccactgagcgtcagaccccgtagaaaagatcaaaggatcttcttgagatcctttttttctgcgcgtaatctgct gcttgcaaacaaaaaaaccaccgctaccagcggtggtttgtttgccggatcaagagctaccaactctttttccgaaggta actggcttcagcagagcgcagataccaaatactgtccttctagtgtagccgtagttaggccaccacttcaagaactctgt agcaccgcctacatacctcgctctgctaatcctgttaccagtggctgctgccagtggcgataagtcgtgtcttaccgggtt ggactcaagacgatagttaccggataaggcgcagcggtcgggctgaacggggggttcgtgcacacagcccagcttg gagcgaacgacctacaccgaactgagatacctacagcgtgagctatgagaaagcgccacgcttcccgaagggagaa aggcggacaggtatccggtaagcggcagggtcggaacaggagagcgcacgagggagcttccagggggaaacgc ctggtatctttatagtcctgtcgggtttcgccacctctgacttgagcgtcgatttttgtgatgctcgtcaggggggcggagc ctatggaaaaacgccagcaacgcggcctttttacggttcctggccttttgctggccttttgctcacatgttctttcctgcgtta tcccctgattctgtggataaccgtattaccgcctttgagtgagctgataccgctcgccgcagccgaacgaccgagcgca gcgagtcagtgagcgaggaagcggaagagcgcctgatgcggtattttctccttacgcatctgtgcggtatttcacaccg catatggtgcactctcagtacaatctgctctgatgccgcatagttaagccagtatacactccgctatcgctacgtgactgg gtcatggctgcgccccgacacccgccaacacccgctgacgcgccctgacgggcttgtctgctcccggcatccgctta cagacaagctgtgaccgtctccgggagctgcatgtgtcagaggttttcaccgtcatcaccgaaacgcgcgaggcagc agatcaattcgcgcgcgaaggcgaagcggcatgcataatgtgcctgtcaaatggacgaagcagggattctgcaaacc ctatgctactccgtcaagccgtcaattgtctgattcgttaccaattatgacaacttgacggctacatcattcactttttcttcac aaccggcacggaactcgctcgggctggccccggtgcattttttaaatacccgcgagaaatagagttgatcgtcaaaac caacattgcgaccgacggtggcgataggcatccgggtggtgctcaaaagcagcttcgcctggctgatacgttggtcct cgcgccagcttaagacgctaatccctaactgctggcggaaaagatgtgacagacgcgacggcgacaagcaaacatg ctgtgcgacgctggcgatatcaaaattgctgtctgccaggtgatcgctgatgtactgacaagcctcgcgtacccgattat ccatcggtggatggagcgactcgttaatcgcttccatgcgccgcagtaacaattgctcaagcagatttatcgccagcag ctccgaatagcgcccttccccttgcccggcgttaatgatttgcccaaacaggtcgctgaaatgcggctggtgcgcttcat ccgggcgaaagaaccccgtattggcaaatattgacggccagttaagccattcatgccagtaggcgcgcggacgaaag taaacccactggtgataccattcgcgagcctccggatgacgaccgtagtgatgaatctctcctggcgggaacagcaaa |
| | | atatcacccggtcggcaaacaaattctcgtccctgatttttcaccaccccctgaccgcgaatggtgagattgagaatataa cctttcattcccagcggtcggtcgataaaaaaatcgagataaccgttggcctcaatcggcgttaaacccgccaccagat gggcattaaacgagtatcccggcagcaggggatcattttgcgcttcagccatacttttcatactcccgccattcagag |
| pBAD-CD19 HK136X mt | 7 | aagaaaccaattgtccatattgcatcagacattgccgtcactgcgtcttttactggctcttctcgctaaccaaaccggtaac cctgattatttgcacggagtcacactttgctatgccatagcatttttatccataagattagcggatcctacctgacgctttttat cgcaactctctactgtttctccatacccgtttttttgggctagaaataattttgtttaactttaagaaggagaatacatcaacta gtacgcaagttcacgtaaaaagggtatctagaggttgaggtgattttatgaaaaagaatatcgcatttcttcttgctagcat gttcgtttttttctattgctacaaacgcatacgctgacatccagatgacacagactacatcctccctgtctgcctctctggga gacagagtcaccatcagttgcagggcaagtcaggacattagtaaatatttaaattggtatcagcagaaaccagatggaa ctgttaaactcctgatctaccatacatcaagattacactcaggagtcccatcaaggttcagtggcagtgggtctggaaca gattattctctcaccattagcaacctggagcaagaagatattgccacttacttttgccaacagggtaatacgcttccgtaca cgttcggaggggggaccaagcttgagatcaaacgaactgtggctgcaccatctgtcttcatcttcccgccatctgatga gcagttgaaatctggaactgcctctgtcgtgtgcctgctgaataacttctatcccagagaggccaaagtacagtggaag gtggataacgccctccaatcgggtaactcccaggagagtgtcacagagcaggacagcaaggacagcacctacagcc tcagcagcaccctgacgctgagcaaagcagactacgagaaacacaaagtctacgcctgcgaagtcacccatcaggg cctgtcctcgcccgtcacaaagagcttcaacaggggagagtgttaagctggggatcctctagaggttgaggtgattttat gaaaaagaatatcgcatttcttcttgcatctatgttcgtttttttctattgctacaaacgcgtacgctgaggtgaaactgcagg agtcaggacctggcctggtggcgccctcacagagcctgtccgtcacatgcactgtctcaggggtctcattacccgacta tggtgtaagctggattcgccagcctccacgaaagggtctggagtggctgggagtaatatggggtagtgaaaccacata ctataattcagctctcaaatccagactgaccatcatcaaggacaactccaagagccaagttttcttaaaaatgaacagtct gcaaactgatgacacagccatttactactgtgccaaacattattactacggtggtagctatgctatggactactggggcca aggaacctcagtcaccgtctcctcagcctccaccaagggcccatcggtcttccccctggcaccctcctccTAGagc acctctgggggcacagcggccctgggctgcctggtcaaggactacttccccgaaccggtgacggtgtcgtggaactc aggcgccctgaccagcggcgtgcacaccttcccggctgtcctacagtcctcaggactctactccctcagcagcgtggt gactgtgccctctagcagcttgggcacccagacctacatctgcaacgtgaatcacaagcccagcaacaccaaggtgg acaagaaagttgagcccaaatcttgtgacaaaactcacacataataagtcgaccgatgcccttgagagccttcaaccca gtcagctccttccggtgggcgcggggcatgactatcgtcgccgcacttatgactgtcttctttatcatgcaactcgtagga caggtgccaaacggtctccagcttggctgttttggcggatgagagaagattttcagcctgatacagattaaatcagaacg cagaagcggtctgataaaacagaatttgcctggcggcagtagcgcggtggtcccacctgaccccatgccgaactcag aagtgaaacgccgtagcgccgatggtagtgtggggtctccccatgcgagagtagggaactgccaggcatcaaataaa acgaaaggctcagtcgaaagactgggcctttcgttttatctgttgtttgtcggtgaacgctctcctgagtaggacaaatcc gccgggagcggatttgaacgttgcgaagcaacggcccggagggtggcgggcaggacgcccgccataaactgcca ggcatcaaattaagcagaaggccatcctgacggatggcctttttgcgtttctacaaactctttttgtttatttttctaaatacatt caaatatgtatccgctcatgagacaataaccctgataaatgcttcaataatattgaaaaaggaagagtatgagtattcaac atttccgtgtcgcccttattcccttttttgcggcattttgccttcctgtttttgctcacccagaaacgctggtgaaagtaaaaga tgctgaagatcagttgggtgcacgagtgggttacatcgaactggatctcaacagcggtaagatccttgagagttttcgcc ccgaagaacgttttccaatgatgagcacttttaaagttctgctatgtggcgcggtattatcccgtgttgacgccgggcaag agcaactcggtcgccgcatacactattctcagaatgacttggttgagtactcaccagtcacagaaaagcatcttacggat ggcatgacagtaagagaattatgcagtgctgccataaccatgagtgataacactgcggccaacttacttctgacaacga tcggaggaccgaaggagctaaccgcttttttgcacaacatgggggatcatgtaactcgccttgatcgttgggaaccgga gctgaatgaagccataccaaacgacgagcgtgacaccacgatgcctgtagcaatggcaacaacgttgcgcaaactatt aactggcgaactacttactctagcttcccggcaacaattaatagactggatggaggcggataaagttgcaggaccactt ctgcgctcggcccttccggctggctggtttattgctgataaatctggagccggtgagcgtgggtctcgcggtatcattgc agcactggggccagatggtaagccctcccgtatcgtagttatctacacgacggggagtcaggcaactatggatgaacg aaatagacagatcgctgagataggtgcctcactgattaagcattggtaactgtcagaccaagtttactcatatatactttag attgatttaaaacttcatttttaatttaaaaggatctaggtgaagatcctttttgataatctcatgaccaaaatcccttaacgtga gttttcgttccactgagcgtcagaccccgtagaaaagatcaaaggatcttcttgagatcctttttttctgcgcgtaatctgct gcttgcaaacaaaaaaaccaccgctaccagcggtggtttgtttgccggatcaagagctaccaactctttttccgaaggta actggcttcagcagagcgcagataccaaatactgtccttctagtgtagccgtagttaggccaccacttcaagaactctgt agcaccgcctacatacctcgctctgctaatcctgttaccagtggctgctgccagtggcgataagtcgtgtcttaccgggtt |
| | | ggactcaagacgatagttaccggataaggcgcagcggtcgggctgaacggggggttcgtgcacacagcccagcttg gagcgaacgacctacaccgaactgagatacctacagcgtgagctatgagaaagcgccacgcttcccgaagggagaa aggcggacaggtatccggtaagcggcagggtcggaacaggagagcgcacgagggagcttccagggggaaacgc ctggtatctttatagtcctgtcgggtttcgccacctctgacttgagcgtcgatttttgtgatgctcgtcaggggggcggagc ctatggaaaaacgccagcaacgcggcctttttacggttcctggccttttgctggccttttgctcacatgttctttcctgcgtta tcccctgattctgtggataaccgtattaccgcctttgagtgagctgataccgctcgccgcagccgaacgaccgagcgca gcgagtcagtgagcgaggaagcggaagagcgcctgatgcggtattttctccttacgcatctgtgcggtatttcacaccg catatggtgcactctcagtacaatctgctctgatgccgcatagttaagccagtatacactccgctatcgctacgtgactgg gtcatggctgcgccccgacacccgccaacacccgctgacgcgccctgacgggcttgtctgctcccggcatccgctta cagacaagctgtgaccgtctccgggagctgcatgtgtcagaggttttcaccgtcatcaccgaaacgcgcgaggcagc agatcaattcgcgcgcgaaggcgaagcggcatgcataatgtgcctgtcaaatggacgaagcagggattctgcaaacc ctatgctactccgtcaagccgtcaattgtctgattcgttaccaattatgacaacttgacggctacatcattcactttttcttcac aaccggcacggaactcgctcgggctggccccggtgcattttttaaatacccgcgagaaatagagttgatcgtcaaaac caacattgcgaccgacggtggcgataggcatccgggtggtgctcaaaagcagcttcgcctggctgatacgttggtcct cgcgccagcttaagacgctaatccctaactgctggcggaaaagatgtgacagacgcgacggcgacaagcaaacatg ctgtgcgacgctggcgatatcaaaattgctgtctgccaggtgatcgctgatgtactgacaagcctcgcgtacccgattat ccatcggtggatggagcgactcgttaatcgcttccatgcgccgcagtaacaattgctcaagcagatttatcgccagcag ctccgaatagcgcccttccccttgcccggcgttaatgatttgcccaaacaggtcgctgaaatgcggctggtgcgcttcat ccgggcgaaagaaccccgtattggcaaatattgacggccagttaagccattcatgccagtaggcgcgcggacgaaag taaacccactggtgataccattcgcgagcctccggatgacgaccgtagtgatgaatctctcctggcgggaacagcaaa atatcacccggtcggcaaacaaattctcgtccctgatttttcaccaccccctgaccgcgaatggtgagattgagaatataa cctttcattcccagcggtcggtcgataaaaaaatcgagataaccgttggcctcaatcggcgttaaacccgccaccagat gggcattaaacgagtatcccggcagcaggggatcatttgcgcttcagccatacttttcatactcccgccattcagag |
| pBAD-CD19 LS202/H K136X mt | 8 | aagaaaccaattgtccatattgcatcagacattgccgtcactgcgtcttttactggctcttctcgctaaccaaaccggtaac cctgattatttgcacggagtcacactttgctatgccatagcatttttatccataagattagcggatcctacctgacgctttttat cgcaactctctactgtttctccatacccgtttttttgggctagaaataattttgtttaactttaagaaggagaatacatcaacta gtacgcaagttcacgtaaaaagggtatctagaggttgaggtgattttatgaaaaagaatatcgcatttcttcttgctagcat gttcgtttttttctattgctacaaacgcatacgctgacatccagatgacacagactacatcctccctgtctgcctctctggga gacagagtcaccatcagttgcagggcaagtcaggacattagtaaatatttaaattggtatcagcagaaaccagatggaa ctgttaaactcctgatctaccatacatcaagattacactcaggagtcccatcaaggttcagtggcagtgggtctggaaca gattattctctcaccattagcaacctggagcaagaagatattgccacttacttttgccaacagggtaatacgcttccgtaca cgttcggaggggggaccaagcttgagatcaaacgaactgtggctgcaccatctgtcttcatcttcccgccatctgatga gcagttgaaatctggaactgcctctgtcgtgtgcctgctgaataacttctatcccagagaggccaaagtacagtggaag gtggataacgccctccaatcgggtaactcccaggagagtgtcacagagcaggacagcaaggacagcacctacagcc tcagcagcaccctgacgctgagcaaagcagactacgagaaacacaaagtctacgcctgcgaagtcacccatcaggg cctgTAGtcgcccgtcacaaagagcttcaacaggggagagtgttaagctggggatcctctagaggttgaggtgattt tatgaaaaagaatatcgcatttcttcttgcatctatgttcgtttttttctattgctacaaacgcgtacgctgaggtgaaactgca ggagtcaggacctggcctggtggcgccctcacagagcctgtccgtcacatgcactgtctcaggggtctcattacccga ctatggtgtaagctggattcgccagcctccacgaaagggtctggagtggctgggagtaatatggggtagtgaaaccac atactataattcagctctcaaatccagactgaccatcatcaaggacaactccaagagccaagttttcttaaaaatgaacag tctgcaaactgatgacacagccatttactactgtgccaaacattattactacggtggtagctatgctatggactactgggg ccaaggaacctcagtcaccgtctcctcagcctccaccaagggcccatcggtcttccccctggcaccctcctccTAG agcacctctgggggcacagcggccctgggctgcctggtcaaggactacttccccgaaccggtgacggtgtcgtgga actcaggcgccctgaccagcggcgtgcacaccttcccggctgtcctacagtcctcaggactctactccctcagcagcg tggtgactgtgccctctagcagcttgggcacccagacctacatctgcaacgtgaatcacaagcccagcaacaccaagg tggacaagaaagttgagcccaaatcttgtgacaaaactcacacataataagtcgaccgatgcccttgagagccttcaac ccagtcagctccttccggtgggcgcggggcatgactatcgtcgccgcacttatgactgtcttctttatcatgcaactcgta ggacaggtgccaaacggtctccagcttggctgttttggcggatgagagaagattttcagcctgatacagattaaatcaga acgcagaagcggtctgataaaacagaatttgcctggcggcagtagcgcggtggtcccacctgaccccatgccgaact cagaagtgaaacgccgtagcgccgatggtagtgtggggtctccccatgcgagagtagggaactgccaggcatcaaat |
| | | aaaacgaaaggctcagtcgaaagactgggcctttcgttttatctgttgtttgtcggtgaacgctctcctgagtaggacaaa tccgccgggagcggatttgaacgttgcgaagcaacggcccggagggtggcgggcaggacgcccgccataaactgc caggcatcaaattaagcagaaggccatcctgacggatggcctttttgcgtttctacaaactcttttgtttatttttctaaatac attcaaatatgtatccgctcatgagacaataaccctgataaatgcttcaataatattgaaaaaggaagagtatgagtattca acatttccgtgtcgcccttattcccttttttgcggcattttgccttcctgtttttgctcacccagaaacgctggtgaaagtaaaa gatgctgaagatcagttgggtgcacgagtgggttacatcgaactggatctcaacagcggtaagatccttgagagttttcg ccccgaagaacgttttccaatgatgagcacttttaaagttctgctatgtggcgcggtattatcccgtgttgacgccgggca agagcaactcggtcgccgcatacactattctcagaatgacttggttgagtactcaccagtcacagaaaagcatcttacgg atggcatgacagtaagagaattatgcagtgctgccataaccatgagtgataacactgcggccaacttacttctgacaac gatcggaggaccgaaggagctaaccgcttttttgcacaacatgggggatcatgtaactcgccttgatcgttgggaaccg gagctgaatgaagccataccaaacgacgagcgtgacaccacgatgcctgtagcaatggcaacaacgttgcgcaaact attaactggcgaactacttactctagcttcccggcaacaattaatagactggatggaggcggataaagttgcaggacca cttctgcgctcggcccttccggctggctggtttattgctgataaatctggagccggtgagcgtgggtctcgcggtatcatt gcagcactggggccagatggtaagccctcccgtatcgtagttatctacacgacggggagtcaggcaactatggatgaa cgaaatagacagatcgctgagataggtgcctcactgattaagcattggtaactgtcagaccaagtttactcatatatacttt agattgatttaaaacttcatttttaatttaaaaggatctaggtgaagatcctttttgataatctcatgaccaaaatcccttaacgt gagttttcgttccactgagcgtcagaccccgtagaaaagatcaaaggatcttcttgagatcctttttttctgcgcgtaatctg ctgcttgcaaacaaaaaaaccaccgctaccagcggtggtttgtttgccggatcaagagctaccaactctttttccgaagg taactggcttcagcagagcgcagataccaaatactgtccttctagtgtagccgtagttaggccaccacttcaagaactct gtagcaccgcctacatacctcgctctgctaatcctgttaccagtggctgctgccagtggcgataagtcgtgtcttaccgg gttggactcaagacgatagttaccggataaggcgcagcggtcgggctgaacggggggttcgtgcacacagcccagc ttggagcgaacgacctacaccgaactgagatacctacagcgtgagctatgagaaagcgccacgcttcccgaaggga gaaaggcggacaggtatccggtaagcggcagggtcggaacaggagagcgcacgagggagcttccagggggaaa cgcctggtatctttatagtcctgtcgggtttcgccacctctgacttgagcgtcgatttttgtgatgctcgtcaggggggcgg agcctatggaaaaacgccagcaacgcggcctttttacggttcctggccttttgctggccttttgctcacatgttctttcctgc gttatcccctgattctgtggataaccgtattaccgcctttgagtgagctgataccgctcgccgcagccgaacgaccgagc gcagcgagtcagtgagcgaggaagcggaagagcgcctgatgcggtattttctccttacgcatctgtgcggtatttcaca ccgcatatggtgcactctcagtacaatctgctctgatgccgcatagttaagccagtatacactccgctatcgctacgtgac tgggtcatggctgcgccccgacacccgccaacacccgctgacgcgccctgacgggcttgtctgctcccggcatccgc ttacagacaagctgtgaccgtctccgggagctgcatgtgtcagaggttttcaccgtcatcaccgaaacgcgcgaggca gcagatcaattcgcgcgcgaaggcgaagcggcatgcataatgtgcctgtcaaatggacgaagcagggattctgcaaa ccctatgctactccgtcaagccgtcaattgtctgattcgttaccaattatgacaacttgacggctacatcattcactttttcttc acaaccggcacggaactcgctcgggctggccccggtgcattttttaaatacccgcgagaaatagagttgatcgtcaaa accaacattgcgaccgacggtggcgataggcatccgggtggtgctcaaaagcagcttcgcctggctgatacgttggtc ctcgcgccagcttaagacgctaatccctaactgctggcggaaaagatgtgacagacgcgacggcgacaagcaaacat gctgtgcgacgctggcgatatcaaaattgctgtctgccaggtgatcgctgatgtactgacaagcctcgcgtacccgatta tccatcggtggatggagcgactcgttaatcgcttccatgcgccgcagtaacaattgctcaagcagatttatcgccagcag ctccgaatagcgcccttccccttgcccggcgttaatgatttgcccaaacaggtcgctgaaatgcggctggtgcgcttcat ccgggcgaaagaaccccgtattggcaaatattgacggccagttaagccattcatgccagtaggcgcgcggacgaaag taaacccactggtgataccattcgcgagcctccggatgacgaccgtagtgatgaatctctcctggcgggaacagcaaa atatcacccggtcggcaaacaaattctcgtccctgatttttcaccaccccctgaccgcgaatggtgagattgagaatataa cctttcattcccagcggtcggtcgataaaaaaatcgagataaccgttggcctcaatcggcgttaaacccgccaccagat gggcattaaacgagtatcccggcagcaggggatcattttgcgcttcagccatacttttcatactcccgccattcagag |
| **LV-EF1a-CD19(F MC63)-BBZ** | 9 | caggtggcacttttcggggaaatgtgcgcggaacccctatttgtttatttttctaaatacattcaaatatgtatccgctcatga gacaataaccctgataaatgcttcaataatattgaaaaaggaagagtatgagtattcaacatttccgtgtcgcccttattcc cttttttgcggcattttgccttcctgtttttgctcacccagaaacgctggtgaaagtaaaagatgctgaagatcagttgggtg cacgagtgggttacatcgaactggatctcaacagcggtaagatccttgagagttttcgccccgaagaacgttttccaatg atgagcacttttaaagttctgctatgtggcgcggtattatcccgtattgacgccgggcaagagcaactcggtcgccgcat acactattctcagaatgacttggttgagtactcaccagtcacagaaaagcatcttacggatggcatgacagtaagagaat tatgcagtgctgccataaccatgagtgataacactgcggccaacttacttctgacaacgatcggaggaccgaaggagc |
| | | taaccgctttttgcacaacatgggggatcatgtaactcgccttgatcgttgggaaccggagctgaatgaagccatacca aacgacgagcgtgacaccacgatgcctgtagcaatggcaacaacgttgcgcaaactattaactggcgaactacttact ctagcttcccggcaacaattaatagactggatggaggcggataaagttgcaggaccacttctgcgctcggcccttccgg ctggctggtttattgctgataaatctggagccggtgagcgtgggtctcgcggtatcattgcagcactggggccagatggt aagccctcccgtatcgtagttatctacacgacggggagtcaggcaactatggatgaacgaaatagacagatcgctgag ataggtgcctcactgattaagcattggtaactgtcagaccaagtttactcatatatactttagattgatttaaaacttcattttta atttaaaaggatctaggtgaagatcctttttgataatctcatgaccaaaatcccttaacgtgagttttcgttccactgagcgtc agaccccgtagaaaagatcaaaggatcttcttgagatcctttttttctgcgcgtaatctgctgcttgcaaacaaaaaaacc accgctaccagcggtggtttgtttgccggatcaagagctaccaactctttttccgaaggtaactggcttcagcagagcgc agataccaaatactgtccttctagtgtagccgtagttaggccaccacttcaagaactctgtagcaccgcctacatacctcg ctctgctaatcctgttaccagtggctgctgccagtggcgataagtcgtgtcttaccgggttggactcaagacgatagttac cggataaggcgcagcggtcgggctgaacggggggttcgtgcacacagcccagcttggagcgaacgacctacaccg aactgagatacctacagcgtgagctatgagaaagcgccacgcttcccgaagggagaaaggcggacaggtatccggt aagcggcagggtcggaacaggagagcgcacgagggagcttccagggggaaacgcctggtatctttatagtcctgtc gggtttcgccacctctgacttgagcgtcgatttttgtgatgctcgtcaggggggcggagcctatggaaaaacgccagca acgcggcctttttacggttcctggccttttgctggccttttgctcacatgttctttcctgcgttatcccctgattctgtggataa ccgtattaccgcctttgagtgagctgataccgctcgccgcagccgaacgaccgagcgcagcgagtcagtgagcgag gaagcggaagagcgcccaatacgcaaaccgcctctccccgcgcgttggccgattcattaatgcagctggcacgaca ggtttcccgactggaaagcgggcagtgagcgcaacgcaattaatgtgagttagctcactcattaggcaccccaggcttt acactttatgcttccggctcgtatgttgtgtggaattgtgagcggataacaatttcacacaggaaacagctatgaccatgat tacgccaagcgcgcaattaaccctcactaaagggaacaaaagctggagctgcaagcttaatgtagtcttatgcaatact cttgtagtcttgcaacatggtaacgatgagttagcaacatgccttacaaggagagaaaaagcaccgtgcatgccgattg gtggaagtaaggtggtacgatcgtgccttattaggaaggcaacagacgggtctgacatggattggacgaaccactgaa ttgccgcattgcagagatattgtatttaagtgcctagctcgatacaataaacgggtctctctggttagaccagatctgagcc tgggagctctctggctaactagggaacccactgcttaagcctcaataaagcttgccttgagtgcttcaagtagtgtgtgcc cgtctgttgtgtgactctggtaactagagatccctcagacccttttagtcagtgtggaaaatctctagcagtggcgcccga acagggacctgaaagcgaaagggaaaccagagctctctcgacgcaggactcggcttgctgaagcgcgcacggcaa gaggcgaggggcggcgactggtgagtacgccaaaaattttgactagcggaggctagaaggagagagatgggtgcg agagcgtcagtattaagcgggggagaattagatcgcgatgggaaaaaattcggttaaggccagggggaaagaaaaa atataaattaaaacatatagtatgggcaagcagggagctagaacgattcgcagttaatcctggcctgttagaaacatcag aaggctgtagacaaatactgggacagctacaaccatcccttcagacaggatcagaagaacttagatcattatataataca gtagcaaccctctattgtgtgcatcaaaggatagagataaaagacaccaaggaagctttagacaagatagaggaagag caaaacaaaagtaagaccaccgcacagcaagcggccgctgatcttcagacctggaggaggagatatgagggacaat tggagaagtgaattatataaatataaagtagtaaaaattgaaccattaggagtagcacccaccaaggcaaagagaaga gtggtgcagagagaaaaaagagcagtgggaataggagctttgttccttgggttcttgggagcagcaggaagcactatg ggcgcagcctcaatgacgctgacggtacaggccagacaattattgtctggtatagtgcagcagcagaacaatttgctga gggctattgaggcgcaacagcatctgttgcaactcacagtctggggcatcaagcagctccaggcaagaatcctggctg tggaaagatacctaaaggatcaacagctcctggggatttggggttgctctggaaaactcatttgcaccactgctgtgcctt ggaatgctagttggagtaataaatctctggaacagattggaatcacacgacctggatggagtgggacagagaaattaa caattacacaagcttaatacactccttaattgaagaatcgcaaaaccagcaagaaaagaatgaacaagaattattggaat tagataaatgggcaagtttgtggaattggtttaacataacaaattggctgtggtatataaaattattcataatgatagtagga ggcttggtaggtttaagaatagtttttgctgtactttctatagtgaatagagttaggcagggatattcaccattatcgtttcag acccacctcccaaccccgaggggacccgacaggcccgaaggaatagaagaagaaggtggagagagagacagag acagatccattcgattagtgaacggatctcgacggttaacttttaaaagaaaaggggggattggggggtacagtgcag gggaaagaatagtagacataatagcaacagacatacaaactaaagaattacaaaaacaaattacaaaaattcaaaattt atcgagctttgcaaagatggataaagttttaaacagagaggaatctttgcagctaatggaccttctaggtcttgaaaggag tgcctCGTGAGGCTCCGGTGCCCGTCAGTGGGCAGAGCGCACATCGCCCA CAGTCCCCGAGAAGTTGGGGGGAGGGGTCGGCAATTGAACCGGTGCCT AGAGAAGGTGGCGCGGGGTAAACTGGGAAAGTGATGTCGTGTACTGGC TCCGCCTTTTTCCCGAGGGTGGGGGAGAACCGTATATAAGTGCAGTAGT |
| | | CGCCGTGAACGTTCTTTTCGCAACGGGTTTGCCGCCAGAACACAGGTA AGTGCCGTGTGTGGTTCCCGCGGGCCTGGCCTCTTTACGGGTTATGGCC CTTGCGTGCCTTGAATTACTTCCACCTGGCTGCAGTACGTGATTCTTGAT CCCGAGCTTCGGGTTGGAAGTGGGTGGGAGAGTTCGAGGCCTTGCGCTT AAGGAGCCCCTTCGCCTCGTGCTTGAGTTGAGGCCTGGCCTGGGCGCTG GGGCCGCCGCGTGCGAATCTGGTGGCACCTTCGCGCCTGTCTCGCTGCT TTCGATAAGTCTCTAGCCATTTAAAATTTTTGATGACCTGCTGCGACGCT TTTTTTCTGGCAAGATAGTCTTGTAAATGCGGGCCAAGATCTGCACACT GGTATTTCGGTTTTTGGGGCCGCGGGCGGCGACGGGGCCCGTGCGTCCC AGCGCACATGTTCGGCGAGGCGGGGCCTGCGAGCGCGGCCACCGAGAA TCGGACGGGGGTAGTCTCAAGCTGGCCGGCCTGCTCTGGTGCCTGGCCT CGCGCCGCCGTGTATCGCCCCGCCCTGGGCGGCAAGGCTGGCCCGGTC GGCACCAGTTGCGTGAGCGGAAAGATGGCCGCTTCCCGGCCCTGCTGC AGGGAGCTCAAAATGGAGGACGCGGCGCTCGGGAGAGCGGGCGGGTG AGTCACCCACACAAAGGAAAAGGGCCTTTCCGTCCTCAGCCGTCGCTTC ATGTGACTCCACGGAGTACCGGGCGCCGTCCAGGCACCTCGATTAGTTC TCGAGCTTTTGGAGTACGTCGTCTTTAGGTTGGGGGGAGGGGTTTTATG CGATGGAGTTTCCCCACACTGAGTGGGTGGAGACTGAAGTTAGGCCAG CTTGGCACTTGATGTAATTCTCCTTGGAATTTGCCCTTTTTGAGTTTGGA TCTTGGTTCATTCTCAAGCCTCAGACAGTGGTTCAAAGTTTTTTTCTTCC ATTTCAGGTGTCGTGAggaattcggtaccgcggccgcccggggatccatggccttaccagtgaccgcc ttgctcctgccgctggccttgctgctccacgccgccaggccggacatccagatgacacagactacatcctccctgtctg cctctctgggagacagagtcaccatcagttgcagggcaagtcaggacattagtaaatatttaaattggtatcagcagaaa ccagatggaactgttaaactcctgatctaccatacatcaagattacactcaggagtcccatcaaggttcagtggcagtgg gtctggaacagattattctctcaccattagcaacctggagcaagaagatattgccacttacttttgccaacagggtaatac gcttccgtacacgttcggaggggggaccaagctggagatcacaggtggcggtggctcgggcggtggtgggtcgggt ggcggcggatctgaggtgaaactgcaggagtcaggacctggcctggtggcgccctcacagagcctgtccgtcacat gcactgtctcaggggtctcattacccgactatggtgtaagctggattcgccagcctccacgaaagggtctggagtggct gggagtaatatggggtagtgaaaccacatactataattcagctctcaaatccagactgaccatcatcaaggacaactcc aagagccaagttttcttaaaaatgaacagtctgcaaactgatgacacagccatttactactgtgccaaacattattactacg gtggtagctatgctatggactactggggccaaggaacctcagtcaccgtctcctcaaccacgacgccagcgccgcga ccaccaacaccggcgcccaccatcgcgtcgcagcccctgtccctgcgcccagaggcgtgccggccagcggcggg gggcgcagtgcacacgagggggctggacttcgcctgtgatatctacatctgggcgcccttggccgggacttgtgggg tccttctcctgtcactggttatcaccctttactgcaaacggggcagaaagaaactcctgtatatattcaaacaaccatttatg agaccagtacaaactactcaagaggaagatggctgtagctgccgatttccagaagaagaagaaggaggatgtgaact gagagtgaagttcagcaggagcgcagacgcccccgcgtacaagcagggccagaaccagctctataacgagctcaat ctaggacgaagagaggagtacgatgttttggacaagagacgtggccgggaccctgagatggggggaaagccgaga aggaagaaccctcaggaaggcctgtacaatgaactgcagaaagataagatggcggaggcctacagtgagattggga tgaaaggcgagcgccggaggggcaaggggcacgatggcctttaccagggtctcagtacagccaccaaggacacct acgacgcccttcacatgcaggccctgccccctcgctaagtcgacaatcaacctctggattacaaaatttgtgaaagattg actggtattcttaactatgttgctccttttacgctatgtggatacgctgctttaatgcctttgtatcatgctattgcttcccgtatg gctttcattttctcctccttgtataaatcctggttgctgtctctttatgaggagttgtggcccgttgtcaggcaacgtggcgtg gtgtgcactgtgtttgctgacgcaacccccactggttggggcattgccaccacctgtcagctcctttccgggactttcgct ttccccctccctattgccacggcggaactcatcgccgcctgccttgcccgctgctggacaggggctcggctgttgggc actgacaattccgtggtgttgtcggggaagctgacgtcctttccatggctgctcgcctgtgttgccacctggattctgcgc gggacgtccttctgctacgtcccttcggccctcaatccagcggaccttccttcccgcggcctgctgccggctctgcggc ctcttccgcgtcttcgccttcgccctcagacgagtcggatctccctttgggccgcctccccgcctggaattcgagctcgg tacctttaagaccaatgacttacaaggcagctgtagatcttagccactttttaaaagaaaaggggggactggaagggcta attcactcccaacgaagacaagatctgctttttgcttgtactgggtctctctggttagaccagatctgagcctgggagctct ctggctaactagggaacccactgcttaagcctcaataaagcttgccttgagtgcttcaagtagtgtgtgcccgtctgttgt |
| | | gtgactctggtaactagagatccctcagacccttttagtcagtgtggaaaatctctagcagtagtagttcatgtcatcttatt attcagtatttataacttgcaaagaaatgaatatcagagagtgagaggaacttgtttattgcagcttataatggttacaaata aagcaatagcatcacaaatttcacaaataaagcatttttttcactgcattctagttgtggtttgtccaaactcatcaatgtatct tatcatgtctggctctagctatcccgcccctaactccgcccagttccgcccattctccgccccatggctgactaattttttttta tttatgcagaggccgaggccgcctcggcctctgagctattccagaagtagtgaggaggcttttttggaggcctaggcttt tgcgtcgagacgtacccaattcgccctatagtgagtcgtattacgcgcgctcactggccgtcgttttacaacgtcgtgact gggaaaaccctggcgttacccaacttaatcgccttgcagcacatccccctttcgccagctggcgtaatagcgaagagg cccgcaccgatcgcccttcccaacagttgcgcagcctgaatggcgaatggcgcgacgcgccctgtagcggcgcatta agcgcggcgggtgtggtggttacgcgcagcgtgaccgctacacttgccagcgccctagcgcccgctcctttcgctttct tcccttcctttctcgccacgttcgccggctttccccgtcaagctctaaatcgggggctccctttagggttccgatttagtgct ttacggcacctcgaccccaaaaaacttgattagggtgatggttcacgtagtgggccatcgccctgatagacggtttttcg ccctttgacgttggagtccacgttctttaatagtggactcttgttccaaactggaacaacactcaaccctatctcggtctatt cttttgatttataagggattttgccgatttcggcctattggttaaaaaatgagctgatttaacaaaaatttaacgcgaatttaa caaaatattaacgtttacaatttcc |
| For Table 12: **BOLD, UNDERLINE, UPPERCASE** = Amber mutant site | | |

| **Table 15. CAR-EC switch target interacting domains (antibodies) -Amino Acid Sequence** | | |
|---|---|---|
| **NAME** | **SEQ ID** | **SEQUENCE** |
| Light chain of wildtype anti-CS1 antibody | 10 | DIQMTQSPSSLSASVGDRVTITCKASQDVGIAVAWYQQKPGK VPKLLIYWASTRHTGVPDRFSGSGSGTDFTLTISSLQPEDVATY YCQQYSSYPYTFGQGTKLEIK |
| Heavy chain of wildtype anti-CS1 antibody | 11 | EVQLVESGGGLVQPGGSLRLSCAASGFDFSRYWMSWVRQAP GKGLEWIGEINPDSSTINYAPSLKDKFIISRDNAKNSLYLQMNS LRAEDTAVYYCARPDGNYWYFDVWGQGTLVTVSS |
| Light chain of anti-EGFRvIII antibody (Hu806 VL) | 12 | DIQMTQSPSSMSVSVGDRVTITCHSSQDINSNIGWLQQKPGKSF KGLIYHGTNLDDGVPSRFSGSGSGTDYTLTISSLQPEDFATYYC VQYAQFPWTFGGGTKLEIK |
| Heavy chain of anti-EGFRvIII antibody (Hu806 HL) | 13 | QLQESGPGLVKPSQTLSLTCTVSGYSISSDFAWNWIRQPPGKG LEWMGYISYSGNTRYQPSLKSRITISRDTSKNQFFLKLNSVTAA DTATYYCVTAGRGFPYWGQGTLVTVSS |
| Light chain of anti-BCMA antibody (BCMA98) | 14 | DIQMTQSPSSLSASVGDRVTITCRANQGISNNLNWYQQKPGKA PKPLIYYTSNLQSGVPSRFSGSGSGTDYTLTISSLQPEDFATYYC QQFTSLPYTFGQGTKLEIK |
| Heavy chain of anti-BCMA antibody (BCMA98) | 15 | EVQLVESGGGLVQPGGSLRLSCAASGFTFSNFDMAWVRQAPG KGLVWVSSITTGGGDTYYADSVKGRFTISRDNAKSTLYLQMD SLRSEDTAVYYCVRHGYYDGYHLFDYWGQGTLVTVSS |
| anti-CD19-Fab Light Chain | 16 | DIQMTQTTSSLSASLGDRVTISCRASQDISKYLNWYQQKPDGT VKLLIYHTSRLHSGVPSRFSGSGSGTDYSLTISNLEQEDIATYFC QQGNTLPYTFGGGTKLEIKRTVAAPSVFIFPPSDEQLKSGTASV VCLLNNFYPREAKVQWKVDNALQSGNSQESVTEQDSKDSTYS LSSTLTLSKADYEKHKVYACEVTHQGLSSPVTKSFNRGEC |
| anti-CD 19-Fab Heavy Chain | 17 | EVKLQESGPGLVAPSQSLSVTCTVSGVSLPDYGVSWIRQPPRK GLEWLGVIWGSETTYYNSALKSRLTIIKDNSKSQVFLKMNSLQ TDDTAIYYCAKHYYYGGSYAMDYWGQGTSVTVSSASTKGPS VFPLAPSSKSTSGGTAALGCLVKDYFPEPVTVSWNSGALTSGV |
| | | HTFPAVLQSSGLYSLSSVVTVPSSSLGTQTYICNVNHKPSNTKV DKKVEPKSCDKTHT |

| **Table 16. CAR-EC small molecule switch TIDs -Amino Acid Sequence** | | |
|---|---|---|
| **NAME** | **SEQ ID** | **SEQUENCE** |
| SS-14 (somatostatin analog) | 18 | Ala-Gly-cyclo(Cys-Lys-Asn-Phe-Phe-Trp-Lys-Thr-Phe-Thr-Ser-Cys) |
| OC (somatostatin analog) | 19 | D-Phe1-cyclo(Cys2-Phe3-D-Trp4-Lys5-Thr6-Cys7)Thr(ol)8 |
| TOC (somatostatin analog) | 20 | D-Phe1-cyclo(Cys2-Tyr3-D-Trp4-Lys5-Thr6-Cys7)Thr(ol)8 |
| TATE (somatostatin analog) | 21 | D-Phe1-cyclo(Cys2-Tyr3-D-Trp4-Lys5-Thr6-Cys7)Thr8 |
| NOC (somatostatin analog) | 22 | D-Phe1-cyclo(Cys2-1-NaI3-D-Trp4-Lys5-Thr6-Cys7)Thr(ol)8 |
| NOC-ATE (somatostatin analog) | 23 | D-Phe1-cyclo(Cys2-1-NaI3-D-Trp4-Lys5-Thr6-Cys7)Thr8 |
| BOC (somatostatin analog) | 24 | D-Phe1-cyclo(Cys2-BzThi3-D-Trp4-Lys5-Thr6-Cys7)Thr(ol)8 |
| BOC-ATE (somatostatin analog) | 25 | D-Phe1-cyclo(Cys2-BzThi3-D-Trp4-Lys5-Thr6-Cys7)Thr8 |
| KE108 (somatostatin analog) | 26 | Tyr-cyclo(DAB-Arg-Phe-Phe-D-Trp-Lys-Thr-Phe) |
| LM3 (somatostatin analog) | 27 | p-Cl-Phe-cyclo(D-Cys-Tyr-D-Aph(Cbm)-LysThr-Cys)D-Tyr-NH2 |
| BN (bombesin analog) | 28 | pGlu1-Gln2-Arg3-Leu4-Gly5-Asn6-Gln7-Trp8-Ala9-Val10-Gly11-His12-Leu13-Met14-NH2 |
| RP527 (bombesin analog) | 29 | N3S-Gly-5-Ava-[Gln7-Trp8-Ala9-Val10-Gly11-His12-Leu13-Met14-NH2] |
| Demobesin 1 (bombesin analog) | 30 | N40-1-bzlg0 [D-Phe6-Gln7-Trp8-Ala9-Val10-Gly11-His12-Leu-NHEt13] |
| Demobesin 4 (bombesin analog) | 31 | N4-[Pro1-Gln2-Arg3-Tyr4-Gly5-Asn6-Gln7-Trp8-Ala9-Val10-Gly11-His12-Leu13-Nle14-NH2] |
| BBS-38 (bombesin analog) | 32 | (NαHis)Ac-β-Ala-β-Ala-[Gln7-Trp8-Ala9-Val10-Gly11-His12-Cha13-Nle14-NH2] |
| BAY 86-4367 (bombesin analog) | 33 | 3-cyano-4-trimethylammonium-benzoyl-Ala(SO3H)-Ala(SO3H)-Ava-[Gln7-Trp8-Ala9-Val10-NMeGly11-His12-Sta13-Leu14-NH2] |
| MG (minigastrin analog) | 34 | Leu1-Glu2-Glu3-Glu4-Glu5-Glu6-Ala7-Tyr8-Gly9-Trp10-Met11-Asp12-Phe13-NH2 |
| MG0 (minigastrin analog) | 35 | D-Glu1-Glu2-Glu3-Glu4-Glu5-Glu6-Ala7-Tyr8-Gly9-Trp10-Met11-Asp12-Phe13-NH2 |
| MG11 (minigastrin analog) | 36 | D-Glu-Ala-Tyr-Gly-Trp-Met-Asp-Phe-NH2 |
| H2-Met (minigastrin analog) | 37 | His-His-Glu-Ala-Tyr-Gly-Trp-Met-Asp-Phe-NH2 |
| H2-Nle (minigastrin analog) | 38 | His-His-Glu-Ala-Tyr-Gly-Trp-Nle-Asp-Phe-NH2 |
| Demogastrin (minigastrin analog) | 39 | N4-D-Glu-(Glu)5-Ala-Tyr-Gly-Trp-Met-Asp-Phe-NH2 |
| Cyclo-MG1 (minigastrin analog) | 40 | c(γ-D-Glu-Ala-Tyr-D-Lys)-Trp-Met-Asp-Phe-NH2 |
| MGD5 (minigastrin analog) | 41 | Gly-Ser-Cys(succinimidopropionyl-Glu-Ala-Tyr-Gly-Trp-Nle-Asp-Phe-NH2)-Glu-Ala-Tyr-Gly-Trp-Nle-Asp-Phe-NH2 |
| Buserelin (GnRH analog) | 42 | pGlu1-His2-Trp3-Ser4-Tyr5-D-Ser(tBu) 6-Leu7-Arg8-Pro9-NHC2H5 |
| Goserelin (GnRH analog) | 43 | pGlu1-His2-Trp3-Ser4-Tyr5-D-Ser(tBu) 6-Leu7-Arg8-Pro9-AzGly10-NH2 |
| Leuprolide (GnRH analog) | 44 | pGlu1-His2-Trp3-Ser4-Tyr5-D-Leu6-Leu7-Arg8-Pro9-NHC2H5 |
| Nafarelin (GnRH analog) | 45 | pGlu1-His2-Trp3-Ser4-Tyr5-D-Nal (2) 6-Leu7-Arg8-Pro9-NHC2H5 |
| Triptorelin (GnRH analog) | 46 | pGlu1-His2-Trp3-Ser4-Tyr5-D-Trp6-Leu7-Arg8-Pro9-Gly10-NH2 |
| Abarelix (GnRH analog) | 47 | Ac-D-Ala1-D-Cpa2-D-Ala3-Ser4-Tyr5-D-Asp6-Leu7-Ilys8-Pro9-D-Ala10-NH2 |
| Acyline (GnRH analog) | 48 | Ac-D-Nal1-D-Cpa2-D-Pal3-Ser4-Aph(Ac)5-D-Aph(Ac)6-Leu7-Ilys8-Pro9-D-Ala10-NH2 |
| Antarelix (GnRH analog) | 49 | Ac-D-Nal1-D-Cpa2-D-Pal3-Ser4-Tyr5-D-Hci6-Leu7-Ilys8-Pro9-D-Ala10-NH2 |
| Antide (GnRH analog) | 50 | Ac-D-Nal1-D-Cpa2-D-Pal3-Ser4-Lys(Nic)5-D-Lys(Nic)6-Leu7-Ilys8-Pro9-D-Ala10-NH2 |
| Azaline B (GnRH analog) | 51 | Ac-D-Nal1-D-Cpa2-D-Pal3-Ser4-Aph (Atz) 5-D-Aph(Atz)6-Leu7-Ilys8-Pro9-D-Ala10-NH2 |
| Cetrorelix (GnRH analog) | 52 | Ac-D-Nal1-D-Cpa2-D-Pal3-Ser4-Tyr5-D-Cit6-Leu7-Arg8-Pro9-D-Ala10-NH2 |
| Degarelix (GnRH analog) | 53 | Ac-D-Nal1-D-Cpa2-D-Pal3-Ser4-Aph(L-hydroorotyl)5-D-Aph(carbamoyl)6-Leu7-Ilys8-Pro9-D-Ala10-NH2 |
| Ganirelix (GnRH analog) | 54 | Ac-D-Nal1-D-Cpa2-D-Pa13-Ser4-Tyr5-D-hArg (Et2)6-Leu7-hArg (Et2)8-Pro9-D-Ala10-NH2 |
| Ozarelix (GnRH analog) | 55 | Ac-D-Nal1-D-Cpa2-D-Pal3-Ser4-N-MeTyr5-D-hCit6-Nle7-Arg8-Pro9-D-Ala10-NH2 |
| LHRH | 56 | Glp-His-Trp-Ser-Tyr-Lys-Leu-Arg-Pro-Gly-NH2 |

| **Table 17. CAR-EC Switch Linker -Amino Acid Sequence** | | |
|---|---|---|
| **NAME** | **SEQ ID** | **SEQUENCE** |
| Linker | 57 | (GGGGS)n, wherein n≥1 |
| 25 amino acid linker | 58 | SSADDAKKDAAKKDDAKKDDAKKDG |

## Claims

1. A switch for activating a chimeric antigen receptor-effector cell (CAR-EC), the switch comprising:
a. a chimeric antigen receptor-interacting domain (CAR-ID) that interacts with a chimeric antigen receptor on the CAR-EC; and
b. a target interacting domain (TID) comprising an unnatural amino acid, wherein the TID interacts with a surface molecule on a target cell, and wherein the CAR-ID is attached to the TID via the unnatural amino acid.

2. The switch of claim 1,
a. further comprising a linker,
(i) wherein the linker attaches the CAR-ID to the TID via the unnatural amino acid,
(ii) wherein the linker site-specifically attaches the CAR-ID to the unnatural amino acid of the TID, or
(iii) wherein the linker preferably comprises an aminooxy group, azide group cyclooctyne group, or a combination thereof at one or more termini; or
b. wherein the CAR-ID comprises a small molecule, wherein the small molecule is
(i) a hapten,
(ii) fluorescein isothiocyanate (FITC), wherein the TID is conjugated to an isothiocyanate of FITC or further comprises a linker that is conjugated to an isothiocyanate of FITC, or
(iii) biotin.

3. The switch of claim 1,
a. wherein the TID is based on or derived from at least a portion of an antibody; or
b. wherein the TID is based on or derived from at least a portion of a single chain variable fragment (scFv); or
c. wherein the TID is based on or derived from at least a portion of an anti-CD 19 antibody; or
d. wherein the TID is based on or derived from at least a portion of a single chain variable domain (scFv) of an anti-CD 19 antibody; or
e. wherein the TID is based on or derived from at least a portion of an antibody selected from the group consisting of anti-CD20, anti-CD22, anti-CD33, anti-BMSA, anti-CEA, anti-CLL1, anti-CS1, anti-EGFR, and anti-Her2; or
f. wherein the TID is based on or derived from at least a portion of a single chain variable domain (scFv) of an antibody selected from the group consisting of anti-CD20, anti-CD22, anti-CD33, anti-BMSA, anti-CEA, anti-CLL1, anti-CS1, anti-EGFR, and anti-Her2 and wherein the unnatural amino acid is inserted in the portion of the antibody from which the TID is based or derived, or replaces an amino acid of the antibody from which the TID is based or derived.

4. A composition comprising a plurality of switches for activating a chimeric antigen receptor-effector cell (CAR-EC), wherein a switch of the plurality of switches comprises (a) a chimeric antigen receptor-interacting domain (CAR-ID) that interacts with a chimeric antigen receptor on the CAR-EC; and (b) a target interacting domain (TID) comprising an unnatural amino acid, wherein the TID interacts with a surface molecule on a target cell, wherein the CAR-ID is attached to the TID via the unnatural amino acid, and wherein at least about 60% of the switches are structurally homologous.

5. The composition of claim 4,
a. wherein the CAR-ID comprises a small molecule; or
b. wherein the CAR-ID comprises fluorescein isothiocyanate (FITC); or
c. wherein the TID comprises a small molecule selected from the group consisting of 2-[3-(1,3-dicarboxypropyl)ureido] pentanedioic acid, folate, or a derivative thereof; or
d. wherein the switch of the plurality of switches further comprises a linker, wherein the linker attaches the CAR-ID to the TID.

6. The composition of claim 4,
a. wherein the CAR-ID is attached to the same predetermined site in the TID in at least about 80% of the switches.

7. A first chimeric antigen receptor-effector cell (CAR-EC) switch for use in a method of treating a disease, the switch comprising:
i. a first chimeric antigen receptor-interacting domain (CAR-ID) that interacts with a chimeric antigen receptor on the CAR-EC; and
ii. a first target interacting domain (TID) comprising an unnatural amino acid, wherein the TID interacts with a first surface molecule on a target cell and wherein the TID comprises a CAR-ID attached site-specifically to the TID via the unnatural amino acid,
wherein the method comprises administering the first CAR-EC switch and further administering a first chimeric antigen receptor-effector cell comprising a chimeric antigen receptor that binds to the first CAR-ID of the first CAR-EC switch to a subject.

8. The switch for use of claim 7, wherein a linker site-specifically attaches the first CAR-ID to the unnatural amino acid of the first TID.

9. The switch for use of claim 7, wherein the first CAR-ID comprises a small molecule.

10. The switch for use of claim 9, wherein the small molecule is selected from (i) a hapten and (ii) a hapten selected from fluorescein isothiocyanate (FITC) or biotin.

11. The switch for use of claim 7, wherein the first TID comprises at least an antigen-binding fragment of an antibody or at least an antigen-binding fragment of a single chain variable fragment (scFv).

12. The switch for use of claim 7, wherein the first TID is comprises at least an antigen binding fragment of an anti-CD19 antibody or at least an antigen-binding fragment of a single chain variable domain (scFv) of an anti-CD 19 antibody.

13. The switch for use of claim 7, wherein the first TID comprises at least an antigen binding fragment of an antibody selected from the group consisting of anti-CD20, anti-CD22, anti-CD33, anti-BMSA, anti-CEA, anti-CLL1, anti-CS1, anti-EGFR, and anti-Her2 or at least an antigen binding fragment of a single chain variable domain (scFv) of an antibody selected from the group consisting of anti-CD20, anti-CD22, anti-CD33, anti-BMSA, anti-CEA, anti-CLL1, anti-CS1, anti-EGFR, and anti-Her2.

14. The switch for use of claim 7, further comprising administering one or more second CAR-EC switch to the subject, each second CAR-EC switch comprising:
i. a second CAR-ID that interacts with a chimeric antigen receptor on an effector cell; and
ii. a second TID comprising an unnatural amino acid, wherein the second TID comprises the second CAR-ID attached site-specifically via the unnatural amino acid; and wherein the second TID interacts with a surface molecule on a target cell;
wherein the second CAR-ID comprised on each second CAR-EC switch is optionally
(i) the same as the first CAR-ID comprised on the first CAR-EC switch or
(ii) a second CAR-ID that differs from the first CAR-ID comprised on the first CAR-EC switch;
provided that if the second CAR-ID that differs from the first CAR-ID, the method further comprises administering a second CAR-EC comprising a chimeric antigen receptor that interacts with the second CAR-ID of the second CAR-EC switch.

15. The switch for use of claim 14, wherein
(i) the first TID comprises an anti-CD20 antibody or an antigen binding portion thereof and the second TID comprises an anti-CD 19 antibody or an antigen binding fragment thereof; or
(ii) the first TID comprises an anti-CD 19 antibody or an antigen binding fragment thereof and the second TID comprises an anti-CD20 antibody or an antigen binding fragment thereof.

16. The switch for use of any one of claim 7-15, wherein the disease is a cancer.

## Patentansprüche

1. Schalter zum Aktivieren einer chimären Antigenrezeptor-Effektorzelle (CAR-EC), wobei der Schalter umfasst:
a. eine chimäre Antigenrezeptor-interagierende Domäne (CAR-ID), die mit einem chimären Antigenrezeptor an der CAR-EC interagiert; und
b. eine Ziel-interagierende Domäne (TID), umfassend eine unnatürliche Aminosäure, wobei die TID mit einem Oberflächenmolekül an einer Zielzelle interagiert und wobei die CAR-ID über die unnatürliche Aminosäure an der TID angehängt ist.

2. Schalter nach Anspruch 1,
a. weiterhin umfassend einen Linker,
(i) wobei der Linker die CAR-ID über die unnatürliche Aminosäure an die TID anhängt,
(ii) wobei der Linker die CAR-ID ortsspezifisch an die unnatürliche Aminosäure der TID anhängt, oder
(iii) wobei der Linker vorzugsweise eine Aminooxygruppe, eine Azidgruppe, Cyclooctyngruppe oder eine Kombination davon an einem oder mehreren Termini umfasst; oder
b. wobei die CAR-ID ein kleines Molekül umfasst, wobei das kleine Molekül
(i) ein Hapten ist,
(ii) Fluoresceinisothiocynat (FITC) ist, wobei die TID an ein Isothiocyanat von FITC konjugiert ist oder weiterhin einen Linker umfasst, der an ein Isothiocyanat von FITC konjugiert ist, oder
(iii) Biotin ist.

3. Schalter nach Anspruch 1,
a. wobei die TID auf wenigstens einem Teil eines Antikörpers basiert oder von ihm stammt, oder
b. wobei die TID auf wenigstens einem Teil eines einkettigen variablen Fragments (scFv) basiert oder von ihm stammt, oder
c. wobei die TID auf wenigstens einem Teil eines Anti-CD19-Antikörpers basiert oder von ihm stammt, oder
d. wobei die TID auf wenigstens einem Teil einer einkettigen variablen Domäne (scFv) eines Anti-CD19-Antikörpers basiert oder von ihm stammt; oder
e. wobei die TID auf wenigstens einem Teil eines Antikörpers basiert oder von ihm stammt, ausgewählt aus der Gruppe, bestehend aus Anti-CD20, Anti-CD22, Anti-CD33, Anti-BMSA, Anti-CEA, Anti-CLL1, Anti-CS1, Anti-EGFR und Anti-Her2; oder
f. wobei die TID auf wenigstens einem Teil einer einkettigen variablen Domäne (scFv) eines Antikörpers basiert oder von ihm stammt, ausgewählt aus der Gruppe, bestehend aus Anti-CD20, Anti-CD22, Anti-CD33, Anti-BMSA, Anti-CEA, Anti-CLL1, Anti-CS1, Anti-EGFR und Anti-Her2 und wobei die unnatürliche Aminosäure in den Teil des Antikörpers eingefügt ist, auf dem die TID basiert oder von dem sie stammt oder eine Aminosäure des Antikörpers ersetzt, auf der die TID basiert oder von der sie stammt.

4. Zusammensetzung, umfassend eine Vielzahl von Schaltern zum Aktivieren einer chimären Antigenrezeptor-Effektorzelle (CAR-EC), wobei ein Schalter der Vielzahl von Schaltern Folgendes umfasst: (a) eine chimäre Antigenrezeptor-interagierende Domäne (CAR-ID), die mit einem chimären Antigenrezeptor an der CAR-EC interagiert; und (b) eine Ziel-interagierende Domäne (TID), umfassend eine unnatürliche Aminosäure, wobei die TID mit einem Oberflächenmolekül an einer Zielzelle interagiert, wobei die CAR-ID über die unnatürliche Aminosäure an der TID angehängt ist, und wobei wenigstens 60% der Schalter strukturhomolog sind.

5. Zusammensetzung nach Anspruch 4,
a. wobei die CAR-ID ein kleines Molekül umfasst, oder
b. wobei die CAR-ID Fluoresceinisothiocyanat (FITC) umfasst, oder
c. wobei die TID ein kleines Molekül umfasst, ausgewählt aus der Gruppe, bestehend aus 2-[3-(1,3-Dicarboxypropyl)Ureido]-Pentandisäure, Folat oder einem Derivat davon, oder
d. wobei der Schalter der Vielzahl von Schaltern weiterhin einen Linker umfasst, wobei der Linker die CAR-ID an die TID anhängt.

6. Zusammensetzung nach Anspruch 4,
a. wobei die CAR-ID bei wenigstens etwa 80% der Schalter an die gleiche vorbestimmte Stelle in der TID angehängt ist.

7. Erster chimärer Antigenrezeptor-Effektorzellen (CAR-EC)-Schalter zur Verwendung in einem Verfahren zur Behandlung einer Krankheit, wobei der Schalter umfasst:
i. eine erste chimäre Antigenrezeptor-interagierende Domäne (CAR-ID), die mit einem chimären Antigenrezeptor an der CAR-EC interagiert; und
ii. eine erste Ziel-interagierende Domäne (TID), umfassend eine unnatürliche Aminosäure, wobei die TID mit einem ersten Oberflächenmolekül an einer Zielzelle interagiert und wobei die TID eine CAR-ID umfasst, die über die unnatürliche Aminosäure ortsspezifisch an der TID angehängt ist,
wobei das Verfahren das Verabreichen des ersten CAR-EC-Schalters und weiterhin das Verabreichen einer ersten chimären Antigenrezeptor-Effektorzelle umfasst, umfassend einen chimären Antigenrezeptor, der an die erste CAR-ID des ersten CAR-EC-Schalters bei einem Patienten bindet.

8. Schalter zur Verwendung nach Anspruch 7, wobei ein Linker die erste CAR-ID ortsspezifisch an die unnatürliche Aminosäure der ersten TID bindet.

9. Schalter zur Verwendung nach Anspruch 7, wobei die erste CAR-ID ein kleines Molekül umfasst.

10. Schalter zur Verwendung nach Anspruch 9, wobei das kleine Molekül ausgewählt ist aus (i) einem Hapten und (ii) einem Hapten, ausgewählt aus Fluoresceinisothiocyanat (FITC) oder Biotin.

11. Schalter zur Verwendung nach Anspruch 7, wobei die erste TID wenigstens ein antigenbindendes Fragment eines Antikörpers oder wenigstens ein antigenbindendes Fragment eines einkettigen variablen Fragments (scFv) umfasst.

12. Schalter zur Verwendung nach Anspruch 7, wobei die erste TID wenigstens ein antigenbindendes Fragment eines Anti-CD19-Antikörpers oder wenigstens ein antigenbindendes Fragment einer einkettigen variablen Domäne (scFv) eines Anti-CD19-Antikörpers umfasst.

13. Schalter zur Verwendung nach Anspruch 7, wobei die erste TID wenigstens ein antigenbindendes Fragment eines Antikörpers umfasst, ausgewählt aus der Gruppe, bestehend aus Anti-CD20, Anti-CD22, Anti-CD33, Anti-BMSA, Anti-CEA, Anti-CLL1, Anti-CS1, Anti-EGFR und Anti-Her2 oder wenigstens einem antigenbindenden Fragment einer einkettigen variablen Domäne (scFv) eines Antikörpers, ausgewählt aus der Gruppe, bestehend aus Anti-CD20, Anti-CD22, Anti-CD33, Anti-BMSA, Anti-CEA, Anti-CLL1, Anti-CS1, Anti-EGFR und Anti-Her2.

14. Schalter zur Verwendung nach Anspruch 7, weiterhin umfassend das Verabreichen eines oder mehrerer zweiter CAR-EC-Schalter an den Patienten, wobei jeder zweite CAR-EC-Schalter umfasst:
i. eine zweite CAR-ID, die mit einem chimären Antigenrezeptor an einer Effektorzelle interagiert, und
ii. eine zweite TID umfassend eine unnatürliche Aminosäure, wobei die zweite TID die zweite CAR-ID umfasst, die über die unnatürliche Aminosäure ortsspezifisch angehängt ist, und wobei die zweite TID mit einem Oberflächenmolekül an einer Zielzelle interagiert,
wobei die zweite CAR-ID an jedem zweiten CAR-EC-Schalter wahlweise
(i) die Gleiche wie die erste CAR-ID an dem ersten CAR-EC-Schalter ist oder
(ii) eine zweite CAR-ID ist, die sich von der ersten CAR-ID an dem ersten CAR-EC-Schalter unterscheidet,
vorausgesetzt dass, wenn die zweite CAR-ID, die sich von der ersten CAR-ID unterscheidet, das Verfahren weiterhin das Verabreichen einer zweiten CAR-EC umfasst, umfassend einen chimären Antigenrezeptor, der mit der zweiten CAR-ID des zweiten CAR-EC-Schalters interagiert.

15. Schalter zur Verwendung nach Anspruch 14, wobei
(i) die erste TID einen Anti-CD20-Antikörper oder einen antigenbindenden Teil davon umfasst und die zweite TID einen Anti-CD19-Antikörper oder ein antigenbindendes Fragment davon umfasst, oder
(ii) die erste TID einen Anti-CD19-Antikörper oder ein antigenbindendes Fragment davon umfasst und die zweite TID einen Anti-CD20-Antikörper oder ein antigenbindendes Fragment davon umfasst.

16. Schalter zur Verwendung nach einem der Ansprüche 7-15, wobei die Krankheit ein Krebs ist.

## Revendications

1. Commutateur pour activer une cellule effectrice de récepteur d'antigène chimérique (CAR-EC), le commutateur comprenant:
a. un domaine d'interaction du récepteur d'antigène chimérique (CAR-ID) qui interagit avec un récepteur d'antigène chimérique sur le CAR-EC; et
b. un domaine d'interaction cible (TID) comprenant un acide aminé non naturel, dans lequel le TID interagit avec une molécule de surface sur une cellule cible, et dans lequel CAR- ID est lié au TID par le biais de l'acide aminé non naturel.

2. Commutateur selon la revendication 1,
a. comprenant en outre un lieur,
(i) dans lequel le lieur relie le CAR-ID au TID par le biais de l'acide aminé non naturel,
(ii) dans lequel le lieur relie de manière spécifique au site le CAR-ID à l'acide aminé non naturel du TID, ou
(iii) dans lequel le lieur comprend de préférence un groupe aminooxy, un groupe azide du groupe cyclooctyne ou une combinaison de ceux-ci à un ou plusieurs groupes terminaux; ou
b. dans lequel le CAR-ID comprend une petite molécule, dans laquelle la petite molécule est
(i) un haptène,
(ii) l'isothiocyanate de fluorescéine (FITC), dans lequel le TID est conjugué à un othiocyanate de FITC ou comprend en outre un lieur qui est conjugué à un isothiocyanate de FITC, ou
(iii) de la biotine.

3. Commutateur selon la revendication 1,
a. dans lequel le TID est à base ou dérivé d'au moins une partie d'un anticorps; ou
b. dans lequel le TID est à base ou dérivé d'au moins une partie d'un fragment variable à chaîne unique (scFv); ou
c. dans lequel le TID est à base ou dérivé d'au moins une partie d'un anticorps anti-CD 19; ou
d. dans lequel le TID est à base ou dérivé d'au moins une partie d'un domaine variable à chaîne unique (scFv) d'un anticorps anti-CD 19; ou
e. dans laquelle le TID est à base ou dérivé d'au moins une partie d'un anticorps choisi dans le groupe constitué des anticorps anti-CD20, anti-CD22, anti-CD33, anti- BMSA, anti-CEA, anti-CLL1, anti-CS1, anti-EGFR et anti-Her2; ou
f. dans lequel le TID est à base ou dérivé d'au moins une partie d'un domaine variable à chaîne unique (scFv) d'un anticorps choisi dans le groupe constitué des anticorps anti-CD20, anti-CD22, anti-CD33, anti-BMSA, anti-CEA , anti-CLL1, anti-CS1, anti- EGFR et anti-Her2 et dans lesquels l'acide aminé non naturel est inséré dans la partie de l'anticorps à partir de laquelle le TID est fondé ou dérivé, ou remplace un acide aminé de l'anticorps à partir duquel le TID est fondé ou dérivé.

4. Composition comprenant une pluralité de commutateurs pour activer une cellule effectrice de récepteur d'antigène chimérique (CAR-EC), dans laquelle un commu-tateur de la pluralité de commutateurs comprend (a) un domaine d'interaction de récepteur d'antigène chimérique (CAR-ID) qui interagit avec un récepteur d'antigène chimérique sur le CAR-EC; et (b) un domaine d'interaction cible (TID) comprenant un acide aminé non naturel, dans lequel le TID interagit avec une molécule de surface sur une cellule cible, et dans lequel CAR- ID est lié au TID par le biais de l'acide aminé non naturel, et dans lequel au moins environ 60% des commutateurs sont structurellement homologues.

5. Composition selon la revendication 4,
a. dans lequel CAR-ID comprend une petite molécule; ou
b. dans lequel CAR-ID comprend de l'isothiocyanate de fluorescéine (FITC); ou
c. dans lequel le TID comprend une petite molécule choisie dans le groupe constitué par l'acide 2-[3-(1,3-dicarboxypropyl) uréido] pentanedioïque, le folate ou un dérivé de celui-ci; ou
d. dans lequel le commutateur de la pluralité de commutateurs comprend en outre un lieur, dans lequel le lieur attache l'identificateur CAR-ID au TID.

6. Composition selon la revendication 4,
a. où CAR-ID est relié au même site prédéterminé dans le TID dans au moins environ 80% des commutateurs.

7. Commutateur de première cellule effectrice antigène chimérique (CAR-EC) pour utilisation dans un procédé de traitement d'une maladie, le commutateur comprenant:
i. un premier domaine d'interaction du récepteur d'antigène chimérique (CAR-ID) qui interagit avec un récepteur d'antigène chimérique sur le CAR-EC; et
ii. un premier domaine d'interaction cible (TID) comprenant un acide aminé non naturel, dans lequel le TID interagit avec une première molécule de surface sur une cellule cible, et
dans lequel le TID comprend un CAR- ID relié au TID de manière spécifique au site par le biais de l'acide aminé non naturel,
dans lequel le procédé comprend l'administration du premier commutateur CAR-EC et en outre l'administration d'une première cellule effectrice récepteur-antigène chimérique comprenant un récepteur d'antigène chimérique qui se lie au premier CAR-ID du premier commutateur CAR-EC à un sujet.

8. Commutateur pour utilisation selon la revendication 7, dans lequel le lieur relie de manière spécifique au site le CAR-ID à l'acide aminé non naturel du TID.

9. Commutateur pour utilisation selon la revendication 7, dans lequel le premier CAR-ID comprend une petite molécule.

10. Commutateur pour utilisation selon la revendication 9, dans lequel la petite molécule est choisie parmi (i) un haptène et (ii) un haptène choisi parmi l'isothiocyanate de fluorescéine (FITC) ou la biotine.

11. Commutateur d'utilisation selon la revendication 7, dans lequel le premier TID comprend au moins un fragment de liaison à l'antigène d'un anticorps ou au moins un fragment de liaison à l'antigène d'un fragment variable à chaîne unique (scFv).

12. Commutateur à utiliser selon la revendication 7, dans lequel le premier TID comprend au moins un fragment de liaison à l'antigène d'un anticorps anti-CD 19 ou au moins un fragment de liaison à l'antigène d'un domaine variable à chaîne unique (scFv) d'un anticorps anti-CD 19.

13. Commutateur pour utilisation selon la revendication 7, dans lequel le premier TID comprend au moins un fragment de liaison à l'antigène d'un anticorps choisi dans le groupe constitué des anticorps anti-CD20, anti-CD22, anti-CD33, anti-BMSA, anti-CEA, anti-anticorps anti-CDA. CLL1, anti-CS1, anti-EGFR et anti-Her2 ou au moins un fragment de liaison à l'antigène d'un domaine variable à chaîne unique (scFv) d'un anticorps choisi dans le groupe constitué des anti-CD20, anti-CD22, anti-CD33, anti- BMSA, anti-CEA, anti-CLL1, anti-CS1, anti-EGFR et anti-Her2.

14. Commutateur pour utilisation selon la revendication 7, comprenant en outre l'administration d'un ou plusieurs seconds commutateurs CAR-EC au sujet, chaque second commutateur CAR-EC comprenant:
i. un second CAR-ID qui interagit avec un récepteur d'antigène chimérique sur une cellule effectrice ; et
ii. dans lequel un second TID comprend un acide aminé non naturel, le second TID comprenant le second CAR-ID attaché de manière spécifique au site par l'intermédiaire de l'acide aminé non naturel; et dans lequel le second TID interagit avec une molécule de surface sur une cellule cible;
dans lequel le second identifiant CAR-ID compris sur chaque second commutateur CAR-EC est éventuellement
(i) identique au premier CAR-ID compris dans le premier commutateur CAR-EC ou
(ii) un second CAR-ID qui diffère du premier CAR-ID compris sur le premier commutateur CAR-EC;
à condition que si le second CAR-ID qui diffère du premier CAR-ID, le procédé comprend en outre l'administration d'un second CAR-EC comprenant un récepteur d'antigène chimérique qui interagit avec le second CAR-ID du second commutateur CAR-EC.

15. Commutateur à utiliser selon la revendication 14, dans lequel
(i) le premier TID comprend un anticorps anti-CD20 ou une partie de sa liaison à l'antigène et le second TID comprend un anticorps anti-CD 19 ou un de ses fragments de liaison à l'antigène; ou
(ii) le premier TID comprend un anticorps 19 ou une partie de sa liaison à l'antigène et le second TID comprend un anticorps anti-CD anti-CD20 ou un de ses fragments de liaison à l'antigène.

16. Commutateur pour utilisation selon l'une quelconque des revendications 7 à 15, dans lequel la maladie est un cancer.
